(19) Europäisches Patentamt
European Patent Office
Office européen des brevets

(11) EP 1 771 570 B1

(12) **EUROPEAN PATENT SPECIFICATION**

(45) Date of publication and mention of the grant of the patent:
**09.03.2011 Bulletin 2011/10**

(21) Application number: **05791283.4**

(22) Date of filing: **21.07.2005**

(51) Int Cl.:
*C12N 15/861* (2006.01) *C12N 15/63* (2006.01)
*C12N 15/64* (2006.01) *C12N 15/10* (2006.01)
*A61K 48/00* (2006.01) *C12N 1/10* (2006.01)
*C07H 21/04* (2006.01) *C12Q 1/68* (2006.01)
*C12Q 1/70* (2006.01)

(86) International application number:
**PCT/US2005/025833**

(87) International publication number:
**WO 2006/012393 (02.02.2006 Gazette 2006/05)**

(54) **ADDITION OF TRANSGENES INTO ADENOVIRAL VECTORS**

Einfügen von Transgenen in adenovirale Vektoren

ADDITION DE TRANSGÈNES DANS DES VECTEURS ADÉNOVIRAUX

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **22.07.2004 US 589804 P**
**11.03.2005 US 660542 P**

(43) Date of publication of application:
**11.04.2007 Bulletin 2007/15**

(73) Proprietor: **BioSante Pharmaceuticals, Inc.**
**Lincolnshire, IL 60069 (US)**

(72) Inventors:
- **HAWKINS, Lynda**
  **San Mateo, CA 94403 (US)**
- **SESHIDAR, Reddy, Police**
  **Chester Springs, PA 19425 (US)**

(74) Representative: **HOFFMANN EITLE**
**Patent- und Rechtsanwälte**
**Arabellastraße 4**
**81925 München (DE)**

(56) References cited:
**WO-A-03/072703**
**US-A- 5 518 913**
**US-A1- 2003 021 768**

- **FUERER C ET AL: "5-Fluorocytosine increases the toxicity of Wnt-targeting replicating adenoviruses that express cytosine deaminase as a late gene." GENE THERAPY, vol. 11, no. 2, January 2004 (2004-01), pages 142-151, XP002467861 ISSN: 0969-7128**
- **PRESCOTT ET AL.: 'Sequence Elements Upstream of the 3' Cleavage Site Confer Substrate Strength to the Adenovirus L1 and L3 Polyadenylation Sites' MOLECULAR AND CELLULAR BIOLOGY vol. 14, no. 7, July 1994, pages 4682 - 4693, XP008093062**
- **PRESCOTT ET AL.: 'Sequence-Mediated Regulation of Adenovirus Gene Expression by Repression of mRNA Accumulation' MOLECULAR AND CELLULAR BIOLOGY vol. 17, no. 4, April 1997, pages 2207 - 2216, XP008093060**
- **GILMARTIN ET AL.: 'Sequence Regulating Poly (A) Site Selection within the Adenovirus Major Late Transcription Unit Influence the Interaction of Constitutive Processing Factors with the Pre-mRNA' JOURNAL OF VIROLOGY vol. 70, no. 3, March 1996, pages 1775 - 1783, XP008092461**
- **PARKS ET AL.: 'Activation of the Adenovirus Major Late Promoter by Transcription Factors MAZ and Sp1' JOURNAL OF VIROLOGY vol. 71, no. 12, December 1997, pages 9600 - 9607, XP008092462**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 771 570 B1

## Description

## BACKGROUND OF THE INVENTION

This application claims priority from U.S. Provisional Application Serial No. 60/589,804, filed July 22, 2004 and U.S. Provisional Application Serial No. 60/660,542, filed March 11, 2005.

## Field of the Invention

The invention relates to adenoviral vectors comprising transgenes inserted into the genome at various locations, such that transgene expression relies on either native adenoviral processing and/or regulatory signals or heterologous transcription signals, and methods for making the same.

## Background of the Technology

Adenoviral vectors have been utilized extensively to deliver and express genes in mammalian cells, *in vitro, ex vivo* and *in vivo.* Adenovirus vectors offer many advantages for gene delivery: wild type adenoviruses cause only minor illness in healthy individuals, they are easy to propagate to high titers, they can infect most cell types regardless of their growth state, they can deliver nucleic acids encoding proteins of interest to cells, and in their most recent embodiments they can be engineered to replicate selectively in certain cells and can be targeted by incorporating or attaching a ligand to capsid proteins.

There are two main types of adenoviral vectors, replication-incompetent (replication defective) and replication-competent. Replication defective vectors traditionally lack one or more genes essential for replication. These replication incompetent viruses are propagated on cells that complement the essential gene(s) which are lacking. Replication competent adenoviral vectors traditionally do not lack any of the adenovirus genes essential for replication. One type of replication competent adenovirus replicates selectively in certain cells. For example, a large number of various cell- and tissue-specific replication competent adenovirus vectors, which preferentially replicate in (and thus destroy) certain cell types, have been described. One method of constructing a cell-specific replication competent virus is to place a heterologous transcriptional regulatory element (TRE) in operative linkage with an essential adenoviral coding sequence. Another method is to delete or inactivate an adenoviral coding sequence that when deleted or inactivated results in preferential replication of the virus in a certain cell-type, for example tumor cells.

Adenoviral vectors are limited by the size of their genome (Bett et al, J Virol 67:5911-5921, 1993). This in turn limits the amount of heterologous DNA that may be inserted into the vector and therefore limits the amount and or length of heterologous coding sequences that maybe incorporated into the adenovirus genome. Therefore, replication defective viruses are capable of the largest heterologous DNA insertions, but are still limited by the size of the adenovirus genomic DNA and the amount of adenoviral DNA deleted.

In the case of replication competent viruses, they are limited not only because they contain the majority of adenoviral coding sequences, but they are limited in the number of insertion sites for heterologous DNA. Traditionally, heterologous insertions of DNA have been inserted in place of an adenovirus E3 coding sequence(s) in replication competent viruses (WO 02/067861, WO 01/02540). This limits the size of the heterologous DNA insertion to a slightly larger size than that of the E3 deletion. Also, for a particular application it may not be desired or advantageous to delete an E3 coding sequence(s) or to insert the heterologous DNA in the E3 region.

Adenoviral infection is divided into early and late phases, separated by viral DNA replication. Viral genes are expressed before (early phase) and/or after (late phase) viral DNA replication. In general, early genes encode proteins that prepare the cell for viral propagation and late proteins are capsid proteins or are involved in packaging and assembly of virions. One method that has been used extensively to express transgenes is to insert an expression cassette into the viral genome in such a way that the expression is constitutive and not regulated by the virus itself. In such cases, typically the transgene is inserted in place of a non-essential gene or in place of an essential gene, wherein the essential gene is complemented by a cell line used for propagation of the viral vector.

In adenoviruses, late transcription initiates predominantly at the major late promoter (MLP) after the initiation of DNA replication. At late time points, transcription from the MLP accounts for approximately 30% of the total RNA synthesis. In all adenoviruses, the primary transcript initiated from the MLP extends towards the right for about 28 kb and terminates at a position close to 99 map units in the viral genome. Each region is characterized by its own polyadenylation signal sequence and usually consists of several differentially spliced messages. The primary transcripts are cleaved and become polyadenylated at one of the five locations along the genome, generating five families (L1 - L5) of mRNAs, each with 3' co termini. After the selection of the 3' end, the primary transcript is processed by splicing in such a way that each mature RNA gains a common set of three short 5' leader segments called tripartite leader (TPL) sequences derived from 16.8, 19.8 and 26.9 map units. Each of the five families expresses more than one protein with the exception of L5 in some adenovirus serotypes. Ad 40 and 41 express more than one protein from the L5 region. The mRNAs within a family differ from each other by positions where the tripartite leader is spliced into a splice acceptor site located upstream of an open reading frame (ORF) in a late region of the genome. For example, four types of mRNAs are produced from the L2 region and all have common 3' ends. Although the messages are polycistronic, only the ORF present at the 5' end is thought to be translated in native Ad transcripts. Fuerer et al. (Gene Therapy 11: 142-151 (2004)) describes inserting a coding sequence for cytosine deaminase (CD) in a replication competent virus using at least two methods. One method relies on the use of the encephalomyocarditis virus internal ribosome entry site (IRES) to convert the L5 transcript into a bicistronic mRNA. Fuerer et al. also describe a method that makes use of the splice acceptor site sequence from the Ad41 long fiber gene to splice the CD cassette onto the tripartite leader exons of the major late transcript.

## SUMMARY OF THE INVENTION

The invention relates to a novel gene delivery system using the adenovirus (Ad) genome as a gene delivery and/or expression vehicle. The adenovirus genome may be either replication competent or incompetent (defective).

The invention provides adenoviral vectors wherein transgenes are inserted into the adenoviral genome at various locations, e.g., upstream or downstream of existing genes, eliminating the need for the addition of large regulatory sequences such as promoters, allowing heterologous DNA (a transgene) to be inserted into the Ad genome, in a location and manner effective to take advantage of and/or utilize native adenoviral processing.

In some aspects of the invention, transcription signals (e.g. branch point sites, splice acceptor sites, IRES, self-processing cleavage sites and/or polyA signals) are added to the adenoviral vectors of the invention. The addition of genes with processing signals such as branch point sequences along with splice acceptor sequences takes advantage of the mRNA processing and expression system utilized by native adenovirus.

## BRIEF DESCRIPTION OF THE FIGURES

Figure 1 is a schematic depiction of the native genome organization of Ad5.

Figure 2 is a schematic depiction of the native Ad5 transcription units.

Figure 3 is a schematic depiction of the L1 region of Ad5.

Figure 4 is a schematic depiction of the L2 region of Ad5.

Figure 5 is a schematic depiction of the L3 region of Ad5.

Figure 6 is a schematic depiction of the E3 transcription units.

Figure 7 is a schematic depiction of the E3A region. The star denotes 192 base pairs in Ad5 that are frequently deleted from adenoviral vectors.

Figure 8 is a schematic depiction of the E3B region.

Figure 9 is a schematic depiction of an exemplary embodiment of the invention wherein an L3 region insertion site is shown with an example of a chimeric adenoviral leader region, wherein a transgene is inserted between the hexon and 23K protease coding sequences. The transgene is operatively linked to (i.e. utilizes for expression) the native splice acceptor site for the 23K mRNA which is located about 95 bases upstream of the hexon stop codon in Ad5. A heterologous splice acceptor site is inserted between the transgene coding sequence and the 23K coding sequence and the heterologous splice acceptor site is operatively linked to the 23K coding sequence. This allows four different mRNAs to be transcribed from the L3 region. In this example, the native L3 polyadenylation sequence is left intact and is utilized by all four mRNAs of the chimeric L3 region.

Figure 10 is a schematic depiction of an exemplary embodiment of the invention wherein an L3 region insertion site is shown with an example of a chimeric adenoviral leader region. In this case, a transgene is inserted between the 23K protease coding sequence and the L3 polyadenylation sequence in the L3 region. The transgene is operatively linked to (i.e. utilizes for expression) a 2A-like sequence or IRES. The 2A-like sequence or IRES is also operatively linked to the 23K coding sequence. Similar to the native L3 region, this chimeric adenoviral L3 region will result in production of three L3 mRNAs. In this case, all three of the L3 mRNAs will contain an IRES (or 2A-like sequence) and therefore translation of all three of these mRNAs will result in the expression of the transgene. For this example, the native L3 polyadenylation sequence is left intact and is utilized by all three mRNAs of the chimeric L3 region.

Figure 11 provides a schematic depiction of OV-1160 comprising in the 5' to 3' direction: the E2F-1 promoter operatively linked to E1A; and in the L3 region: pVI, hexon, a splice acceptor and the TRAIL coding sequence. The vector carries the packaging signal in the native location and carries a polyadenylation signal upstream of the E2F-1 promoter to inhibit transcriptional read-through from the LITR.

Figure 12 provides a schematic depiction of OV-1164 comprising in the 5' to 3' direction: the E2F-1 promoter operatively linked to E1A; and in the L3 region: pVI, hexon, an IRES, the TRAIL coding sequence and the 23K gene. The vector carries the packaging signal in the native location and carries a polyadenylation signal upstream of the E2F-1 promoter to inhibit transcriptional read-through from the LITR.

## DETAILED DESCRIPTION OF THE INVENTION

The present invention provides recombinant adenoviral vectors as defined in the claims which rely on the use of existing (native) Transcriptional and/or translational regulatory elements for the expression of one or more transgenes. In one example, the major late promoter is used for expression of a transgene during the late phase of infection by insertion of one or more transgenes into a late region. In another example, this strategy is used to insert transgenes into an adenoviral early region. A transgene may be inserted in operative linkage with any endogenous adenoviral promoter, so long as the open reading frames (ORF) of essential adenoviral genes and native adenoviral processing signals, such as endogenous polyadenylation and transcriptional signals are not disturbed and the virus can effectively replicate.

In addition to a transgene, heterologous a branch point, splice acceptor site, or other Similar sequence, are included in an adenoviral vector of the invention to allow correct processing of the transcript. The quantity and kinetics of transgene expression can be manipulated by altering the particular sequence of the processing signals, such as splicing efficiency, translation initiation efficiency or the efficiency of the signals, so that the desired kinetics are obtained. Multiple transgenes may be included in the same vector, either in the same or different regions of the genome. Other endogenous adenoviral genes may be deleted, to achieve a desired effect or to allow for the inclusion of longer sequences of exogenous DNA.

In one aspect, the present invention "restores transcription signals" to a adenoviral vector. By "restoring transcription signals" it is meant that a non-native transcription signal is used to perform a similar function or to affect transcription in a manner similar to that of the native transcription signal. For example, in the L5 region the fiber stop codon is embedded in the L5 polyA signal. Insertion of a transgene downstream of the fiber coding region will usually include a mutation that disrupts the function of the native L5 polyA site, but maintains the fiber stop codon. Therefore, the polyA site must be restored for correct processing of the L5 message. This entails adding a polyA signal to the L5 region. This polyA signal may be the same sequence as the one that was disrupted. In an exemplary embodiment where a transgene is inserted downstream of the fiber coding region, the polyA signal is inserted downstream of the transgene coding region. In a different embodiment of the invention where a transgene is inserted between existing adenoviral coding sequences, the restoration/addition of an additional splice acceptor site is typically employed. For example, the transgene may use an adenoviral native splice site that would have been the native splice site for the next adenoviral coding sequence downstream of the transgene coding sequence. In this case, a splice site has to be restored for the next adenoviral coding sequence downstream of the transgene coding sequence. This "restored" splice site could be the same sequence as the native sequence now utilized for splicing upstream of the transgene sequence or it could be a heterologous splice site. Different splice sites are known in the art to have different splicing efficiencies. Therefore, one skilled in the art can choose splice sites with different efficiencies as desired for a particular application.

The advantages provided by the present invention are several-fold: a) by utilizing existing expression signals, larger size transgene(s) may be incorporated in a size-limited vector; b) the known and predictable expression patterns of adenoviral genes allows for design of adenoviral vectors with desired transgene expression kinetics, such as expression level and timing, wherein expression is tailored dependent upon the nature of the transgene; c) the adenoviral vectors of the invention take advantage of native regulation systems that are already in place in the adenoviral genome; d) the adenoviral vectors of the invention include introduced regulatory sequences such as self-processing cleavage sites, branch point sequences and splice acceptor sequences which are small compared to other elements typically used for transgene expression; and e) the selection of heterologous transcription factors can be used to further regulate expression. Numerous transgenes and/or transgene categories exist that can be transgene candidates, as further described herein.

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, "Molecular Cloning: A Laboratory Manual", second edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology" (D.M. Weir & C.C. Blackwell, eds.); "Gene Transfer Vectors for Mammalian Cells" (J.M. Miller & M.P. Calos, eds., 1987); "Current Protocols in Molecular Biology" (F.M. Ausubel et al., eds., 1987); "PCR: The Polymerase Chain Reaction", (Mullis et al., eds., 1994); and "Current Protocols in Immunology" (J.E. Coligan et al., eds., 1991).

### Definitions

Unless otherwise indicated, all terms used herein have the same meaning as they would to one skilled in the art and the practice of the present invention will employ, conventional techniques of microbiology and recombinant DNA technology, which are within the knowledge of those of skill of the art.

The terms "virus," "viral particle," "vector particle," "viral vector particle," and "virion" are used interchangeably and are to be understood broadly as meaning infectious viral particles that are formed when, e.g., a viral vector of the invention is transduced into an appropriate cell or cell line for the generation of infectious particles. Viral particles according to the invention may be utilized for the purpose of transferring DNA into cells either *in vitro* or *in vivo*. For purposes of the present invention, these terms refer to adenoviruses, including recombinant adenoviruses formed when an adenoviral vector of the invention is encapsulated in an adenovirus capsid.

An "adenovirus vector" or "adenoviral vector" (used interchangeably) as referred to herein is a polynucleotide construct, which is replication competent or replication incompetent (defective).

Exemplary adenoviral vectors of the invention include, but are not limited to, DNA, DNA encapsulated in an adenovirus coat, adenoviral DNA packaged in another viral or viral-like form (such as herpes simplex, and AAV), adenoviral DNA encapsulated in liposomes, adenoviral DNA complexed with polylysine, adenoviral DNA complexed with synthetic polycationic molecules, conjugated with transferrin, or complexed with compounds such as PEG to immunologically "mask" the antigenicity and/or increase half-life, or conjugated to a nonviral protein. Hence, the terms" adenovirus vector" or "adenoviral vector" as used herein include adenovirus or adenoviral particles.

The terms "polynucleotide" and "nucleic acid", used interchangeably herein, refer to a polymeric form of nucleotides of any length, either ribonucleotides or deoxyribonucleotides. These terms include a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. Preferably, a vector of the invention comprises DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides.

The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs, uracyl, other sugars and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support. Preferably, the polynucleotide is DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides. A nucleic acid sequence is "operatively linked" or "operably linked" when it is placed into a functional relationship with another nucleic acid sequence. For example, a promoter or regulatory DNA sequence is said to be "operatively linked" or "operably linked" to a DNA sequence that codes for an RNA and/or a protein if the two sequences situated such that the promoter or regulatory DNA sequence affects the expression level of the coding or structural DNA sequence. Operatively linked means that the DNA sequences being linked are generally contiguous and, where necessary to join two protein coding regions, contiguous and in the same reading frame. However, since enhancers generally function when separated from the promoter by several kilobases and intronic sequences may be of variable length, some polynucleotide elements may be operatively linked but not contiguous.

The term "coding region", as used herein, refers to a region of a nucleic acid that contains the coding sequence. The coding region may contain other regions from the corresponding gene including introns. The term "coding sequence" (CDS) refers to the nucleic acid sequence containing the codons that encode a protein. The coding sequence generally begins with a translation start codon (e.g. ATG) and ends with a translation stop codon. Sequences said to be upstream of a coding sequence are 5' to the translational start codon and sequences downstream of a CDS are 3' of the translational stop codon.

The term "ORF" means open reading frame.

The term "gene" refers to a defined region that is located within a genome and that, in addition to the aforementioned coding sequence, comprises other, primarily regulatory, nucleic acid sequences responsible for the control of expression, i.e., transcription and translation of the coding portion. A gene may also comprise other 5' and 3' untranslated sequences and termination sequences. Depending on the source of the gene, further elements that may be present are, for example, introns.

The terms "heterologous" and "exogenous" as used herein with reference to nucleic acid molecules such as promoters and gene coding sequences, refer to sequences that originate from a source foreign (non-native) to a particular virus or host cell or, if from the same source, are modified from their original form. Thus, a heterologous gene in a virus or cell includes a gene that is endogenous to the particular virus or cell but has been modified through, for example, codon optimization. The terms also include non-naturally occurring multiple copies of a naturally occurring nucleic acid sequence. Thus, the terms refer to a nucleic acid segment that is foreign or heterologous to the virus or cell, or homologous to the virus or cell but in a position within the host viral or cellular genome in which it is not ordinarily found.

The term "native" refers to a gene that is present in the genome of the wildtype virus or cell.

The term "naturally occurring" or "wildtype" is used to describe an object that can be found in nature as distinct from being artificially produced by man. For example, a protein or nucleotide sequence present in an organism (including a virus), which can be isolated from a source in nature and which has not been intentionally modified by man in the laboratory, is naturally occurring.

The terms "complement" and "complementary" refer to two nucleotide sequences that comprise antiparallel nucleotide sequences capable of pairing with one another upon formation of hydrogen bonds between the complementary base residues in the antiparallel nucleotide sequences. The term "recombinant" as used herein with reference to nucleic acid molecules refers to a combination of nucleic acid molecules that are joined together using recombinant DNA technology into a progeny nucleic acid molecule. As used herein with reference to viruses, cells, and organisms, the terms "recombinant," "transformed," and "transgenic" refer to a host virus, cell, or organism into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Recombinant viruses, cells, and organisms are understood to encompass not only the end product of a transformation process, but also recombinant progeny thereof. A "non-transformed," "non-transgenic," or "non-recombinant" host refers to a wildtype virus, cell, or organism that does not contain the heterologous nucleic acid molecule.

"Regulatory elements" are sequences involved in controlling the expression of a nucleotide sequence. Regulatory elements include promoters, enhancers, and termination signals. They also typically encompass sequences required for proper translation of the nucleotide sequence.

The term "promoter" refers to an untranslated DNA sequence usually located upstream of the coding region that contains the binding site for RNA polymerase II and initiates transcription of the DNA. The promoter region may also include other elements that act as regulators of gene expression. The term "minimal promoter" refers to a promoter element, particularly a TATA element that is inactive or has greatly reduced promoter activity in the absence of upstream activation elements.

The term "enhancer" within the meaning of the invention may be any genetic element, e.g., a nucleotide sequence that increases transcription of a coding sequence operatively linked to a promoter to an extent greater than the transcription activation effected by the promoter itself when operatively linked to the coding sequence, i.e. it increases transcription from the promoter.

A "chimeric adenoviral region" is defined as a portion of an adenoviral vector that contains both a portion of a native adenovirus genome and a portion of a heterologous DNA sequence. For example, a "chimeric adenoviral leader region" comprises at least a portion of a native adenoviral leader region, such as L1, L2, L3, L4, L5, and further comprises a heterologous DNA sequence. In one embodiment, the heterologous DNA sequence comprises a transgene CDS. In another embodiment, the heterologous DNA sequence comprises a transgene CDS, a branch site and a splice acceptor site.

The terms "transcriptional regulation elements" and "translational regulation elements" are those elements that affect transcription and/or translation of nucleic acids. These elements include, but are not limited to, splice donor and acceptor sites, translation stop and start codons, and adenylation signals.

As used herein, a "transcriptional response element" or "transcriptional regulatory element", or "TRE" is a polynucleotide sequence, preferably a DNA sequence, comprising one or more enhancer(s) and/or promoter(s) and/or promoter elements such as a transcriptional regulatory protein response sequence or sequences, which increases transcription of an operatively linked polynucleotide in a host cell that allows a TRE to function.

"Under transcriptional control" is a term well understood in the art and indicates that transcription of a polynucleotide sequence, usually a DNA sequence, depends on its being operatively linked to an element which contributes to the initiation of, or promotes, transcription.

The term "vector", as used herein, refers to a nucleic acid construct designed for transfer between different host cells. Vectors may be, for example, "cloning vectors" which are designed for isolation, propagation and replication of inserted nucleotides, "expression vectors" which are designed for expression of a nucleotide sequence in a host cell, or a "viral vector" which is designed to result in the production of a recombinant virus or virus-like particle, or "shuttle vectors", which comprise the attributes of more than one type of vector. Any vector for use in gene introduction can basically be used as a "vector" into which the DNA having the desired sequence is to be introduced. Plasmid vectors will find use in practicing the present invention. The term vector as it applies to the present invention is used to describe a recombinant vector, e.g., a plasmid or viral vector (including a replication defective or replication competent virus).

The terms "vector," "polynucleotide vector," "polynucleotide vector construct," "nucleic acid vector construct," and "vector construct" are used interchangeably herein to mean any nucleic acid construct for gene transfer, as understood by one skilled in the art. The term "replication defective" as used herein relative to a viral vector of the invention means the viral vector cannot further replicate and package its genomes. For example, when the cell of a subject are infected with an adenoviral vector that has the entire E1 and the E4 coding region deleted or inactivated, the heterologous transgene is expressed in the patient's cells if the transgene is transcriptionally active in the cell. However, due to the fact that the patient's cells lack the Ad E1 and E4 coding sequences, the Ad vector is replication defective and viral particles cannot be formed in these cells.

The term "replication competent" means the vector can replicate in particular cell types ("target cells"), e.g., cancer cells and preferentially effect cytolysis of those cells. Specific replication competent viral vectors have been developed for which selective replication in cancer cells preferentially destroys those cells. Various cell-specific replication competent adenovirus constructs, which preferentially replicate in (and thus destroy) certain cell types. Such viral vectors may be referred to as "oncolytic viruses" or "oncolytic vectors" and may be considered to be "cytolytic" or "cytopathic" and to effect "selective cytolysis" of target cells. Examples of "replication competent" or "oncolytic" viral vectors are described in, for example PCT Publication Nos. WO98/39466, WO95/19434, WO97/01358, WO98/39467, WO98/39465, WO01/72994, WO 04/009790, WO 00/15820, WO 98/14593, WO 00/46355, WO 02/067861, WO 98/39464, WO 98/13508, WO 20004/009790; U.S. Provisional Application Serial Nos. 60/511,812, 60/423,203 and U.S. Patent Publication No. US 2001/0053352, each of which is expressly incorporated by reference herein.

The terms "replication conditional viruses", "preferentially replicating viruses", "specifically replicating viruses" and "selectively replicating viruses" are terms that are used interchangeably and are replication competent viral vectors and particles that preferentially replicate in certain types of cells or tissues but to a lesser degree or not at all in other types. In one embodiment of the invention, the viral vector and/or particle selectively replicates in tumor cells and or abnormally proliferating tissue, such as solid tumors and other neoplasms. Such viruses may be referred to as "oncolytic viruses" or "oncolytic vectors" and may be considered to be "cytolytic" or "cytopathic" and to effect "selective cytolysis" of target cells. "Preferential replication" and "selective replication" and "specific replication" may be used interchangeably and mean that the virus replicates more in a target cell than in a non-target cell. The virus replicates at a higher rate in target cells than non target cells, e.g. at least about 3-fold higher, at least about 10-fold higher, at least about 50-fold higher, and in some instances at least about 100-fold, 400-fold, 500-fold, 1000-fold or even 1 x $10^6$ higher. In one embodiment, the virus replicates only in the target cells (that is, does not replicate at all or replicates at a very low level in non-target cells).

The term "plasmid" as used herein refers to a DNA molecule that is capable of autonomous replication within a host cell, either extrachromosomally or as part of the host cell chromosome(s). The starting plasmids herein are commercially available, are publicly available on an unrestricted basis, or can be constructed from such available plasmids as disclosed herein and/or in accordance with published procedures. In certain instances, as will be apparent to the ordinarily skilled artisan, other plasmids known in the art may be used interchangeable with plasmids described herein.

The term "expression" refers to the transcription and/or translation of an endogenous gene, transgene or coding region in a cell.

A "polyadenylation signal sequence" is a recognition region for endonuclease cleavage of a RNA transcript that is followed by a polyadenylation consensus sequence AATAAA. A polyadenylation signal sequence provides a "polyA site", i.e. a site on a RNA transcript to which adenine residues will be added by post-transcriptional polyadenylation. Generally, a polyadenylation signal sequence includes a core poly(A) signal that consists of two recognition elements flanking a cleavage-polyadenylation site (e.g., Figure 1 of WO 02/067861 and WO 02/068627). The choice of a suitable polyadenylation signal sequence will consider the strength of the polyadenylation signal sequence, as completion of polyadenylation process correlates with poly(A) site strength (Chao et al., Molecular and Cellular Biology, 1999, 19: 5588-5600). For example, the strong SV40 late poly(A) site is committed to cleavage more rapidly than the weaker SV40 early poly(A) site. The person skilled in the art will consider choosing a stronger polyadenylation signal sequence if desired. In principle, any polyadenylation signal sequence may be useful for the purposes of the present invention. However, in some embodiments of this invention the termination signal sequence is the SV40 late polyadenylation signal sequence or the SV40 early polyadenylation signal sequence. Usually, the termination signal sequence is isolated from its genetic source or synthetically constructed and inserted into a vector of the invention at a suitable position.

A "multicistronic transcript" refers to a mRNA molecule that contains more than one protein coding region, or cistron. A mRNA comprising two coding regions is denoted a "bicistronic transcript." The "5'-proximal" coding region or cistron is the coding region whose translation initiation codon (usually AUG) is closest to the 5'-end of a multicistronic mRNA molecule. A "5'-distal" coding region or cistron is one whose translation initiation codon (usually AUG) is not the closest initiation codon to the 5' end of the mRNA. The terms "5'-distal" and "downstream" are used synonymously to refer to coding regions that are not adjacent to the 5' end of a mRNA molecule.

As used herein, an "internal ribosome entry site" or "IRES" refers to an element that promotes direct internal ribosome entry to the initiation codon, such as ATG, of a cistron (a protein encoding region), thereby leading to the cap-independent translation of the gene (Jackson R J, Howell M T, Kaminski A (1990) Trends Biochem Sci 15(12):477-83) and Jackson R J and Kaminski, A. (1995) RNA 1(10):985-1000). The present invention encompasses the use of any IRES element, which is able to promote direct internal ribosome entry to the initiation codon of a cistron. PCT publication WO 01/55369 describes examples of IRES sequences including synthetic sequences and these sequences may also be used according to the present invention. "Under translational control of an IRES" as used herein means that translation is associated with the IRES and proceeds in a cap-independent manner. Examples of "IRES" known in the art include, but are not limited, to IRES obtainable from picornavirus (Jackson et al., 1990, Trends Biochem Sci 15(12):477-483); and IRES obtainable from viral or cellular mRNA sources, such as for example, immunoglobulin heavy-chain binding protein (BiP), the vascular endothelial growth factor (VEGF) (Huez et al. (1998) Mol. Cell. Biol. 18(11):6178-6190), the fibroblast growth factor 2, and insulin-like growth factor, the translational initiation factor eIF4G, yeast transcription factors TFIID and HAP4. IRES have also been reported in different viruses such as cardiovirus, rhinovirus, aphthovirus, HCV, Friend murine leukemia virus (FrMLV) and Moloney murine leukemia virus (MoMLV). As used herein, "IRES" encompasses functional variations of IRES sequences as long as the variation is able to promote direct internal ribosome entry to the initiation codon of a cistron. In some embodiments, the IRES is mammalian. In other embodiments, the IRES is viral or protozoan. In one embodiment, the IRES is obtainable from encephelomycarditis virus (EQMV) (commercially available from Novogen, Duke et al. (1992) J. Virol 66(3):1602-1609). In another illustrative embodiment disclosed herein, the IRES is from VEGF. Examples of IRES sequences are described in U.S. Patent No. 6,692,736.

A "self-processing cleavage site" or "self-processing cleavage sequence" as referred to herein is a DNA or amino acid sequence, wherein upon translation, rapid intramolecular (cis) cleavage of a polypeptide comprising the self-processing cleavage site occurs to result in expression of discrete mature protein or polypeptide products. Such a "self-processing cleavage site", may also be referred to as a post-translational or co-translational processing cleavage site, e.g., a 2A site, sequence or domain. A 2A site, sequence or domain demonstrates a translational effect by modifying the activity of the ribosome to promote hydrolysis of an ester linkage, thereby releasing the polypeptide from the translational complex in a manner that allows the synthesis of a discrete downstream translation product to proceed (Donnelly, 2001). Alternatively, a 2A site, sequence or domain demonstrates "auto-proteolysis" or "cleavage" by cleaving its own C-terminus in cis to produce primary cleavage products (Furler; Palmenberg, Ann. Rev. Microbiol. 44:603-623 (1990)).

The term "splice acceptor site" or "3' splice acceptor site" (hereinafter referred to as a 3' SAS) is a sequence that may be engineered into different locations of the wild type Ad5 genome. Introduction of the site provides a means to facilitate the expression of heterologous stretches of DNA (i.e. transgenes) by utilizing the endogenous alternative splicing mechanisms of adenoviruses. An exemplary 3' SAS site is:

TACTTATGACTCGTACTATTGTTATTCATCCAG↓G

(SEQ ID NO: 45) where the underlined bases are the consensus branch site (which is variable), A refers to a branch point, which is highly conserved and the down arrow refers to the splice site (such that sequences 5' to (before) the arrow are cleaved out, and sequences after the arrow are part of the exon).

The terms "branch point" and "branch point sequence" are used interchangeably and refer to a nucleotide sequence involved in splicing and are understood in the art to be a recognition signal for the site of lariat formation. When referring to a DNA vector, a branch point sequence is the DNA sequence that codes for the RNA branch point sequence.

As used herein, "transgene" refers to a polynucleotide that can be expressed, via recombinant techniques, in a non-native environment or heterologous cell under appropriate conditions. In the present invention, the transgene coding region is inserted in a viral vector. In one embodiment, the viral vector is an adenoviral vector. The transgene may be derived from the same type of cell in which it is to be expressed, but introduced from an exogenous source, modified as compared to a corresponding native form and/or expressed from a non-native site, or it may be derived from a heterologous cell. "Transgene" is synonymous with "exogenous gene", "foreign gene", "heterologous coding sequence" and "heterologous gene". In the context of a vector for use in practicing the present invention, a "heterologous polynucleotide" or "heterologous gene" or "transgene" is any polynucleotide or gene that is not present in the corresponding wild-type vector or virus. The transgene coding sequence may be a sequence found in nature that codes for a certain protein. The transgene coding sequence may alternatively be a non-natural coding sequence. For example, one skilled in the art can readily recode a coding sequence to optimize the codons for expression in a certain species using a codon usage chart. In one embodiment, the recoded sequence still codes for the same amino acid sequence as a natural coding sequence for the transgene. Examples of preferred transgenes for inclusion in the vectors of the invention, are provided herein. A transgene may be a therapeutic gene. A transgene does not necessarily code for a protein.

As used herein, a "therapeutic" gene refers to a transgene that, when expressed, confers a beneficial effect on a cell, tissue or mammal in which the gene is expressed. Examples of beneficial effects include amelioration of a sign or symptom of a condition or disease, prevention or inhibition of a condition or disease, or conferral of a desired characteristic. Numerous examples of therapeutic genes are known in the art, a number of which are further described below.

In the context of a vector for use in practicing the present invention, a "heterologous" sequence or element is one which is not associated with or derived from the corresponding wild-type vector or virus.

In the context of a vector for use in practicing the present invention, an "endogenous" sequence or element is native to or derived from the corresponding wild-type vector or virus.

"Replication" and "propagation" are used interchangeably and refer to the ability of a viral vector of the invention to reproduce or proliferate. These terms are well understood in the art. For purposes of this invention, replication involves production of virus proteins and is generally directed to reproduction of virus. Replication can be measured using assays standard in the art and described herein, such as a virus yield assay, burst assay or plaque assay. "Replication" and "propagation" include any activity directly or indirectly involved in the process of virus manufacture, including, but not limited to, viral gene expression; production of viral proteins, replication of nucleic acids or other components; packaging of viral components into complete viruses and cell lysis.

As used herein, a "packaging cell" is a cell that is able to package adenoviral genomes or modified genomes to produce viral particles. It can provide a missing gene product or its equivalent. Thus, packaging cells can provide complementing functions for the genes deleted in an adenoviral genome and are able to package the adenoviral genomes into the adenovirus particle. The production of such particles requires that the genome be replicated and that those proteins necessary for assembling an infectious virus are produced. The particles also can require certain proteins necessary for the maturation of the viral particle. Such proteins can be provided by the vector or by the packaging cell.

"Producer cells" for viral vectors are well known in the art. A producer cell is a cell in which the adenoviral vector is delivered and the adenoviral vector is replicated and packaged into virions. If the viral vector has an essential gene deleted or inactivated, then the producer cell complements for the inactivated gene. Examples of adenoviral vector producer cells are PerC.6 (Fallaux et al. Hum Gene Ther. 1998 Sep 1;9(13):1909-17) and 293 cells (Graham et al. J Gen Virol. 1977 Jul;36(1):59-74). In the case of selectively replicating viruses, producer cells may be of a cell type in which the virus selectively replicates. Alternatively or in addition, the producer cell may express the genes that are selectively controlled or inactivated in the viral vector.

The term "HeLa-S3" means the human cervical tumor-derived cell line available from American Type Culture Collection (ATCC, Manassas, VA) and designated as ATCC number CCL-2.2. HeLa-S3 is a clonal derivative of the parent HeLa line (ATCC CCL-2). HeLa-S3 was cloned in 1955 by T.T. Puck et al. (J. Exp. Med. 103: 273-284 (1956)).

An "individual" is a vertebrate, a mammal, or a human. Mammals include, but are not limited to, farm animals, sport animals, rodents, primates, and pets. A "host cell" includes an individual cell or cell culture which can be or has been a recipient of an adenoviral vector(s) of this invention. Host cells include progeny of a single host cell, and the progeny may not necessarily be completely identical (in morphology or in total DNA complement) to the original parent cell due to natural, accidental, or deliberate mutation and/or change. A host cell includes cells transfected or infected *in vivo* or *in vitro* with an adenoviral vector of this invention.

As used herein, "cytotoxicity" is a term well understood in the art and refers to a state in which a cell's usual biochemical or biological activities are compromised (i.e., inhibited). These activities include, but are not limited to, metabolism; cellular replication; DNA replication; transcription; translation; uptake of molecules. "Cytotoxicity" includes cell death and/or cytolysis. Assays are known in the art which indicate cytotoxicity, such as dye exclusion, 3H-thymidine uptake, and plaque assays.

As used herein, the terms "neoplastic cells", "neoplasia", "tumor", "tumor cells", "carcinoma", "carcinoma cells", "cancer" and "cancer cells", (used interchangeably) refer to cells which exhibit relatively autonomous growth, so that they exhibit an aberrant growth phenotype characterized by a significant loss of control of cell proliferation. Neoplastic cells can be malignant or benign.

## Adenoviral Vectors

As used herein, the terms "adenovirus" and "adenoviral particle" are used to include any and all viruses that may be categorized as an adenovirus, including any adenovirus that infects a human or an animal, including all known and later discovered groups, subgroups, and serotypes. Thus, as used herein, "adenovirus" ("Ad") and "adenovirus particle" refer to the virus itself or derivatives thereof and cover all serotypes and subtypes and both naturally occurring and recombinant forms, except where indicated otherwise. Such adenoviruses may be wildtype or may be modified in various ways known in the art or as disclosed herein. Such modifications include changes to the adenovirus genome that are packaged in the particle in order to make an infectious virus. Such modifications also include deletions known in the art, such as deletions in one or more of the adenoviral genes that are essential for replicion, e.g., the E1a, E1b, E2a, E2b, E3, or E4 coding regions. The term "gene essential for replication" refers to a nucleic acid sequence whose transcription is required for a viral vector to replicate in a target cell. For example, in an adenoviral vector of the invention, a gene essential for replication may be selected from the group consisting of the E1a, E1b, E2a, E2b, and E4 genes. The terms also include replication-specific adenoviruses; that is, viruses that preferentially replicate in certain types of cells or tissues but to a lesser degree or not at all in other types of cells or tissues. Such viruses are sometimes referred to as "cytolytic" or "cytopathic" viruses (or vectors), and, if they have such an effect on neoplastic cells, are referred to as "oncolytic" viruses (or vectors).

Exemplary adenoviral vectors of the invention include, but are not limited to, DNA, DNA encapsulated in an adenovirus coat, adenoviral DNA packaged in another viral or viral-like form (such as herpes simplex, and AAV), adenoviral DNA encapsulated in liposomes, adenoviral DNA complexed with polylysine, adenoviral DNA complexed with synthetic polycationic molecules, conjugated with transferrin, or complexed with compounds such as PEG to immunologically "mask" the antigenicity and/or increase half-life, or conjugated to a nonviral protein.

In the context of adenoviral vectors, the term "5' " is used interchangeably with "upstream" and means in the direction of the left inverted terminal repeat (ITR). In the context of adenoviral vectors, the term "3' " is used interchangeably with "downstream" and means in the direction of the right ITR.

Standard systems for generating adenoviral vectors for expression of inserted sequences are known in the art and are available from commercial sources, for example the Adeno-X expression system from Clontech (Clontechniques (January 2000) p. 10-12).

The adenoviral vectors of the invention include replication defective and replication competent vectors. A replication defective vector does not replicate, or does so at very low levels, in the target cell. In one embodiment, a replication defective vector has at least one coding region in E1a, E1b, E2a, E2b or E4 inactivated, usually by deleting or mutating, part or all of the coding region. Methods for propagating these vectors are well known in the art.

The present invention contemplates the use of all adenoviral serotypes to construct adenoviral vectors and virus particles according to the present invention. Adenoviral stocks that can be employed according to the invention include any adenovirus serotype. Adenovirus serotypes 1 through 47 are currently available from American Type Culture Collection (ATCC, Manassas, VA), and the invention includes any other serotype of adenovirus available from any source. The adenoviruses that can be employed according to the invention may be of human or non-human origin. For instance, an adenovirus can be of subgroup A (e.g., serotypes 12, 18, 31), subgroup B (e.g., serotypes 3, 7, 11, 14, 16, 21, 34, 35), subgroup C (e.g., serotypes 1, 2, 5, 6), subgroup D (e.g., serotypes 8, 9, 10, 13, 15, 17, 19, 20, 22-30, 32, 33, 36-39, 42-47), subgroup E (serotype 4), subgroup F (serotype 40,41), or any other adenoviral serotype. Throughout the specification reference is made to specific nucleotides in adenovirus type 5. Based on knowledge generally available to those skilled in the art, one can determine the corresponding nucleotides in other serotypes and therefore construct similar adenoviral vectors in adenovirus serotypes other than serotype 5. In one preferred embodiment, the adenoviral nucleic acid backbone is derived from adenovirus serotype 2 (Ad2), 5 (Ad5) or 35 (Ad35), or a chimeric adenovirus backbone comprising a combination of a portion of adenovirus serotype 2(Ad2) or 5 (Ad5) with a portion of adenovirus serotype 35 (Ad35).

The DNA and protein sequences of Adenovirus serotypes 2 and 5 can be found in GenBank under Accession Number N_001405 (Ad2) and AY339865 (Ad5), both of which are incorporated herein in their entirety. Along with the complete genome DNA sequence, the GenBank entries include useful details such as references, location of splicing signals, polyadenylation sites, TATA signals, introns, start and stop codons for each identified gene, protein sequence, cDNA for each gene, and a list of sequence variations that exist throughout the literature. Also, of special interest with regards to the present invention, the mRNA structures for each region can be deduced from the indicated splicing site and polyadenylation cleavage site for each gene or region and the reference list of relevant publications in these GenBank records.

The following references provide details of adenovirus gene locations, gene splicing, locations of transcription elements (e.g. splice sites, polyadenylation signal sequences): Nevins and Chen-Kiang, Adv Virus Res. 1981;26:1-35.; Akusjarvi and Stevenin, Curr Topics Microbiol Immunology 272:253-86 (2003); Prescott and Falck-Pedersen, Mol Cell Biol 1994 14:4682-4693; Muhlemann et al. 1995 J Virol 69:7324-7327; Nevins and Wilson, Nature. 1981 Mar12;290 (5802):113-8; Prescott and Falck-Pederson, J Biol Chem 267:8175; 1992; Larsson, Svensson, and Akusjarvi J Mol Biol 225:287; 1992; Farley, Brown, and Leppard, J Virol 78: 1782; 2004. Adenovirus encodes genes and processing signals for genes on both strands (upper and lower) of its genome.

Care is taken when making any changes that there are no undesired changes on the complimentary strand. Also, in some cases, transgenes are relatively small and therefore multiple transgenes can potentially be inserted into one adenoviral vector genome. Transgenes can be essentially inserted anywhere between the Ad genes as long as essential genes and processing signals on both DNA strands are kept intact. Alternatively, in some situations, it may be desirable to alter existing genes and/or signals to change viral gene expression to suit the purpose of the vector. Also, it may be necessary to add branch point and splice acceptor sites or other processing signals along with the hansgene (s) for proper processing of the newly introduced mRNA for the new CDS.

In one preferred aspect, the adenoviral vector is replication-competent or replication conditional. Such vectors are able to replicate in a target cell. Replication competent viruses include wild-type viruses and viruses engineered to replicate in target cells. These include vectors designed to replicate specifically or preferentially in one type of target cell as compared to another. The target cell can be of a certain cell type, tissue type or have a certain cell status. Replication competent adenoviral vectors wherein replication is dependent upon cell type, tissue type or cell status are further described below.

In one embodiment, an adenoviral vector of the invention comprises one or more adenoviral genes essential for replication under transcriptional control of a heterologous transcriptional regulatory element (TRE) which confers selective replication on the adenovirus. The adenoviral gene essential for replication is an early gene, selected from the group consisting of E1A, E1B, E2a, E2b and E4. In a further embodiment, one or more additional TREs is operatively linked to one or more adenoviral genes essential for replication or a transgene, e.g., a therapeutic gene.

The adenoviral E1B 19-kDa region refers to the genomic region of the adenovirus E1B gene encoding the E1B 19-kDa product. According to wild-type Ad5, the E1B 19-kDa region is a 261bp region located between nucleotide (nt) 1714 and nt 2244. The E1B 19-kDa region has been described in, for example, Rao et al., Proc. Natl. Acad. Sci. USA, 89:7742-7746. In one embodiment, the present invention encompasses deletion of part or all of the E1B 19-kDa region as well as embodiments wherein the E1B 19-kDa region is mutated, as long as the deletion or mutation lessens or eliminates the inhibition of apoptosis associated with E1B-19kDa.

In an embodiment of the invention, adenovirus vectors replicate preferentially in carcinoma target cells, which replication preference is indicated by comparing the level of replication (e.g., cytolysis or cell killing and/or titer) in carcinoma target cells to the level of replication in non-target, non-carcinoma cells, normal or control cells. Comparison of the titer of a particular adenovirus in a target cell to the titer of the adenovirus in a non-target (or "TRE inactive" cell) indicates that the replication preference is enhanced in target cells and/or depressed in non-target cells.

An adenovirus vector may further include one or more heterologous TREs, which may or may not be operatively linked to the same gene. For example a cell type-specific, cell status-specific or tissue-type specific TRE, (all described herein as forms of "cell-specific" or "target cell specific" TREs) may be juxtaposed to a second TRE of the same or a different type. "Juxtaposed" means a first TRE and a second TRE transcriptionally control the same gene. For these embodiments, the more than one TRE may be in any of a number of configurations, including, but not limited to, (a) next to each other (i.e., abutting); (b) both 5' to the gene that is transcriptionally controlled (wherein the TREs may have intervening sequences between them); (c) one TRE may be 5' to the gene and the other TRE 3' to the gene.

The viral vectors of this invention can be prepared using recombinant techniques that are standard in the art. Methods of modifying replication-competent or replication-incompetent viral vectors are well known in the art and are described herein and in publications cited herein. Various methods for cloning transgenes and desired transcriptional elements into adenovirus are described herein and are standard and well known in the art. The transgene and desired transcriptional elements are cloned into various sites, as described herein, in the adenoviral vector genome. For example, there are various plasmids in the art that contain the different portions of the adenovirus genome, including plasmids that contain the entire adenovirus genome. The construction of these plasmids is also well described in the art (e.g. US20030104625). Once a site is selected for transgene(s) insertion an appropriate plasmid can be used to perform the modifications. Then the modifications may be introduced into a full-length adenoviral vector genome by, for example homologous recombination or *in vitro* ligation. The homologous recombination may take place in a mammalian cell (e.g. PerC6) or in a bacterial cell (e.g. E. Coli, see WO9617070). Manipulation of the viral vector genome can alternatively or in addition include well known molecular biology methods including, but not limited to, polymerase chain reaction (PCR), PCR-SOEing, restriction digests. If homologous recombination is employed, the two plasmids typically share at least about 500 bp of sequence overlap, although smaller regions of overlap will recombine, but usually with lower efficiencies. Each plasmid, as desired, may be independently manipulated, followed by cotransfection in a competent host, providing complementing genes as appropriate for propagation of the adenoviral vector. Plasmids are generally introduced into a suitable host cell (e.g. 293, PerC.6, Hela-S3 cells) using appropriate means of transduction, such as cationic liposomes or calcium phosphate. Alternatively, *in vitro* ligation of the right and left-hand portions of the adenovirus genome can also be used to construct recombinant adenovirus derivative containing all the replication-essential portions of adenovirus genome. Berkner et al. (1983) Nucleic Acid Research 11: 6003-6020; Bridge et al. (1989) J. Virol. 63: 631-638.

For convenience, plasmids are available that provide the necessary portions of adenovirus. Plasmid pXC.1 (McKinnon (1982) Gene 19:33-42) contains the wild-type left-hand end ofAd5. pBHG10 (Bett et al. (1994); Microbix Biosystems Inc., Toronto) provides the right-hand end of Ad5, with a deletion in E3. Deletion in E3 provides more room in the viral vector to insert heterologous sequences. The gene for E3 is located on the opposite strand from E4 (r-strand). pBHG11 provides an even larger E3 deletion, an additional 0.3 kb is deleted (Bett et al. (1994). Alternatively, the use of pBHGE3 (Microbix Biosystems, Inc.) provides the right hand end of Ad5, with a full-length of E3.

Methods of packaging polynucleotides into adenovirus particles are known in the art and are also described in PCT/US98/04080. The preferred packaging cells are those that have been designed to limit homologous recombination that could lead to wildtype adenoviral particles. Cells that may be used to produce the adenoviral particles of the invention include the human embryonic kidney cell line 293 (Graham et al., J Gen. Virol. 36:59-72 (1977)), the human embryonic retinoblast cell line PER.C6 (U.S. Patent Nos. 5,994,128 and 6,033,908; Fallaux et al., Hum. Gene Ther. 9: 1909-1917 (1998)), and the human cervical tumor-derived cell line HeLa-S3 (PCT Application No. US 04/11855). The viral vectors may be delivered to the target cell in a variety of ways, including, but not limited to, liposomes, general transfection methods that are well known in the art (such as calcium phosphate precipitation or electroporation), direct injection, and intravenous infusion. The means of delivery will depend in large part on the particular vector (including its form) as well as the type and location of the target cells (i.e., whether the cells are *in vitro* or *in vivo*).

The invention further provides a recombinant adenovirus particle comprising a recombinant viral vector according to the invention. In one embodiment, a capsid protein of the adenovirus particle comprises a targeting ligand. In one approach, the capsid protein is a fiber protein or pIX. In one aspect, the capsid protein is a fiber protein and the ligand is in the C terminus (carboxyl end) or HI loop of the fiber protein. The adenoviral vector particle may also include other mutations to the fiber protein. In an additional embodiment, the virus is targeted by replacing the fiber knob with a fiber knob from another adenovirus serotype. Examples of these mutations include, but are not limited to those described in US Application No. 10/403,337; US Application Publication No. 2004/0002060; PCT Publication Nos. WO 98/07877; WO 99/39734; WO 00/67576; WO 01/92299; and U.S. Patent Nos. 5,543,328; 5,731,190; 5,756,086; 5,770,442; 5,846,782; 5,962,311; 5,922,315; 6,057,155; 6,127,525; 6,153,435; 6,455,314; 6,555,368 and 6,683,170 and Wu et al. (J Virol. 2003 Jul 1;77(13):7225-7235). These include, but are not limited to, mutations that decrease binding of the viral vector particle to a particular cell type or more than one cell type, enhance the binding of the viral vector particle to a particular cell type or more than one cell type and/or reduce the immune response to the adenoviral vector particle in a mammal.

The adenoviral major late transcription unit (MLTU) is divided into five regions in Group C Adenoviruses. Each region is characterized by its own polyadenylation signal sequence and usually consists of several differentially spliced messages. All late messages are spliced at their 5' end to the tripartite leader (TPL). A description of each region follows.

In exemplary embodiments of the present invention, a transgene is inserted in at least one of the following locations: A) L1 region, insert between the 52, 55K and IIIa CDS or after the pIIIa CDS and upstream of the polyadenylation signal sequence of L1 region (Figure 3).

In one embodiment, the transgene is expressed as part of the L1 transcripts.

In one embodiment, the transgene in the vector utilizes (i.e is operatively linked to) the same polyA signal used by at least one of the viral mRNAs. For example, , when inserting the transgene in a major late transcription unit, the method for inserting the transgene does not create a new or 6th leader sequence . The same polyA signal is a native Ad polyA signal.

Modifications of sequences near a polyadenylation signal sequence may influence the efficiency of the cleavage. Therefore modifications to these regions may increase, decrease or result in equivalent cleavage efficiency. One skilled in the art can consider the desired timing of expression and amount of expression of the transgene when selecting an insertion site.

In one embodiment of the invention, an IRES or self-processing cleavage sequence, is operatively linked to a transgene that is inserted upstream of a polyadenylation signal sequence and downstream of an adenoviral coding sequence. The polyadenylation signal sequence is usually an adenoviral polyadenylation signal sequence. In one embodiment of the invention, the IRES or self-processing cleavage sequence and i.e. transgene are inserted into an adenovirus transcription unit or leader sequence (i.e. L1) that codes for more than one protein (e.g. due to differential splicing). This embodiment gives the advantage that the IRES or self-processing cleavage sequence will be functional with each mRNA from said transcript or leader region. In other words, each mRNA from said transcription unit should operatively code and express the transgene in addition to the adenoviral coding sequence (CDS). In a comparative example, an IRES or self-processing cleavage sequence followed by a transgene is inserted upstream of the L2 polyadenylation signal, all four types of mRNAs produced from the L2 region would contain the inserted sequences and thus all four types of mRNAs would express the transgene. Without being bound by theory, it is believed that the closer the IRES or self-processing cleavage sequence and transgene are placed to the polyadenylation signal, the higher the expression level of the transgene. One skilled in the art can consider the desired timing of expression and amount of expression of the transgene when selecting an insertion site. For example, if expression of transgenes at an intermediate time in relation to the viral replication cycle is desired, then an IRES or self-processing cleavage sequence transgene cassette may also be inserted upstream of the polyA signal of either pIX or IVa2. Various types of IRESs and self-processing cleavage sequences are available and each one varies in its efficiency to mediate translation. One skilled in the art can choose a particular IRES or self-processing cleavage sequence with a known efficiency for tailored transgene expression efficiency. When adding an IRES or self-processing cleavage sequence and transgene, care must be taken not to disrupt other adenovirus sequences including those coded for on the other strand (i.e. anti-sense strand) unless disruption of certain adenovirus gene expression is desired. Due to the extensive characterization of adenoviral gene expression, one skilled in the art can determine sites that will and will not disrupt adenoviral gene expression.

L1 Region

The L1 region includes the coding regions for 52/55K and IIIa, as shown in Figures 1-3. Both genes have only one base upstream of the ATG after the splice acceptor site. In all late regions, the mRNAs encoded in each region are attached to the tripartite leader at their splice acceptor site. The presence and location of splicing enhancer sequences (25 base pair sequences) between 52/55K and IIIa coding regions has been identified. In addition, there is a splice repressor region directly upstream of the splice enhancer. Upstream of the L1 region are the VA RNA coding regions and the region that encodes E2B, which is present on the complementary strand. In fact, VA RNA II ends about 10 base pairs from the splice acceptor site for 52/55K mRNA. The splice acceptor site for the downstream L2 region is upstream of the L1 polyadenylation signal sequence. These overlapping features should be considered and usually will be left intact when inserting transgenes into the L1 region. L1 mRNA begins to be synthesized during the early phase of infection, although only detectable levels of the 52/55K gene are made. After entering the late phase, the IIIa coding region is translated and the level of 52/55K translation is much lower. Again the mechanism of this shift in regulation is due to differential splice site usage which is affected by entry into the late phase of infection.

Transgene(s) can potentially be placed between the two L1 coding regions, or upstream of the 52/55K coding region, or immediately downstream of the IIIa coding region and upstream of the L1 polyadenylation signal sequence. In one embodiment, a branch point sequence and splice acceptor site is added for the transgene and/or downstream for the adenoviral coding region. In another embodiment, a self-processing cleavage site is added downstream of and operatively linked to the IIIa region and then the self-processing cleavage site is operably linked to a transgene located downstream of the self-processing cleavage site. Also, when designing insertions downstream of the IIIa coding region, care must be taken to avoid disruption of the L1 polyadenylation signal sequence and the L2 splice acceptor site. Alternatively, these signals can be restored or altered to change the protein expression, if desired. It is thought that transgenes inserted upstream and downstream of 52/55K will be expressed in the early phase and transgenes inserted downstream of IIIa will be expressed during the late phase of infection, similar to the viral proteins. This may be altered depending on, for example, the disposition of the splice enhancer and repressor regions.

In another embodiment an IRES operatively linked to a transgene is inserted downstream of the IIIa coding region and upstream of (i.e. operatively linked to) the L1 polyadenylation signal sequence or a heterologous polyadenylation signal sequence.

<u>L2 region</u>

The L2 region encodes four identified coding regions: penton (aka m), proVII (aka pVII), pV, and pX (sometimes called pV precursor or "mu"), as shown in Figures 1-2 and 4. Penton has a splice acceptor site 2 bases upstream of its start codon. The splice site for proVII mRNA is embedded in the penton coding region and its start codon is 7 base pairs downstream of the stop codon for penton. The stop codon for proVII, the splice site for pV mRNA, and start codon for pV are separated by 46 and 23 base pairs, respectively. The pX, and its ATG start codon is 124 bases downstream of the pV stop codon. The polyadenylation signal sequence for the L2 region is 26 bases after the pX stop codon, and cleavage takes place about 12 bases after the polyA signal.

Options for transgene insertion include, but are not limited to: a) inserting after pV, or pX CDSs and upstream of a L2 polyadenylation signal sequence, b) inserting downstream of a splice acceptor site for pV mRNA and upstream of the pV CDS, and c) inserting between a penton stop codon and a pVII start codon. When the transgene insertion method and expression relies on alternative splicing, it is desirable to have a splice acceptor site for each coding region, be it an existing or an exogenously added sequence. All L2 mRNA messages will usually use the L2 polyadenylation signal, but in some embodiments a non-native polyA signal may be utilized.

A transgene may also be expressed from these regions of the L2 region using a self-processing cleavage site. The self-processing cleavage site may be operatively linked to the 3' end of the Ad CDS (e.g. penton, pro VII, pV, and pX) and the transgene CDS is operatively linked to the 3' end of the self-processing cleavage site. In an alternative embodiment, the transgene is inserted up stream of an Ad CDS, wherein the self-processing cleavage site is operatively linked to the 3' end of the transgene and the Ad CDS is operatively linked to the 3' end of the self-processing cleavage site. In this case, the transgene is inserted with the proper transcriptional elements (as described herein) so that it is transcribed from the vector. This may include inserting the transgene so it is operatively linked to an Ad splice site and branch point. In one embodiment, the Ad splice site and branch point are the ones linked in the native virus to the downstream Ad CDS. In another embodiment, the transgene is operatively linked to a splice site and branch point wherein either one or both are heterologous.

AN IRES may be operatively linked to a transgene is inserted downstream of the pV coding region and upstream of (i.e. operatively linked to) the L2 polyA signal or a heterologous polyadenylation signal sequence.

<u>L3 region</u>

The L3 region contains coding regions for pVI, hexon (II), and 23K (viral protease), as shown in Figures 5-7. The splice acceptor site for the pVI mRNA is downstream of the L2 polyadenylation site and one base upstream of the pVI start codon. The stop codon for pVI is about 50 bases upstream of the splice acceptor site for the hexon message, and the hexon start codon is about 35 bases from its mRNA splice acceptor site. The splice acceptor site for the 23K mRNA is contained in the coding sequence for hexon, about 95 bases upstream of the hexon stop codon. The start codon for 23K is 32 bases downstream of the hexon stop codon. The stop codon for 23K is about 25 bases upstream of the L3 polyA signal. The L3 polyadenylation region overlaps with the polyadenylation signal for E2A located on the complementary strand. In most cases, it is desirable to keep the polyadenylation signal sequence on both strands intact for efficient processing.

There are several options for transgene insertions into the L3 region: a) upstream of the pVI coding region, b) between pVI and hexon coding region, c) between the hexon and 23K coding region, and d) at the end of 23K CDS. Note that the L3 polyA signal is very strong and hexon mRNA is very abundantly expressed. It is predicted that transgenes inserted into L3 will also be expressed abundantly. Also, for insertions between the hexon and 23K coding regions, it may be desirable to use the splice acceptor site within the hexon coding region (normally used for the 23K mRNA) for the transgene mRNA and add a new branch point and splice acceptor site for synthesis of the 23K message. Alternatively, the transgene is linked to the hexon CDS with a self-processing cleavage site. In another embodiment an IRES operatively linked to a transgene is inserted downstream of the 23K coding region and upstream of (i.e. operatively linked to) the L3 polyA signal or a heterologous polyadenylation signal sequence.

L4 region

The L4 region contains coding regions for the 100K, 33K, and pVIII proteins, as shown in Figures 1 and 2. A portion of the CDS for 33K overlaps the CDS for 100K. Although there are non-coding base pairs between these two genes, it is not a desirable location to insert a transgene since this may disrupt the intron for 33K or alter its usage. The pVIII CDS and the polyA region overlaps the E2 and E3 promoters. On the complementary strand are the leader sequences for the E2A and E2B transcription units. Therefore when utilizing this region for transgene insertion, the designer should be aware of this. It is likely that any insertions or disruptions in this region may alter or disrupt expression of essential genes in the E2, E3, and/or L4 region.

Moreover, an IRES or self processing cleavage site may be operatively linked to a transgene is inserted downstream of the 100K coding region and upstream of (i.e. operatively linked to) the L4 polyA signal or a heterologous polyadenylation signal sequence. In this case, the E3 12.5K CDS will likely be interrupted since its CDS overlaps the L4 polyA sequence. However, some mutants that have this gene deleted have not shown a change in phenotype *in vitro,* so this location/method for transgene insertion can lead to a functional adenoviral vector.

L5 region

The L5 region in Ad5 encodes only the fiber protein as shown in Figures 1 and 2. The splice acceptor site for fiber mRNA is 2 nucleotides (nts) upstream of the fiber start codon. The fiber stop codon is embedded in the L5 polyA signal. Transgenes can be inserted either upstream or downstream of the fiber coding region. However, for downstream insertions, the native L5 polyadenylation signal sequence is modified so the sequence no longer provides a polyadenylation function. A splice acceptor site and a transgene may be inserted for synthesis of the mRNA. A polyadenylation signal is then restored downstream of the inserted transgene for use by the altered L5 region: Alternatively, a self-processing cleavage site is operatively linked to the downstream end of the fiber CDS and a transgene CDS is inserted downstream of and operatively linked to the self-processing cleavage site.

Additional late region

Another approach to inserting transgenes into a viral vector, such as Ad is to insert an additional late region. This can be done, for example, by inserting just downstream of an existing region or transcription unit (such as L3, for example), a cassette consisting of a branch point and a splice acceptor site, a transgene(s), and a polyadenylation signal (and any other necessary signals). This cassette could also consist of an IRES or self processing cleavage site operatively linked to a second transgene. The second transgene could code for the same or a different protein as the first transgene. In the case of the same protein, it is preferred that the coding sequence of one of the transgenes be "recoded". In other words, use different codons to code for the same amino acids. This is done to reduce the amount of homology between the two transgenes at the DNA level, thus reducing or eliminating homologous recombination between the two transgenes.

E3 Region

The E3 region in adenovirus encodes the genes for the 12.5K, 6.7K, gp19K, ADP, RID-alpha, RID-beta and 14.7K identified proteins. A transgene may be inserted downstream of the gp19K coding region and upstream of the ADP CDS. Interestingly, there are 128 bps in the Ad5 virus located between the gp19K and ADP coding regions (base pairs 29209-29336 in Ad genome accession number AY339865; SEQ ID NO:41).

128 bps or the majority of these 128 bps may be deleted from an Ad5 vector. In one case, the vector contains the gp19K and/or ADP coding regions and is deleted for these 128 bps in an Ad5 vector. Ad2 virus does not contain these 128 bps or similar sequences in this region. In another situation, a transgene is inserted after the 14.7K coding region and upstream of the E3B polyadenylation signal sequence. In another situation, a transgene is inserted after the ADP coding region. However the ADP stop codon is embedded in the E3A polyA site. Therefore, the polyA site would be modified so the sequence no longer provides a polyadenylation function, but still contains a stop codon in frame with the ADP coding sequence. A transgene can be added (e.g. along with a splice acceptor site or self-processing cleavage site) downstream of the ADP CDS. Then a polyadenylation signal sequence is inserted downstream of the transgene coding region. The inserted polyadenylation signal sequence may be the native E3A polyA site or another polyA site (e.g. SV40 polyA site).

The E3 coding regions are not required for viral replication. Therefore, the E3 coding regions are not necessary in an adenoviral vector. As a result, viral vectors of the invention may have one or more E3 coding regions deleted or alternatively, may contain all of the E3 coding regions. In embodiments of the invention, a E3 14.7, 14.5 or 10.4k coding regions or combinations thereof are retained in the adenoviral vector. In one embodiment of the invention, the transgene is not inserted in place of an E3 CDS. For example, the transgene is inserted between two native E3 CDSs, wherein said two native coding sequences are adjacent to each other in the native virus and do not have another E3 CDS located between them. In some embodiments, adenoviral vectors of the invention contain a heterologous splice acceptor site operatively linked to a transgene, which is transcribed as part of the E3 transcription unit. For example, a heterologous splice acceptor site is operatively linked to a transgene and both are inserted into the E3 region.

A self processing cleavage site may be operatively linked to a transgene CDS inserted so the self-processing cleavage site is also operatively linked to an adenoviral E3 CDS. The transgene CDS may be downstream of the self-processing cleavage site, with the E3 CDS being upstream of the self-processing cleavage site. Furthermore, the E3 CDS may be downstream of the self-processing cleavage site, with the transgene CDS being upstream of the self-processing cleavage site.

An IRES operatively linked to a transgene may be inserted downstream of the 14.7K coding region and upstream of (i.e. operatively linked to) the E3B polyA signal or a heterologous polyadenylation signal sequence. In one situation, an IRES operatively linked to a transgene is inserted downstream of the ADP coding region and upstream of (i.e. operatively linked to) the E3A polyA signal or a heterologous polyadenylation signal sequence. The E3 region codes for proteins that are not necessary for viral replication and therefore are dispensable for certain type of and uses of adenoviral vectors. Therefore, alternatively one or more E3 coding sequences are deleted or mutated and the heterologous DNA comprised of an IRES operatively linked to a transgene is inserted between the E3A or E3B polyadenylation signal sequence and the E3 coding sequence present immediately 5' to the E3A or E3B polyadenylation signal sequence, respectively.

Any of the methods for transgene insertions described herein may be combined with any other method of transgene insertion described herein or elsewhere as long as the sites of insertion are compatible. For example, a first transgene operatively linked to a splice acceptor site and a branch point may be inserted in a transcriptional unit. The inserted first transgene may then be operatively linked to a self-processing cleavage site inserted downstream of said first transgene and a second transgene is then inserted downstream of and operatively linked to the self-processing cleavage site. It is appreciated that one skilled in the art can rely on the teachings herein and insert transgenes using combinations of the disclosed transgene insertion and expression methods, all of which methods and insertion sites are encompassed by the present invention.

If the adenoviral vector DNA is to be packaged into a viral virion, then care must be taken not to exceed the packaging capacity of the virus. For example, for Ad5 when the genomic DNA is larger than about 105% of the size of the wild-type Ad5 genome, the packaging efficiency greatly decreases (Bett et al. Virol. 1993 Oct;67(10):5911-21). However, Ad5 vectors of larger sizes have been reported to be stable.

For the purposes of simplicity, the descriptions hereinabove, unless otherwise stated, refer to Ad serotype 5. One skilled in the art can readily deduce without undue experimentation equivalent insertion sites for transgenes in other adenoviruses or other viruses.

Depending on the properties of the transgene(s), it may be desirable to express it during the early or late phase of infection; additionally, it may be desirable to have either high or low levels of expression. For example, in some cases, there may be a preference for expression during the late phase of infection following viral DNA replication. If the vector is an oncolytic Ad vector with a tumor-specific promoter driving expression of early genes, then late expression allows an extra regulation mechanism because late gene expression requires DNA replication. If the transgene is inserted in a late transcript, expression will be specific since viral replication will be specific for the target cells if the Ad vector is designed to replicate selectively.

Another mechanism that can be used to help regulate expression of transgenes or change expression of viral genes, is to modify the sequence of the added elements (e.g. branch point sequences and/or splice acceptor sites or self-processing cleavage sites) as compared to a consensus sequence. This changes the efficiency of usage. For example, in the case of a splice acceptor site, if high expression levels are desired, one would use sequences very close to a consensus splice acceptor sequence. A consensus splice acceptor site sequence was determined to be $(T/C)_8$, N, C/T,A,G,G (Mount, Nucleic Acids Res 10:459; 1982). Mutational analysis has been performed that changes the efficiency of cleavage (Roscigno, et al. J Biol Chem 268: 11222; 1993; Lee et al. Gene Therapy 11: 94; 2004). It should be noted that the general accepted consensus sequence definitions and usage can and do change after the virus goes into late phase (Akusjarvi and Svevenin, Curr Top Microbiol Immunol 272: 253; 2003; Nevins and Wilson, Nature 290:113; 1981; Akusjarvi and Persson, J Virol 38: 469; 1981). For example, non-consensus splice acceptor site usage is enhanced during the late phase of infection (Muhlemann et al. J Virol 69: 7324; 1995). Most of these changes are due to the effect that viral proteins have on the cellular machinery for transcription and translation.

In addition to the heterologous splice acceptor site, a heterologous branch point sequence may be included in the adenoviral vectors of the invention. A branch point sequence is necessary for efficient splicing of an intron (Vandenbroucke et al., BMC Genomics 3: 13; 2002; Hall et al., PNAS 85:704-708; 1988; Harris et al. Nucl Acid Res 18(10) 3015-3019 1990). The distance of the branch point to the splice acceptor site also influences the efficiency of splicing and is usually, but not always, located 10-50 and even more frequently 18-37 base pairs upstream of the splice acceptor site (Vandenbroucke et al., 2002; Hall et al. 1988; Harris et al. 1990). The consensus sequence of a branch point sequence is believed to be YNYURAY (SEQ ID NO:40) (where Y is a pyrimidine, N is any nucleotide, and R is a purine) and the underlined A is invariant (Liao et al., Virology 323:131; 2004). Any deviations in this sequence may result in changes in efficiency of usage of the branch point and splice acceptor site.

In one embodiment of the invention, the sequence of the branch point plus splice acceptor sequence is: TACTTAT GACTCGTACTATTGTTATTCATCC AG↓G (SEQ ID NO:39) The underlined sequence is the branch point sequence and the arrow indicates the location of the splice site according to splicing rules. Since the rules governing the consensus sequence are not invariant, other similar sequences that conform to the rules can be used. In one embodiment, a branch point plus splice acceptor site is used from another Ad serotype. In other words, the branch point plus splice acceptor site is hetereologous, i.e., not from the native adenovirus that the Ad vector is based on, but is from a different Ad serotype.

Other optional sequences can be added that change the efficiency of splicing and/or expression of the transgene as desired. For example, cis-acting elements present in the exon have been identified that can enhance or suppress splicing. These sequences are called exonic splicing enhancer (ESE) or exonic splicing suppressor (ESS) (reviewed in Zheng, J Biomed Sci 11:278; 2004). Although there are not consensus sequences for these elements, many examples of ESSs and ESEs have been identified in other viral and non-viral organisms and these may be used. One skilled in the art is able to survey the literature and data and choose appropriate sequences.

In summary, the provided are methods for inserting transgene coding regions in specific regions of the viral vector genome. The methods take advantage of known viral transcription elements and the mechanisms for expression of Ad genes, reduce the size of the DNA sequence for transgene expression that is inserted into the Ad genome since no additional promoter is necessary and the regulation signals encompass a smaller size DNA fragment, provide flexibility in temporal regulation of the transgene (e.g. early versus late stage of infection; early versus intermediate stage of infection), and provide techniques to regulate the amount of transgene expressed. For example, a higher amount of transgene can be expressed by inserting the transgene into a transcript that is expressed normally at high levels and/or by operatively linking a high efficiency splice acceptor site to the transgene coding region. Expression levels are also affected by how close the regulating signals are to their consensus sequences; changes can be made to tailor expression as desired.

In some cases expression of a transgene may inhibit the life cycle of a replication competent virus. In this case the transgene may be inserted in a way that the transgene is only or mostly expressed at the late stages of infection (after viral DNA replication). For example, the transgene may be inserted in L3. For some transgenes, it may be desired to express the transgene early in the viral life cycle. For example, the transgene may be inserted in any of the early regions (e.g. E3) or into the upstream L1 region.

## Transcriptional Regulatory Elements (TREs)

Transcriptional regulatory element (TREs), as well as methods for their identification, isolation, characterization, genetic manipulation and use for regulation of operatively linked coding sequences, are known in the art. A TRE can be derived from the transcriptional regulatory sequence of a single gene, sequences from different genes can be combined to produce a functional TRE, or a TRE can be synthetically generated (e.g. the CTP4 promoter).

A TRE can be tissue-specific, tumor-specific, developmental stage-specific, cell status specific, etc., depending on the type of cell present in the target tissue or tumor. Such TREs are collectively referred to herein as tissue-specific or target cell-specific. As described in more detail below, a target cell-specific TRE can comprise any number of configurations, including, but not limited to, a target cell-specific promoter and target cell-specific enhancer; a heterologous promoter and a target cell-specific enhancer; a target cell-specific promoter and a heterologous enhancer; a heterologous promoter and a heterologous enhancer; and multimers of the foregoing. The promoter and enhancer components of a target cell-specific TRE may be in any orientation and/or distance from the coding sequence of interest, as long as the desired target cell-specific transcriptional activity is obtained.

Transcriptional activation can be measured in a number of ways known in the art (and described in more detail below), but is generally measured by detection and/or quantitation of mRNA or the protein product of the coding sequence under control of (i.e., operably linked to) the target cell-specific TRE.

As further discussed herein, a target cell-specific TRE can be of varying lengths, and of varying sequence composition. A target cell-specific TRE is preferentially functional in a limited population (or type) of cells, e.g., prostate cells, liver cells, melanoma cells, etc. Accordingly, in some embodiments, the TRE used is preferentially functional in any of the following tissue types: prostate; liver; breast; urothelial (bladder); colon; lung; ovarian; pancreas; stomach; uterine, etc.

As is readily appreciated by one skilled in the art, a TRE is a polynucleotide sequence, and, as such, can exhibit function over a variety of sequence permutations. Methods of nucleotide substitution, addition, and deletion are known in the art, and readily-available functional assays (such as the CAT or luciferase reporter gene assay) allow one of ordinary skill to determine whether a sequence variant exhibits requisite cell-specific transcription regulatory function. Hence, functionally preserved variants of TREs, comprising nucleic acid substitutions, additions, and/or deletions, can be used in the vectors disclosed herein. Accordingly, variant TREs retain function in the target cell but need not exhibit maximal function. In fact, maximal transcriptional activation activity of a TRE may not always be necessary to achieve a desired result, and the level of induction afforded by a fragment of a TRE may be sufficient for certain applications. For example, if used for treatment or palliation of a disease state, less-than-maximal responsiveness may be sufficient if, for example, the target cells are not especially virulent and/or the extent of disease is relatively confined.

Certain base modifications may result in enhanced expression levels and/or cell-specificity. For example, nucleic acid sequence deletions or additions within a TRE can move transcription regulatory protein binding sites closer or farther away from each other than they exist in their normal configuration, or rotate them so they are on opposite sides of the DNA helix, thereby altering spatial relationship among TRE-bound transcription factors, resulting in a decrease or increase in transcription, as is known in the art. Thus, while not wishing to be bound by theory, the present disclosure contemplates the possibility that certain modifications of a TRE will result in modulated expression levels as directed by the TRE, including enhanced cell-specificity. Achievement of enhanced expression levels may be especially desirable in the case of more aggressive forms of neoplastic growth, and/or when a more rapid and/or aggressive pattern of cell killing is warranted (for example, in an immunocompromised subject).

A TRE for use in the present vectors may or may not comprise a silencer. The presence of a silencer (i.e., a negative regulatory- element known in the art) can assist in shutting off transcription (and thus replication) in non-target cells. Thus, presence of a silencer can confer enhanced cell-specific vector replication by more effectively preventing replication in non-target cells. Alternatively, lack of a silencer may stimulate replication in target cells, thus conferring enhanced target cell-specificity.

Transcriptional activity directed by a TRE (including both inhibition and enhancement) can be measured in a number of ways known in the art, but is generally measured by detection and/or quantitation of mRNA and/or of a protein product encoded by the sequence under control of (i.e., operably linked to) a TRE.

As discussed herein, a TRE can be of varying lengths, and of varying sequence composition. The size of a heterologous TRE will be determined in part by the capacity of the viral vector, which in turn depends upon the contemplated form of the vector. Generally minimal sizes are preferred for TREs, as this provides potential room for insertion of other sequences which may be desirable, such as transgenes and/or additional regulatory sequences. In a preferred embodiment, such an additional regulatory sequence is an IRES, a self-processing cleavage sequence such as a 2A or 2A-like sequence, a splicing sequence or a branch site.

By way of example, an adenoviral vector can be packaged with extra sequences totaling up to about 105% of the genome size, or approximately 1.8 kb, without requiring deletion of viral sequences. If non-essential sequences are removed from the adenovirus genome, an additional 4.6 kb of insert can be tolerated (i.e., for a total insertion capacity of about 6.4 kb).

In the case of replication-competent adenoviral vectors, in order to minimize non-specific replication, endogenous (adenovirus) TREs (i.e., the native E1A and/or E1B promoter) are preferably removed from the vector. Besides facilitating target cell-specific replication, removal of endogenous TREs also provides greater insert capacity in a vector, which is of special concern if an adenoviral vector is to be packaged within a virus particle. Even more importantly, deletion of endogenous TREs prevents the possibility of a recombination event whereby a heterologous TRE is deleted and the endogenous TRE assumes transcriptional control of its respective adenovirus coding sequences (thus allowing non-specific replication). In one embodiment, an adenoviral vector is constructed such that the endogenous transcription control sequences of one or more adenoviral genes are deleted and replaced by one or more heterologous TREs. However, endogenous TREs can be maintained in the adenovirus vector(s), provided that sufficient cell-specific replication preference is preserved. These embodiments are constructed by inserting heterologous TREs between an endogenous TRE and a gene coding segment required for replication. Requisite cell-specific replication preference is determined by conducting assays that compare replication of the adenovirus vector in a cell which allows function of the heterologous TREs with replication in a cell which does not.

Generally, a TRE will increase replication of a vector in a target cell by at least about 2-fold, preferably at least about 5-fold, preferably at least about 10-fold more preferably at least about 20-fold, more preferably at least about 50-fold, more preferably at least about 100-fold, more preferably at least about 200-fold, even more preferably at least about 400- to about 500- fold, even more preferably at least about 1000-fold, compared to basal levels of replication in the absence of a TRE. The acceptable differential can be determined empirically (by measurement of mRNA levels using, for example, RNA blot assays, RNase protection assays or other assays known in the art) and will depend upon the anticipated use of the vector and/or the desired result.

Adenoviral vectors directed at specific target cells can be generated using TREs that are preferentially functional in a target cell. In one embodiment of the present invention, a target cell-specific or cell status-specific, heterologous TRE is tumor cell-specific. A vector can comprise a single tumor cell-specific TRE or multiple heterologous TREs which are tumor cell-specific and functional in the same cell. In another embodiment, a vector comprises one or more heterologous TREs which are tumor cell-specific and additionally comprises one or more heterologous TREs which are tissue specific, whereby all TREs are functional in the same cell.

In a preferred embodiment for the oncolytic adenovirus platform, bicistronic or multicistronic cassettes containing an IRES or a self processing cleavage sequence such as a 2A or 2A-like sequence comprise adenoviral early viral genes (E1A, E1B, E2, E3, and/and or E4) or genes expressed later in the viral life cycle (fiber, penton, and hexon).

In certain instances, it may be desirable to enhance the degree and/or rate of cytotoxic activity, due to, for example, the relatively refractory nature or particular aggressiveness of the cancerous target cell. An example of a viral gene that contributes to cytotoxicity includes, but is not limited to, the adenovirus death protein (ADP) gene. In another embodiment disclosed herein, the adenovirus comprises the adenovirus E1B gene which has a deletion in or of its endogenous promoter. In other embodiments disclosed herein, the 19-kDa region of E1B is deleted.

To provide enhanced cytotoxicity to target cells, one or more transgenes having a cytotoxic effect may be present in the vector. Additionally, or alternatively, an adenovirus gene that contributes to cytotoxicity and/or cell death, such as the adenovirus death protein (ADP) gene, can be included in the vector, optionally under the selective transcriptional control of a heterologous TRE and optionally under the translational control of an IRES or a self-processing cleavage sequence, such as a 2A or 2A-like sequence. This could be accomplished by coupling the target cell-specific cytotoxic activity with cell-specific expression of, a heterologous gene or transgene.

Any of a number of heterologous therapeutic genes or transgenes may be included in the replication competent viral vectors of the invention, as further described below.

Typically, the aforementioned bicistronic or multicistronic cassettes are placed under the control of a transcriptional response element, generally a cell type or cell status associated transcriptional regulatory element that is preferentially expressed in cancer or tumor cells. Accordingly, the therapeutic gene included in a given construct will vary dependent upon the type of target cell.

As is known in the art, activity of TREs can be inducible. Inducible TREs generally exhibit low activity in the absence of inducer, and are up-regulated in the presence of an inducer. Inducers include, for example, nucleic acids, polypeptides, small molecules, organic compounds and/or environmental conditions such as temperature, pressure or hypoxia. Inducible TREs may be preferred when expression is desired only at certain times or at certain locations, or when it is desirable to titrate the level of expression using an inducing agent. For example, transcriptional activity from the *PSE*-TRE, *PB*-TRE and *hKLK2*-TRE is inducible by androgen, as described herein and in PCT/US98/04080, expressly incorporated by reference herein. Accordingly, in one embodiment of the present invention, the adenovirus vector comprises an inducible heterologous TRE.

A TRE as used in the present invention can be present in a variety of configurations. A TRE can comprise multimers. For example, a TRE can comprise a tandem series of at least two, at least three, at least four, or at least five target cell-specific TREs. These multimers may also contain heterologous promoter and/or enhancer sequences.

Alternatively, a TRE can comprise one or more promoter regions along with one or more enhancer regions. TRE multimers can also comprise promoter and/or enhancer sequences from different genes. The promoter and enhancer components of a TRE can be in any orientation with respect to each other and can be in any orientation and/or any distance from the coding sequence of interest, as long as the desired cell-specific transcriptional activity is obtained.

As used herein, a TRE derived from a specific gene is referred to by the gene from which it was derived and is a polynucleotide sequence which regulates transcription of an operably linked polynucleotide sequence in a host cell that expresses the gene. For example, as used herein, a "human glandular kallikrein transcriptional regulatory element", or "*hLk2*-TRE" is a polynucleotide sequence, preferably a DNA sequence, which increases transcription of an operably linked polynucleotide sequence in a host cell that allows an *hKLK2*-TRE to function, such as a cell (preferably a mammalian cell, even more preferably a human cell) that expresses androgen receptor, such as a prostate cell. An *hKLK2*-TRE is thus responsive to the binding of androgen receptor and comprises at least a portion of an *HULK2* promoter and/or an *hKLK2* enhancer (i.e., the ARE or androgen receptor binding site). A human glandular kallikrein enhancer and adenoviral vectors comprising the enhancer are described in WO99/06576, expressly incorporated by reference herein.

As used herein, a "probasin *(PB)* transcriptional regulatory element", or "*PB*-TRE" is a polynucleotide sequence, preferably a DNA sequence, which selectively increases transcription of an operably-linked polynucleotide sequence in a host cell that allows a *PB*-TRE to function, such as a cell (preferably a mammalian cell, more preferably a human cell, even more preferably a prostate cell) that expresses androgen receptor. A *PB*-TRE is thus responsive to the binding of androgen receptor and comprises at least a portion of a *PB* promoter and/or a *PB* enhancer (i.e., the ARE or androgen receptor binding site). Adenovirus vectors specific for cells expressing androgen are described in WO98/39466, expressly incorporated by reference herein.

As used herein, a "prostate-specific antigen (*PSA*) transcriptional regulatory element", or "*PSA*-TRE", or "*PSE*-TRE" is a polynucleotide sequence, preferably a DNA sequence, which selectively increases transcription of an operably linked polynucleotide sequence in a host cell that allows a *PSA*-TRE to function, such as a cell (preferably a mammalian cell, more preferably a human cell, even more preferably a prostate cell) that expresses androgen receptor. A *PSA*-TRE is thus responsive to the binding of androgen receptor and comprises at least a portion of a *PSA* promoter and/or a *PSA* enhancer (i.e., the ARE or androgen receptor binding site). A tissue-specific enhancer active in prostate and use in adenoviral vectors is described in WO.95/19434 and WO 9.7/01358, each of which is expressly incorporated by reference herein.

As used herein, a "carcinoembryonic antigen (*CEA*) transcriptional regulatory element", or *"CEA*-TRE" is a polynucleotide sequence, preferably a DNA sequence, which selectively increases transcription of an operably linked polynucleotide sequence in a host cell that allows a *CEA*-TRE to function, such as a cell (preferably a mammalian cell, even more preferably a human cell) that expresses *CEA.* The *CEA*-TRE is responsive to transcription factors and/or co-factor(s) associated with *CEA*-producing cells and comprises at least a portion of the *CEA* promoter and/or enhancer. Adenovirus vectors specific for cells expressing carcinoembryonic antigen are described in WO 98/39467, expressly incorporated by reference herein.

As used herein, an "alpha-fetoprotein (*AFP*) transcriptional regulatory element", or "*AFP*-TRE" is a polynucleotide sequence, preferably a DNA sequence, which selectively increases transcription (of an operably linked polynucleotide sequence) in a host cell that allows an *AFP*-TRE to function, such as a cell (preferably a mammalian cell, even more preferably a human cell) that expresses *AFP.* The *AFP*-TRE is responsive to transcription factors and/or co-factor (s) associated with *AFP*-producing cells and comprises at least a portion of the *AFP* promoter and/or enhancer. Adenovirus vectors specific for cells expressing alpha fetoprotein are described in WO 98/39465, expressly incorporated by reference herein.

As used herein, an "a mucin gene *(MUC)* transcriptional regulatory element", or *"MUC1*-TRE" is a polynucleotide sequence, preferably a DNA sequence, which selectively increases transcription (of an operably-linked polynucleotide sequence) in a host cell that allows a *MUC1*-TRE to function, such as a cell (preferably a mammalian cell, even more preferably a human cell) that expresses *MUC1.* The *MUC1*-TRE is responsive to transcription factors and/or co-factor (s) associated with *MUC1*-producing cells and comprises at least a portion of the *MUC1* promoter and/or enhancer.

As used herein, a "urothelial cell-specific transcriptional response element", or "urothelial cell-specific TRE" is polynucleotide sequence, preferably a DNA sequence, which increases transcription of an operably linked polynucleotide sequence in a host cell that allows a urothelial-specific TRE to function, i.e., a target cell. A variety of urothelial cell-specific TREs are known, are responsive to cellular proteins (transcription factors and/or cofactor(s)) associated with urothelial cells, and comprise at least a portion of a urothelial-specific promoter and/or a urothelial-specific enhancer. Exemplary urothelial cell specific transcriptional regulatory sequences include a human or rodent uroplakin (UP), e.g., UPI, UPII, UPIII and the like. Human urothelial cell specific uroplakin transcriptional regulatory sequences and adenoviral vectors comprising the same are described in WO 01/72994, expressly incorporated by reference herein.

As used herein, a "melanocyte cell-specific transcriptional response element", or "melanocyte cell-specific TRE" is a polynucleotide sequence, preferably a DNA sequence, which increases transcription of an operably linked polynucleotide sequence in a host cell that allows a melanocyte-specific TRE to function, i.e., a target cell. A variety of melanocyte cell-specific TREs are known, are responsive to cellular proteins (transcription factors and/or co-factor(s)) associated with melanocyte cells, and comprise at least a portion of a melanocyte-specific promoter and/or a melanocyte-specific enhancer. Methods are described herein for measuring the activity of a melanocyte cell-specific TRE and thus for determining whether a given cell allows a melanocyte cell-specific TRE to function. Examples of a melanocyte-specific TRE for use in practicing the invention include but are not limited to a TRE derived from the 5' flanking region of a tyrosinase gene, a tyrosinase related protein-1 gene, a TRE derived from the 5'-flanking region of a tyrosinase related protein-2 gene, a TRE derived from the 5' flanking region of a MART-1 gene or a TRE derived from the 5'-flanking region of a gene which is aberrantly expressed in melanoma.

In another aspect, the invention provides adenoviral vectors comprising a metastatic colon cancer specific TRE derived from a PRL-3 gene operably linked to a gene essential for adenovirus replication or a transgene. As used herein, a "metastatic colon cancer specific TRE derived from a PRL-3 gene" or a "PRL-3 TRE" is a polynucleotide sequence, preferably a DNA sequence, which selectively increases transcription of an operably linked polynucleotide sequence in a host cell that allows a PRL-3 TRE to function, such as a cell (preferably a mammalian cell, more preferably a human cell, even more preferably a metastatic colon cancer cell). The metastatic colon cancer-specific TRE may comprise one or more regulatory sequences, e.g. enhancers, promoters, transcription factor binding sites and the like, which may be derived from the same or different genes. In one preferred aspect, the PRL-3 TRE comprises a PRL-3 promoter. The PRL-3 protein tyrosine phosphatase gene has been found to be specifically expressed at a high level in metastatic colon cancers (Saha et al. (2001) Science 294:1343). Originally identified as a member of a group of up-regulated genes in a metastatic colon cancer library, identified by the serial analysis of gene expression (SAGE), the PRL-3 gene was confirmed to be elevated in only the metastases, not the primary cancer or pre-malignant adenomas. Replication competent adenoviral vectors comprising PRL-3 transcriptional regulatory sequences are described in WO 20004/009790. Examples of relevant sequences are presented as a 0.6 kb and 1 kb sequence upstream of the translational start codon for the PRL-3 gene (identified as SEQ ID NO:1 and SEQ ID NO:2 in WO 20004/009790).

In another aspect, the invention provides adenoviral vectors comprising a liver cancer specific TREs derived from the CRG-L2 gene operably linked to a gene essential for adenovirus replication or a transgene. As used herein, a "liver cancer specific TREs derived from the CRG-L2 gene" or a "CRG-L2 TRE" is a polynucleotide sequence, preferably a DNA sequence, which selectively increases transcription of an operably linked polynucleotide sequence in a host cell that allows a CRG-L2 to function, such as a cell (preferably a mammalian cell, more preferably a human cell, even more preferably a hepatocellular carcinoma cell). The hepatocellular carcinoma specific TRE may comprise one or more regulatory sequences, e.g. enhancers, promoters, transcription factor binding sites and the like, which may be derived from the same or different genes. In one preferred aspect, the CRG-L2 TRE may be derived from the 0.8 kb sequence upstream of the translational start codon for the CRG-L2 gene, or from a 0.7 kb sequence contained within the 0.8 kb sequence (residues 119-803); or from an EcoRI to NcoI fragment derived from the 0.8 kb sequence, as described in US Provisional Application Serial No. 60/511,812, expressly incorporated by reference herein.

In another aspect, the invention provides adenoviral vectors comprising an EBV-specific transcriptional regulatory element (TRE) operably linked to a gene essential for adenovirus replication or a transgene. In one aspect, the EBV specific TRE is derived from a sequence upstream of the translational start codon for the LMP1, LMP2A or LMP2B genes, as further described in US Provisional Application Serial No. 60/423,203, expressly incorporated by reference herein. The EBV-specific TRE may comprise one or more regulatory sequences, e.g. enhancers, promoters, transcription factor binding sites and the like, which may be derived from the same or different genes.

In yet another aspect, the invention provides adenoviral vectors comprising a hypoxia-responsive element ("here") operably linked to a gene essential for adenovirus replication or a transgene. HRE is a transcriptional regulatory element comprising a binding site for the transcriptional complex HIF-1, or hypoxia inducible factor-1, which interacts with a in the regulatory regions of several genes, including vascular endothelial growth factor, and several genes encoding glycolytic enzymes, including enolase-1. Accordingly, in one embodiment, an adenovirus vector comprises an adenovirus gene, preferably an adenoviral gene essential for replication, under transcriptional control of a cell status-specific TRE such as a HRE, as further described in WO 00/15820, expressly incorporated by reference herein.

In yet another aspect, the invention provides adenoviral vectors comprising a "telomerase promoter" or "TERT promoter" operably linked to a gene essential for adenovirus replication or a transgene. The term "telomerase promoter" or "TERT promoter" as used herein refers to a native TERT promoter and functional fragments, mutations and derivatives thereof. The TERT promoter does not have to be the full-length or wild type promoter. One skilled in the art knows how to derive fragments from a TERT promoter and test them for the desired selectivity. A TERT promoter fragment of the present invention has promoter activity selective for tumor cells, i.e. drives tumor selective expression of an operatively linked coding sequence. In one embodiment, the TERT promoter of the invention is a mammalian TERT promoter. In another embodiment, the mammalian TERT promoter is a human TERT (hTERT) promoter. See, e.g., WO 98/14593 and WO 00/46355 for exemplary TERT promoters that find utility in the compositions and methods of the present invention.

In yet another aspect, the invention provides adenoviral vectors comprising an "E2F promoter" operably linked to a gene essential for adenovirus replication or a transgene. The term "E2F promoter" as used herein refers to a native E2F promoter and functional fragments, mutations and derivatives thereof. The E2F promoter does not have to be the full-length or wild type promoter. One skilled in the art knows how to derive fragments from an E2F promoter and test them for the desired selectivity. An E2F promoter fragment of the present invention has promoter activity selective for tumor cells, i.e. drives tumor selective expression of an operatively linked coding sequence. The term "tumor selective promoter activity" as used herein means that the promoter activity of a promoter fragment of the present invention in tumor cells is higher than in non-tumor cell types. An E2F-responsive promoter has at least one E2F binding site. In one embodiment, the E2F-responsive promoter is a mammalian E2F promoter. In another embodiment, it is a human E2F promoter. For example, the E2F promoter may be the human E2F-1 promoter. Further, the human E2F-1 promoter may be, for example, a E2F-1 promoter having the sequence as described in SEQ ID NO:43. A number of examples of E2F promoters are known in the art (e.g. Parr et al. Nature Medicine 1997:3(10) 1145-1149, WO 02/067861, US20010053352 and WO 98/13508). E2F responsive promoters typically share common features such as Sp I and/or ATT7 sites in proximity to their E2F site(s), which are frequently located near the transcription start site, and lack of a recognizable TATA box. E2F-responsive promoters include E2F promoters such as the E2F-1 promoter, dihydrofolate reductase (DHFR) promoter, DNA polymerase A (DPA) promoter, c-myc promoter and the B-myb promoter. The E2F-1 promoter contains four E2F sites that act as transcriptional repressor elements in serum-starved cells. In one embodiment, an E2F-responsive promoter has at least two E2F sites. In another embodiment, an E2F promoter is operatively linked to the adenovirus E1a region. In a further embodiment, an E2F promoter is operatively linked to the adenovirus E1b region. In yet a further embodiment, an E2F promoter is operatively linked to the adenovirus E4 region.

In one embodiment of the invention, the recombinant viral vectors of the present invention selectively replicate in and lyse Rb-pathway defective cells. In one embodiment, the E2F promoter of the invention is a mammalian E2F promoter. In another embodiment, the mammalian E2F promoter is a human E2F promoter, for example a human E2F promoter which comprises or consists essentially of SEQ ID NO:43. Embodiments of the invention include adenoviral vectors comprising an E2F promoter wherein the E2F promoter comprises a nucleotide sequence selected from the group consisting of: (a) the nucleotide sequence shown in SEQ ID NO:43; (b) a fragment of the nucleotide sequence shown in SEQ ID NO: 43, wherein the fragment has tumor selective promoter activity; (c) a nucleotide sequence having at least 90, 91, 92, 93, 94, 95, 96, 97, 98, 99% or more % identity over its entire length to the nucleotide sequence shown in SEQ ID NO:43 wherein the nucleotide sequence has tumor selective promoter activity; and (d) a nucleotide sequence having a full-length complement that hybridizes under stringent conditions to the sequence shown in SEQ ID NO:43, wherein the nucleotide sequence has tumor selective promoter activity. In another embodiment of the invention, the E2F promoter comprises nucleotides 7 to 270 of SEQ ID NO:43. In another embodiment of the invention, the E2F promoter comprises nucleotides 7 to 270 of SEQ ID NO:43, wherein nucleotide 75 of SEQ ID NO:43 is a T instead of a C.

In other embodiments, a E2F promoter according to the present invention has at least 80, 85, 87, 89, 90, 91, 92, 93, 94, 95, 96, 97, 98, 99% or more sequence identity to the nucleotide sequence shown in SEQ ID NO:43, when compared and aligned for maximum correspondence, as measured using one of the following sequence comparison algorithms or by visual inspection. In one embodiment, the given % sequence identity exists over a region of the sequences that is at least about 50 nucleotides in length. In another embodiment, the given % sequence identity exists over a region of at least about 100 nucleotides in length. In another embodiment, the given % sequence identity exists over a region of at least about 200 nucleotides in length. In another embodiment, the given % sequence identity exists over the entire length of the sequence.

The E2F-responsive promoter does not have to be the full-length or wild type promoter, but should have a tumor-selectivity of at least 3-fold, at least 5-fold, at least 10-fold, at least 20-fold, at least 30-fold, at least 50-fold, at least 100-fold or even at least 300-fold. Tumor-selectivity can be determined by a number of assays using known techniques, such as the techniques employed in WO 02/067861, Example 4, for example RT-PCR or a comparison of replication in selected cell types.

Without being bound by theory, the selectivity of E2F-responsive promoters (hereinafter sometimes referred to as E2F promoters) is reported to be based on the derepression of the E2F promoter/transactivator in Rb-pathway defective tumor cells. In quiescent cells, E2F binds to the tumor suppressor protein pRB in ternary complexes.

The protein urokinase plasminogen activator (uPA) and its cell surface receptor, urokinase plasminogen activator receptor (uPAR), are expressed in many of the most frequently-occurring neoplasms and appear to represent important proteins in cancer metastasis. Both proteins are implicated in breast, colon, prostate, liver, renal, lung and ovarian cancer. Sequence elements that regulate uPA and uPAR transcription have been extensively studied. (Riccio et al. (1985) Nucleic Acids Res. 13:2759-2771; Cannio et al. (1991) Nucleic Acids Res. 19:2303-2308; See also, WO 98/39464).

<u>Heterologous TRE(s) Operatively Linked To Essential Ad Coding Regions</u>

For manipulation of the early genes, the transcription start site of Ad5 E1A is at 498 and the ATG start site of the E1A coding segment is at 560 in the virus genome. This region can be used for insertion of a heterologous TRE. Figure 1 depicts the native genome organization of Ad5 and Figure 2 depicts the native Ad5 transcription units.

A restriction site may be introduced by employing polymerase chain reaction (PCR), where the primer that is employed may be limited to the Ad5 genome, or may involve a portion of the plasmid carrying the Ad5 genomic DNA. For example, where pBR322 is used, the primers may use the EcoRI site in the pBR322 backbone and the XbaI site at nt 1339 of Ad5. By carrying out the PCR in two steps, where overlapping primers at the center of the region introduce a nucleotide sequence change resulting in a unique restriction site, one can provide for insertion of a heterologous TRE at that site.

A similar strategy may also be used for insertion of a heterologous TRE element in operative linkage to E1B. The E1B promoter of Ad5 consists of a single high-affinity recognition site for Spl and a TATA box. This region extends from Ad5 nt 1636 to 1701. By insertion of a cell-specific heterologous TRE in this region, one can provide for cell-specific transcription of the E1B gene. By employing the left-hand region modified with the cell-specific response element regulating E1A, as the template for introducing a heterologous TRE to regulate E1B, the resulting adenovirus vector will be dependent upon the cell-specific transcription factors for expression of both E1A and E1B. In some embodiments, part or all of the 19-kDa region of E1B is deleted.

Similarly, a heterologous TRE can be inserted upstream of the E2 gene to make its expression cell-specific. The E2 early promoter, mapping in Ad5 from about 27050-27150, consists of a major and a minor transcription initiation site, the latter accounting for about 5% of the E2 transcripts, two non-canonical TATA boxes, two E2F transcription factor binding sites and an ATF transcription factor binding site (for a detailed review of the E2 promoter architecture see Swaminathan et al., Curr. Topics in Micro. and Immunol. (1995) 199(part 3):177-194.

The E2 late promoter overlaps with the coding sequences of a gene encoded by the counterstrand and is therefore not amenable for genetic manipulation. However, the E2 early promoter overlaps only for a few base pairs with sequences coding for a 33 kD protein on the counterstrand. Notably, the SpeI restriction site (Ad5 position 27082) is part of the stop codon for the above mentioned 33 kD protein and conveniently separates the major E2 early transcription initiation site and TATA-binding protein site from the upstream transcription factor binding sites E2F and ATF. Therefore, insertion of a heterologous TRE having SpeI ends into the SpeI site would disrupt the endogenous E2 early promoter of Ad5 and should allow cell-specific expression of E2 transcripts. For E4, one must use the right hand portion of the adenovirus genome. The E4 transcription start site is predominantly at about nt 35605 for Ad5, the TATA box at about nt 35631 and the first AUG/CUG of ORF I is at about nt 35532. Virtanen et al. (1984) J. Virol. 51: 822-831. Using any of the above strategies for the other genes, a heterologous TRE may be introduced upstream from the transcription start site. For the construction of full-length adenovirus with a heterologous TRE inserted in the E4 region, the co-transfection and homologous recombination may be performed in W162 cells (Weinberg et al. (1983) Proc. Natl. Acad. Sci. 80: 5383-5386) which provide E4 proteins in trans to complement defects in synthesis of these proteins.

An "E3 region" (used interchangeably with "E3 ") is a term well understood in the art and means the region of the adenoviral genome that encodes the E3 gene products. The E3 region has been described in various publications, including, for example, Wold et al. (1995) Curr. Topics Microbiol. Immunol. 199:237-274. A "portion" of the E3 region means less than the entire E3 region, and as such includes polynucleotide deletions as well as polynucleotides encoding one or more polypeptide products of the E3 region. See Figures 6, 7 and 8.

Adenoviral constructs containing an E3 region can be generated wherein homologous recombination between an E3-containing adenoviral plasmid, for example, BHGE3 (Microbix Biosystems Inc., Toronto) and a non-E3-containing adenoviral plasmid, is carried out.

Alternatively, an adenoviral vector comprising an E3 region can be introduced into cells, for example 293 cells, along with an adenoviral construct or an adenoviral plasmid construct, where they can undergo homologous recombination to yield adenovirus containing an E3 region. In this case, the E3-containing adenoviral vector and the adenoviral construct or plasmid construct contain complementary regions of adenovirus, for example, one contains the left-hand and the other contains the right-hand region, with sufficient sequence overlap as to allow homologous recombination.

Alternatively, an E3-containing adenoviral vector of the invention can be constructed using other conventional methods including standard recombinant methods (e.g., using restriction nucleases and/or PCR), chemical synthesis, or a combination of any of these. Further, deletions of portions of the E3 region can be created using standard techniques of molecular biology.

In some embodiments, the adenovirus death protein (ADP), encoded within the E3 region, is maintained in an adenovirus vector. The ADP gene, under control of the major late promoter (MLP), appears to code for a protein (ADP) that is important in expediting host cell lysis. Tollefson et al. (1996) J. Virol. 70(4):2296; Tollefson et al. (1992) J. Virol. 66(6):3633. Thus, adenoviral vectors containing the ADP gene may render the adenoviral vector more potent, making possible more effective treatment and/or a lower dosage requirement.

Accordingly in one embodiment, the invention provides adenovirus vectors in which an adenovirus gene is under transcriptional control of a first TRE and a polynucleotide sequence encoding an ADP under control of a second TRE element, and wherein preferably the adenovirus gene is essential for replication. The DNA sequence encoding ADP and the amino acid sequence of an ADP are publicly available. Briefly, an ADP coding sequence is obtained from Ad using techniques known in the art, such as PCR. Preferably, the Y leader (which is an important sequence for correct expression of late genes) is also obtained and ligated to the ADP coding sequence. The ADP coding sequence (with or without the Y leader) can then be introduced into the adenoviral genome, for example, in the E3 region (where the ADP coding sequence will be driven by the MLP). The ADP coding sequence could also be inserted in other locations of the adenovirus genome, such as the E4 region. Alternatively, the ADP coding sequence could be operatively linked to a different type of TRE, including, but not limited to, another viral TRE. In one embodiment, the vector of the invention had ADP operatively linked to its native TREs.

Internal Ribosome Entry Sites

To express two or more proteins from a single viral or non-viral vector, an internal ribosome entry site (IRES) sequence is commonly used to drive expression of the second, third, fourth gene, etc. The adenovirus vectors of the present invention may comprise one or more intergenic IRES elements, which link the translation of two or more coding sequences. Adenovirus vectors comprising an IRES linking two adenoviral coding regions are stable and may provide better specificity than vectors not containing an IRES. An adenovirus vector comprising an intergenic IRES rather than a second TRE may provide for additional space in the vector for inclusion of additional gene(s), e.g., a therapeutic gene. Examples of adenoviral vectors comprising an IRES are described in US 6,692,736, expressly incorporated by reference herein. In one aspect of the invention, the viral vectors comprise at least one IRES within a multicistronic transcript, wherein production of the multicistronic transcript is regulated by a heterologous, cell type-, tissue type- or cell status-specific TRE. For adenovirus vectors comprising a second adenoviral coding region under control of an IRES, it is preferred that the endogenous promoter of the coding region under translational control of the IRES be deleted so that the endogenous promoter does not interfere with transcription of the second coding region. It is preferred that the second coding region be in frame with the IRES if the IRES contains an initiation codon. If an initiation codon, such as ATG, is present in the IRES, it is preferred that the initiation codon of the second coding sequence is removed and that the IRES and the second coding sequence are in frame. Alternatively, if the IRES does not contain an initiation codon or if the initiation codon is removed from the IRES, the initiation codon of the second coding region is used. In one embodiment, the adenovirus vectors comprise the adenovirus essential genes, E1A and E1B genes, under the transcriptional control of a heterologous TRE, and an IRES introduced between E1A and E1B. Thus, both E1A and E1B are under common transcriptional control, and translation of E1B coding region is obtained by virtue of the presence of the IRES. In one embodiment, E1A has its endogenous promoter deleted. In another embodiment, E1A has an endogenous enhancer deleted and in yet an additional embodiment, E1A has its endogenous promoter deleted and E1A enhancer deleted. In another embodiment, E1B has its endogenous promoter deleted. In yet further embodiments, E1B has a deletion of part or all of the 19-kDa region of E1B.

Insertion of an IRES into a vector is accomplished by methods and techniques that are known in the art and described herein supra, including but not limited to, restriction enzyme digestion, ligation, and PCR. A DNA copy of an IRES can be obtained by chemical synthesis, or by making a cDNA copy of, for example, a picornavirus IRES. See, for example, Duke et al. (1995) J.Virol. 66(3):1602-9) for a description of the EMCV IRES and Huez et al. (1998), Mol. Cell. Biol. 18(11):6178-90) for a description of the VEGF IRES. The internal translation initiation sequence is inserted into a vector genome at a site such that it lies upstream of a 5'-distal coding region in a multicistronic mRNA. For example, in one embodiment of an adenovirus vector in which production of a bicistronic E1A-E1B mRNA is under the control of a heterologous TRE, the E1B promoters is deleted or inactivated, and an IRES sequence is placed between E1A and E1B. In other embodiments, part or all of the 19-kDa region of E1B is deleted. IRES sequences of cardioviruses and certain aphthoviruses contain an AUG codon at the 3' end of the IRES that serves as both a ribosome entry site and as a translation initiation site. Accordingly, this type of IRES is introduced into a vector so as to replace the translation initiation codon of the protein whose translation it regulates. However, in an IRES of the entero/rhinovirus class, the AUG at the 3' end of the IRES is used for ribosome entry only, and translation is initiated at the next downstream AUG codon. Accordingly, if an entero/rhinovirus IRES is used in a vector for translational regulation of a downstream coding region, the AUG (or other translation initiation codon) of the downstream gene is retained in the vector construct.

In another embodiments, an IRES is operatively linked to a transgene inserted downstream of an adenovirus CDS that is not immediately upstream of a polyA signal. For example, the IRES transgene is not inserted after the furthest downstream Ad CDS in a leader sequence. For example, the IRES transgene cassette is operatively linked to a one of the following Ad CDSs: 52/55K, pV, penton, pVI, or hexon.

Multiple coding sequences can be linked with IRESs. In one embodiment, an Ad CDS is operatively linked by a first IRES to a first transgene and said first transgene is operatively linked by a second IRES to a second transgene. In one embodiment, the first and second transgenes encode for the same or different proteins. In the case of the same proteins, it is advantageous that the coding sequence of one of the transgenes be "recoded". In other words, use different codons to code for the same amino acids. This is done to reduce the amount of homology between the two transgenes at the DNA level, thus reducing or eliminating homologous recombination between the two transgenes. Other embodiments include two adenovirus CDSs operatively linked by an IRES. This may accompany a deletion of adenoviral DNA sequences. For example, two adenoviral CDSs that are located in the same leader region and are adjacent to each other may be operatively linked by an IRES and a portion or all of the intervening Ad sequence may be deleted as long as the deletion does not disrupt other sequences or elements necessary for viral vector production, being especially mindful of the complementary strand. The deleted portion may be 1-5 nucleotides (nts), 6-15 nts, 16-25 nts, 26-35 nts, 36-40 nts, or greater than 40 nts. In one embodiment, a first transgene CDS is operatively linked by a first IRES to an Ad CDS and a second IRES operatively links a second transgene to said Ad CDS. Other embodiments include various combinations of Ad CDSs, and both Ad CDSs and transgene CDSs operatively linked with IRES and/or self-processing peptide sequences.

When using multiple IRES sequences in a vector, it is preferable that the two IRES sequences have minimal or no homology at the DNA level to reduce the frequency of homologous recombination.

Self-Processing Cleavage Sites Or Sequences

In another aspect of the invention a "self-processing cleavage site" (e.g. 2A-like sequence) is utilized to express two polypeptides from one mRNA. A "self-processing cleavage site" or "self-processing cleavage sequence" is defined as a DNA or amino acid sequence, wherein upon translation, rapid intramolecular (cis) cleavage of a polypeptide comprising the self-processing cleavage site occurs to result in expression of discrete mature protein or polypeptide products. Such a "self-processing cleavage site", may also be referred to as a post-translational or co-translational processing cleavage site, exemplified herein by a 2A site, sequence or domain. As used herein, a "self-processing peptide" is defined herein as the peptide expression product of the DNA sequence that encodes a self-processing cleavage site or sequence, which upon translation, mediates rapid intramolecular (cis) cleavage of a protein or polypeptide comprising the self-processing cleavage site to yield discrete mature protein or polypeptide products. It has been reported that a 2A site, sequence or domain demonstrates a translational effect by modifying the activity of the ribosome to promote hydrolysis of an ester linkage, thereby releasing the polypeptide from the translational complex in a manner that allows the synthesis of a discrete downstream translation product to proceed (Donnelly et al. J Gen Virol. 2001 May;82(Pt 5):1013-25). Alternatively, it has also been reported that a 2A site, sequence or domain demonstrates "auto-proteolysis" or "cleavage" by cleaving its own C-terminus in cis to produce primary cleavage products (Furler; Palmenberg, Ann. Rev. Microbiol. 44:603-623 (1990)).

Although the mechanism is not part of the invention, the activity of a 2A-like sequence may involve ribosomal skipping between codons which prevents formation of peptide bonds (de Felipe et al., Human Gene Therapy 11: 1921-1931 (2000); Donnelly et al., J. Gen. Virol. 82:1013-1025 (2001); Donnelly et al. J Gen Virol. 2001 May;82(Pt 5): 1027-41); ; Szymczak et al. Nature Biotechnology 22:589-594 and 760 (2004), although it has been considered that the domain acts more like an autolytic enzyme (Ryan et al., Virol. 173:35-45 (1989)). Studies in which the Foot and Mouth Disease Virus (FMDV) 2A coding region was cloned into expression vectors and transfected into target cells showed FMDV 2A cleavage of artificial reporter polyproteins in wheat-germ lysate and transgenic tobacco plants (Halpin et al., US 5,846,767; 1998 and Halpin et al., Plant J 17:453-459, 1999); Hs 683 human glioma cell line (de Felipe et al., Gene Therapy 6:198-208, 1999); hereinafter referred to as "de Felipe II"); rabbit reticulocyte lysate and human HTK-143 cells (Ryan et al., EMBO J. 13:928-933 (1994)); and insect cells (Roosien et al., J. Gen. Virol. 71:1703-1711, 1990). The FMDV 2A-mediated cleavage of a heterologous polyprotein has been shown for IL-12 (p40/p35 heterodimer; Chaplin et al., J. Interferon Cytokine Res. 19:235-241, 1999). The reference demonstrates that in transfected COS-7 cells, FMDV 2A mediated the cleavage of a p40-2A-p35 polyprotein into biologically functional subunits p40 and p35 having activities associated with IL-12.

’ The FMDV 2A sequence has been incorporated into retroviral vectors, alone or combined with different IRES sequences to construct bicistronic, tricistronic and tetracistronic vectors. The efficiency of 2A-mediated gene expression in animals was demonstrated by Furler et al. (Gene Ther. 2001 Jun;B(11):864-73) using recombinant adeno-associated viral (AAV) vectors encoding a-synuclein and EGFP or Cu/Zn superoxide dismutase (SOD-1) and EGFP linked via the FMDV 2A sequence. EGFP and a-synuclein were expressed at substantially higher levels from vectors which included a 2A sequence relative to corresponding IRES-based vectors, while SOD-1 was expressed at comparable or slightly higher levels. Furler also demonstrated that the 2A sequence results in bicistronic gene expression *in* vivo after injection of 2A-containing AAV vectors into rat substantia nigra. Syzmczak et al. (Nature Biotechnology 22:589-594&760 (2004)) describe a retroviral vector with four coding regions linked with three 2A sequences.

For the present invention, the DNA sequence encoding a self-processing cleavage site is exemplified by viral sequences derived from a picornavirus, including but not limited to an entero-, rhino-, cardio-, aphtho- or Foot-and-Mouth Disease Virus (FMDV). In a preferred embodiment, the self-processing cleavage site coding sequence is derived from a FMDV. Self-processing cleavage sites include but are not limited to 2A and 2A-like sites, sequences or domains (Donnelly et al., J. Gen. Virol. 82:1027-1041 (2001)).

FMDV 2A is a polyprotein region, which functions in the FMDV genome to direct a single cleavage at its own C-terminus, thus functioning in cis. The FMDV 2A domain is typically reported to be about nineteen amino acids in length ((LLNFDLLKLAGDVESNPGP (SEQ ID NO:1); TLNFDLLKLAGDVESNPGP (SEQ ID NO:2); Ryan et al., J. Gen. Virol. 72:2727-2732 (1991)), however oligopeptides of as few as fourteen amino acid residues ((LLKLAGDVESNPGP (SEQ ID NO:3)) have also been shown to mediate cleavage at the 2A C-terminus in a fashion similar to its role in the native FMDV polyprotein processing. Variations of the 2A sequence have been studied for their ability to mediate efficient processing of polyproteins (Donnelly et al., J. Gen. Virol. 82:1027-1041 (2001)). Homologues and variant 2A sequences are included within the scope of the invention and include, but are not limited to, the sequences presented as SEQ ID NOs: 1-32.

In one embodiment, the FMDV 2A sequence included in a vector according to the invention encodes amino acid residues comprising the sequence presented as SEQ ID NO:1. Alternatively, a vector according to the invention may encode amino acid residues for other 2A-like regions as discussed in Donnelly et al., J. Gen. Virol. 82:1027-1041 (2001) and including, but not limited to, a 2A-like domain from picornavirus, insect virus, Type C rotavirus, trypanosome repeated sequences or the bacterium, Thermatoga maritima.

The invention contemplates the use of nucleic acid sequence variants that encode a self-processing cleavage site, such as a 2A or 2A-like polypeptide, and nucleic acid coding sequences that have a different codon for one or more of the amino acids relative to that of the parent (native) nucleotide. Such variants are specifically contemplated and encompassed by the present invention. Sequence variants of self-processing cleavage peptides and polypeptides are included within the scope of the invention as well.

In accordance with the present invention, also encompassed are sequence variants which encode self-processing cleavage polypeptides and polypeptides themselves that have 80, 85, 88, 89, 90, 91, 92, 93, 94, 95, 96, 97, 98, 99% or more sequence identity to the native sequence.

In one embodiment of the invention, a self-processing cleavage sequence (e.g. 2A or 2A-like sequence) is operably linked to an adenovirus protein coding region and a transgene. The adenovirus protein CDS may be upstream of the self-processing cleavage site, with the transgene being downstream. Alternatively, the transgene CDS may be upstream of the self-processing cleavage site, with the adenovirus protein CDS being downstream.

Multiple CDSs may be linked with self-processing cleavage sites. In one embodiment, an Ad CDS is operatively linked by a self processing cleavage site to a first transgene and said first transgene is operatively linked by a self-processing cleavage site to a second transgene. In one embodiment, the first and second transgenes encodes for the same or different proteins. In the case of the same proteins, it is advantageous that the coding sequence of one of the transgenes be "recoded". In other words, use different codons to code for the same amino acids. This is done to reduce the amount of homology between the two transgenes at the DNA level, thus reducing or eliminating homologous recombination between the two transgenes. Other embodiments include two Ad CDSs operatively linked by a self-processing cleavage site. This may accompany a deletion of adenoviral sequence. For example, two adenoviral CDSs that are located in the same leader region and are adjacent to each other may be operatively linked by a self-processing cleavage site and a portion or all of the intervening Ad sequence may be deleted as long the deletion does not disrupt other sequences or elements necessary for viral vector production, being especially mindful of the complementary strand. The deleted portion may be 1-5 nucleotides (nts), 6-15 nts, 16-25 nts, 26-35 nts, 36-40 nts, or greater than 40 nts.

In one embodiment, a first transgene CDS is operatively linked by a first self-processing cleavage site to an Ad CDS and the Ad CDS is operatively linked by a second self-processing cleavage site to a second transgene. Other embodiments include various combinations of Ad CDSs, and both Ad CDSs and transgene CDSs operatively linked with IRES and/or self-processing peptide sequences.

When using more than one self-processing peptide sequences in a vector, it is preferable that the self-processing peptide sequences have minimal or no homology at the DNA level to reduce the frequency of homologous recombination. For example, the self-processing peptide sequences may be derived from different sources wherein the multiple coding sequences for self-processing peptide sequences have minimal or no homology. In another embodiment, a coding sequence for a self-processing peptide sequence is recoded. In other words, different codons are used to code for the same amino acids of the self-processing peptide sequence. This is done to reduce the amount of homology between the more than one self-processing peptide coding sequences, thus reducing or eliminating homologous recombination between the two transgenes.

A self-processing peptide sequence is operatively linked to a CDS when the sequence encoding the self-processing peptide sequence is inserted in frame with the upstream and downstream CDS.

<u>Removal of Self Processing Peptide Sequences.</u>

One concern associated with the use of self-processing peptides, such as a 2A or 2A-like sequence is that the C terminus of the expressed polypeptide contains amino acids derived from the self-processing peptide, i.e. 2A-derived amino acid residues. These amino acid residues are "foreign" to the host and may elicit an immune response when the recombinant protein is expressed *in vivo* or delivered *in vivo* following *in vitro* or *ex vivo* expression. In addition, if not removed, self-processing peptide-derived amino acid residues may interfere with protein function and/or alter protein conformation, resulting in a less than optimal expression level and/or reduced biological activity of the recombinant protein. In other words, depending on the application it may be advantageous that the resulting proteins not contain all of the 2A-derived amino acid residues.

The invention includes vectors, engineered such that an additional proteolytic cleavage site is provided between a first protein or polypeptide coding sequence (the first or 5' ORF) and the self processing cleavage site as a means for removal of self processing cleavage site derived amino acid residues that are present in the expressed protein product.

Examples of additional proteolytic cleavage sites are furin cleavage sites with the consensus sequence RXK(R)R (SEQ ID NO:33), which can be cleaved by endogenous subtilisin-like proteases, such as furin and other serine proteases. As shown in Example 6 of USSN 10/831304, the inventors have demonstrated that self processing 2A amino acid residues at the C terminus of a first expressed protein can be efficiently removed by introducing a furin cleavage site RAKR (SEQ ID NO:33) between the first polypeptide and a self processing 2A sequence. In addition, use of a plasmid containing a 2A sequence and a furin cleavage site adjacent to the 2A sequence resulting in a higher level of protein expression than achieved using a plasmid containing the 2A sequence alone. This improvement provides a further advantage in that when 2A amino acid residues are removed from the C-terminus of the protein, longer 2A- or 2A like sequences or other self-processing sequences can be used, as described in USSN 10/831304.

As detailed herein, the 2A peptide sequence provides a "cleavage" site that facilitates the generation of both chains of an immunoglobulin or other protein during the translation process. In one exemplary embodiment, the C-terminus of the first protein, for example the immunoglobulin heavy chain, contains approximately 13 amino acid residues which are derived from the 2A sequence itself. The number of residual amino acids is dependent upon the 2A sequence used. As set forth above and shown in the Examples, when a furin cleavage site sequence, e.g., RAKR, is inserted between the first protein and the 2A sequence, the 2A residues are removed from the C-terminus of the first protein. However, mass spectrum data indicates that the C-terminus of the first protein expressed from the RAKR-2A construct contains two additional amino acid residues, RA, derived from the furin cleavage site RAKR.

In one embodiment, the invention provides a method for removal of these residual amino acids and a composition for expression of the same. A number of novel constructs have been designed that provide for removal of these additional amino acids from the C-terminus of the protein. Furin cleavage occurs at the C-terminus of the cleavage site, which has the consensus sequence RXR(K)R, where X is any amino acid. In one aspect, the invention provides a means for removal of the newly exposed basic amino acid residues R or K from the C-terminus of the protein by use of an enzyme selected from a group of enzymes called carboxypeptidases (CPs), which include, but not limited to, carboxypeptidase D, E and H (CPD, CPE, CPH). Since CPs are able to remove basic amino acid residues at the C-terminus of a protein, all amino acid resides derived from a furin cleavage site which contain exclusively basic amino acids R or K, such as RKKR, RKRR, RRRR, etc, can be removed by a CP. A series of immunoglobulin expression constructs that contain a 2A sequence and a furin cleavage site and which have basic amino acid residues at the C terminus have been constructed to evaluate efficiency of cleavage and residue removal. An exemplary construct design is the following: H chain - furin (e.g, RKKR, RKRR, RRKR or RRRR)- 2A - L chain or L chain - furin (e.g, RKKR, RKRR, RRKR or RRRR) - 2A - H chain.

As will be apparent to those of skill in the art, there is a basic amino acid residue (K) at the C terminus of the immunoglobulin heavy (H) chain (rendering it subject to cleavage with carboxypeptidase), while the immunoglobulin light (L) chain, terminates with a non-basic amino acid C. In one preferred embodiment of the invention, an antibody expression construct comprising a furin site and a 2A sequence is provided wherein the immunoglobulin L chain is 5' to the immunoglobulin H chain such that following translation, the additional furin amino acid residues are cleaved with carboxypeptidase.

It is often advantageous to produce therapeutic proteins, polypeptides, fragments or analogues thereof with fully human characteristics. These reagents avoid the undesired immune responses induced by proteins, polypeptides, fragments or analogues thereof originating from different species. To address possible host immune responses to amino acid residues derived from self-processing peptides, the coding sequence for a proteolytic cleavage site may be inserted (using standard methodology known in the art) between the coding sequence for a first protein and the coding sequence for a self-processing peptide so as to remove the self-processing peptide sequence from the expressed protein or polypeptide.

Any additional proteolytic cleavage site known in the art that can be expressed using recombinant DNA technology may be employed in practicing the invention. Exemplary additional proteolytic cleavage sites which can be inserted between a polypeptide or protein coding sequence and a self processing cleavage sequence include, but are not limited to a:

a). Furin consensus sequence or site: RXK(R)R (SEQ ID. NO:33);
b). Factor Xa cleavage sequence or site: IE(D)GR (SEQ ID NO:34);
c). Signal peptidase I cleavage sequence or site: e.g., LAGFATVAQA (SEQ ID. NO:35); and
d). Thrombin cleavage sequence or site: LVPRGS (SEQ ID NO:36).
e). Adenoviral consensus protease sequence or site (M,L,I)XGG/X (SEQ ID NO:37) and (M,L,I)XGX/G (SEQ ID NO:38) see Webster et al. J Gen Virol 70:3215-3223 (1989); Weber, Curr Top Microbiol Immunol 1991:227-235 (1995) and Balakirev et al. J of Virol 76:6323-6331 (2002)

In the case of an adenovirus protease sequence or site, the invention is not meant to be limited to the consensus sequences provided above. The invention contemplates the use of any adenoviral protease. In one embodiment, the adenoviral protease is from the same adenovirus serotype as from which the adenoviral vector genome is derived.

Transgenes

The vectors of the invention may include one or more transgenes. In this way, various genetic capabilities may be introduced into target cells. In one embodiment, the transgene encodes a selectable marker. In another embodiment, the transgene encodes a cytotoxic protein. These vectors encoding a cytotoxic protein may be used to eliminate certain cells in either an investigational setting or to achieve a therapeutic effect. For example, in certain instances, it may be desirable to enhance the degree of therapeutic efficacy by enhancing the rate of cytotoxic activity. This could be accomplished by coupling the cell-specific replicative cytotoxic activity with expression of, one or more metabolic enzymes such as HSV-tk, nitroreductase, cytochrome P450 or cytosine deaminase (CD) which render cells capable of metabolizing 5-fluorocytosine (5-FC) to the chemotherapeutic agent 5-fluorouracil (5-FU), carboxylesterase (CA), deoxycytidine kinase (dCK), purine nucleoside phosphorylase (PNP), carboxypeptidase G2 (CPG2; Niculescu-Duvaz et al. J Med Chem. 2004 May 6;47(10):2651-2658), thymidine phosphorylase (TP), thymidine kinase (TK) or xanthine-guanine phosphoribosyl transferase (XGPRT). This type of transgene may also be used to confer a bystander effect.

Additional transgenes that may be introduced into a vector of the invention include a factor capable of initiating apoptosis, antisense or ribozymes, which among other capabilities may be directed to mRNAs encoding proteins essential for proliferation of the cells or a pathogen, such as structural proteins, transcription factors, polymerases, etc., viral or other pathogenic proteins, where the pathogen proliferates intracellularly, cytotoxic proteins, e.g., the chains of diphtheria, ricin, abrin, etc., genes that encode an engineered cytoplasmic variant of a nuclease (e.g., RNase A) or protease (e.g., trypsin, papain, proteinase K, carboxypeptidase, etc.), chemokines, such as MCP3 alpha or MIP-1, pore-forming proteins derived from viruses, bacteria, or mammalian cells, fusgenic genes, chemotherapy sensitizing genes and radiation sensitizing genes. Other genes of interest include cytokines, antigens, transmembrane proteins, and the like, such as IL-1, IL-2, IL-4, IL-5, IL-6, IL-10, IL-12, IL-18 or flt3, GM-CSF, G-CSF, M-CSF, IFN-$\alpha$, $\beta$, -$\gamma$, TNF-$\alpha$ -$\beta$, TGF-$\alpha$ -$\beta$, NGF, MDA-7 (Melanoma differentiation associated gene-7, mda-7/interleukin-24), and the like. Further examples include, proapoptotic genes such as Fas, Bax, Caspase, TRAIL, Fas ligands, nitric oxide synthase (NOS) and the like; fusion genes which can lead to cell fusion or facilitate cell fusion such as V22, VSV and the like; tumor suppressor gene such as p53, RB, p16, p17, W9 and the like; genes associated with the cell cycle and genes which encode anti-angiogenic proteins such as endostatin, angiostatin and the like.

Other opportunities for specific genetic modification include T cells, such as tumor infiltrating lymphocytes (TILs), where the TELs may be modified to enhance expansion, enhance cytotoxicity, reduce response to proliferation inhibitors, enhance expression of lymphokines, etc. One may also wish to enhance target cell vulnerability by providing for expression of specific surface membrane proteins, e.g., B7, SV40 T antigen mutants, etc.

Although any gene or coding sequence of relevance can be used in the practice of the invention, certain genes, or fragments thereof, are particularly suitable. For example, coding regions encoding immunogenic polypeptides, toxins, immunotoxins and cytokines are useful in the practice of the invention. These coding regions include those hereinabove and additional coding regions include those that encode the following: proteins that stimulate interactions with immune cells such as B7, CD28, MHC class I, MHC class II, TAPs, tumor-associated antigens such as immunogenic sequences from MART-1, gp 100(pmel-17), tyrosinase, tyrosinase-related protein 1, tyrosinase-related protein 2, melanocyte-stimulating hormone receptor, MAGE1, MAGE2, MAGE3, MAGE12, BAGE, GAGE, NY-ESO-1, -catenin, MIJM-1, CDK-4, caspase 8, KIA 0205, HLA-A2R1701, a-fetoprotein, telomerase catalytic protein, G-250, *MUC*-1, carcinoembryonic protein, p53, Her2/neu, triosephosphate isomerase, CDC-27, LDLR-FUT, telomerase reverse transcriptase, PSMA, cDNAs of antibodies that block inhibitory signals (CTLA4 blockade), chemokines (MIP1, MIP3, CCR7 ligand, and calreticulin), anti-angiogenic genes include, but are not limited to, genes that encode METH-1, METH -2, TrpRS fragments, proliferin-related protein, prolactin fragment, PEDF, vasostatin, various fragments of extracellular matrix proteins and growth factor/cytokine inhibitors, various fragments of extracellular matrix proteins which include, but are not limited to, angiostatin, endostatin, kininostatin, fibrinogen-E fragment, thrombospondin, tumstatin, canstatin, restin, growth factor/ cytokine inhibitors which include, but are not limited to, VEGF/VEGFR antagonist, sFlt-1, sFlk, sNRPI, murine Flt3 ligand (mFLT3L), angiopoietin/tie antagonist, sTie-2, chemokines (IP-10, PF-4, Gro-beta, IFN-gamma (Mig), IFN, FGF/FGFR antagonist (sFGFR), Ephriri/Eph antagonist (sEphB4 and sephrinB2), PDGF, TGF and IGF-1. Genes suitable for use in the practice of the invention can encode enzymes (such as, for example, urease, renin, thrombin, metalloproteases, nitric oxide synthase, superoxide dismutase, catalase and others known to those of skill in the art), enzyme inhibitors (such as, for example, alpha1-antitrypsin, antithrombin III, cellular or viral protease inhibitors, plasminogen activator inhibitor-1, tissue inhibitor of metalloproteases, etc.), the cystic fibrosis transmembrane conductance regulator (CFTR) protein, insulin, dystrophin, or a Major Histocompatibility Complex (MHC) antigen of class I or II. Also useful are genes encoding polypeptides that can inodulate/regulate expression of corresponding genes, polypeptides capable of inhibiting a bacterial, parasitic or viral infection or its development (for example, antigenic polypeptides, antigenic epitopes, and transdominant protein variants inhibiting the action of a native protein by competition), apoptosis inducers or inhibitors (for example, Bax, Bc12, Bc1X and others known to those of skill in the art), cytostatic agents (e.g., p21, p16, Rb, etc.), apolipoproteins (e.g., ApoAI, ApoAIV, ApoE, etc.), oxygen radical scavengers, polypeptides having an anti-tumor effect, antibodies, toxins, immunotoxins, markers (e.g., beta-galactosidase, luciferase, etc.) or any other genes of interest that are recognized in the art as being useful for treatment or prevention of a clinical condition. Further transgenes include those coding for a polypeptide which inhibits cellular division or signal transduction, a tumor suppressor protein (such as, for example, p53, Rb, p73), a polypeptide which activates the host immune system, a tumor-associated antigen (e.g., *MUC*-1, BRCA-1, an HPV early or late antigen such as E6, E7, L1, L2, etc), optionally in combination with a cytokine.

TRAIL has been shown to induce apoptosis in a wide variety of transformed cell lines (Jeremias I et al, Eur J Immunol 1998, 28:143-152 and Walczak H et al., Nat Med 1999, 5:157-163). The physiological role of TRAIL appears to involve both the innate and adaptive immune responses (NK and T-cells regulation of virally infected and transformed cells). Although TRAIL expression is widespread, normal cells appear to be resistant to TRAIL-induced apoptosis allegedly due to the expression of intracellular proteins (bc1-2, IAPs, FLIP, etc. which mitigate the apoptosis signaling response.

Although the precise molecular mechanism of the anti-tumor specificity of TRAIL remains unclear at present, TRIAL was chosen as a transgene of interest due to a number of features of TRAIL that have been described in the literature and suggest that late expression of TRAIL by adenovirus should enhance cell killing. E1A has been descried as able to enhance killing by TRAIL (E1A and TRAIL: Routes et al., J Immunol. 2000 Oct 15;165(8):4522-7); the E1B 19K and 55K proteins reportedly reduce the effects of TRAIL (E1B 19K and TRAIL: Routes et al., J Immunol. 2000 Oct 15;165(8):4522-7 and anti-apoptotic activity: Tollefson et al., J Virol. 2001 Oct;75(19):8875-87); E3: 10.4K and 14.5k (RID) remove FAS and TRAIL receptors on the cell surface by inducing their degradation in lysosomes (RID, FAS and TRAIL: Tollefson et al., Nature. 1998 Apr 16;392(6677):726-30; Shisler et al., J Virol. 1997 Nov;71(11):8299-306; Lichtenstein et al., J Virol. 2002 Nov;76(22):11329-42; Tollefson et al., J Virol. 2001; E3: 14.7K inhibits apoptosis by TNF, Fas and TRAIL and binds to caspase 8 (E3 14.7K and TRAIL: Chen et al., J Biol. Chem. 1998 Mar 6;273(10):5815-20 and Tollefson et al., J Virol. 2001) and E3: 6.7K prevents apoptosis by TRAIL and maintains ER Ca homeostasis (E3 6.7K and TRAIL/ER Ca2++ homeostasis: Benedict et al., J Biol. Chem. 2001 Feb 2;276(5):3270-8 and E3-6.7K protein of adenovirus/localization in the endoplasmic reticulum: Wilson-Rawls et al., Virology. 1993 Jul;195(1):6-15).

The invention further comprises combinations of two or more transgenes with synergistic, complementary and/or non-overlapping toxicities and methods of action. In summary, the present invention provides methods for inserting transgene coding regions in specific regions of the viral vector genome. The methods take advantage of known viral transcription elements and the mechanisms for expression of Ad genes, reduce the size of the DNA sequence for transgene expression that is inserted into the Ad genome, since no additional promoter is necessary and the regulation signals encompass a smaller size DNA fragment, provide flexibility in temporal regulation of the transgene (e.g., early versus late stage of infection; early versus intermediate stage of infection), and provide techniques to regulate the amount of transgene expressed. For example, a higher amount of transgene can be expressed by inserting the transgene into a transcript that is expressed normally at high levels and/or by operatively linking a high efficiency splice acceptor site to the transgene coding region. Expression levels are also affected by how close the regulating signals are to their consensus sequences; changes can be made to tailor expression as desired.

In designing the adenoviral vectors of the invention the biological activity of the transgene is considered, e.g. in some cases it is advantageous that the transgene be inserted in the vector such that the transgene is only or mostly expressed at the late stages of infection (after viral DNA replication). For example, the transgene may be inserted, in L3, as further described herein. For some transgenes, it may be preferred to express the transgene early in the viral life cycle. In such cases, the transgene may be inserted in any of the early regions (for example, E3) or into the upstream L1 region.

Therapeutic Methods

An effective amount of a vector of the invention is administered to a mammal (e.g., a human) as a composition in a pharmaceutically acceptable excipient, which may include one or more of the following: a saline solution, a suitable buffer, preservatives, stabilizers, and the like. A vector of the invention may be administered in conjunction with suitable agents such as antiemetics. An effective amount is an amount sufficient to effect beneficial or desired results, including clinical efficacy. An effective amount can be administered in one or more administrations or doses. For purposes of this invention, an effective amount of vector is an amount that is sufficient to palliate, ameliorate, stabilize, reverse, slow or delay the progression of the disease state or alleviate one or more symptoms of the disease. The amount to be given will be determined by the condition of the individual, the extent of disease, the route of administration, how many doses will be administered, and the desired objective.

Delivery of vectors of the invention is often accomplished by either site-specific injection or intravenous injection. Site-specific injections of vector may include, for example, injection into tumors, as well as intraperitoneal, intrapleural, intrathecal, intra-arterial, subcutaneous injection, intradermal injection, intramuscular injection or topical application. These methods are easily accommodated in treatments using vector alone or a combination of vector and chemotherapeutic agent. The invention also contemplates the use of the vector to infect cells from a subject *ex vivo.* For example, cells are isolated from a mammal. The isolated cells may contain a mixture of tumor cells and non-tumor cells. The cells are infected with a virus that is replication competent and the virus specifically replicates in the tumor cells. Therefore, the tumor cells are eliminated and if desired the remaining non-tumor cells may be administered back to the same mammal or if desired to a different mammal.

If used as a packaged adenovirus, adenovirus vectors may be administered in an appropriate physiologically acceptable carrier at a dose of about $10^4$ to about $10^{14}$. If administered as a polynucleotide construct (i.e., not packaged as a virus) about 0.01 μg to about 1000 μg an adenoviral vector can be administered. The exact dosage to be administered is dependent upon a variety of factors including the age, weight, and sex of the patient, and the size and severity of the tumor being treated. The adenoviral vector(s) may be administered one or more times, depending upon the intended use and the immune response potential of the host, and may also be administered as multiple, simultaneous injections. If an immune response is undesirable, the immune response may be diminished by employing a variety of immunosuppressants, or by employing a technique such as an immunoadsorption procedure (e.g., immunoapheresis) that removes adenovirus antibody from the blood, so as to permit repetitive administration, without a strong immune response. If packaged as another viral form, such as HSV, an amount to be administered is based on standard knowledge about that particular virus (which is readily obtainable from, for example, published literature) and can be determined empirically.

In one embodiment the host organism is a human patient. For human patients, if a therapeutic coding region is included in the vector, the therapeutic coding region may be of human origin although genes of closely related species that exhibit high homology and biologically identical or equivalent function in humans may be used if the gene does not produce an adverse immune reaction in the recipient A therapeutically active amount of a nucleic acid sequence or a therapeutic gene is an amount effective at dosages and for a period of time necessary to achieve the desired result. This amount may vary according to various factors including but not limited to sex, age, weight of a subject, and the like.

Disclosed herein are methods for the administration of combinations of a cancer-specific vector of the invention and a second anti-neoplastic therapy, which may include radiation, administration of an anti-neoplastic or chemotherapeutic agent, etc., to an individual with neoplasia, as detailed in U.S. Application 20030068307. The cancer-specific vector and chemotherapeutic agent may be administered simultaneously or sequentially, with various time intervals for sequential administration. In some embodiments, an effective amount of vector and an effective amount of at least one antineoplastic or chemotherpeutic agent are combined with a suitable excipient and/or buffer solutions and administered simultaneously from the same solution by any of the methods listed herein or those known in the art. This may be applicable when the chemotherapeutic agent does not compromise the viability and/or activity of the vector itself.

Where more than one chemotherapeutic agent is administered, the agents may be administered together in the same composition; sequentially in any order; or, alternatively, administered simultaneously in different compositions. If the agents are administered sequentially, administration may further comprise a time delay. Sequential administration may be in any order, and accordingly encompasses the administration of an effective amount of a vector first, followed by the administration of an effective amount of the chemotherapeutic agent. The interval between administration of the cancer-specific vector and chemotherapeutic agent may be in terms of at least (or, alternatively, less than) minutes, hours, or days. Sequential administration also encompasses administration of a chosen antineoplastic agent followed by the administration of the vector. The interval between administration may be in terms of at least (or, alternatively, less than) minutes, hours, or days.

Administration of the above-described methods may also include repeat doses or courses of a cancer-specific vector and chemotherapeutic agent depending, inter alia, upon the individual's response and the characteristics of the individual's disease. Repeat doses may be undertaken immediately following the first course of treatment (i.e., within one day), or after an interval of days, weeks or months to achieve and/or maintain suppression of tumor growth. A particular course of treatment according to the above-described methods, for example, combined cancer-specific vector and chemotherapy, may later be followed by a course of combined radiation and cancer-specific vector therapy.

Anti-neoplastic (chemotherapeutic) agents include those from each of the major classes of chemotherapeutics, including but not limited to: alkylating agents, alkaloids, antimetabolites, anti-tumor antibiotics, nitrosoureas, hormonal agonists/antagonists and analogs, immunomodulators, photosensitizers, enzymes and others. In some embodiments, the antineoplastic is an alkaloid, an antimetabolite, an antibiotic or an alkylating agent. In certain embodiments the antineoplastic agents include, for example, thiotepa, interferon alpha-2a, and the M-VAC combination (methotrexate-vinblastine, doxorubicin, cyclophosphamide). Preferred antineoplastic agents include, for example, 5-fluorouracil, cisplatin, 5-azacytidine, and gemcitabine. Particularly preferred embodiments include, but are not limited to, 5-fluorouracil, gemcitabine, doxorubicin, miroxantrone, mitomycin, dacarbazine, carmustine, vinblastine, lomustine, tamoxifen, docetaxel, paclitaxel or cisplatin. The specific choice of chemotherapeutic agent(s) is dependent upon, inter alia, the characteristics of the disease to be treated. These characteristics include, but are not limited to, location of the tumor, stage of the disease and the individual's response to previous treatments, if any.

In addition to the use of single antineoplastic agents in combination with a particular cancer-specific vector, the invention also includes the use of more than one agent in conjunction with the cancer-specific vector. These combinations of antineoplastics when used to treat neoplasia are often referred to as combination chemotherapy and are often part of a combined modality treatment which may also include surgery and/or radiation, depending on the characteristics of an individual's cancer. It is contemplated that the combined cancer-specific vector/chemotherapy of the present invention can also be used as part of a combined modality treatment program.

There are a variety of delivery methods for the administration of antineoplastic agents, which are well known in the art, including oral and parenteral methods. There are a number of drawbacks to oral administration for a large number of antineoplastic agents, including low bioavailability, irritation of the digestive tract and the necessity of remembering to administer complicated combinations of drugs. The majority of parenteral administration of antineoplastic agents is intravenously, as intramuscular and subcutaneous injection often leads to irritation or damage to the tissue. Regional variations of parenteral injections include intra-arterial, intravesical, intra-tumor, intrathecal, intrapleural, intraperitoneal and intracavity injections.

Delivery methods for chemotherapeutic agents include intravenous, intraparenteral and intraperitoneal methods as well as oral administration. Intravenous methods also include delivery through a vein of the extremities as well as including more site specific delivery, such as an intravenous drip into the portal vein. Other intraparenteral methods of delivery include direct injections of an antineoplastic solution, for example, subcutaneously, intracavity or intra-tumor.

Assessment of the efficacy of a particular treatment regimen may be determined by any of the techniques known in the art, including diagnostic methods such as imaging techniques, analysis of serum tumor markers, biopsy, the presence, absence or amelioration of tumor associated symptoms. It will be understood that a given treatment regime may be modified, as appropriate, to maximize efficacy.

In a further aspect, a pharmaceutical composition comprising the recombinant viral vectors and/or particles of the invention and a pharmaceutically acceptable carrier is disclosed. Such compositions, which can comprise an effective amount of cancer-specific vector and/or viral particles of the invention in a pharmaceutically acceptable carrier, are suitable for local or systemic administration to individuals in unit dosage forms, sterile parenteral solutions or suspensions, sterile non-parenteral solutions or oral solutions or suspensions, oil in water or water in oil emulsions and the like. Formulations for parenteral and non-parenteral drug delivery are known in the art. Compositions also include lyophilized and/or reconstituted forms of the cancer-specific vector or particles of the invention. Acceptable pharmaceutical carriers are, for example, saline solution, protamine sulfate (Elltins-Sinn, Inc., Cherry Hill, N.J.), water, aqueous buffers, such as phosphate buffers and Tris buffers, or Polybrene (Sigma Chemical, St. Louis MO) and phosphate-buffered saline and sucrose. The selection of a suitable pharmaceutical carrier is deemed to be apparent to those skilled in the art from the teachings contained herein. These solutions are sterile and generally free of particulate matter other than the desired cancer-specific vector. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. Excipients that enhance uptake of the cancer-specific vector by cells may be included.

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g., amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. For example, due to codon redundancy, changes can be made in the underlying DNA sequence without affecting the protein sequence. Moreover, due to biological functional equivalency considerations, changes can be made in protein structure without affecting the biological action in kind or amount. All such modifications are intended to be included within the scope of the preferred embodiments.

## EXAMPLES

It will be appreciated that the methods and compositions of the instant invention can be incorporated in the form of a variety of embodiments, only a few of which are disclosed herein. It will be apparent to the artisan that other embodiments exist and do not depart from the spirit of the invention. Thus, the described embodiments are illustrative and should not be construed as restrictive.

The following examples are offered by way of illustration and not by way of limitation. In the following examples, the sequence of the branch point plus splice acceptor sequence is: TACTTAT GACTCGTACTATTGTTATTCATCC AG↑TG (SEQ ID NO:39) The underlined sequence is the branch point sequence and the arrow indicates the location of the splice site according to splicing rules. Since the rules governing the consensus sequence are not invariant, other similar sequences that conform to the rules may be used. Alternatively, a branch point plus splice acceptor site that exists in another Ad serotype can be used. The following examples pertain to exemplary adenoviral vectors derived from human Ad5 serotype. Similar constructs can be made from other adenoviruses.

**Example 1:** Constructing Ad vectors and propagating Ad vector virions coding a transgene(s).

The following examples describe inserting at least one transgene in vectors that contain either a portion of the adenovirus vector genome or the whole adenoviral vector genome and the inserted transgene. In the case of the vectors coding for the whole adenoviral vector genome and the inserted transgene, the vector is transfected into an adenoviral producer cell line and virus is propagated using standard techniques. Prior to transfecting, the vector may be digested with a restriction enzyme, which does not cut within the viral vector genome, but cuts the vector (e.g. plasmid) backbone.

Alternatively, the transgene is inserted into a vector that contains a portion of the viral genome. In this case, the portion of the vector containing part of the viral genome is cloned into a vector that contains the rest of the viral vector genome. Therefore creating a vector that encodes the complete viral vector. Next, the complete viral vector genome is transfected into a producer cell line and virus is propagated. In another method, the vector containing the transgene and portion of the viral genome is transfected into a producer cell line along with the appropriate fragment(s) of the viral vector genome, that as a result of homologous recombination, will form a complete viral vector genome, which will replicate and propagate in the producer cell lines.

The following references provide more details of viral vector construction and viral propagation: Ghosh-Choudhury et al. Gene. 1986;50(1-3):161-71; Toietta et al. Mol Ther. 2002 Feb;5(2):204-10. This example describes inserting a transgene(s) in the various locations in the L2 region of Ad.

**Example 2:** Cloning Ad vectors with modifications and for cloning further modifications in the L2 region.

For recombination using the bacterial system, it is an advantage to have a full length Ad genomic plasmid (of any specific modifications) that also contains at least one unique restriction site in the region that will be altered. In some examples below, the targeted region is the L2 region. However a unique site does not exist. Therefore, a plasmid was created that possesses these features by the following method. The BamHI to AscI region of Ad corresponding to bases 15672 to 21562 of wild type Ad was inserted into the cloning plasmid pNEB193 (New England Biolabs) and this plasmid is called CP1563. Site directed mutagenesis was performed to incorporate a unique SwaI site into position 16530 (base corresponding to Ad wild type) and this plasmid was designated CP 1564. A plasmid was made that contains the Ad5 sequences from 13259 to 21562, the BamHI to PmeI fragment, inserted into the cloning plasmid pMCS5 and is called cp1165. cp1165 was altered so that it no longer contains the AscI site that was present in the cloning vector and becomes CP1166. The SwaI-containing Ad fragment from CP1564 was removed by digestion with AscI and BamHI and inserted into AscI and BamHI-cut CP1566 and this plasmid is called CP1567. This is built back into any full length Ad genomic plasmid to generate a genomic plasmid that contains a unique SwaI site in the L2 region that can be used for cutting for recombination into the L2 region.

**Example 3:** Transgene insertions in the L2 region between the pVII CDS and pV CDS

The following cloning steps are performed in a vector (e.g. plasmid) containing either the whole adenoviral vector genome (i.e. ITR to ITR) or a vector that contains the L2 region of the adenoviral genome or a relevant portion thereof. A transgene CDS, i.e., an open reading frame (ORF) or cDNA, was inserted downstream of the pVII CDS and upstream of the pV CDS. There is a splice acceptor sequence between these two genes already and it is used for the expression of pV; the associated branch point sequence has not been exactly mapped. The transgene CDS or cDNA is inserted upstream of the endogenous pV splice site and an additional branch point and splice acceptor site is inserted upstream of the transgene. These additional splicing sequences are used for transgene expression. The sequence for an exemplary splice acceptor for the L2 region differs from the one used for the L3 region. The new splice acceptor site was chosen according to the existing splice site for pVII: TACTTATAGTGAA AACG TTCCTGCTCTCACAG by conserving the strong bases to obtain TACTTATAGTAATCTAATTCCTGCTCTCTCAG (SEQ ID NO:42). Alternatively, an additional branch point and splice acceptor site operatively linked to the transgene CDS is inserted upstream of the native pV branch point and splice acceptor site.

**Example 4:** Transgene insertions in the L2 region between the penton CDS and pVII CDS

A transgene CDS is inserted downstream of the penton CDS and upstream of the pVII CDS. The presumed branch point and splice acceptor site for pVII is present in the coding region for penton. Therefore, the inserted transgene uses the endogenous splicing signals present in the penton CDS and an additional branch point and splice acceptor site is placed after the transgene CDS and upstream of (and operatively linked to) the pVII CDS to synthesize the mRNA for pVII gene.

**Example 5:** Transgene insertions in the L2 region between the Mu CDS and L4 polyA signal

A transgene is inserted downstream of the Mu (aka pX) CDS and upstream of the L2 polyadenylation signal sequence. A branch point, splice acceptor site and transgene CDS are inserted downstream of the Mu CDS and upstream of the L2 polyadenylation signal sequence. The inserted branch point and splice acceptor site are operatively linked to transgene CDS

**Example 6:** Transgene insertions in the L2 region downstream of the Mu CDS and L4 polyA signal

An IRES operatively linked to a transgene is inserted downstream of the Mu CDS and upstream of the L2 polyadenylation signal sequence.

**Example 7:** Cloning splicing elements and hIL-24 into the L3 region between 23K CDS and L3 polyA

pCR2.1 Topo (Invitrogen, Carlsbad, California) is cut with EcoRI and religated to remove one EcoRI site. This plasmid is then cut with BspHI, filled in with Klenow, and religated to remove the BspHI site. This plasmid is then cut with ApaI and RsrII, filled in with Klenow, and religated to remove excess sequences between these sites. The portion containing the ampicillin resistance gene is retained. This plasmid is designated CP1601. pBHGE3 (Microbix Biosystems, Toronto, Ontario, Canada) is cut with HindIII and XhoI and the HindIII/XhoI fragment containing nucleotides 18318 to 24795 of the wild-type Ad5 genome is ligated into the HindIII- and XhoI-cut fragment (vector backbone) of CP1601. The resulting plasmid is designated CP1603.

To insert the transgene human-IL24 (hIL-24, also known as MDA-7) downstream of the 23K CDS and upstream of the L3 polyadenylation signal sequence and to allow expression by a splicing mechanism, the following steps are performed: Using PCR SOEing (Horton et al. Biotechniques. 1990 May;8(5):528-35) a fragment containing the branch point and splice acceptor site and the hIL-24 CDS is amplified. (The IL-24 CDS is obtained from Invivogen plasmid reference "porf-hil24"). The branch point and splice acceptor site are coded for in the PCR primers. The same branch point and splice acceptor site sequence outlined above is used. The PCR product is TA cloned into pCR2.1 Topo (Invitrogen, Carlsbad, California) creating CP1602. Then the HindIII/XhoI region from CP1602 which contains the Ad and hIL-24 sequences, is ligated into HindIII/XhoI digested CP1601 resulting in plasmid CP1606 Rev (SEQ ID NO:48). The KpnI/SfiI fragment of CP1606 Rev is ligated into KpnI/SfiI-cut CP1524 to create CP1610. CP1610 is the shuttle plasmid for "shuttling" the modified L3 region in to a complete adenoviral vector genome. With this plasmid, any transgene can be inserted here after digestion of the transgene fragment (often generated by PCR with the appropriate ends) and CP 1610 with BspHI and NheI. Generation of the Ad vector or virus is accomplished through homologous recombination of CP1606 Rev (SEQ ID NO:48) with an adenoviral vector genome backbone plasmid. The adenoviral vector genome backbone contains all of the other necessary adenoviral genes and regions of DNA that provide homology with the region to be inserted, thereby generating a vector that contains the complete sequence of the adenoviral vector genome including the modifications to the L3 region. The recombination can be performed in a strain of E. coli to generate a plasmid that contains the full length of Ad genome with the desired changes. This vector is then used to create adenoviral viral particles by transfection into mammalian cells and amplification of virus.

Other transgenes can be inserted into the L3 region just upstream of the L2 polyadenylation site using this same strategy. Alternatively, transgenes can be amplified with the restriction sites BspHI and NheI on their 5' and 3' ends, respectively, and inserted into CP1606 Rev (SEQ ID NO:48) and generation of the corresponding Ad virus can be done using the same strategy.

**Example 8:** Cloning mIL21 into the L3 region between the hexon CDS and 23K CDS

Insertion of a murine interleukin-21 transgene downstream of the L3 hexon CDS and upstream of the 23K CDS utilizing splicing for expression is performed as follows. Using methods routinely employed by those of skill in the art, a splice acceptor site and a mIL21 cDNA (Invivogen reference: porf-mIL21) are inserted downstream of the hexon CDS and upstream of the 23K CDS. The branch point and splice acceptor site are coded for in PCR primers. The same branch point and splice acceptor site sequence outlined above is used. In this case, the branch point plus splice acceptor site is added to the 3' end of the transgene because the normal 23K splicing signals are present in the ORF for hexon and, as such, upstream of where the transgene can be inserted. This strategy utilizes this endogenous (native viral) splicing sequence for the transgene and adds an additional branch point plus splice acceptor to be utilized for expression of the 23K CDS. PCR products containing fragments of the L3 region of the Ad5 genome (modified with restriction enzyme sites for cloning and/or the splice processing signals) and the hIL24 cDNA are digested with the appropriate restriction endnonucleases. The fragments are then ligated into a CP1601 equivalent vector to create CP1623. Then the BamHI/KpnI fragment of CP1623 is ligated into BamHI/KpnI -cut CP1524 (Example 7) to create CP1631; a shuttle in which the engineered cassette (the 3' splice acceptor site preceded by the hIL24 cDNA) is inserted after the stop codon for Hexon and the start codon for 23K. CP1623 is the shuttle plasmid that can be used for other transgenes to be expressed in this manner. A complete adenoviral vector containing the inserted mIL21 transgene is attained through homologous recombination of CP1631 with the desired full length vector containing the desired adenoviral genome backbone. The resulting plasmid, CP1631, is then used to create the new Ad virus, OV1153, which will express the transgene.

**Example 9:** Cloning an IRES and hIL-24 into the L3 region between 23K CDS and the L3 polyA signal

A transgene such as hIL-24 (Invivogen reference: porf-hil24) can be inserted into the L3 region just upstream of the L3 polyadenylation site and downstream of all the L3 coding regions using an IRES for expression. The IRES and transgene are expected to be included in and translated from all the L3 mRNAs. Using PCR SOEing (Horton et al. Biotechniques. 1990 May;8(5): 528-35), a PCR product is generated so that an IRES, such as that obtained from FMDV or ECMV, is operatively linked to a hIL-24 CDS and is inserted downstream of the 23K CDS and upstream of the L3 polyadenylation signal sequence. The PCR product is TA cloned into pCR2.1 creating CP1604. The HindlllhoI region from CP1604 which contains the Ad and hIL-24 sequences, is ligated into Hindm/XhoI digested CP1601-NotI resulting in CP1607. CP1601-NotI was made by cutting CP1601 with NotI, filling in the ends with Klenow, and then religated the plasmid so that the NotI site is destroyed. The BamHI/SfiII fragment of CP1607 is ligated into BamHI/SfiI -cut CP1603 to create the plasmid CP1609. CP1607 is the shuttle plasmid that can be used for other transgenes to be expressed in this manner. A complete adenoviral vector containing the inserted mFLT3L transgene is attained through homologous recombination of CP1609 with the desired full length adenoviral genome backbone plasmid. The resulting plasmid is then used to generate an Ad virus by transfection into mammalian cells.

Example 10. Construction of OV 1165 control virus

OV 1165 is a control virus comprising the E2F-1 promoter operatively linked to E1A. The vector carries the packaging signal in the native location and carries a polyadenylation signal upstream of the E2F-1 promoter to inhibit transcriptional read-through from the LITR. These sequences contained in OV1165 were derived from the plasmid pFLAr21pAE2Ff.

The full-length recombinant adenoviral genome (pFLAr21pAE2Ff) was generated as follows. First, a full-length plasmid, pFLAd5 was generated by combining the SmaI-linearized pAd5LtRtSmaI shuttle plasmid containing I-SceI restriction sites introduced by PCR with genomic DNA of Ad5 in E.coli resulting in pFLAd5 which contained the Ad5 genome bordered by I-SceI sites. Next, pFLAd5 was digested with SmaI and the fragments containing the left and right terminal fragments of Ad5 were gel purified and self-ligated to generate pAd5LtRtSmaI. The plasmid pAd5LtRtSmaI containing the left (1006 bp) and right terminal (582 bp) restriction fragments of Ad5 was digested with SmaI and combined with genomic viral DNA of Ar21pAE2f in E-coli to generate the full length plasmid pFLAr21pAE2Ff.

A plasmid called CP 1585 containing a full-length adenoviral-derived genome of the type described above (pFLAr21pAE2Ff) was generated as follows:

> A 4648 bp SmaI fragment was isolated from the pFLAr21pAE2fF plasmid (pFLAr21 Sma shuttle) which contains the E2F promoter upstream of E1A. The BamHI site was removed from pFLAr21 Sma shuttle by digestion with Bam HI, filled in with klenow polymerase to destroy the site and religated. A 4646 bp plasmid product called pE2f LtRT shuttle was isolated. Plasmid CP 1585 (38998 bp) was generated by bacterial homologous recombination in BJ 5183 cells using CV802 viral DNA containing the wild-type Ad 5 genome and linearized pE2f LtRt Shuttle. The final product CP 1585 has the full-length recombinant adenoviral genome with the E2F-1 promoter operatively linked to E1A. CP 1585 also has a unique BamHI site (map site 21796) near the L4 region which can be used to facilitate gene insertion in the late region.

The OV 1165 virus was generated by lipofectamine transfection of A549 cells with the 36,246 base pair I-SceI fragment derived from CP 1585. OV 1165 was grown on A549 cells and scaled up to 5 roller bottles.

The virus was purified by CsCl centrifugation and formulated in ARCA buffer with a yield of 4.97 ml and a particle titer of 2.33 x $10^{12}$ vp/ml (2.3 x $10^{12}$ vp per roller bottle).

Example 11. Generation of L3 vectors

Shuttle plasmids for use in generating vectors expressing transgenes from the Ad5 L3 region, utilizing alternative splicing mechanisms were constructed as follows:

Plasmids containing recombinant, full-length adenoviral genomes were generated using the BJ5183 bacterial recombination system.

Plasmids containing recombinant, full-length adenoviral genomes were generated using the BJ5183 bacterial recombination system. In this application of the system, modifications were made to the CP1606 and CP 1524 shuttle plasmids containing smaller fragments of the adenoviral genome.

L3 vectors were also generated by the BJ5183 bacterial recombination system. A large, linearized vector containing a full-length adenoviral genome in a plasmid backbone was co-transformed with a smaller fragment of DNA derived from a similar adenoviral genome into BJ5183 bacteria, a strain with functional recB, recC, sbcB, and sbcC genes. The smaller fragment contains significant stretches of nucleotide sequence homologous to the larger vector flanking the coding sequence for TRAIL. Co-transformation of the large, linearized vector and the smaller fragment of DNA into BJ5183 cells was used to promote homologous recombination of the smaller fragment into the larger, linearized vector. The resulting progeny plasmid contains a full-length, adenoviral-derived genome incorporating the TRAIL coding sequence (cDNA; SEQ ID NO: 46).

A fragment was excised from the final shuttle plasmid (CP1581, derived from CP1524) and co-transformed with a linearized plasmid containing the full-length, recombinant adenoviral genome (pFLAr21pAE2Ff). Progeny plasmids are derivatives of pFLAr21pAE2Ff . Prior to recombination, introduction of deliberate alterations to the Ad sequence contained in CP 1524 were performed in a smaller base vectors, CP1606 Rev (SEQ ID NO:48). CP1524 (SEQ ID NO: 47) and CP1606 Rev are further described below.

CP1524 (SEQ ID NO:47) is a base shuttle vector containing nucleotides 18318 - 24795 of the wild-type Ad5 genome in a pcDNA3.1+ (Invitrogen) derived plasmid backbone. The stretch of the Ad5 genome from nucleotides 18318 - 24795 contains a portion of the wt Ad5 L3 region.

CP1524 was constructed by using molecular cloning methods routinely employed by those of skill in the art. Initially, pcDNA3.1+ was digested with the restriction endonucleases BstZ17 I and Xba I (New England Biolabs). The resulting digested vector was treated with DNA Polymerase I (Klenow) (New England Biolabs) to fill in the single-stranded stretch of DNA created by Xba I digestion. The resulting "blunt" ends were ligated together to create a modified pcDNA3.1+ derived vector, CP1523, with excess sequence removed. The Ad5 sequence of CP1524 was derived from pBHGE3 (Microbix), a commercially available plasmid containing the wt Ad5 genome, with the exception of a large deletion in the E1 region. The region representing the wt Ad5 sequence from nucleotides 18318 - 24795 was excised from pBHGE3 with the restriction endonucleases HindIII and XhoI (New England Biolabs) and ligated into the similarly cut CP1523 to yield the progeny plasmid, CP1524.

Example 12. Cloning splicing elements into the L3 region between 23K CDS and L3 polyA (CP1606 Rev Construction)

CP 1606 Rev contains a modified segment of the wt Ad5 L3 region representing nucleotides 22181 to 23008 of the wt Ad5 genome in a truncated pCR2.1 Topo plasmid backbone (Invitrogen). Modifications to the region include a 3' splice acceptor site (hereinafter referred to as 3' SAS) preceding the hIL24 cDNA. The hIL24 cDNA is flanked by BspHI (compatible with NcoI) and NheI sites for replacement with a different cDNA sequence, e.g., TRAIL.

CP1606 Rev was constructed by standard molecular cloning methods. Initially, a PCR fragment was generated encompassing nucleotides 22181 to 23008 of the wt Ad5 genome, modified to include a 3' SAS and the hIL24 cDNA between the stop codon for the wt Ad5 23K gene (hereinafter referred to as 23K) and the L3 polyA. The fragment was constructed by PCR splice overlap extension.

Briefly, 3 precursor PCR fragments were generated. Fragment PCRp 1618.95.1/2 contains the portion of the wt Ad5 genome from nucleotides 22181 to 22354 amplified from pBHGE3 with the primers 1618.95.1 (SEQ ID NO:52) and 1618.95.2 (SEQ ID NO: 56). PCRp 1618.95.1/2 incorporates a 3' SAS (SEQ ID NO: 45) at its downstream end.

Fragment PCRp 1618.97.1 (SEQ ID NO:54)/1618.97.2 (SEQ ID NO:55) contains the hIL24 cDNA, amplified from pORF9-hIL24 (Invivogen) with the primers 1618.97.1 and 1618.97.2. PCRp 1618.97.1/2 incorporates a 3' SAS and a portion of the wt Ad5 L3 region at its upstream and downstream ends, respectively.

Nucleotides 22355 to 23008 of the wt Ad5 genome were amplified with primers 1618.95.5 (SEQ ID NO:56) and 1618.95.6 (SEQ ID NO:53). Fragments PCRp 1618.95.1/2 and PCRp 1618.97.1/2 have overlapping ends. A mixture of the two fragments was placed in a PCR reaction with flanking primers 1618.95.1 (SEQ ID NO:52) and 1618.97.2 (SEQ ID NO:55). The resulting product, PCRp 1618.95.1/1618.97.2, joined the portion of the wt Ad5 genome from nucleotides 22181 to 22354 with a 3' SAS and the hIL24 cDNA.

Likewise, fragments PCRp 1618.97.1/2 and PCRp 1618.95.5/6 have overlapping ends. A mixture of the two fragments was placed in a PCR reaction with flanking primers 1618.97.1 and 1618.95.6. The resulting product, PCRp 1618.97.1/1618.95.6, has a 3' SAS and the hIL24 cDNA with the portion of the wt Ad5 genome from positions 22355 to 23008.

Therefore, PCRp 1618.95.1/1618.97.2 and PCRp 1618.97.1/1618.95.6 overlap at the engineered 3' SAS and hIL24 cDNA. A mixture of the two fragments was placed in a PCR reaction with flanking primers 1618.95.1 (SEQ ID NO:52) and 1618.95.6 (SEQ ID NO:53). The resulting product, PCRp 1618.95.1/6, represents nucleotides 22181 to 23008 of the original wt Ad5 genome incorporating an engineered 3' SAS and the hIL24 cDNA between the original positions 22354 and 22355 (so that the introduced features are between the stop codon for 23K and the L3 polyA site). PCRp 1618.95.1/6 was placed in the vector pCR2.1 Topo by Topo TA cloning resulting in plasmid CP1602.

To generate a base shuttle for the removal and insertion of transgenes into the modified 23K region described above, a plasmid backbone was constructed by modification of pCR2.1 Topo. Initially, pCR 2.1 Topo was digested with the restriction endonuclease EcoRI (New England Biolabs) and religated to remove one of the EcoRI sites and the excess nucleotide sequence between the original two sites, yielding pCR2.1 TopoEcoRI. In order to facilitate the downstream cloning of transgenes, the BspHI restriction site was removed from pCR2.1 TopoEcoRI by digestion with the restriction endonuclease BspHI (New England Biolabs), treatment with DNA Polymerase I (Klenow), followed by ligation of the treated vector to yield pCR2.1 Topo EcoRI-BspHI. To ease downstream cloning efforts, excess sequence was removed from pCR.21 Topo EcoRI-BspHI by digestion with the restriction endonucleases ApaI and RsrII (New England Biolabs), treatment with DNA Polymerase I (Klenow), followed by ligation of the treated vector to yield CP 1601.

The final base shuttle, CP1606 Rev (SEQ ID NO:48), was constructed by excising the HindIII to XhoI fragment of CP1602 and placing it into a similarly cut CP1601 vector.

To generate a larger shuttle plasmid suitable for recombination with the linearized vector pFLAr21pAE2Ff, the Kpn I to SFiI (New England Biolabs) fragment was excised from CP1606 Rev and placed into a similarly cut CP1524 vector, resulting in plasmid CP1610.

<u>Example 13. Generation of an Oncolytic Vector with a Splice Acceptor (SA) Site and TRAIL in the L3 region between 23K CDS and L3 polyA (OV 1160)</u>

In order to generate a recombinant derivative of pFLAr21pAE2Ff containing the engineered 3' SAS and TRAIL cDNA (Invivogen; reference pORF-TRAIL), two sub-clonings were carried out. Plasmid C1580 was constructed by amplification of the TRAIL cDNA (SEQ ID NO: 46) from pORF-hTTRAIL by PCR. This fragment was subjected to restriction digest using NcoI-NheI sites and ligated into CP1606 Rev following digestion with BspHI-NheI. The BspHI site is compatible with the NcoI site. The Cap1581 plasmid was constructed from the KpnI-SfiI fragment of CP1580 which contains TRAIL 3' to the splice site of the 23K protein ligated into the Cap1524 shuttle plasmid for use in recombination. Plasmid CP1582 was obtained following recombination of the XhoI-HindIII fragment of CP1581 with the full length pFLar21paE2f. Recombinant derivatives of pFLAR21 pAE2Ff containing the engineered 3' SAS (SEQ ID NO:45) and TRAIL cDNA (SEQ ID NO:46) were isolated and designated CP1582.

OV 1160 virus (SEQ ID NO:51) was generated by restriction digest of CP 1582 with I-SceI enzyme liberating the full length adenoviral sequence (containing the new splice acceptor site and the TRAIL).

<u>Example 14. Generation of an Oncolytic Vector with an IRES and TRAIL in the L3 region between 23K CDS and L3 polyA (OV 1164)</u>

OV1164 (SEQ ID NO:50) has an IRES after the stop codon of the Hexon coding sequence. The plasmids involved in the generation of OV1164 are CP 1590, CP1591 and CP1592 (full length).

The full length CP 1592 plasmid was generated following three sub-clonings. The assembly of the HexonFmdvTrail-containing plasmid was performed by PCR Soeing. In order to generate a fragment that would have an overlap sufficient to generate the next plasmid by recombination, lkb of sequence from Ad5 was included on both sides of that sequence.PCR SOEing was performed as follows:

(1) the Ad5 sequence from pBHG3 was amplified using 1706.83.1(SEQ ID NO:57)/1706.83.2 (SEQ ID NO:58); (2) HexFmdvTRAIL fragment from pmcsHexFmdvTRAIL was amplified using oligos 1706.95.1(SEQ ID NO:59) /1618.116.3 (SEQ ID NO:60): and (3) the fragments were assembled using the 1706.83.1 and 1618.116.3 cloned new fragment in PCRstopo.

(2) CP 1590 is a PCR2.1 topo vector containing the PCR assembled fragment (ad5HexFmdvTRAILAd5). CP1591 was generated by recombination of the HpaI-XhoI frag (Ad5HexFmdvTRAILAd5) of CP1590 with CP1524 linearized with BamHI. The final full length CP1592 plasmid was generated by recombination of the XhoI-HindIII fragment of CP1591 with the pFLar21paE2f linearized with SgfI.

TRAIL was cloned in pmcs HexFMDV by amplification of TRAIL from pORF-hTRAIL with oligos 1619.144.1 (SEQ ID NO:61)/1619.144.3 (SEQ ID NO:62) using Expand polymerase. The amplified fragment was digested with BSTZ171 and ligated into CP1557 (pmcsHexFmdv bamHI-EcoRI) to generate pmcsHexFmdvTrail.

Virus was generated by restriction digest of CP 1592 with I-SceI enzyme liberating the full length adenoviral sequence (containing the IRES and TRAIL).

Example 15. Virus Production, Trail Expression and Biological Activity *In Vitro*.

A549 cells (1-2 X 10e7 cells) were transferred from plates into a roller bottle (Falcon 35-3069 pleated surface roller bottle with vented cap) on day one in RPMI 1640 medium with 2mM L-Glutamine and 10% FBS. On day 4, the cells were infected for 3-6 hours with 5-10 viral particles/cell. The cells were harvested at 72-96 hours post infection and stored at - 80°C until purification by CsCl centrifugation. A titer of 2.55 e12, 2.05 e12 and 2.11 e12 was obtained for OV 1160, lot 1; OV 1160, lot 2; and OV 1164, respectively (as determined by HPLC). The titer was determine by OD at 260nm (viruses were diluted 1:10 and 1:20 in TE containing 0.1% of SDS, incubated 20 min at 56° C and the absorbance was read at 260 and 280nm on a spectrophotometer.

The virus yield was performed at 50ppc for each viruses on A549, SW780 and Hela S3 cells. Infection was carried out for 4hrs, then the cells were washed twice with PBS before adding 3ml of fresh complete media. The cells were harvested at Day 3 (72 hours following transduction), freeze-thawed 3 times and analyzed. Table 1 shows the virus yield at Day 3.

The virus yield was determined at Day 3 (72 hours following transduction) and titrated using a Hexon Assay on 293 cells on Collagen-coated 12-well plates. Table 1 shows the virus yield at Day 3.

**Table 1.**

| | **A549** | | | | | **SW780** | | |
|---|---|---|---|---|---|---|---|---|
| | **total PFU** | **PFU/Cell** | **Fold Diff** | | | **total PFL** | **PFU/Cell** | **Fold Diff** |
| OV1165 | 3.50E+10 | 7.00E+04 | 1.00E+00 | | OV1165 | 4.55E+10 | 9.10E+04 | 1.00E+00 |
| OV1160#2 | 4.87E+09 | 9.74E+03 | 7.19E+00 | | OV1160# 2 | 2.97E+09 | 5.94E+03 | 1.53E+01 |
| OV1160#1 | 4.27E+09 | 8.54E+03 | 8.20E+00 | | OV1160# 1 | 2.98E+09 | 5.96E+03 | 1.53E+01 |
| OV1164 | 1.23E+09 | 2.46E+03 | 2.85E+01 | | OV1164 | n/a | | |
| 802 | 5.83E+10 | 1.17E+05 | 6.00E-01 | | 802 | 8.90E+10 | 1.78E+05 | 5.11E-01 |
| | | | | | | | | |
| | **HelaS3** | | | | | | | |
| | **total PFU** | **PFU/Cel** | **Fold Diff** | | | | | |
| OV1165 | 3.20E+10 | 6.40E+04 | 1.00E+00 | | | | | |
| OV1160#2 | 2.45E+09 | 4.90E+03 | 1.31E+01 | | | | | |
| OV1160#1 | 2.24E+09 | 4.48E+03 | 1.43E+01 | | | | | |
| OV1164 | 5.50E+08 | 1.10E+03 | 5.82E+01 | | | | | |
| 802 | 7.30E+10 | 1.46E+05 | 4.38E-01 | | | | | |

The Hexon assay is a biological assay to measure, by immunostaining, the production of the adenovirus hexon protein in a given culture of cells. Briefly, cells are infected with serial dilutions of CVL or purified virus and incubated at 37°C. 48 hours post-infection, the cells are fixed with methanol then probed with an anti-Hexon primary antibody followed by a horseradish peroxidase (hereinafter referred to as HRP) conjugated secondary antibody. Hexon-producing cells are then visualized by exposing the fixed culture to a diaminobenzidine (hereinafter referred to as DAB) substrate which is converted, by the HRP of the secondary antibody, into a dark precipitate readily visualized under a microscope. The spots where the dark precipitate has formed are then visually scored. The infectious units per milliliter (hereinafter referred to as IU/mL) were calculated based on the spots scored per microscope field, the total number of fields in the culture dish, the volume of serially diluted virus used for infection, and the dilution factor at which the spots were scored:

**IU/mL = [(scored spots per field)*(total fields)]/[(volume diluted virus)*(dilution factor)]**

In this case, 293 cells were infected with serially diluted OV1160 (lot 1), OV1160 (lot 2), or OV1164. An anti-hexon primary antibody (Chemicon; MAB8043), a secondary antibody (Amersham Biosciences; NA931V) and DAB substrate (Pierce; 1856090) was used to carry out the assay. PBS (Mediatech; 1-031-CV) supplemented with 1% BSA (Boehringer Mannheim; 100-350) was used as diluent for antibodies and for all intermediate washing steps.

The EC50 of the OV1165, 2 stocks of OV1160, OV1164, OV802, a laboratory-derived wild-type Ad5 isolate (Yu et al., 1999) and Ad*dl*312 (an E1a deleted replication defective virus) was determined on a number of cell lines using an MTS assay at day 7. The results are provided in Table 2, below as the dose a which 50% of the cells are killed (reported as PPC).

Table 2.

| **Cells/virus** | **OV1165** | **OV1160#1** | **OV1160#2** | **OV1164** | **802** | **d1312** |
|---|---|---|---|---|---|---|
| A549 | 0.12 | 0.07 | 0.21 | 0.86 | 0.02 | 543 |
| lung carcinoma | | | | | | |
| SW780 | 6 | 2 | 2 | 52 | 0.63 | 895 |
| Transitional cell carcinoma | | | | | | |
| SKMel-28 | 133 | 148 | 230 | 1033 | 15 | 4787 |
| melanoma | | | | | | |
| HT29 | 147 | 90 | 106 | 784 | 5 | 4301 |
| Colorectal adenocarcinoma | | | | | | |
| H460 | 0.62 | 0.43 | 0.7 | 10 | 0.02 | 1862 |
| Lung carcinoma | | | | | | |
| DLD-1 | 64 | 28 | 34 | 525 | 18 | na |
| Colorectal adenocarcinoma | | | | | | |

Western Blot and ELISA Analysis for TRAIL Expression

Western blot analysis was performed using methods widely known in the art (e.g., Anton and Graham, J. Virology, 69, 4600-4606, 1995, Sambrook and Russell, supra). A first series of Western blots was performed using the CVL (Crude viral lysate) of OV1164 and OV1160 following infection of A549 cells. For OV1164, A549 cells were infected at 1000, 100 or 10 particles per cell (ppc) for 24, 48 and 72hrs and the viral particle (vp) count as determined by HPLC was 8.6e9 vp/ml.

Various volumes of untitered OV1160 CVL were also used to infect A549 cells for 72 hrs. Following infection A549 cells were harvested using a cell scraper, centrifuged at 14,000 for 3 min and the cell pellet from cells infected with OV1160 or OV1164 was resuspended in 200ul of cold Western lysis buffer, incubated 30 min on ice and stored at -80 oC until use. The quantity of protein in each sample was evaluated using the Bradford protein assay. 10 or 30ug/ml of total cellular protein from OV1160 or OV 1164 infected cells was combined in a solution containing 100mM DTT and loading dye, denatured 5 min at 85□C and loaded onto a 4-12% gradient Bis-Tris SDS-Page gel (Novex from Invitrogen).

A second series of Western blots was performed using purified OV1160, OV1164 and OV1165 virus to infect A549 cells. The infection of A549 cells was carried out at PPC of 10 and 100 and the supernatant was harvested at 6, 24 and 48hrs post infection. The cells were collected at 24 and 48 hrs post-infection. 30ug of total protein or 30ul of supernatant were loaded onto a 4-12% gradient Bis-Tris SDS-Page gel (Novex from Invitrogen). The gel was subjected to 100Volts for 1.5hers in 1X MOPS Buffer, and then transferred onto a PDVF membrane for 1hr at 400mAmp in 1X MOPS buffer with 10% ofMeOH. The first antibody used was a Goat IgG anti-human TRAIL polyclonal (R&D Systems; AF375). A goat anti-HRP secondary antibody (Santa Cruz Bio SC-2056) was used. The results were detected using a ECL-Plus kit (Amersham) following exposure on Biomax MS film (Kodak).

TRAIL protein was detected by Western blot in cell lysates from A549 cells infected for 24, 48 or 72 hrs. with OV1160 and OV164 at a PPC of 10, 100 and 1000. The 19 kDa cleaved portion of TRAIL (soluble form) was also detected in the suprernatant of A549 cells infected for 48hrs with OV1160 at a PPC of 100.

TRAIL expression was quantitated by ELISA and the bystander effect of TRAIL produced by virally infected cells was performed by evaluation of the degree of apoptosis (as evaluated in a bioassay which is further described below). The analysis was carried out in order to determine whether TRAIL was produced following viral infection and if it was biologically active.

A549 cells were infected at a PPC of 100 for 24 and 48hrs, respectively, with OV1160, OV1164 and OV1165. Supernatants were harvested and frozen at-80°C until an assay was performed to determine the amount of TRAIL in the supernatant by ELISA.

Conditioned medium was collected and assayed for sTRAIL expression using a sandwich ELISA. 96-well microtiter plates were coated with mouse anti-human TRAIL monoclonal antibody (BioSource) in 0.1M carbonate pH 9.6 buffer and incubated overnight at 4°C. The plates were washed extensively with PBS-0.05%Tween-20, and blocked with PBS-1% BSA-0.05% Tween-20 buffer for 1 hr. Recombinant human TRAIL protein (R&D Systems, Minneapolis, MN) was used for standard curves after serial dilutions. Samples were incubated in the wells for 1 hr, washed extensively, and incubated with goat anti-human TRAIL polyclonal antibody (R&D Systems, Minneapolis, MN) for 1hr. After extensive washing, the samples were incubated with HRP-conjugated anti-goat IgG antibody (Sigma Chemical Co.) for 1 hr, washed again, and detected using Sure Blue TMB substrate (KPL, Gaithersburg, MD) at an optical density of 450/650 nm. A standard curve was generates using recombinant Human TRAIL (R&D Systems; 375 TEC). The results of this assay is provided in Table 3A, below.

Table 3A.

| | **PPC100** | | | |
|---|---|---|---|---|
| | **24hr** | | **Mean** | **TRAIL (ng/ml)** |
| OV1160#1 | 0.093 | 0.11 | 0.1015 | 1.1 |
| OV1160#2 | 0.1 | 0.1 | 0.1 | 0.9 |
| OV1165 | 0.078 | 0.079 | 0.0785 | |
| OV1164 | | | | |
| | | | | |
| | **48hr** | | **Mean** | **TRAIL (ng/ml)** |
| OV1160#1 | 0.192 | 0.191 | 0.1915 | 9.6 |
| OV1160#2 | 0.208 | 0.207 | 0.2075 | 13.3 |
| OV 1165 | 0.079 | 0.08 | 0.0795 | |
| OV1164 | 0.082 | 0.08 | 0.081 | |

A second ELISA was performed on 6 cell lines (SW780, A549, DID-I, SKMe128, HT29, H460) infected at their respective EC50 (determine by MTS at day 7) with OV 1160 or OV1165 for 40hrs. This study was carried out to evaluate the amount of TRAIL produced by a number of different cell lines. The results shown in Table 3B indicate that SW780, A549 and HT460 cells infected with OV 1160 produced the greatest amount of TRAIL.

Table 3B.

| **TRAIL (ng/ml)** | | **SW780** | **A549** | **DLD1** | **SK-Mel28** | **HT29** | **H460** |
|---|---|---|---|---|---|---|---|
| mean | OV1165 | 0 | 0 | 0.201301 | 0 | 0 | 0 |
| | OV1160#1 | 10.048 | 110.3783 | 4.790433 | 1.839404 | 0.727786 | 13.83983 |
| | OV1160#2 | 5.921467 | 87.18996 | 5.231936 | 1.890064 | 0.616181 | 13.55111 |
| | | | | | | | |
| sd | | 0 | 0 | 0.284682 | 0 | 0 | 0 |
| | | 1.997327 | 7.356895 | 0.159734 | 0.365634 | 0.072695 | 0 |

(continued)

| TRAIL (ng/ml) | | SW780 | A549 | DLD1 | SK-Mel28 | HT29 | H460 |
|---|---|---|---|---|---|---|---|
| | | | 5.811357 | 1.379575 | 0.437278 | 0.04107 | 1.353553 |

The bystander effect of TRAIL produced by cells infected with OV1160, OV1164 and OV1165 was evaluated by transfer of supernatant derived from infected cells and transferred onto fresh non-infected cells. The degree of apoptosis was evaluated by DNA fragmentation using an ELISA kit (Cell death detection kit Elisa plus/Roche 1774425).

A first assay was performed on 100 ul of supernatant collected following infection of A549 cells at a PPC 100 with OV1165, OV 1164 or one of two lots of OV1160 for 48 hrs with and without a general caspase inhibitor (R&D systems: FMK001). The analysis was performed 16hrs following addition of the supernatant. The results shown in Table 4 indicate that OV1160 and not OV1165 is capable of inducing apoptosis on SW780 cells and when a caspase inhibitor is included in the culture, the effect is decreased.

Table 4.

| | SW780 | A549 |
|---|---|---|
| Cells alone | 0.35 | 0.24 |
| 1165 | 0.68 | 0.26 |
| 1165 + inhibitor | 1 | 0.3 |
| 1160 | 3 | 0.175 |
| 1160 + inhibitor | 0.56 | 0.15 |
| rTrail | 3.75 | 1.45 |
| Positive control from kit | 3.3 | |
| Incubation buffer | 0.21 | |
| ABTS (dye) | 0.18 | |

A second assay is performed using 6 cell lines (SW780, DLD-1, HT29, SkMe128, H460 and A549) infected with OV1165 and OV1160 at their respective EC50s as determined by MTS on day 7. Freshly harvested supernatant from each virus-infected cell line is added to the corresponding non-infected cells with and without caspase inhibitor for 40hrs before performing the ELISA. As shown in Table 3B, the level of TRAIL produced was variable for different cell lines. This study is designed to evaluate the biological activity of TRAIL produced by a particular cell line.

Example 16. Anti-Tumor Efficacy In The Subcutaneous Human Bladder SW780 Xenograft Tumor Model.

A study was performed to evaluate the anti-tumor efficacy of TRAIL-expressing (OV1160) and control (OV1165) virus using a human bladder TCC cell line, SW780. In this current study, xenograft tumors of SW780 cells in nude mice are treated by five intratumoral injections of OV1160 ad OV 1165, respectively. The parameters measured include survival, changes in tumor volume, body weight, as well as the extent of viral infection, TRAIL expression and degree of apoptosis in tumors.

Tumors are established by injecting 2 x $10^6$ SW780 cells subcutaneously into female mice (10 animals per group). Intratumoral virus treatment is initiated when the mean tumor volume reaches approximately 150 $mm^3$. Starting on Study Day (SD 1), tumors are injected five times at a dose of 1 x 10e8, 1 x 10e9, or 1 x 10e10 virus particles (vp) per injection, every other day for a total of 5 administrations. Tumor volume [($W^2$ x L)/2] is measured twice per week; body weight is measured once per week. Hexon staining and TRAIL ELISA is performed at various time points post infection.

**Brief Description Of The Sequences**

The following is a description of the sequences employed in practicing the invention.

LLNFDLLKLAGDVESNPGP (SEQ ID NO:1)
TLNFDLLKLAGDVESNPGP (SEQ ID NO:2);

LLKLAGDVESNPGP (SEQ ID NO:3)
NFDLLKLAGDVESNPGP (SEQ ID NO:4)
QLLNFDLLKLAGDVESNPGP (SEQ ID NO:5)
APVKQTLNFDLLKLAGDVESNPGP (SEQ ID NO:6).

VTELLYRMKRAETYCPRPLLAIHPTEARHKQKIVAPVKQTLNFDLLKLAGDVESNPGP (SEQ ID NO:7)

LLAIHPTEARHKQKIVAPVKQTLNFDLLKLAGDVESNPGP (SEQ ID NO:8)
EARHKQKIVAPVKQTLNFDLLKLAGDVESNPGP (SEQ ID NO:9)
NFDLLKLAGDVESNPGPFF (SEQ ID NO:10)
GIFNAHYAGYFADLLIHDIETNPGP (SEQ ID NO:11)
RIFNAHYAGYFADLLIHDIETNPGP (SEQ ID NO:12)
HVFETHYAGYFADLLIHDVETNPGP (SEQ ID NO:13)
KAVRGYHADYYKQRLIHDVEMNPGP (SEQ ID NO:14)
RAVRAYHADYYKQRLIHDVEMNPGP (SEQ ID NO:15)
KAVRGYHADYYRQRLIHDVETNPGP (SEQ ID NO:16)
LTNFDLLKLAGDVESNPGP (SEQ ID NO:17)
LLNFDLLKLAGDMESNPGP (SEQ ID NO:18)
MCNFDLLKLAGDVESNPGP (SEQ ID NO:19)
CTNYALLKLAGDVESNPGP (SEQ ID NO:20)
GATNFSLLKLAGDVELNPGP (SEQ ID NO:21)
GPGATNFSLLKQAGDVEENPGP (SEQ ID NO:22)
EAARQMLLLLSGDVETNPGP (SEQ ID NO:23)
FLRKRTQLLMSGDVESNPGP (SEQ ID NO:24)
GSWTDILLLLSGDVETNPGP (SEQ ID NO:25)
RAEGRGSLLTCGDVEENPGP (SEQ ID NO:26)
TRAEIEDELIRAGIESNPGP (SEQ ID NO:27)
SKFQIDRILISGDIELNPGP (SEQ ID NO:28)
AKFQIDKLISGDVELNPGP (SEQ ID NO:29)
SKFQIDKILISGDIELNPGP (SEQ ID NO:30)
SSIIRTKMLVSGDVEENPGP (SEQ ID NO:31)
CDAQRQKLLLSGDIEQNPGP (SEQ ID NO:32)
SEQ ID NO:33 IS A FURIN CONSENSUS SEQUENCE OR SITE: RXK(R)R
SEQ ID NO:34 IS A FACTOR XA CLEAVAGE SEQUENCE OR SITE: IE(D)GR
SEQ ID NO:35 IS A SIGNAL PEPTIDASE I CLEAVAGE SEQUENCE OR SITE: LAGFATVAQA
SEQ ID NO:36 IS A THROMBIN CLEAVAGE SEQUENCE OR SITE: LVPRGS
SEQ ID NO:37 IS AN Adenoviral consensus protease sequence or site (M,L,I)XGG/X
SEQ ID NO:38 IS AN Adenoviral consensus protease sequence or site (M,L,I)XGX/G
SEQ ID NO:39 is an exemplary sequence for a branch point plus splice acceptor
SEQ ID NO:40 is a branch point consensus sequence
SEQ ID NO:41 is nucleotides 29209 to 29336 of GenBank AY339865
SEQ ID NO:42 is an L2 region splice site
SEQ ID NO:43 is the E2F PROMOTER sequence
SEQ ID NO:44 is an FMDV (VIRUS STRAIN C) IRES Sequence
SEQ ID NO:45 is a 3' splice acceptor site or SAS
SEQ ID NO:46 is the hTRAIL ORF (INVIVOGEN)
SEQ ID NO:47 is the CP1524 SEQUENCE (9587 BASE PAIRS)
SEQ ID NO:48 is the CP1606 REV SEQUENCE (3855 BASE PAIRS)
SEQ ID NO:49 is the OV 1165 sequence
SEQ ID NO:50 is the OV1164 sequence
SEQ ID NO:51 is the OV1160 sequence
SEQ ID NO:52 is the sequence for oligonucleotide primer 1618.95.1
SEQ ID NO:53 is the sequence for oligonucleotide primer 1618.95.
SEQ ID NO:54 is the sequence for oligonucleotide primer 1618.97.1

SEQ ID NO:55 is the sequence for oligonucleotide primer 1618.97.2
SEQ ID NO:56 is the sequence for-oligonucleotide primer 1618.95.5
SEQ ID NO:57 is the sequence for oligonucleotide primer 1706.83.1
SEQ ID NO:58 is the sequence for oligonucleotide primer 1706.83.2
SEQ ID NO:59 is the sequence for oligonucleotide primer 1706.95.1
SEQ ID NO:60 is the sequence for oligonucleotide primer 1618.116.3
SEQ ID NO:61 is the sequence for oligonucleotide primer 1619.144.1
SEQ ID NO:62 is the sequence for oligonucleotide primer 1619.144.3
The following is a description of the sequences employed in practicing the invention.
LLNFDLLKLAGDVESNPGP (SEQ ID NO:1)
TLNFDLLKLAGDVESNPGP (SEQ ID NO:2);
LLKLAGDVESNPGP (SEQ ID NO:3)
NFDLLKLAGDVESNPGP (SEQ ID NO:4)
QLLNFDLLKLAGDVESNPGP (SEQ ID NO:5)
APVKQTLNFDLLKLAGDVESNPGP (SEQ ID NO:6).

VTELLYRMKRAETYCPRPLLAIHPTEARHKQKIVAPVKQTLNFDLLKLAGDVESNPG P (SEQ ID NO:7)

LLAIHPTEARHKQKIVAPVKQTLNFDLLKLAGDVESNPGP (SEQ ID NO:8)
EARHKQKIVAPVKQTLNFDLLKLAGDVESNPGP (SEQ ID NO:9)
NFDLLKLAGDVESNPGPFF (SEQ ID NO:10)
GIFNAHYAGYFADLLIHDIETNPGP (SEQ ID NO:11)
RIFNAHYAGYFADLLIHDIETNPGP (SEQ ID NO:12)
HVFETHYAGYFADLLIHDVETNPGP (SEQ ID NO:13)
KAVRGYHADYYKQRLIHDVEMNPGP (SEQ ID NO:14)
RAVRAYHADYYKQRLIHDVEMNPGP (SEQ ID NO:15)
KAVRGYHADYYRQRLIHDVETNPGP (SEQ ID NO:16)
LTNFDLLKLAGDVESNPGP (SEQ ID NO:17)
LLNFDLLKLAGDMESNPGP (SEQ ID NO:18)
MCNFDLLKLAGDVESNPGP (SEQ ID NO:19)
CTNYALLKLAGDVESNPGP (SEQ ID NO:20)
GATNFSLLKLAGDVELNPGP (SEQ ID NO:21)
GPGATNFSLLKQAGDVEENPGP (SEQ ID NO:22)
EAARQMLLLLSGDVETNPGP (SEQ ID NO:23)
FLRKRTQLLMSGDVESNPGP (SEQ ID NO:24)
GSWTDILLLLSGDVETNPGP (SEQ ID NO:25)
RAEGRGSLLTCGDVEENPGP (SEQ ID NO:26)
TRAEIEDELIRAGIESNPGP (SEQ ID NO:27)
SKFQIDRILISGDIELNPGP (SEQ ID NO:28)
AKFQIDKILISGDVELNPGP (SEQ ID NO:29)
SKFQIDKILISGDIELNPGP (SEQ ID NO:30)
SSIIRTKMLVSGDVEENPGP (SEQ ID NO:31)
CDAQRQKLLLSGDIEQNPGP (SEQ ID NO:32)
SEQ ID NO:33 IS A FURIN CONSENSUS SEQUENCE OR SITE: RXK(R)R
SEQ ID NO:34 IS A FACTOR XA CLEAVAGE SEQUENCE OR SITE: IE(D)GR
SEQ ID NO:35 IS A SIGNAL PEPTIDASE I CLEAVAGE SEQUENCE OR SITE: LAGFATVAQA
SEQ ID NO:36 IS A THROMBIN CLEAVAGE SEQUENCE OR SITE: LVPRGS
SEQ ID NO:37 IS AN Adenoviral consensus protease sequence or site (M,L,I)XGG/X
SEQ ID NO:38 IS AN Adenoviral consensus protease sequence or site (M,L,I)XGX/G
SEQ ID NO:39 is an exemplary sequence for a branch point plus splice acceptor TACTTAT GACTCGTACTATTGTTATTCATCC AG6G
SEQ ID NO:40 is a branch point consensus sequence YNYURAY (where Y is a pyrimidine, N is any nucleotide, and R is a purine)

SEQ ID NO:41 is nucleotides 29209 to 29336 of GenBank AY339865

TAATTTACTAAGTTACAAAGCTAATGTCACCACTAACTGCTTTACTCGCTGCTTG
CAAAACAAATTCAAAAAGTTAGCATTATAATTAGAATAGGATTTAAACCCCCCG
GTCATTTCCTGCTCAATAC

SEQ ID NO:42 is an L2 region splice site
TAC TTAT AGT AAT CTA A TT CCT G CT CTC TC AG

SEQ ID NO:43 is the E2F PROMOTER sequence

CATCCGGACAAAGCCTGCGCGCGCCCCGCCCCGCCATTGGCCGTACCGCCCCGC
GCCGCCGCCCCATCTCGCCCCTCGCCGCCGGGTCCGGCGCGTTAAAGCCAATAG
GAACCGCCGCCGTTGTTCCCGTCACGGCCGGGGCAGCCAATTGTGGCGGCGCTC
GGCGGCTCGTGGCTCTTTCGCGGCAAAAAGGATTTGGCGCGTAAAAGTGGCCGG
GACTTTGCAGGCAGCGGCGGCCGGGGGCGGAGCGGGATCGAGCCCTCGATGAT
ATCA

SEQ ID NO:44 is an FMDV (VIRUS STRAIN C) IRES Sequence

AGCAGGTTTCCCCAACTG

ACACAAAACGTGCAACTTGAAACTCCGCCTGGTCTTTCCAGGTCTAGAGGGGTAACA

CTTTGTACTGCGT

TTGGCTCCACGCTCGATCCACTGGCGAGTGTTAGTAACAGCACTGTTGCTTCGTAGCG

GAGCATGACGGC

CGTGGGAACTCCTCCTTGGTAACAAGGACCCACGGGGCCAAAAGCCACGCCCACACG

GGCCCGTCATGTG

TGCAACCCCAGCACGGCGACTTTACTGCGAAACCCACTTTAAAGTGACATTGAAACT

GGTACCCACACAC

TGGTGACAGGCTAAGGATGCCCTTCAGGTACCCCGAGGTAACACGCGACACTCGGGA

TCTGAGAAGGGGA

CTGGGGCTTCTATAAAAGCGCTCGGTTTAAAAAGCTTCTATGCCTGAATAGGTGACC

GGAGGTCGGCACC

TTTCCTTTGCAATTAATGACCCT

SEQ ID NO:45 is a 3' splice acceptor site or SAS
TACTTATGACTCGTACTATTGTTATTCATCCAG G

SEQ ID NO:46 is the hTRAIL ORF (INVIVOGEN) (SEQ ID NO:46)

ATGGCTATGATGGAGGTCCAGGGGGGACCCAGCCTGGGACAGACCTGCGTGCTGATC

GTGATCTTTACAGTG

CTCCTGCAGTCTCTCTGTGTGGCTGTAACTTACGTGTACTTTACCAACGAGCTGAAGC

AGATGCAGGACAAG

TACTCCAAAAGTGGCATTGCTTGTTTCTTAAAAGAAGATGACAGTTATTGGGACCCCA

ATGACGAAGAGAGT

ATGAACAGCCCCTGCTGGCAAGTCAAGTGGCAACTCCGTCAGCTCGTTAGAAAGATG

ATTTTGAGAACCTCT

GAGGAAACCATTTCTACAGTTCAAGAAAAGCAACAAAATATTTCTCCCCTAGTGAGA

GAAAGAGGTCCTCAG

AGAGTAGCAGCTCACATAACTGGGACCAGAGGAAGAAGCAACACATTGTCTTCTCCA

AACTCCAAGAATGAA

AAGGCTCTGGGCCGCAAAATAAACTCCTGGGAATCATCAAGGAGTGGGCATTCATTC

CTGAGCAACTTGCAC

TTGAGGAATGGTGAACTGGTCATCCATGAAAAAGGGTTTTACTACATCTATTCCCAAA

CATACTTTCGATTT

CAGGAGGAAATAAAAGAAAACACAAAGAACGACAAACAAATGGTCCAATATATTTA

CAAATACACAAGTTAT

CCTGACCCTATATTGTTGATGAAAAGTGCTAGAAATAGTTGTTGGTCTAAAGATGCAG

AATATGGACTCTAT

TCCATCTATCAAGGGGGAATATTTGAGCTTAAGGAAAATGACAGAATTTTTGTTTCTG

TAACAAATGAGCAC

TTAATAGACATGGACCATGAAGCCAGTTTTTTCGGGGCCTTTTTAGTTGGCTAA

SEQ ID NO:47 is the CP1524 SEQUENCE (9587 BASE PAIRS)

GACGGATCGGGAGATCTCCCGATCCCCTATGGTGCACTCTCAGTACAATCTGCTCT

GATGCCGCATAGTTAAG

CCAGTATCTGCTCCCTGCTTGTGTGTTGGAGGTCGCTGAGTAGTGCGCGAGCAAAA TTTAAGCTACAACAAGG

CAAGGCTTGACCGACAATTGCATGAAGAATCTGCTTAGGGTTAGGCGTTTTGCGCT GCTTCGCGATGTACGGG

CCAGATATACGCGTTGACATTGATTATTGACTAGTTATTAATAGTAATCAATTACG GGGTCATTAGTTCATAG

CCCATATATGGAGTTCCGCGTTACATAACTTACGGTAAATGGCCCGCCTGGCTGAC CGCCCAACGACCCCCGC

CCATTGACGTCAATAATGACGTATGTTCCCATAGTAACGCCAATAGGGACTTTCCA TTGACGTCAATGGGTGG

AGTATTTACGGTAAACTGCCCACTTGGCAGTACATCAAGTGTATCATATGCCAAGT ACGCCCCCTATTGACGT

CAATGACGGTAAATGGCCCGCCTGGCATTATGCCCAGTACATGACCTTATGGGACT TTCCTACTTGGCAGTAC

ATCTACGTATTAGTCATCGCTATTACCATGGTGATGCGGTTTTGGCAGTACATCAA TGGGCGTGGATAGCGGT

TTGACTCACGGGGATTTCCAAGTCTCCACCCCATTGACGTCAATGGGAGTTTGTTTT GGCACCAAAATCAACG

GGACTTTCCAAAATGTCGTAACAACTCCGCCCCATTGACGCAAATGGGCGGTAGG CGTGTACGGTGGGAGGTC

TATATAAGCAGAGCTCTCTGGCTAACTAGAGAACCCACTGCTTACTGGCTTATCGA AATTAATACGACTCACT

ATAGGGAGACCCAAGCTGGCTAGCGTTTAAACTTAAGCTTGATCCCCGCCCTCCCG TAGAGGAGCCTCCACCG

GCCGTGGAGACAGTGTCTCCAGAGGGGCGTGGCGAAAAGCGTCCGCGCCCCGACA GGGAAGAAACTCTGGTGA

CGCAAATAGACGAGCCTCCCTCGTACGAGGAGGCACTAAAGCAAGGCCTGCCCAC CACCCGTCCCATCGCGCC

CATGGCTACCGGAGTGCTGGGCCAGCACACACCCGTAACGCTGGACCTGCCTCCCC CCGCCGACACCCAGCAG

AAACCTGTGCTGCCAGGCCCGACCGCCGTTGTTGTAACCCGTCCTAGCCGCGCGTC CCTGCGCCGCGCCGCCA

GCGGTCCGCGATCGTTGCGGCCCGTAGCCAGTGGCAACTGGCAAAGCACACTGAA CAGCATCGTGGGTCTGGG

GGTGCAATCCCTGAAGCGCCGACGATGCTTCTGAATAGCTAACGTGTCGTATGTGT GTCATGTATGCGTCCAT

GTCGCCGCCAGAGGAGCTGCTGAGCCGCCGCGCCCGCTTTCCAAGATGGCTAC CCCTTCGATGATGCCGCA

GTGGTCTTACATGCACATCTCGGGCCAGGACGCCTCGGAGTACCTGAGCCCCGGGC TGGTGCAGTTTGCCCGC

GCCACCGAGACGTACTTCAGCCTGAATAACAAGTTTAGAAACCCCACGGTGGCGC CTACGCACGACGTGACCA

CAGACCGGTCCCAGCGTTTGACGCTGCGGTTCATCCCTGTGGACCGTGAGGATACT GCGTACTCGTACAAGGC

GCGGTTCACCCTAGCTGTGGGTGATAACCGTGTGCTGGACATGGCTTCCACGTACT TTGACATCCGCGGCGTG

CTGGACAGGGGCCCTACTTTTAAGCCCTACTCTGGCACTGCCTACAACGCCCTGGC TCCCAAGGGTGCCCCAA

ATCCTTGCGAATGGGATGAAGCTGCTACTGCTCTTGAAATAAACCTAGAAGAAGA GGACGATGACAACGAAGA

CGAAGTAGACGAGCAAGCTGAGCAGCAAAAAACTCACGTATTTGGGCAGGCGCCT TATTCTGGTATAAATATT

ACAAAGGAGGGTATTCAAATAGGTGTCGAAGGTCAAACACCTAAATATGCCGATA AAACATTTCAACCTGAAC

CTCAAATAGGAGAATCTCAGTGGTACGAAACTGAAATTAATCATGCAGCTGGGAG AGTCCTTAAAAAGACTAC

CCCAATGAAACCATGTTACGGTTCATATGCAAAACCCACAAATGAAAATGGAGGG CAAGGCATTCTTGTAAAG

CAACAAAATGGAAAGCTAGAAAGTCAAGTGGAAATGCAATTTTTCTCAACTACTG AGGCGACCGCAGGCAATG

GTGATAACTTGACTCCTAAAGTGGTATTGTACAGTGAAGATGTAGATATAGAAACC CCAGACACTCATATTTC

TTACATGCCCACTATTAAGGAAGGTAACTCACGAGAACTAATGGGCCAACAATCT ATGCCCAACAGGCCTAAT

TACATTGCTTTTAGGGACAATTTTATTGGTCTAATGTATTACAACAGCACGGGTAA TATGGGTGTTCTGGCGG

GCCAAGCATCGCAGTTGAATGCTGTTGTAGATTTGCAAGACAGAAACACAGAGCT TTCATACCAGCTTTTGCT

TGATTCCATTGGTGATAGAACCAGGTACTTTTCTATGTGGAATCAGGCTGTTGACA GCTATGATCCAGATGTT

AGAATTATTGAAAATCATGGAACTGAAGATGAACTTCCAAATTACTGCTTTCCACT GGGAGGTGTGATTAATA

CAGAGACTCTTACCAAGGTAAAACCTAAAACAGGTCAGGAAAATGGATGGGAAA
AAGATGCTACAGAATTTTC

AGATAAAAATGAAATAAGAGTTGGAAATAATTTTGCCATGGAAATCAATCTAAAT
GCCAACCTGTGGAGAAAT

TTCCTGTACTCCAACATAGCGCTGTATTTGCCCGACAAGCTAAAGTACAGTCCTTC
CAACGTAAAAATTTCTG

ATAACCCAAACACCTACGACTACATGAACAAGCGAGTGGTGGCTCCCGGGTTAGT
GGACTGCTACATTAACCT

TGGAGCACGCTGGTCCCTTGACTATATGGACAACGTCAACCCATTTAACCACCACC
GCAATGCTGGCCTGCGC

TACCGCTCAATGTTGCTGGGCAATGGTCGCTATGTGCCCTTCCACATCCAGGTGCC
TCAGAAGTTCTTTGCCA

TTAAAAACCTCCTTCTCCTGCCGGGCTCATACACCTACGAGTGGAACTTCAGGAAG
GATGTTAACATGGTTCT

GCAGAGCTCCCTAGGAAATGACCTAAGGGTTGACGGAGCCAGCATTAAGTTTGAT
AGCATTTGCCTTTACGCC

ACCTTCTTCCCCATGGCCCACAACACCGCCTCCACGCTTGAGGCCATGCTTAGAAA
CGACACCAACGACCAGT

CCTTTAACGACTATCTCTCCGCCGCCAACATGCTCTACCCTATACCCGCCAACGCT
ACCAACGTGCCCATATC

CATCCCCTCCCGCAACTGGGCGGCTTTCCGCGGCTGGGCCTTCACGCGCCTTAAGA
CTAAGGAAACCCCATCA

CTGGGCTCGGGCTACGACCCTTATTACACCTACTCTGGCTCTATACCCTACCTAGAT
GGAACCTTTTACCTCA

ACCACACCTTTAAGAAGGTGGCCATTACCTTTGACTCTTCTGTCAGCTGGCCTGGC
AATGACCGCCTGCTTAC

CCCCAACGAGTTTGAAATTAAGCGCTCAGTTGACGGGGAGGGTTACAACGTTGCC
CAGTGTAACATGACCAAA

GACTGGTTCCTGGTACAAATGCTAGCTAACTACAACATTGGCTACCAGGGCTTCTA
TATCCCAGAGAGCTACA

AGGACCGCATGTACTCCTTCTTTAGAAACTTCCAGCCCATGAGCCGTCAGGTGGTG
GATGATACTAAATACAA

GGACTACCAACAGGTGGGCATCCTACACCAACACAACAACTCTGGATTTGTTGGCT
ACCTTGCCCCCACCATG

CGCGAAGGACAGGCCTACCCTGCTAACTTCCCCTATCCGCTTATAGGCAAGACCGC
AGTTGACAGCATTACCC

AGAAAAAGTTTCTTTGCGATCGCACCCTTTGGCGCATCCCATTCTCCAGTAACTTTA
TGTCCATGGGCGCACT

CACAGACCTGGGCCAAAACCTTCTCTACGCCAACTCCGCCCACGCGCTAGACATGA
CTTTTGAGGTGGATCCC

ATGGACGAGCCCACCCTTCTTTATGTTTTGTTTGAAGTCTTTGACGTGGTCCGTGTG
CACCGGCCGCACCGCG

GCGTCATCGAAACCGTGTACCTGCGCACGCCCTTCTCGGCCGGCAACGCCACAACA
TAAAGAAGCAAGCAACA

TCAACAACAGCTGCCGCCATGGGCTCCAGTGAGCAGGAACTGAAAGCCATTGTCA
AAGATCTTGGTTGTGGGC

CATATTTTTTGGGCACCTATGACAAGCGCTTTCCAGGCTTTGTTTCTCCACACAAGC
TCGCCTGCGCCATAGT

CAATACGGCCGGTCGCGAGACTGGGGGCGTACACTGGATGGCCTTTGCCTGGAAC
CCGCACTCAAAAACATGC

TACCTCTTTGAGCCCTTTGGCTTTTCTGACCAGCGACTCAAGCAGGTTTACCAGTTT
GAGTACGAGTCACTCC

TGCGCCGTAGCGCCATTGCTTCTTCCCCCGACCGCTGTATAACGCTGGAAAAGTCC
ACCCAAAGCGTACAGGG

GCCCAACTCGGCCGCCTGTGGACTATTCTGCTGCATGTTTCTCCACGCCTTTGCCAA
CTGGCCCCAAACTCCC

ATGGATCACAACCCCACCATGAACCTTATTACCGGGGTACCCAACTCCATGCTCAA
CAGTCCCCAGGTACAGC

CCACCCTGCGTCGCAACCAGGAACAGCTCTACAGCTTCCTGGAGCGCCACTCGCCC
TACTTCCGCAGCCACAG

TGCGCAGATTAGGAGCGCCACTTCTTTTGTCACTTGAAAAACATGTAAAAATAAT
GTACTAGAGACACTTTC

AATAAAGGCAAATGCTTTTATTTGTACACTCTCGGGTGATTATTTACCCCCACCCTT
GCCGTCTGCGCCGTTT

AAAAATCAAAGGGGTTCTGCCGCGCATCGCTATGCGCCACTGGCAGGGACACGTT
GCGATACTGGTGTTTAGT

GCTCCACTTAAACTCAGGCACAACCATCCGCGGCAGCTCGGTGAAGTTTTCACTCC
ACAGGCTGCGCACCATC

ACCAACGCGTTTAGCAGGTCGGGCGCCGATATCTTGAAGTCGCAGTTGGGGCCTCC
GCCCTGCGCGCGCGAGT

TGCGATACACAGGGTTGCAGCACTGGAACACTATCAGCGCCGGGTGGTGCACGCT
GGCCAGCACGCTCTTGTC

GGAGATCAGATCCGCGTCCAGGTCCTCCGCGTTGCTCAGGGCGAACGGAGTCAAC TTTGGTAGCTGCCTTCCC

AAAAAGGGCGCGTGCCCAGGCTTTGAGTTGCACTCGCACCGTAGTGGCATCAAAA GGTGACCGTGCCCGGTCT

GGGCGTTAGGATACAGCGCCTGCATAAAAGCCTTGATCTGCTTAAAAGCCACCTG AGCCTTTGCGCCTTCAGA

GAAGAACATGCCGCAAGACTTGCCGGAAAACTGATTGGCCGGACAGGCCGCGTCG TGCACGCAGCACCTTGCG

TCGGTGTTGGAGATCTGCACCACATTTCGGCCCCACCGGTTCTTCACGATCTTGGC CTTGCTAGACTGCTCCT

TCAGCGCGCGCTGCCCGTTTTCGCTCGTCACATCCATTTCAATCACGTGCTCCTTAT TTATCATAATGCTTCC

GTGTAGACACTTAAGCTCGCCTTCGATCTCAGCGCAGCGGTGCAGCCACAACGCGC AGCCCGTGGGCTCGTGA

TGCTTGTAGGTCACCTCTGCAAACGACTGCAGGTACGCCTGCAGGAATCGCCCCAT CATCGTCACAAAGGTCT

TGTTGCTGGTGAAGGTCAGCTGCAACCCGCGGTGCTCCTCGTTCAGCCAGGTCTTG CATACGGCCGCCAGAGC

TTCCACTTGGTCAGGCAGTAGTTTGAAGTTCGCCTTTAGATCGTTATCCACGTGGTA CTTGTCCATCAGCGCG

CGCGCAGCCTCCATGCCCTTCTCCCACGCAGACACGATCGGCACACTCAGCGGGTT CATCACCGTAATTTCAC

TTTCCGCTTCGCTGGGCTCTTCCTCTTCCTCTTGCGTCCGCATACCACGCGCCACTG GGTCGTCTTCATTCAG

CCGCCGCACTGTGCGCTTACCTCCTTTGCCATGCTTGATTAGCACCGGTGGGTTGCT GAAACCCACCATTTGT

AGCGCCACATCTTCTCTTTCTTCCTCGCTGTCCACGATTACCTCTGGTGATGGCGGG CGCTCGGGCTTGGGAG

AAGGGCGCTTCTTTTTCTTCTTGGGCGCAATGGCCAAATCCGCCGCCGAGGTCGAT GGCCGCGGGCTGGGTGT

GCGCGGCACCAGCGCGTCTTGTGATGAGTCTTCCTCGTCCTCGGACTCGATACGCC GCCTCATCCGCTTTTTT

GGGGGCGCCCGGGGAGGCGGCGACGGGGACGGGGACGACACGTCCTCCATG GTTGGGGGACGTCGCGCCG

CACCGCGTCCGCGCTCGGGGGTGGTTTCGCGCTGCTCCTCTTCCCGACTGGCCATTT CCTTCTCCTATAGGCA

GAAAAAGATCATGGAGTCAGTCGAGAAGAAGGACAGCCTAACCGCCCCCTCTGAG TTCGCCACCACCGCCTCC

ACCGATGCCGCCAACGCGCCTACCACCTTCCCCGTCGAGGCACCCCCGCTTGAGGA GGAGGAAGTGATTATCG

AGCAGGACCCAGGTTTTGTAAGCGAAGACGACGAGGACCGCTCAGTACCAACAGA GGATAAAAAGCAAGACCA

GGACAACGCAGAGGCAAACGAGGAACAAGTCGGGCGGGGGGACGAAAGGCATGG CGACTACCTAGATGTGGGA

GACGACGTGCTGTTGAAGCATCTGCAGCGCCAGTGCGCCATTATCTGCGACGCGTT GCAAGAGCGCAGCGATG

TGCCCCTCGCCATAGCGGATGTCAGCCTTGCCTACGAACGCCACCTATTCTCACCG CGCGTACCCCCCAAACG

CCAAGAAAACGGCACATGCGAGCCCAACCCGCGCCTCAACTTCTACCCCGTATTTG
CCGTGCCAGAGGTGCTT

GCCACCTATCACATCTTTTTCCAAAACTGCAAGATACCCCTATCCTGCCGTGCCAA
CCGCAGCCGAGCGGACA

AGCAGCTGGCCTTGCGGCAGGGCGCTGTCATACCTGATATCGCCTCGCTCAACGAA
GTGCCAAAAATCTTTGA

GGGTCTTGGACGCGACGAGAAGCGCGCGGCAAACGCTCTGCAACAGGAAAACAG
CGAAAATGAAAGTCACTCT

GGAGTGTTGGTGGAACTCGAGTTACCGTCGACCTCTAGCTAGAGCTTGGCGTAATC
ATGGTCATAGCTGTTTC

CTGTGTGAAATTGTTATCCGCTCACAATTCCACACAACATACGAGCCGGAAGCATA
AAGTGTAAAGCCTGGGG

TGCCTAATGAGTGAGCTAACTCACATTAATTGCGTTGCGCTCACTGCCCGCTTTCC
AGTCGGGAAACCTGTCG

TGCCAGCTGCATTAATGAATCGGCCAACGCGCGGGGAGAGGCGGTTTGCGTATTG
GGCGCTCTTCCGCTTCCT

CGCTCACTGACTCGCTGCGCTCGGTCGTTCGGCTGCGGCGAGCGGTATCAGCTCAC
TCAAAGGCGGTAATACG

GTTATCCACAGAATCAGGGGATAACGCAGGAAAGAACATGTGAGCAAAAGGCCA
GCAAAAGGCCAGGAACCGTA

AAAAGGCCGCGTTGCTGGCGTTTTTCCATAGGCTCCGCCCCCCTGACGAGCATCAC
AAAAATCGACGCTCAAGT

CAGAGGTGGCGAAACCCGACAGGACTATAAAGATACCAGGCGTTTCCCCCTGGAA
GCTCCCTCGTGCGCTCTCC

TGTTCCGACCCTGCCGCTTACCGGATACCTGTCCGCCTTTCTCCCTTCGGGAAGCGT GGCGCTTTCTCATAGCT

CACGCTGTAGGTATCTCAGTTCGGTGTAGGTCGTTCGCTCCAAGCTGGGCTGTGTG CACGAACCCCCCGTTCAG

CCCGACCGCTGCGCCTTATCCGGTAACTATCGTCTTGAGTCCAACCCGGTAAGACA CGACTTATCGCCACTGGC

AGCAGCCACTGGTAACAGGATTAGCAGAGCGAGGTATGTAGGCGGTGCTACAGAG TTCTTGAAGTGGTGGCCTA

ACTACGGCTACACTAGAAGAACAGTATTTGGTATCTGCGCTCTGCTGAAGCCAGTT ACCTTCGGAAAAAGAGTT

GGTAGCTCTTGATCCGGCAAACAAACCACCGCTGGTAGCGGTTTTTTTGTTTGCAA GCAGCAGATTACGCGCAG

AAAAAAAGGATCTCAAGAAGATCCTTTGATCTTTTCTACGGGGTCTGACGCTCAGT GGAACGAAAACTCACGTT

AAGGGATTTTGGTCATGAGATTATCAAAAAGGATCTTCACCTAGATCCTTTTAAAT TAAAAATGAAGTTTTAAA

TCAATCTAAAGTATATATGAGTAAACTTGGTCTGACAGTTACCAATGCTTAATCAG TGAGGCACCTATCTCAGC

GATCTGTCTATTTCGTTCATCCATAGTTGCCTGACTCCCCGTCGTGTAGATAACTAC GATACGGGAGGGCTTAC

CATCTGGCCCCAGTGCTGCAATGATACCGCGAGACCCACGCTCACCGGCTCCAGAT TTATCAGCAATAAACCAG

CCAGCCGGAAGGGCCGAGCGCAGAAGTGGTCCTGCAACTTTATCCGCCTCCATCC AGTCTATTAATTGTTGCCG

GGAAGCTAGAGTAAGTAGTTCGCCAGTTAATAGTTTGCGCAACGTTGTTGCCATTG

CTACAGGCATCGTGGTGT

CACGCTCGTCGTTTGGTATGGCTTCATTCAGCTCCGGTTCCCAACGATCAAGGCGA

GTTACATGATCCCCCATG

TTGTGCAAAAAAGCGGTTAGCTCCTTCGGTCCTCCGATCGTTGTCAGAAGTAAGTT

GGCCGCAGTGTTATCACT

CATGGTTATGGCAGCACTGCATAATTCTCTTACTGTCATGCCATCCGTAAGATGCTT

TTCTGTGACTGGTGAGT

ACTCAACCAAGTCATTCTGAGAATAGTGTATGCGGCGACCGAGTTGCTCTTGCCCG

GCGTCAATACGGGATAAT

ACCGCGCCACATAGCAGAACTTTAAAAGTGCTCATCATTGGAAAACGTTCTTCGGG

GCGAAAACTCTCAAGGAT

CTTACCGCTGTTGAGATCCAGTTCGATGTAACCCACTCGTGCACCCAACTGATCTT

CAGCATCTTTTACTTTCA

CCAGCGTTTCTGGGTGAGCAAAAACAGGAAGGCAAAATGCCGCAAAAAAGGGAA

TAAGGGCGACACGGAAATGT

TGAATACTCATACTCTTCCTTTTTCAATATTATTGAAGCATTTATCAGGGTTATTGT

CTCATGAGCGGATACAT

ATTTGAATGTATTTAGAAAAATAAACAAATAGGGGTTCCGCGCACATTTCCCCGAA

AAGTGCCACCTGACGTC

SEQ ID NO:48 is the CP1606 REV SEQUENCE (3855 BASE PAIRS)

AGCGCCCAATACGCAAACCGCCTCTCCCCGCGCGTTGGCCGATTCATTAATGCAG

CTGGCACGACAGGTTTCCCG

ACTGGAAAGCGGGCAGTGAGCGCAACGCAATTAATGTGAGTTAGCTCACTCATTA

GGCACCCCAGGCTTTACACT

TTATGCTTCCGGCTCGTATGTTGTGTGGAATTGTGAGCGGATAACAATTTCACACA

GGAAACAGCTATGACCATG

ATTACGCCAAGCTTGGTACCGAGCTCGGATCCACTAGTAACGGCCGCCAGTGTGC

TGGAATTCGCCCTTGACGCG

GCCTGTCCGGCCAATCAGTTTTCCGGCAAGTCTTGCGGCATGTTCTTCTCTGAAGG

CGCAAAGGCTCAGGTGGCT

TTTAAGCAGATCAAGGCTTTTATGCAGGCGCTGTATCCTAACGCCCAGACCGGGC

ACGGTCACCTTTTGATGCCA

CTACGGTGCGAGTGCAACTCAAAGCCTGGGCACGCGCCCTTTTTGGGAAGGCAGC

TACCAAAGTTGACTCCGTTC

GCCCTGAGCAACGCGGAGGACCTGGACGCGGATCTGATCTCCGACAAGAGCGTG

CTGGCCAGCGTGCACCACCCG

GCGCTGATAGTGTTCCAGTGCTGCAACCCTGTGTATCGCAACTCGCGCGCGCAGG

GCGGAGGCCCCAACTGCGAC

TTCAAGATATCGGCGCCCGACCTGCTAAACGCGTTGGTGATGGTGCGCAGCCTGT

GGAGTGAAAACTTCACCGAG

CTGCCGCGGATGGTTGTGCCTGAGTTTAAGTGGAGCACTAAACACCAGTATCGCA

ACGTGTCCCTGCCAGTGGCG

CATAGCGATGCGCGGCAGAACCCCTTTGATTTTTAAACGGCGCAGACGGCAAGGG

TGGGGGTAAATAATCACCCG

AGAGTGTACAAATAAAAGCATTTGCCTTTATTGAAAGTGTCTCTAGTAGCTAGCG

GGAGGGAGGTCCTGGTCTAG

ACATTCAGAGCTTGTAGAATTTCTGCATCCAGGTCAGAAGAATGTCCACTTCCCCA
AGGGCTTTGGTCAGAGCTG

CTTCTACGTCCAACTGTTTGAATGCTCTCCGGAATAGCAGAAACCGCCTGTGTGCA
CTGTCTCTGATGGAAAACA

TCTCATTTTCTTGACTGGGTTGCAGTTGTGACACGATGAGAACAAAGTTGTTGGCC
AGAGTAGAGAATGACTTCA

GAGTCCTGACTTCAACTGTTCTATTGTGGTGGTTTTTGAAAACAGTTTTCAAGTAG
AACTCCAGCAGGGTGTGGA

CAAGGTAACAGCTCTCAGCATCCGAGACGTTCTGCAGAACCTCCTGCTGCAGCAG
CCGGGCACTCGTGATGTTAT

CCTGAGCTTGCATAGTGTCTTTCACAGCCCAGAAGGCTTCCCACAGTTTCTGGGGA
ACAACCCCCTTCACTTGGC

AGGGCCCAAAGTGGAATTCTTGGCCCTGGGCCCCTGATACCTGGCTCCAGAGAAG
CAGGGTAAAACCCAGGCAAG

GGAGCACAACCATCTGCATTTGAGAGGCTGTCGCCAGCAAAGGAGGGCAGAAGG
GTCTGGCTAAAGTCCACAGGC

TTTGCAGCCTCTGTTGAAAATTCATGATGGCCCTCCTACCGCCTGGATGAATAACA
ATAGTACGAGTCATAAGTA

ATTATTTTACATGTTTTTCAAGTGACAAAAAGAAGTGGCGCTCCTAATCTGCGCA
CTGTGGCTGCGGAAGTAGG

GCGAGTGGCGCTCCAGGAAGCTGTAGAGCTGTTCCTGGTTGCGACGCAGGGTGGG
CTGTACCTGGGGACTGTTGA

GCATGGAGTTGGGTACCCCGGTAAAAAGGGCGAATTCTGCAGATATCCATCACAC
TGGCGGCCGCTCGAGCATGC

ATCTAGAGGACCGCTATCAGGACATAGCGTTGGCTACCCGTGATATTGCTGAAGA
GCTTGGCGGCGAATGGGCTG

ACCGCTTCCTCGTGCTTTACGGTATCGCCGCTCCCGATTCGCAGCGCATCGCCTTC
TATCGCCTTCTTGACGAGT

TCTTCTGAATTGAAAAAGGAAGAGTATGAGTATTCAACATTTCCGTGTCGCCCTTA
TTCCCTTTTTTGCGGCATT

TTGCCTTCCTGTTTTTGCTCACCCAGAAACGCTGGTGAAAGTAAAAGATGCTGAA
GATCAGTTGGGTGCACGAGT

GGGTTACATCGAACTGGATCTCAACAGCGGTAAGATCCTTGAGAGTTTTCGCCCC
GAAGAACGTTTTCCAATGAT

GAGCACTTTTAAAGTTCTGCTATGTGGCGCGGTATTATCCCGTATTGACGCCGGGC
AAGAGCAACTCGGTCGCCG

CATACACTATTCTCAGAATGACTTGGTTGAGTACTCACCAGTCACAGAAAAGCAT
CTTACGGATGGCATGACAGT

AAGAGAATTATGCAGTGCTGCCATAACCATGAGTGATAACACTGCGGCCAACTTA
CTTCTGACAACGATCGGAGG

ACCGAAGGAGCTAACCGCTTTTTTGCACAACATGGGGGATCATGTAACTCGCCTT
GATCGTTGGGAACCGGAGCT

GAATGAAGCCATACCAAACGACGAGCGTGACACCACGATGCCTGTAGCAATGGC
AACAACGTTGCGCAAACTATT

AACTGGCGAACTACTTACTCTAGCTTCCCGGCAACAATTAATAGACTGGATGGAG
GCGGATAAAGTTGCAGGACC

ACTTCTGCGCTCGGCCCTTCCGGCTGGCTGGTTTATTGCTGATAAATCTGGAGCCG
GTGAGCGTGGGTCTCGCGG

TATCATTGCAGCACTGGGGCCAGATGGTAAGCCCTCCCGTATCGTAGTTATCTACA
CGACGGGGAGTCAGGCAAC
TATGGATGAACGAAATAGACAGATCGCTGAGATAGGTGCCTCACTGATTAAGCAT
TGGTAACTGTCAGACCAAGT
TTACTCATATATACTTTAGATTGATTTAAAACTTCATTTTTAATTTAAAAGGATCTA
GGTGAAGATCCTTTTTGA
TAATCTCATGCATGACCAAAATCCCTTAACGTGAGTTTTCGTTCCACTGAGCGTCA
GACCCCGTAGAAAAGATCA
AAGGATCTTCTTGAGATCCTTTTTTTCTGCGCGTAATCTGCTGCTTGCAAACAAAA
AAACCACCGCTACCAGCGG
TGGTTTGTTTGCCGGATCAAGAGCTACCAACTCTTTTTCCGAAGGTAACTGGCTTC
AGCAGAGCGCAGATACCAA
ATACTGTTCTTCTAGTGTAGCCGTAGTTAGGCCACCACTTCAAGAACTCTGTAGCA
CCGCCTACATACCTCGCTC
TGCTAATCCTGTTACCAGTGGCTGCTGCCAGTGGCGATAAGTCGTGTCTTACCGGG
TTGGACTCAAGACGATAGT
TACCGGATAAGGCGCAGCGGTCGGGCTGAACGGGGGGTTCGTGCACACAGCCCA
GCTTGGAGCGAACGACCTACA
CCGAACTGAGATACCTACAGCGTGAGCTATGAGAAAGCGCCACGCTTCCCGAAGG
GAGAAAGGCGGACAGGTATC
CGGTAAGCGGCAGGGTCGGAACAGGAGAGCGCACGAGGGAGCTTCCAGGGGGAA
ACGCCTGGTATCTTTATAGTC
CTGTCGGGTTTCGCCACCTCTGACTTGAGCGTCGATTTTTGTGATGCTCGTCAGGG
GGGCGGAGCCTATGGAAAA

ACGCCAGCAACGCGGCCTTTTTACGGTTCCTGGCCTTTTGCTGGCCTTTTGCTCAC

ATGTTCTTTCCTGCGTTAT

CCCCTGATTCTGTGGATAACCGTATTACCGCCTTTGAGTGAGCTGATACCGCTCGC

CGCAGCCGAACGACCGAGC

GCAGCGAGTCAGTGAGCGAGGAAGCGGAAG

SEQ ID NO:49 is the sequence for OV 1165

CAGGGTAATCATCATCAATAATATACCTTATTTTGGATTGAA

GCCAATATGATAATGAGG

GGGTGGAGTTTGTGACGTGGCGCGGGGCGTGGGAACGGGGC

GGGTGACGTAGTAGTGTGG

CGGAAGTGTGATGTTGCAAGTGTGGCGGAACACATGTAAGC

GACGGATGTGGCAAAAGTG

ACGTTTTTGGTGTGCGCCGGTGTACACAGGAAGTGACAATTT

TCGCGCGGTTTTAGGCGG

ATGTTGTAGTAAATTTGGGCGTAACCGAGTAAGATTTGGCCA

TTTTCGCGGGAAAACTGA

ATAAGAGGAAGTGAAATCTGAATAATTTTGTGTTACTCATAG

CGCGTAATATTTGTCTAG

GGCCGGGATCTCTGCAGGAATTTGATATCAAGCTTATCGATA

CCGTCGAAACTTGTTTAT

TGCAGCTTATAATGGTTACAAATAAAGCAATAGCATCACAAA

TTTCACAAATAAAGCATT

TTTTTCACTGCATTCTAGTTGTGGTTTGTCCAAACTCATCAAT

GTATCTTATCATGTCTG

GATCGATCCGCTAGCGGCGCGCCGTTTCATCCGGACAAAGCC TGCGCGCGCCCCGCCCCG

CCATTGGCCGTACCGCCCCGCGCCGCCGCCCCATCTCGCCCC TCGCCGCCGGGTCCGGCG

CGTTAAAGCCAATAGGAACCGCCGCCGTTGTTCCCGTCACGG CCGGGGCAGCCAATTGTG

GCGGCGCTCGGCGGCTCGTGGCTCTTTCGCGGCAAAAAGGAT TTGGCGCGTAAAAGTGGC

CGGGACTTTGCAGGCAGCGGCGGCCGGGGGCGGAGCGGGAT CGAGCCCTCGATGATATCA

GATCAAACGATATCACCGGTCGACTGAAAATGAGACATATTA TCTGCCACGGAGGTGTTA

TTACCGAAGAAATGGCCGCCAGTCTTTTGGACCAGCTGATCG AAGAGGTACTGGCTGATA

ATCTTCCACCTCCTAGCCATTTTGAACCACCTACCCTTCACGA ACTGTATGATTTAGACG

TGACGGCCCCCGAAGATCCCAACGAGGAGGCGGTTTCGCAG ATTTTTCCCGACTCTGTAA

TGTTGGCGGTGCAGGAAGGGATTGACTTACTCACTTTTCCGC CGGCGCCCGGTTCTCCGG

AGCCGCCTCACCTTTCCCGGCAGCCCGAGCAGCCGGAGCAGA GAGCCTTGGGTCCGGTTT

CTATGCCAAACCTTGTACCGGAGGTGATCGATCTTACCTGCC ACGAGGCTGGCTTTCCAC

CCAGTGACGACGAGGATGAAGAGGGTGAGGAGTTTGTGTTA
GATTATGTGGAGCACCCCG

GGCACGGTTGCAGGTCTTGTCATTATCACCGGAGGAATACGG
GGGACCCAGATATTATGT

GTTCGCTTTGCTATATGAGGACCTGTGGCATGTTTGTCTACAG
TAAGTGAAAATTATGGG

CAGTGGGTGATAGAGTGGTGGGTTTGGTGTGGTAATTTTTTT
TTAATTTTTACAGTTTT

GTGGTTTAAAGAATTTTGTATTGTGATTTTTTTAAAAGGTCCT
GTGTCTGAACCTGAGCC

TGAGCCCGAGCCAGAACCGGAGCCTGCAAGACCTACCCGCC
GTCCTAAAATGGCGCCTGC

TATCCTGAGACGCCCGACATCACCTGTGTCTAGAGAATGCAA
TAGTAGTACGGATAGCTG

TGACTCCGGTCCTTCTAACACACCTCCTGAGATACACCCGGT
GGTCCCGCTGTGCCCCAT

TAAACCAGTTGCCGTGAGAGTTGGTGGGCGTCGCCAGGCTGT
GGAATGTATCGAGGACTT

GCTTAACGAGCCTGGGCAACCTTTGGACTTGAGCTGTAAACG
CCCCAGGCCATAAGGTGT

AAACCTGTGATTGCGTGTGTGGTTAACGCCTTTGTTTGCTGAA
TGAGTTGATGTAAGTTT

AATAAAGGGTGAGATAATGTTTAACTTGCATGGCGTGTTAAA
TGGGGCGGGGCTTAAAGG

GTATATAATGCGCCGTGGGCTAATCTTGGTTACATCTGACCTC
ATGGAGGCTTGGGAGTG

TTTGGAAGATTTTTCTGCTGTGCGTAACTTGCTGGAACAGAG
CTCTAACAGTACCTCTTG

GTTTTGGAGGTTTCTGTGGGGCTCATCCCAGGCAAAGTTAGT
CTGCAGAATTAAGGAGGA

TTACAAGTGGGAATTTGAAGAGCTTTTGAAATCCTGTGGTGA
GCTGTTTGATTCTTTGAA

TCTGGGTCACCAGGCGCTTTTCCAAGAGAAGGTCATCAAGAC
TTTGGATTTTTCCACACC

GGGGCGCGCTGCGGCTGCTGTTGCTTTTTGAGTTTTATAAAG
GATAAATGGAGCGAAGA

AACCCATCTGAGCGGGGGGTACCTGCTGGATTTTCTGGCCAT
GCATCTGTGGAGAGCGGT

TGTGAGACACAAGAATCGCCTGCTACTGTTGTCTTCCGTCCG
CCCGGCGATAATACCGAC

GGAGGAGCAGCAGCAGCAGCAGGAGGAAGCCAGGCGGCGG
CGGCAGGAGCAGAGCCCATG

GAACCCGAGAGCCGGCCTGGACCCTCGGGAATGAATGTTGTA
CAGGTGGCTGAACTGTAT

CCAGAACTGAGACGCATTTTGACAATTACAGAGGATGGGCA
GGGGCTAAAGGGGGTAAAG

AGGGAGCGGGGGGCTTGTGAGGCTACAGAGGAGGCTAGGAA
TCTAGCTTTTAGCTTAATG

ACCAGACACCGTCCTGAGTGTATTACTTTTCAACAGATCAAG GATAATTGCGCTAATGAG

CTTGATCTGCTGGCGCAGAAGTATTCCATAGAGCAGCTGACC ACTTACTGGCTGCAGCCA

GGGGATGATTTTGAGGAGGCTATTAGGGTATATGCAAAGGTG GCACTTAGGCCAGATTGC

AAGTACAAGATCAGCAAACTTGTAAATATCAGGAATTGTTGC TACATTTCTGGGAACGGG

GCCGAGGTGGAGATAGATACGGAGGATAGGGTGGCCTTTAG ATGTAGCATGATAAATATG

TGGCCGGGGGTGCTTGGCATGGACGGGGTGGTTATTATGAAT GTAAGGTTTACTGGCCCC

AATTTTAGCGGTACGGTTTTCCTGGCCAATACCAACCTTATCC TACACGGTGTAAGCTTC

TATGGGTTTAACAATACCTGTGTGGAAGCCTGGACCGATGTA AGGGTTCGGGGCTGTGCC

TTTTACTGCTGCTGGAAGGGGGTGGTGTGTCGCCCCAAAAGC AGGGCTTCAATTAAGAAA

TGCCTCTTTGAAAGGTGTACCTTGGGTATCCTGTCTGAGGGTA ACTCCAGGGTGCGCCAC

AATGTGGCCTCCGACTGTGGTTGCTTCATGCTAGTGAAAAGC GTGGCTGTGATTAAGCAT

AACATGGTATGTGGCAACTGCGAGGACAGGGCCTCTCAGATG CTGACCTGCTCGGACGGC

AACTGTCACCTGCTGAAGACCATTCACGTAGCCAGCCACTCT

CGCAAGGCCTGGCCAGTG

TTTGAGCATAACATACTGACCCGCTGTTCCTTGCATTTGGGTA

ACAGGAGGGGGGTGTTC

CTACCTTACCAATGCAATTTGAGTCACACTAAGATATTGCTTG

AGCCCGAGAGCATGTCC

AAGGTGAACCTGAACGGGGTGTTTGACATGACCATGAAGATC

TGGAAGGTGCTGAGGTAC

GATGAGACCCGCACCAGGTGCAGACCCTGCGAGTGTGGCGG

TAAACATATTAGGAACCAG

CCTGTGATGCTGGATGTGACCGAGGAGCTGAGGCCCGATCAC

TTGGTGCTGGCCTGCACC

CGCGCTGAGTTTGGCTCTAGCGATGAAGATACAGATTGAGGT

ACTGAAATGTGTGGGCGT

GGCTTAAGGGTGGGAAAGAATATATAAGGTGGGGGTCTTAT

GTAGTTTTGTATCTGTTTT

GCAGCAGCCGCCGCCGCCATGAGCACCAACTCGTTTGATGGA

AGCATTGTGAGCTCATAT

TTGACAACGCGCATGCCCCCATGGGCCGGGGTGCGTCAGAAT

GTGATGGGCTCCAGCATT

GATGGTCGCCCCGTCCTGCCCGCAAACTCTACTACCTTGACCT

ACGAGACCGTGTCTGGA

ACGCCGTTGGAGACTGCAGCCTCCGCCGCCGCTTCAGCCGCT

GCAGCCACCGCCCGCGGG

ATTGTGACTGACTTTGCTTTCCTGAGCCCGCTTGCAAGCAGTG
CAGCTTCCCGTTCATCC

GCCCGCGATGACAAGTTGACGGCTCTTTTGGCACAATTGGAT
TCTTTGACCCGGGAACTT

AATGTCGTTTCTCAGCAGCTGTTGGATCTGCGCCAGCAGGTTT
CTGCCCTGAAGGCTTCC

TCCCCTCCCAATGCGGTTTAAAACATAAATAAAAAACCAGAC
TCTGTTTGGATTTGGATC

AAGCAAGTGTCTTGCTGTCTTTATTTAGGGGTTTTGCGCGCGC
GGTAGGCCCGGGACCAG

CGGTCTCGGTCGTTGAGGGTCCTGTGTATTTTTTCCAGGACGT
GGTAAAGGTGACTCTGG

ATGTTCAGATACATGGGCATAAGCCCGTCTCTGGGGTGGAGG
TAGCACCACTGCAGAGCT

TCATGCTGCGGGGTGGTGTTGTAGATGATCCAGTCGTAGCAG
GAGCGCTGGGCGTGGTGC

CTAAAAATGTCTTTCAGTAGCAAGCTGATTGCCAGGGGCAGG
CCCTTGGTGTAAGTGTTT

ACAAAGCGGTTAAGCTGGGATGGGTGCATACGTGGGGATAT
GAGATGCATCTTGGACTGT

ATTTTTAGGTTGGCTATGTTCCCAGCCATATCCCTCCGGGGAT
TCATGTTGTGCAGAACC

ACCAGCACAGTGTATCCGGTGCACTTGGGAAATTTGTCATGT
AGCTTAGAAGGAAATGCG

TGGAAGAACTTGGAGACGCCCTTGTGACCTCCAAGATTTTCC

ATGCATTCGTCCATAATG

ATGGCAATGGGCCCACGGGCGGCGGCCTGGGCGAAGATATT

TCTGGGATCACTAACGTCA

TAGTTGTGTTCCAGGATGAGATCGTCATAGGCCATTTTTACA

AAGCGCGGGCGGAGGGTG

CCAGACTGCGGTATAATGGTTCCATCCGGCCCAGGGGCGTAG

TTACCCTCACAGATTTGC

ATTTCCCACGCTTTGAGTTCAGATGGGGGGATCATGTCTACCT

GCGGGGCGATGAAGAAA

ACGGTTTCCGGGGTAGGGGAGATCAGCTGGGAAGAAAGCAG

GTTCCTGAGCAGCTGCGAC

TTACCGCAGCCGGTGGGCCCGTAAATCACACCTATTACCGGG

TGCAACTGGTAGTTAAGA

GAGCTGCAGCTGCCGTCATCCCTGAGCAGGGGGGCCACTTCG

TTAAGCATGTCCCTGACT

CGCATGTTTTCCCTGACCAAATCCGCCAGAAGGCGCTCGCCG

CCCAGCGATAGCAGTTCT

TGCAAGGAAGCAAAGTTTTTCAACGGTTTGAGACCGTCCGCC

GTAGGCATGCTTTTGAGC

GTTTGACCAAGCAGTTCCAGGCGGTCCCACAGCTCGGTCACC

TGCTCTACGGCATCTCGA

TCCAGCATATCTCCTCGTTTCGCGGGTTGGGGCGGCTTTCGCT

GTACGGCAGTAGTCGGT

GCTCGTCCAGACGGGCCAGGGTCATGTCTTTCCACGGGCGCA

GGGTCCTCGTCAGCGTAG

TCTGGGTCACGGTGAAGGGGTGCGCTCCGGGCTGCGCGCTGG

CCAGGGTGCGCTTGAGGC

TGGTCCTGCTGGTGCTGAAGCGCTGCCGGTCTTCGCCCTGCG

CGTCGGCCAGGTAGCATT

TGACCATGGTGTCATAGTCCAGCCCCTCCGCGGCGTGGCCCT

TGGCGCGCAGCTTGCCCT

TGGAGGAGGCGCCGCACGAGGGGCAGTGCAGACTTTTGAGG

GCGTAGAGCTTGGGCGCGA

GAAATACCGATTCCGGGGAGTAGGCATCCGCGCCGCAGGCC

CCGCAGACGGTCTCGCATT

CCACGAGCCAGGTGAGCTCTGGCCGTTCGGGGTCAAAAACCA

GGTTTCCCCCATGCTTTT

TGATGCGTTTCTTACCTCTGGTTTCCATGAGCCGGTGTCCACG

CTCGGTGACGAAAAGGC

TGTCCGTGTCCCCGTATACAGACTTGAGAGGCCTGTCCTCGA

GCGGTGTTCCGCGGTCCT

CCTCGTATAGAAACTCGGACCACTCTGAGACAAAGGCTCGCG

TCCAGGCCAGCACGAAGG

AGGCTAAGTGGGAGGGGTAGCGGTCGTTGTCCACTAGGGGG

TCCACTCGCTCCAGGGTGT

GAAGACACATGTCGCCCTCTTCGGCATCAAGGAAGGTGATTG

GTTTGTAGGTGTAGGCCA

CGTGACCGGGTGTTCCTGAAGGGGGGCTATAAAAGGGGGTG
GGGGCGCGTTCGTCCTCAC

TCTCTTCCGCATCGCTGTCTGCGAGGGCCAGCTGTTGGGGTG
AGTACTCCCTCTGAAAAG

CGGGCATGACTTCTGCGCTAAGATTGTCAGTTTCCAAAAACG
AGGAGGATTTGATATTCA

CCTGGCCCGCGGTGATGCCTTTGAGGGTGGCCGCATCCATCT
GGTCAGAAAAGACAATCT

TTTTGTTGTCAAGCTTGGTGGCAAACGACCCGTAGAGGGCGT
TGGACAGCAACTTGGCGA

TGGAGCGCAGGGTTTGGTTTTTGTCGCGATCGGCGCGCTCCTT
GGCCGCGATGTTTAGCT

GCACGTATTCGCGCGCAACGCACCGCCATTCGGGAAAGACG
GTGGTGCGCTCGTCGGGCA

CCAGGTGCACGCGCCAACCGCGGTTGTGCAGGGTGACAAGG
TCAACGCTGGTGGCTACCT

CTCCGCGTAGGCGCTCGTTGGTCCAGCAGAGGCGGCCGCCCT
TGCGCGAGCAGAATGGCG

GTAGGGGGTCTAGCTGCGTCTCGTCCGGGGGGTCTGCGTCCA
CGGTAAAGACCCCGGGCA

GCAGGCGCGCGTCGAAGTAGTCTATCTTGCATCCTTGCAAGT
CTAGCGCCTGCTGCCATG

CGCGGGCGGCAAGCGCGCGCTCGTATGGGTTGAGTGGGGGA
CCCCATGGCATGGGGTGGG

TGAGCGCGGAGGCGTACATGCCGCAAATGTCGTAAACGTAG AGGGGCTCTCTGAGTATTC

CAAGATATGTAGGGTAGCATCTTCCACCGCGGATGCTGGCGC GCACGTAATCGTATAGTT

CGTGCGAGGGAGCGAGGAGGTCGGGACCGAGGTTGCTACGG GCGGGCTGCTCTGCTCGGA

AGACTATCTGCCTGAAGATGGCATGTGAGTTGGATGATATGG TTGGACGCTGGAAGACGT

TGAAGCTGGCGTCTGTGAGACCTACCGCGTCACGCACGAAGG AGGCGTAGGAGTCGCGCA

GCTTGTTGACCAGCTCGGCGGTGACCTGCACGTCTAGGGCGC AGTAGTCCAGGGTTTCCT

TGATGATGTCATACTTATCCTGTCCCTTTTTTTCCACAGCTC GCGGTTGAGGACAAACT

CTTCGCGGTCTTTCCAGTACTCTTGGATCGGAAACCCGTCGGC CTCCGAACGGTAAGAGC

CTAGCATGTAGAACTGGTTGACGGCCTGGTAGGCGCAGCATC CCTTTTCTACGGGTAGCG

CGTATGCCTGCGCGGCCTTCCGGAGCGAGGTGTGGGTGAGCG CAAAGGTGTCCCTGACCA

TGACTTTGAGGTACTGGTATTTGAAGTCAGTGTCGTCGCATCC GCCCTGCTCCCAGAGCA

AAAAGTCCGTGCGCTTTTTGGAACGCGGATTTGGCAGGGCGA AGGTGACATCGTTGAAGA

GTATCTTTCCCGCGCGAGGCATAAAGTTGCGTGTGATGCGGA

AGGGTCCCGGCACCTCGG

AACGGTTGTTAATTACCTGGGCGGCGAGCACGATCTCGTCAA

AGCCGTTGATGTTGTGGC

CCACAATGTAAAGTTCCAAGAAGCGCGGGATGCCCTTGATGG

AAGGCAATTTTTTAAGTT

CCTCGTAGGTGAGCTCTTCAGGGGAGCTGAGCCCGTGCTCTG

AAAGGGCCCAGTCTGCAA

GATGAGGGTTGGAAGCGACGAATGAGCTCCACAGGTCACGG

GCCATTAGCATTTGCAGGT

GGTCGCGAAAGGTCCTAAACTGGCGACCTATGGCCATTTTTT

CTGGGGTGATGCAGTAGA

AGGTAAGCGGGTCTTGTTCCCAGCGGTCCCATCCAAGGTTCG

CGGCTAGGTCTCGCGCGG

CAGTCACTAGAGGCTCATCTCCGCCGAACTTCATGACCAGCA

TGAAGGGCACGAGCTGCT

TCCCAAAGGCCCCCATCCAAGTATAGGTCTCTACATCGTAGG

TGACAAAGAGACGCTCGG

TGCGAGGATGCGAGCCGATCGGGAAGAACTGGATCTCCCGC

CACCAATTGGAGGAGTGGC

TATTGATGTGGTGAAAGTAGAAGTCCCTGCGACGGGCCGAAC

ACTCGTGCTGGCTTTTGT

AAAAACGTGCGCAGTACTGGCAGCGGTGCACGGGCTGTACA

TCCTGCACGAGGTTGACCT

GACGACCGCGCACAAGGAAGCAGAGTGGGAATTTGAGCCCC TCGCCTGGCGGGTTTGGCT

GGTGGTCTTCTACTTCGGCTGCTTGTCCTTGACCGTCTGGCTG CTCGAGGGGAGTTACGG

TGGATCGGACCACCACGCCGCGCGAGCCCAAAGTCCAGATGT CCGCGCGCGGCGGTCGGA

GCTTGATGACAACATCGCGCAGATGGGAGCTGTCCATGGTCT GGAGCTCCCGCGGCGTCA

GGTCAGGCGGGAGCTCCTGCAGGTTTACCTCGCATAGACGGG TCAGGGCGCGGGCTAGAT

CCAGGTGATACCTAATTTCCAGGGGCTGGTTGGTGGCGGCGT CGATGGCTTGCAAGAGGC

CGCATCCCCGCGGCGCGACTACGGTACCGCGCGGCGGGCGGT GGGCCGCGGGGGTGTCCT

TGGATGATGCATCTAAAAGCGGTGACGCGGGCGAGCCCCCG GAGGTAGGGGGGGCTCCGG

ACCCGCCGGGAGAGGGGGCAGGGGCACGTCGGCGCCGCGCG CGGGCAGGAGCTGGTGCTG

CGCGCGTAGGTTGCTGGCGAACGCGACGACGCGGCGGTTGAT CTCCTGAATCTGGCGCCT

CTGCGTGAAGACGACGGGCCCGGTGAGCTTGAGCCTGAAAG AGAGTTCGACAGAATCAAT

TTCGGTGTCGTTGACGGCGGCCTGGCGCAAAATCTCCTGCAC GTCTCCTGAGTTGTCTTG

ATAGGCGATCTCGGCCATGAACTGCTCGATCTCTTCCTCCTGGAGATCTCCGCGTC

CGGC

TCGCTCCACGGTGGCGGCGAGGTCGTTGGAAATGCGGGCCATGAGCTGCGAGAA

GGCGTT

GAGGCCTCCCTCGTTCCAGACGCGGCTGTAGACCACGCCCCCTTCGGCATCGCGG

GCGCG

CATGACCACCTGCGCGAGATTGAGCTCCACGTGCCGGGCGAAGACGGCGTAGTTT

CGCAG

GCGCTGAAAGAGGTAGTTGAGGGTGGTGGCGGTGTGTTCTGCCACGAAGAAGTAC

ATAAC

CCAGCGTCGCAACGTGGATTCGTTGATATCCCCCAAGGCCTCAAGGCGCTCCATG

GCCTC

GTAGAAGTCCACGGCGAAGTTGAAAAACTGGGAGTTGCGCGCCGACACGGTTAA

CTCCTC

CTCCAGAAGACGGATGAGCTCGGCGACAGTGTCGCGCACCTCGCGCTCAAAGGCT

ACAGG

GGCCTCTTCTTCTTCTTCAATCTCCTCTTCCATAAGGGCCTCCCCTTCTTCTTCTTCT

GG

CGGCGGTGGGGGAGGGGGGACACGGCGGCGACGACGGCGCACCGGGAGGCGGT

CGACAAA

GCGCTCGATCATCTCCCCGCGGCGACGGCGCATGGTCTCGGTGACGGCGCGGCCG

TTCTC

GCGGGGGCGCAGTTGGAAGACGCCGCCCGTCATGTCCCGGTTATGGGTTGGCGGG

GGGCT

GCCATGCGGCAGGGATACGGCGCTAACGATGCATCTCAACAATTGTTGTGTAGGT ACTCC

GCCGCCGAGGGACCTGAGCGAGTCCGCATCGACCGGATCGGAAAACCTCTCGAG AAAGGC

GTCTAACCAGTCACAGTCGCAAGGTAGGCTGAGCACCGTGGCGGGCGGCAGCGG GCGGCG

GTCGGGGTTGTTTCTGGCGGAGGTGCTGCTGATGATGTAATTAAAGTAGGCGGTC TTGAG

ACGGCGGATGGTCGACAGAAGCACCATGTCCTTGGGTCCGGCCTGCTGAATGCGC AGGCG

GTCGGCCATGCCCCAGGCTTCGTTTTGACATCGGCGCAGGTCTTTGTAGTAGTCTT GCAT

GAGCCTTTCTACCGGCACTTCTTCTTCTCCTTCCTCTTGTCCTGCATCTCTTGCATC TAT

CGCTGCGGCGGCGGCGGAGTTTGGCCGTAGGTGGCGCCCTCTTCCTCCCATGCGT GTGAC

CCCGAAGCCCCTCATCGGCTGAAGCAGGGCTAGGTCGGCGACAACGCGCTCGGCT AATAT

GGCCTGCTGCACCTGCGTGAGGGTAGACTGGAAGTCATCCATGTCCACAAAGCGG TGGTA

TGCGCCCGTGTTGATGGTGTAAGTGCAGTTGGCCATAACGGACCAGTTAACGGTC TGGTG

ACCCGGCTGCGAGAGCTCGGTGTACCTGAGACGCGAGTAAGCCCTCGAGTCAAAT ACGTA

GTCGTTGCAAGTCCGCACCAGGTACTGGTATCCCACCAAAAAGTGCGGCGGCGGC

TGGCG

GTAGAGGGGCCAGCGTAGGGTGGCCGGGGCTCCGGGGGCGAGATCTTCCAACAT

AAGGCG

ATGATATCCGTAGATGTACCTGGACATCCAGGTGATGCCGGCGGCGGTGGTGGAG

GCGCG

CGGAAAGTCGCGGACGCGGTTCCAGATGTTGCGCAGCGGCAAAAAGTGCTCCATG

GTCGG

GACGCTCTGGCCGGTCAGGCGCGCGCAATCGTTGACGCTCTAGACCGTGCAAAAG

GAGAG

CCTGTAAGCGGGCACTCTTCCGTGGTCTGGTGGATAAATTCGCAAGGGTATCATG

GCGGA

CGACCGGGGTTCGAGCCCCGTATCCGGCCGTCCGCCGTGATCCATGCGGTTACCG

CCCGC

GTGTCGAACCCAGGTGTGCGACGTCAGACAACGGGGGAGTGCTCCTTTTGGCTTC

CTTCC

AGGCGCGGCGGCTGCTGCGCTAGCTTTTTTGGCCACTGGCCGCGCGCAGCGTAAG

CGGTT

AGGCTGGAAAGCGAAAGCATTAAGTGGCTCGCTCCCTGTAGCCGGAGGGTTATTT

TCCAA

GGGTTGAGTCGCGGGACCCCCGGTTCGAGTCTCGGACCGGCCGGACTGCGGCGAA

CGGGG

GTTTGCCTCCCCGTCATGCAAGACCCCGCTTGCAAATTCCTCCGGAAACAGGGAC

GAGCC

CCTTTTTTGCTTTTCCCAGATGCATCCGGTGCTGCGGCAGATGCGCCCCCCTCCTC AGCA

GCGGCAAGAGCAAGAGCAGCGGCAGACATGCAGGGCACCCTCCCCTCCTCCTACC GCGTC

AGGAGGGGCGACATCCGCGGTTGACGCGGCAGCAGATGGTGATTACGAACCCCC GCGGCG

CCGGGCCCGGCACTACCTGGACTTGGAGGAGGGCGAGGGCCTGGCGCGGCTAGG AGCGCC

CTCTCCTGAGCGGTACCCAAGGGTGCAGCTGAAGCGTGATACGCGTGAGGCGTAC GTGCC

GCGGCAGAACCTGTTTCGCGACCGCGAGGGAGAGGAGCCCGAGGAGATGCGGGA TCGAAA

GTTCCACGCAGGGCGCGAGCTGCGGCATGGCCTGAATCGCGAGCGGTTGCTGCGC GAGGA

GGACTTTGAGCCCGACGCGCGAACCGGGATTAGTCCCGCGCGCGCACACGTGGCG GCCGC

CGACCTGGTAACCGCATACGAGCAGACGGTGAACCAGGAGATTAACTTTCAAAA AAGCTT

TAACAACCACGTGCGTACGCTTGTGGCGCGCGAGGAGGTGGCTATAGGACTGATG CATCT

GTGGGACTTTGTAAGCGCGCTGGAGCAAAACCCAAATAGCAAGCCGCTCATGGCG CAGCT

GTTCCTTATAGTGCAGCACAGCAGGGACAACGAGGCATTCAGGGATGCGCTGCTA AACAT

AGTAGAGCCCGAGGGCCGCTGGCTGCTCGATTTGATAAACATCCTGCAGAGCATA

GTGGT

GCAGGAGCGCAGCTTGAGCCTGGCTGACAAGGTGGCCGCCATCAACTATTCCATG

CTTAG

CCTGGGCAAGTTTTACGCCCGCAAGATATACCATACCCCTTACGTTCCCATAGACA

AGGA

GGTAAAGATCGAGGGGTTCTACATGCGCATGGCGCTGAAGGTGCTTACCTTGAGC

GACGA

CCTGGGCGTTTATCGCAACGAGCGCATCCACAAGGCCGTGAGCGTGAGCCGGCGG

CGCGA

GCTCAGCGACCGCGAGCTGATGCACAGCCTGCAAAGGGCCCTGGCTGGCACGGG

CAGCGG

CGATAGAGAGGCCGAGTCCTACTTTGACGCGGGCGCTGACCTGCGCTGGGCCCCA

AGCCG

ACGCGCCCTGGAGGCAGCTGGGGCCGGACCTGGGCTGGCGGTGGCACCCGCGCG

CGCTGG

CAACGTCGGCGGCGTGGAGGAATATGACGAGGACGATGAGTACGAGCCAGAGGA

CGGCGA

GTACTAAGCGGTGATGTTTCTGATCAGATGATGCAAGACGCAACGGACCCGGCGG

TGCGG

GCGGCGCTGCAGAGCCAGCCGTCCGGCCTTAACTCCACGGACGACTGGCGCCAGG

TCATG

GACCGCATCATGTCGCTGACTGCGCGCAATCCTGACGCGTTCCGGCAGCAGCCGC

AGGCC

AACCGGCTCTCCGCAATTCTGGAAGCGGTGGTCCCGGCGCGCGCAAACCCCACGC
ACGAG

AAGGTGCTGGCGATCGTAAACGCGCTGGCCGAAAACAGGGCCATCCGGCCCGAC
GAGGCC

GGCCTGGTCTACGACGCGCTGCTTCAGCGCGTGGCTCGTTACAACAGCGGCAACG
TGCAG

ACCAACCTGGACCGGCTGGTGGGGGATGTGCGCGAGGCCGTGGCGCAGCGTGAG
CGCGCG

CAGCAGCAGGGCAACCTGGGCTCCATGGTTGCACTAAACGCCTTCCTGAGTACAC
AGCCC

GCCAACGTGCCGCGGGGACAGGAGGACTACACCAACTTTGTGAGCGCACTGCGG
CTAATG

GTGACTGAGACACCGCAAAGTGAGGTGTACCAGTCTGGGCCAGACTATTTTTTCC
AGACC

AGTAGACAAGGCCTGCAGACCGTAAACCTGAGCCAGGCTTTCAAAAACTTGCAGG
GGCTG

TGGGGGGTGCGGGCTCCCACAGGCGACCGCGCGACCGTGTCTAGCTTGCTGACGC
CCAAC

TCGCGCCTGTTGCTGCTGCTAATAGCGCCCTTCACGGACAGTGGCAGCGTGTCCCG
GGAC

ACATACCTAGGTCACTTGCTGACACTGTACCGCGAGGCCATAGGTCAGGCGCATG
TGGAC

GAGCATACTTTCCAGGAGATTACAAGTGTCAGCCGCGCGCTGGGGCAGGAGGAC
ACGGGC

AGCCTGGAGGCAACCCTAAACTACCTGCTGACCAACCGGCGGCAGAAGATCCCCT

CGTTG

CACAGTTTAAACAGCGAGGAGGAGCGCATTTTGCGCTACGTGCAGCAGAGCGTGA

GCCTT

AACCTGATGCGCGACGGGGTAACGCCCAGCGTGGCGCTGGACATGACCGCGCGC

AACATG

GAACCGGGCATGTATGCCTCAAACCGGCCGTTTATCAACCGCCTAATGGACTACT

TGCAT

CGCGCGGCCGCCGTGAACCCCGAGTATTTCACCAATGCCATCTTGAACCCGCACT

GGCTA

CCGCCCCCTGGTTTCTACACCGGGGGATTCGAGGTGCCCGAGGGTAACGATGGAT

TCCTC

TGGGACGACATAGACGACAGCGTGTTTTCCCCGCAACCGCAGACCCTGCTAGAGT

TGCAA

CAGCGCGAGCAGGCAGAGGCGGCGCTGCGAAAGGAAAGCTTCCGCAGGCCAAGC

AGCTTG

TCCGATCTAGGCGCTGCGGCCCCGCGGTCAGATGCTAGTAGCCCATTTCCAAGCTT

GATA

GGGTCTCTTACCAGCACTCGCACCACCCGCCCGCGCCTGCTGGGCGAGGAGGAGT

ACCTA

AACAACTCGCTGCTGCAGCCGCAGCGCGAAAAAAACCTGCCTCCGGCATTTCCCA

ACAAC

GGGATAGAGAGCCTAGTGGACAAGATGAGTAGATGGAAGACGTACGCGCAGGAG

CACAGG

GACGTGCCAGGCCCGCGCCCGCCCACCCGTCGTCAAAGGCACGACCGTCAGCGGG

GTCTG

GTGTGGGAGGACGATGACTCGGCAGACGACAGCAGCGTCCTGGATTTGGGAGGG

AGTGGC

AACCCGTTTGCGCACCTTCGCCCCAGGCTGGGGAGAATGTTTTAAAAAAAAAAAA

GCATG

ATGCAAAATAAAAAACTCACCAAGGCCATGGCACCGAGCGTTGGTTTTCTTGTAT

TCCCC

TTAGTATGCGGCGCGCGGCGATGTATGAGGAAGGTCCTCCTCCCTCCTACGAGAG

TGTGG

TGAGCGCGGCGCCAGTGGCGGCGGCGCTGGGTTCTCCCTTCGATGCTCCCCTGGA

CCCGC

CGTTTGTGCCTCCGCGGTACCTGCGGCCTACCGGGGGGAGAAACAGCATCCGTTA

CTCTG

AGTTGGCACCCCTATTCGACACCACCCGTGTGTACCTGGTGGACAACAAGTCAAC

GGATG

TGGCATCCCTGAACTACCAGAACGACCACAGCAACTTTCTGACCACGGTCATTCA

AAACA

ATGACTACAGCCCGGGGGAGGCAAGCACACAGACCATCAATCTTGACGACCGGT

CGCACT

GGGGCGGCGACCTGAAAACCATCCTGCATACCAACATGCCAAATGTGAACGAGTT

CATGT

TTACCAATAAGTTTAAGGCGCGGGTGATGGTGTCGCGCTTGCCTACTAAGGACAA

TCAGG

TGGAGCTGAAATACGAGTGGGTGGAGTTCACGCTGCCCGAGGGCAACTACTCCGA
GACCA

TGACCATAGACCTTATGAACAACGCGATCGTGGAGCACTACTTGAAAGTGGGCAG
ACAGA

ACGGGGTTCTGGAAAGCGACATCGGGGTAAAGTTTGACACCCGCAACTTCAGACT
GGGGT

TTGACCCCGTCACTGGTCTTGTCATGCCTGGGGTATATACAAACGAAGCCTTCCAT
CCAG

ACATCATTTTGCTGCCAGGATGCGGGGTGGACTTCACCCACAGCCGCCTGAGCAA
CTTGT

TGGGCATCCGCAAGCGGCAACCCTTCCAGGAGGGCTTTAGGATCACCTACGATGA
TCTGG

AGGGTGGTAACATTCCCGCACTGTTGGATGTGGACGCCTACCAGGCGAGCTTGAA
AGATG

ACACCGAACAGGGCGGGGGTGGCGCAGGCGGCAGCAACAGCAGTGGCAGCGGCG
CGGAAG

AGAACTCCAACGCGGCAGCCGCGGCAATGCAGCCGGTGGAGGACATGAACGATC
ATGCCA

TTCGCGGCGACACCTTTGCCACACGGGCTGAGGAGAAGCGCGCTGAGGCCGAAG
CAGCGG

CCGAAGCTGCCGCCCCCGCTGCGCAACCCGAGGTCGAGAAGCCTCAGAAGAAAC
CGGTGA

TCAAACCCCTGACAGAGGACAGCAAGAAACGCAGTTACAACCTAATAAGCAATG
ACAGCA

CCTTCACCCAGTACCGCAGCTGGTACCTTGCATACAACTACGGCGACCCTCAGAC
CGGAA
TCCGCTCATGGACCCTGCTTTGCACTCCTGACGTAACCTGCGGCTCGGAGCAGGTC
TACT
GGTCGTTGCCAGACATGATGCAAGACCCCGTGACCTTCCGCTCCACGCGCCAGAT
CAGCA
ACTTTCCGGTGGTGGGCGCCGAGCTGTTGCCCGTGCACTCCAAGAGCTTCTACAA
CGACC
AGGCCGTCTACTCCCAACTCATCCGCCAGTTTACCTCTCTGACCCACGTGTTCAAT
CGCT
TTCCCGAGAACCAGATTTTGGCGCGCCCGCCAGCCCCCACCATCACCACCGTCAG
TGAAA
ACGTTCCTGCTCTCACAGATCACGGGACGCTACCGCTGCGCAACAGCATCGGAGG
AGTCC
AGCGAGTGACCATTACTGACGCCAGACGCCGCACCTGCCCCTACGTTTACAAGGC
CCTGG
GCATAGTCTCGCCGCGCGTCCTATCGAGCCGCACTTTTTGAGCAAGCATGTCCATC
CTTA
TATCGCCCAGCAATAACACAGGCTGGGGCCTGCGCTTCCCAAGCAAGATGTTTGG
CGGGG
CCAAGAAGCGCTCCGACCAACACCCAGTGCGCGTGCGCGGGCACTACCGCGCGCC
CTGGG
GCGCGCACAAACGCGGCCGCACTGGGCGCACCACCGTCGATGACGCCATCGACG
CGGTGG

TGGAGGAGGCGCGCAACTACACGCCCACGCCGCCACCAGTGTCCACAGTGGACG

CGGCCA

TTCAGACCGTGGTGCGCGGAGCCCGGCGCTATGCTAAAATGAAGAGACGGCGGA

GGCGCG

TAGCACGTCGCCACCGCCGCCGACCCGGCACTGCCGCCCAACGCGCGGCGGCGGC

CCTGC

TTAACCGCGCACGTCGCACCGGCCGACGGGCGGCCATGCGGGCCGCTCGAAGGCT

GGCCG

CGGGTATTGTCACTGTGCCCCCCAGGTCCAGGCGACGAGCGGCCGCCGCAGCAGC

CGCGG

CCATTAGTGCTATGACTCAGGGTCGCAGGGGCAACGTGTATTGGGTGCGCGACTC

GGTTA

GCGGCCTGCGCGTGCCCGTGCGCACCCGCCCCCCGCGCAACTAGATTGCAAGAAA

AAACT

ACTTAGACTCGTACTGTTGTATGTATCCAGCGGCGGCGGCGCGCAACGAAGCTAT

GTCCA

AGCGCAAAATCAAAGAAGAGATGCTCCAGGTCATCGCGCCGGAGATCTATGGCC

CCCCGA

AGAAGGAAGAGCAGGATTACAAGCCCCGAAAGCTAAAGCGGGTCAAAAAGAAA

AAGAAAG

ATGATGATGATGAACTTGACGACGAGGTGGAACTGCTGCACGCTACCGCGCCCAG

GCGAC

GGGTACAGTGGAAAGGTCGACGCGTAAAACGTGTTTTGCGACCCGGCACCACCGT

AGTCT

TTACGCCCGGTGAGCGCTCCACCCGCACCTACAAGCGCGTGTATGATGAGGTGTA
CGGCG

ACGAGGACCTGCTTGAGCAGGCCAACGAGCGCCTCGGGGAGTTTGCCTACGGAA
AGCGGC

ATAAGGACATGCTGGCGTTGCCGCTGGACGAGGGCAACCCAACACCTAGCCTAAA
GCCCG

TAACACTGCAGCAGGTGCTGCCCGCGCTTGCACCGTCCGAAGAAAAGCGCGGCCT
AAAGC

GCGAGTCTGGTGACTTGGCACCCACCGTGCAGCTGATGGTACCCAAGCGCCAGCG
ACTGG

AAGATGTCTTGGAAAAAATGACCGTGGAACCTGGGCTGGAGCCCGAGGTCCGCGT
GCGGC

CAATCAAGCAGGTGGCGCCGGGACTGGGCGTGCAGACCGTGGACGTTCAGATAC
CCACTA

CCAGTAGCACCAGTATTGCCACCGCCACAGAGGGCATGGAGACACAAACGTCCCC
GGTTG

CCTCAGCGGTGGCGGATGCCGCGGTGCAGGCGGTCGCTGCGGCCGCGTCCAAGAC
CTCTA

CGGAGGTGCAAACGGACCCGTGGATGTTTCGCGTTTCAGCCCCCCGGCGCCCGCG
CGGTT

CGAGGAAGTACGGCGCCGCCAGCGCGCTACTGCCCGAATATGCCCTACATCCTTC
CATTG

CGCCTACCCCCGGCTATCGTGGCTACACCTACCGCCCCAGAAGACGAGCAACTAC
CCGAC

GCCGAACCACCACTGGAACCCGCCGCCGCCGTCGCCGTCGCCAGCCCGTGCTGGC

CCCGA

TTTCCGTGCGCAGGGTGGCTCGCGAAGGAGGCAGGACCCTGGTGCTGCCAACAGC

GCGCT

ACCACCCCAGCATCGTTTAAAAGCCGGTCTTTGTGGTTCTTGCAGATATGGCCCTC

ACCT

GCCGCCTCCGTTTCCCGGTGCCGGGATTCCGAGGAAGAATGCACCGTAGGAGGGG

CATGG

CCGGCCACGGCCTGACGGGCGGCATGCGTCGTGCGCACCACCGGCGGCGGCGCG

CGTCGC

ACCGTCGCATGCGCGGCGGTATCCTGCCCCTCCTTATTCCACTGATCGCCGCGGCG

ATTG

GCGCCGTGCCCGGAATTGCATCCGTGGCCTTGCAGGCGCAGAGACACTGATTAAA

AACAA

GTTGCATGTGGAAAAATCAAAATAAAAAGTCTGGACTCTCACGCTCGCTTGGTCC

TGTAA

CTATTTTGTAGAATGGAAGACATCAACTTTGCGTCTCTGGCCCCGCGACACGGCTC

GCGC

CCGTTCATGGGAAACTGGCAAGATATCGGCACCAGCAATATGAGCGGTGGCGCCT

TCAGC

TGGGGCTCGCTGTGGAGCGGCATTAAAAATTTCGGTTCCACCGTTAAGAACTATG

GCAGC

AAGGCCTGGAACAGCAGCACAGGCCAGATGCTGAGGGATAAGTTGAAAGAGCAA

AATTTC

CAACAAAAGGTGGTAGATGGCCTGGCCTCTGGCATTAGCGGGGTGGTGGACCTGG
CCAAC
CAGGCAGTGCAAAATAAGATTAACAGTAAGCTTGATCCCCGCCCTCCCGTAGAGG
AGCCT
CCACCGGCCGTGGAGACAGTGTCTCCAGAGGGGCGTGGCGAAAAGCGTCCGCGC
CCCGAC
AGGGAAGAAACTCTGGTGACGCAAATAGACGAGCCTCCCTCGTACGAGGAGGCA
CTAAAG
CAAGGCCTGCCCACCACCCGTCCCATCGCGCCCATGGCTACCGGAGTGCTGGGCC
AGCAC
ACACCCGTAACGCTGGACCTGCCTCCCCCCGCCGACACCCAGCAGAAACCTGTGC
TGCCA
GGCCCGACCGCCGTTGTTGTAACCCGTCCTAGCCGCGCGTCCCTGCGCCGCGCCG
CCAGC
GGTCCGCGATCGTTGCGGCCCGTAGCCAGTGGCAACTGGCAAAGCACACTGAACA
GCATC
GTGGGTCTGGGGGTGCAATCCCTGAAGCGCCGACGATGCTTCTGAATAGCTAACG
TGTCG
TATGTGTGTCATGTATGCGTCCATGTCGCCGCCAGAGGAGCTGCTGAGCCGCCGC
GCGCC
CGCTTTCCAAGATGGCTACCCCTTCGATGATGCCGCAGTGGTCTTACATGCACATC
TCGG
GCCAGGACGCCTCGGAGTACCTGAGCCCCGGGCTGGTGCAGTTTGCCCGCGCCAC
CGAGA

CGTACTTCAGCCTGAATAACAAGTTTAGAAACCCCACGGTGGCGCCTACGCACGA
CGTGA

CCACAGACCGGTCCCAGCGTTTGACGCTGCGGTTCATCCCTGTGGACCGTGAGGA
TACTG

CGTACTCGTACAAGGCGCGGTTCACCCTAGCTGTGGGTGATAACCGTGTGCTGGA
CATGG

CTTCCACGTACTTTGACATCCGCGGCGTGCTGGACAGGGGCCCTACTTTTAAGCCC
TACT

CTGGCACTGCCTACAACGCCCTGGCTCCCAAGGGTGCCCCAAATCCTTGCGAATG
GGATG

AAGCTGCTACTGCTCTTGAAATAAACCTAGAAGAAGAGGACGATGACAACGAAG
ACGAAG

TAGACGAGCAAGCTGAGCAGCAAAAAACTCACGTATTTGGGCAGGCGCCTTATTC
TGGTA

TAAATATTACAAAGGAGGGTATTCAAATAGGTGTCGAAGGTCAAACACCTAAATA
TGCCG

ATAAAACATTTCAACCTGAACCTCAAATAGGAGAATCTCAGTGGTACGAAACTGA
AATTA

ATCATGCAGCTGGGAGAGTCCTTAAAAAGACTACCCCAATGAAACCATGTTACGG
TTCAT

ATGCAAAACCCACAAATGAAAATGGAGGGCAAGGCATTCTTGTAAAGCAACAAA
ATGGAA

AGCTAGAAAGTCAAGTGGAAATGCAATTTTTCTCAACTACTGAGGCGACCGCAGG
CAATG

GTGATAACTTGACTCCTAAAGTGGTATTGTACAGTGAAGATGTAGATATAGAAAC
CCCAG
ACACTCATATTTCTTACATGCCCACTATTAAGGAAGGTAACTCACGAGAACTAAT
GGGCC
AACAATCTATGCCCAACAGGCCTAATTACATTGCTTTTAGGGACAATTTTATTGGT
CTAA
TGTATTACAACAGCACGGGTAATATGGGTGTTCTGGCGGGCCAAGCATCGCAGTT
GAATG
CTGTTGTAGATTTGCAAGACAGAAACACAGAGCTTTCATACCAGCTTTTGCTTGAT
TCCA
TTGGTGATAGAACCAGGTACTTTTCTATGTGGAATCAGGCTGTTGACAGCTATGAT
CCAG
ATGTTAGAATTATTGAAAATCATGGAACTGAAGATGAACTTCCAAATTACTGCTTT
CCAC
TGGGAGGTGTGATTAATACAGAGACTCTTACCAAGGTAAAACCTAAAACAGGTCA
GGAAA
ATGGATGGGAAAAAGATGCTACAGAATTTTCAGATAAAAATGAAATAAGAGTTG
GAAATA
ATTTTGCCATGGAAATCAATCTAAATGCCAACCTGTGGAGAAATTTCCTGTACTCC
AACA
TAGCGCTGTATTTGCCCGACAAGCTAAAGTACAGTCCTTCCAACGTAAAAATTTCT
GATA
ACCCAAACACCTACGACTACATGAACAAGCGAGTGGTGGCTCCCGGGTTAGTGGA
CTGCT

ACATTAACCTTGGAGCACGCTGGTCCCTTGACTATATGGACAACGTCAACCCATTT AACC

ACCACCGCAATGCTGGCCTGCGCTACCGCTCAATGTTGCTGGGCAATGGTCGCTA TGTGC

CCTTCCACATCCAGGTGCCTCAGAAGTTCTTTGCCATTAAAAACCTCCTTCTCCTG CCGG

GCTCATACACCTACGAGTGGAACTTCAGGAAGGATGTTAACATGGTTCTGCAGAG CTCCC

TAGGAAATGACCTAAGGGTTGACGGAGCCAGCATTAAGTTTGATAGCATTTGCCT TTACG

CCACCTTCTTCCCCATGGCCCACAACACCGCCTCCACGCTTGAGGCCATGCTTAGA AACG

ACACCAACGACCAGTCCTTTAACGACTATCTCTCCGCCGCCAACATGCTCTACCCT ATAC

CCGCCAACGCTACCAACGTGCCCATATCCATCCCCTCCCGCAACTGGGCGGCTTTC CGCG

GCTGGGCCTTCACGCGCCTTAAGACTAAGGAAACCCCATCACTGGGCTCGGGCTA CGACC

CTTATTACACCTACTCTGGCTCTATACCCTACCTAGATGGAACCTTTTACCTCAAC CACA

CCTTTAAGAAGGTGGCCATTACCTTTGACTCTTCTGTCAGCTGGCCTGGCAATGAC CGCC

TGCTTACCCCCAACGAGTTTGAAATTAAGCGCTCAGTTGACGGGGAGGGTTACAA CGTTG

CCCAGTGTAACATGACCAAAGACTGGTTCCTGGTACAAATGCTAGCTAACTACAA
CATTG
GCTACCAGGGCTTCTATATCCCAGAGAGCTACAAGGACCGCATGTACTCCTTCTTT
AGAA
ACTTCCAGCCCATGAGCCGTCAGGTGGTGGATGATACTAAATACAAGGACTACCA
ACAGG
TGGGCATCCTACACCAACACAACAACTCTGGATTTGTTGGCTACCTTGCCCCCACC
ATGC
GCGAAGGACAGGCCTACCCTGCTAACTTCCCCTATCCGCTTATAGGCAAGACCGC
AGTTG
ACAGCATTACCCAGAAAAAGTTTCTTTGCGATCGCACCCTTTGGCGCATCCCATTC
TCCA
GTAACTTTATGTCCATGGGCGCACTCACAGACCTGGGCCAAAACCTTCTCTACGCC
AACT
CCGCCCACGCGCTAGACATGACTTTTGAGGTGGATCCCATGGACGAGCCCACCCT
TCTTT
ATGTTTTGTTTGAAGTCTTTGACGTGGTCCGTGTGCACCGGCCGCACCGCGGCGTC
ATCG
AAACCGTGTACCTGCGCACGCCCTTCTCGGCCGGCAACGCCACAACATAAAGAAG
CAAGC
AACATCAACAACAGCTGCCGCCATGGGCTCCAGTGAGCAGGAACTGAAAGCCATT
GTCAA
AGATCTTGGTTGTGGGCCATATTTTTGGGCACCTATGACAAGCGCTTTCCAGGCT
TTGT

TTCTCCACACAAGCTCGCCTGCGCCATAGTCAATACGGCCGGTCGCGAGACTGGG GGCGT

ACACTGGATGGCCTTTGCCTGGAACCCGCACTCAAAAACATGCTACCTCTTTGAG CCCTT

TGGCTTTTCTGACCAGCGACTCAAGCAGGTTTACCAGTTTGAGTACGAGTCACTCC TGCG

CCGTAGCGCCATTGCTTCTTCCCCCGACCGCTGTATAACGCTGGAAAAGTCCACCC AAAG

CGTACAGGGGCCCAACTCGGCCGCCTGTGGACTATTCTGCTGCATGTTTCTCCACG CCTT

TGCCAACTGGCCCCAAACTCCCATGGATCACAACCCCACCATGAACCTTATTACC GGGGT

ACCCAACTCCATGCTCAACAGTCCCCAGGTACAGCCCACCCTGCGTCGCAACCAG GAACA

GCTCTACAGCTTCCTGGAGCGCCACTCGCCCTACTTCCGCAGCCACAGTGCGCAG ATTAG

GAGCGCCACTTCTTTTTGTCACTTGAAAAACATGTAAAAATAATGTACTAGAGAC ACTTT

CAATAAAGGCAAATGCTTTTATTTGTACACTCTCGGGTGATTATTTACCCCCACCC TTGC

CGTCTGCGCCGTTTAAAAATCAAAGGGGTTCTGCCGCGCATCGCTATGCGCCACT GGCAG

GGACACGTTGCGATACTGGTGTTTAGTGCTCCACTTAAACTCAGGCACAACCATC CGCGG

CAGCTCGGTGAAGTTTTCACTCCACAGGCTGCGCACCATCACCAACGCGTTTAGC AGGTC

GGGCGCCGATATCTTGAAGTCGCAGTTGGGGCCTCCGCCCTGCGCGCGAGTTG CGATA

CACAGGGTTGCAGCACTGGAACACTATCAGCGCCGGGTGGTGCACGCTGGCCAGC ACGCT

CTTGTCGGAGATCAGATCCGCGTCCAGGTCCTCCGCGTTGCTCAGGGCGAACGGA GTCAA

CTTTGGTAGCTGCCTTCCCAAAAAGGGCGCGTGCCCAGGCTTTGAGTTGCACTCGC ACCG

TAGTGGCATCAAAAGGTGACCGTGCCCGGTCTGGGCGTTAGGATACAGCGCCTGC ATAAA

AGCCTTGATCTGCTTAAAAGCCACCTGAGCCTTTGCGCCTTCAGAGAAGAACATG CCGCA

AGACTTGCCGGAAAACTGATTGGCCGGACAGGCCGCGTCGTGCACGCAGCACCTT GCGTC

GGTGTTGGAGATCTGCACCACATTTCGGCCCCACCGGTTCTTCACGATCTTGGCCT TGCT

AGACTGCTCCTTCAGCGCGCGCTGCCCGTTTTCGCTCGTCACATCCATTTCAATCA CGTG

CTCCTTATTTATCATAATGCTTCCGTGTAGACACTTAAGCTCGCCTTCGATCTCAG CGCA

GCGGTGCAGCCACAACGCGCAGCCCGTGGGCTCGTGATGCTTGTAGGTCACCTCT GCAAA

CGACTGCAGGTACGCCTGCAGGAATCGCCCCATCATCGTCACAAAGGTCTTGTTG

CTGGT

GAAGGTCAGCTGCAACCCGCGGTGCTCCTCGTTCAGCCAGGTCTTGCATACGGCC

GCCAG

AGCTTCCACTTGGTCAGGCAGTAGTTTGAAGTTCGCCTTTAGATCGTTATCCACGT

GGTA

CTTGTCCATCAGCGCGCGCGCAGCCTCCATGCCCTTCTCCCACGCAGACACGATCG

GCAC

ACTCAGCGGGTTCATCACCGTAATTTCACTTTCCGCTTCGCTGGGCTCTTCCTCTTC

CTC

TTGCGTCCGCATACCACGCGCCACTGGGTCGTCTTCATTCAGCCGCCGCACTGTGC

GCTT

ACCTCCTTTGCCATGCTTGATTAGCACCGGTGGGTTGCTGAAACCCACCATTTGTA

GCGC

CACATCTTCTCTTTCTTCCTCGCTGTCCACGATTACCTCTGGTGATGGCGGGCGCTC

GGG

CTTGGGAGAAGGGCGCTTCTTTTTCTTCTTGGGCGCAATGGCCAAATCCGCCGCCG

AGGT

CGATGGCCGCGGGCTGGGTGTGCGCGGCACCAGCGCGTCTTGTGATGAGTCTTCC

TCGTC

CTCGGACTCGATACGCCGCCTCATCCGCTTTTTTGGGGGCGCCCGGGGAGGCGGC

GGCGA

CGGGGACGGGGACGACACGTCCTCCATGGTTGGGGGACGTCGCGCCGCACCGCGT

CCGCG

CTCGGGGGTGGTTTCGCGCTGCTCCTCTTCCCGACTGGCCATTTCCTTCTCCTATAG

GCA

GAAAAAGATCATGGAGTCAGTCGAGAAGAAGGACAGCCTAACCGCCCCCTCTGA

GTTCGC

CACCACCGCCTCCACCGATGCCGCCAACGCGCCTACCACCTTCCCCGTCGAGGCA

CCCCC

GCTTGAGGAGGAGGAAGTGATTATCGAGCAGGACCCAGGTTTTGTAAGCGAAGA

CGACGA

GGACCGCTCAGTACCAACAGAGGATAAAAAGCAAGACCAGGACAACGCAGAGGC

AAACGA

GGAACAAGTCGGGCGGGGGACGAAAGGCATGGCGACTACCTAGATGTGGGAGA

CGACGT

GCTGTTGAAGCATCTGCAGCGCCAGTGCGCCATTATCTGCGACGCGTTGCAAGAG

CGCAG

CGATGTGCCCCTCGCCATAGCGGATGTCAGCCTTGCCTACGAACGCCACCTATTCT

CACC

GCGCGTACCCCCCAAACGCCAAGAAAACGGCACATGCGAGCCCAACCCGCGCCT

CAACTT

CTACCCCGTATTTGCCGTGCCAGAGGTGCTTGCCACCTATCACATCTTTTTCCAAA

ACTG

CAAGATACCCCTATCCTGCCGTGCCAACCGCAGCCGAGCGGACAAGCAGCTGGCC

TTGCG

GCAGGGCGCTGTCATACCTGATATCGCCTCGCTCAACGAAGTGCCAAAAATCTTT

GAGGG

TCTTGGACGCGACGAGAAGCGCGGCAAACGCTCTGCAACAGGAAAACAGCGA

AAATGA

AAGTCACTCTGGAGTGTTGGTGGAACTCGAGGGTGACAACGCGCGCCTAGCCGTA

CTAAA

ACGCAGCATCGAGGTCACCCACTTTGCCTACCCGGCACTTAACCTACCCCCCAAG

GTCAT

GAGCACAGTCATGAGTGAGCTGATCGTGCGCCGTGCGCAGCCCCTGGAGAGGGAT

GCAAA

TTTGCAAGAACAAACAGAGGAGGGCCTACCCGCAGTTGGCGACGAGCAGCTAGC

GCGCTG

GCTTCAAACGCGCGAGCCTGCCGACTTGGAGGAGCGACGCAAACTAATGATGGCC

GCAGT

GCTCGTTACCGTGGAGCTTGAGTGCATGCAGCGGTTCTTTGCTGACCCGGAGATG

CAGCG

CAAGCTAGAGGAAACATTGCACTACACCTTTCGACAGGGCTACGTACGCCAGGCC

TGCAA

GATCTCCAACGTGGAGCTCTGCAACCTGGTCTCCTACCTTGGAATTTTGCACGAAA

ACCG

CCTTGGGCAAAACGTGCTTCATTCCACGCTCAAGGGCGAGGCGCGCCGCGACTAC

GTCCG

CGACTGCGTTTACTTATTTCTATGCTACACCTGGCAGACGGCCATGGGCGTTTGGC

AGCA

GTGCTTGGAGGAGTGCAACCTCAAGGAGCTGCAGAAACTGCTAAAGCAAAACTT

GAAGGA

CCTATGGACGGCCTTCAACGAGCGCTCCGTGGCCGCGCACCTGGCGGACATCATT TTCCC

CGAACGCCTGCTTAAAACCCTGCAACAGGGTCTGCCAGACTTCACCAGTCAAAGC ATGTT

GCAGAACTTTAGGAACTTTATCCTAGAGCGCTCAGGAATCTTGCCCGCCACCTGCT GTGC

ACTTCCTAGCGACTTTGTGCCCATTAAGTACCGCGAATGCCCTCCGCCGCTTTGGG GCCA

CTGCTACCTTCTGCAGCTAGCCAACTACCTTGCCTACCACTCTGACATAATGGAAG ACGT

GAGCGGTGACGGTCTACTGGAGTGTCACTGTCGCTGCAACCTATGCACCCCGCAC CGCTC

CCTGGTTTGCAATTCGCAGCTGCTTAACGAAAGTCAAATTATCGGTACCTTTGAGC TGCA

GGGTCCCTCGCCTGACGAAAAGTCCGCGGCTCCGGGGTTGAAACTCACTCCGGGG CTGTG

GACGTCGGCTTACCTTCGCAAATTTGTACCTGAGGACTACCACGCCCACGAGATT AGGTT

CTACGAAGACCAATCCCGCCCGCCAAATGCGGAGCTTACCGCCTGCGTCATTACC CAGGG

CCACATTCTTGGCCAATTGCAAGCCATCAACAAAGCCCGCCAAGAGTTTCTGCTA CGAAA

GGGACGGGGGGTTTACTTGGACCCCCAGTCCGGCGAGGAGCTCAACCCAATCCCC CCGCC

GCCGCAGCCCTATCAGCAGCAGCCGCGGGCCCTTGCTTCCCAGGATGGCACCCAA

AAAGA

AGCTGCAGCTGCCGCCGCCACCCACGGACGAGGAGGAATACTGGGACAGTCAGG

CAGAGG

AGGTTTTGGACGAGGAGGAGGAGGACATGATGGAAGACTGGGAGAGCCTAGACG

AGGAAG

CTTCCGAGGTCGAAGAGGTGTCAGACGAAACACCGTCACCCTCGGTCGCATTCCC

CTCGC

CGGCGCCCCAGAAATCGGCAACCGGTTCCAGCATGGCTACAACCTCCGCTCCTCA

GGCGC

CGCCGGCACTGCCCGTTCGCCGACCCAACCGTAGATGGGACACCACTGGAACCAG

GGCCG

GTAAGTCCAAGCAGCCGCCGCCGTTAGCCCAAGAGCAACAACAGCGCCAAGGCT

ACCGCT

CATGGCGCGGGCACAAGAACGCCATAGTTGCTTGCTTGCAAGACTGTGGGGGCAA

CATCT

CCTTCGCCCGCCGCTTTCTTCTCTACCATCACGGCGTGGCCTTCCCCCGTAACATC

CTGC

ATTACTACCGTCATCTCTACAGCCCATACTGCACCGGCGGCAGCGGCAGCGGCAG

CAACA

GCAGCGGCCACACAGAAGCAAAGGCGACCGGATAGCAAGACTCTGACAAAGCCC

AAGAAA

TCCACAGCGGCGGCAGCAGCAGGAGGAGGAGCGCTGCGTCTGGCGCCCAACGAA

CCCGTA

TCGACCCGCGAGCTTAGAAACAGGATTTTTCCCACTCTGTATGCTATATTTCAACA
GAGC
AGGGGCCAAGAACAAGAGCTGAAAATAAAAAACAGGTCTCTGCGATCCCTCACC
CGCAGC
TGCCTGTATCACAAAAGCGAAGATCAGCTTCGGCGCACGCTGGAAGACGCGGAG
GCTCTC
TTCAGTAAATACTGCGCGCTGACTCTTAAGGACTAGTTTCGCGCCCTTTCTCAAAT
TTAA
GCGCGAAAACTACGTCATCTCCAGCGGCCACACCCGGCGCCAGCACCTGTCGTCA
GCGCC
ATTATGAGCAAGGAAATTCCCACGCCCTACATGTGGAGTTACCAGCCACAAATGG
GACTT
GCGGCTGGAGCTGCCCAAGACTACTCAACCCGAATAAACTACATGAGCGCGGGA
CCCCAC
ATGATATCCCGGGTCAACGGAATCCGCGCCCACCGAAACCGAATTCTCTTGGAAC
AGGCG
GCTATTACCACCACACCTCGTAATAACCTTAATCCCCGTAGTTGGCCCGCTGCCCT
GGTG
TACCAGGAAAGTCCCGCTCCCACCACTGTGGTACTTCCCAGAGACGCCCAGGCCG
AAGTT
CAGATGACTAACTCAGGGGCGCAGCTTGCGGGCGGCTTTCGTCACAGGGTGCGGT
CGCCC
GGGCAGGGTATAACTCACCTGACAATCAGAGGGCGAGGTATTCAGCTCAACGAC
GAGTCG

GTGAGCTCCTCGCTTGGTCTCCGTCCGGACGGGACATTTCAGATCGGCGGCGCCG

GCCGT

CCTTCATTCACGCCTCGTCAGGCAATCCTAACTCTGCAGACCTCGTCCTCTGAGCC

GCGC

TCTGGAGGCATTGGAACTCTGCAATTTATTGAGGAGTTTGTGCCATCGGTCTACTT

TAAC

CCCTTCTCGGGACCTCCCGGCCACTATCCGGATCAATTTATTCCTAACTTTGACGC

GGTA

AAGGACTCGGCGGACGGCTACGACTGAATGTTAAGTGGAGAGGCAGAGCAACTG

CGCCTG

AAACACCTGGTCCACTGTCGCCGCCACAAGTGCTTTGCCCGCGACTCCGGTGAGT

TTTGC

TACTTTGAATTGCCCGAGGATCATATCGAGGGCCCGGCGCACGGCGTCCGGCTTA

CCGCC

CAGGGAGAGCTTGCCCGTAGCCTGATTCGGGAGTTTACCCAGCGCCCCCTGCTAG

TTGAG

CGGGACAGGGGACCCTGTGTTCTCACTGTGATTTGCAACTGTCCTAACCTTGGATT

ACAT

CAAGATCTTTGTTGCCATCTCTGTGCTGAGTATAATAAATACAGAAATTAAAATAT

ACTG

GGGCTCCTATCGCCATCCTGTAAACGCCACCGTCTTCACCCGCCCAAGCAAACCA

AGGCG

AACCTTACCTGGTACTTTTAACATCTCTCCCTCTGTGATTTACAACAGTTTCAACCC

AGA

CGGAGTGAGTCTACGAGAGAACCTCTCCGAGCTCAGCTACTCCATCAGAAAAAAC

ACCAC

CCTCCTTACCTGCCGGGAACGTACGAGTGCGTCACCGGCCGCTGCACCACACCTA

CCGCC

TGACCGTAAACCAGACTTTTTCCGGACAGACCTCAATAACTCTGTTTACCAGAAC

AGGAG

GTGAGCTTAGAAAACCCTTAGGGTATTAGGCCAAAGGCGCAGCTACTGTGGGGTT

TATGA

ACAATTCAAGCAACTCTACGGGCTATTCTAATTCAGGTTTCTCTAGAATCGGGGTT

GGGG

TTATTCTCTGTCTTGTGATTCTCTTTATTCTTATACTAACGCTTCTCTGCCTAAGGC

TCG

CCGCCTGCTGTGTGCACATTTGCATTTATTGTCAGCTTTTTAAACGCTGGGGTCGC

CACC

CAAGATGATTAGGTACATAATCCTAGGTTTACTCACCCTTGCGTCAGCCCACGGTA

CCAC

CCAAAAGGTGGATTTTAAGGAGCCAGCCTGTAATGTTACATTCGCAGCTGAAGCT

AATGA

GTGCACCACTCTTATAAAATGCACCACAGAACATGAAAAGCTGCTTATTCGCCAC

AAAAA

CAAAATTGGCAAGTATGCTGTTTATGCTATTTGGCAGCCAGGTGACACTACAGAG

TATAA

TGTTACAGTTTTCCAGGGTAAAAGTCATAAAACTTTTATGTATACTTTTCCATTTTA

TGA

AATGTGCGACATTACCATGTACATGAGCAAACAGTATAAGTTGTGGCCCCCACAA
AATTG
TGTGGAAAACACTGGCACTTTCTGCTGCACTGCTATGCTAATTACAGTGCTCGCTT
TGGT
CTGTACCCTACTCTATATTAAATACAAAAGCAGACGCAGCTTTATTGAGGAAAAG
AAAAT
GCCTTAATTTACTAAGTTACAAAGCTAATGTCACCACTAACTGCTTTACTCGCTGC
TTGC
AAAACAAATTCAAAAAGTTAGCATTATAATTAGAATAGGATTTAAACCCCCCGGT
CATTT
CCTGCTCAATACCATTCCCCTGAACAATTGACTCTATGTGGGATATGCTCCAGCGC
TACA
ACCTTGAAGTCAGGCTTCCTGGATGTCAGCATCTGACTTTGGCCAGCACCTGTCCC
GCGG
ATTTGTTCCAGTCCAACTACAGCGACCCACCCTAACAGAGATGACCAACACAACC
AACGC
GGCCGCCGCTACCGGACTTACATCTACCACAAATACACCCCAAGTTTCTGCCTTTG
TCAA
TAACTGGGATAACTTGGGCATGTGGTGGTTCTCCATAGCGCTTATGTTTGTATGCC
TTAT
TATTATGTGGCTCATCTGCTGCCTAAAGCGCAAACGCGCCCGACCACCCATCTATA
GTCC
CATCATTGTGCTACACCCAAACAATGATGGAATCCATAGATTGGACGGACTGAAA
CACAT

GTTCTTTTCTCTTACAGTATGATTAAATGAGACATGATTCCTCGAGTTTTATATTA
CTG
ACCCTTGTTGCGCTTTTTTGTGCGTGCTCCACATTGGCTGCGGTTTCTCACATCGAA
GTA
GACTGCATTCCAGCCTTCACAGTCTATTTGCTTTACGGATTTGTCACCCTCACGCT
CATC
TGCAGCCTCATCACTGTGGTCATCGCCTTTATCCAGTGCATTGACTGGGTCTGTGT
GCGC
TTTGCATATCTCAGACACCATCCCCAGTACAGGGACAGGACTATAGCTGAGCTTC
TTAGA
ATTCTTTAATTATGAAATTTACTGTGACTTTTCTGCTGATTATTTGCACCCTATCTG
CGT
TTTGTTCCCCGACCTCCAAGCCTCAAAGACATATATCATGCAGATTCACTCGTATA
TGGA
ATATTCCAAGTTGCTACAATGAAAAAAGCGATCTTTCCGAAGCCTGGTTATATGC
AATCA
TCTCTGTTATGGTGTTCTGCAGTACCATCTTAGCCCTAGCTATATATCCCTACCTTG
ACA
TTGGCTGGAACGCAATAGATGCCATGAACCACCCAACTTTCCCCGCGCCCGCTAT
GCTTC
CACTGCAACAAGTTGTTGCCGGCGGCTTTGTCCCAGCCAATCAGCCTCGCCCACCT
TCTC
CCACCCCCACTGAAATCAGCTACTTTAATCTAACAGGAGGAGATGACTGACACCC
TAGAT

CTAGAAATGGACGGAATTATTACAGAGCAGCGCCTGCTAGAAAGACGCAGGGCA

GCGGCC

GAGCAACAGCGCATGAATCAAGAGCTCCAAGACATGGTTAACTTGCACCAGTGCA

AAAGG

GGTATCTTTTGTCTGGTAAAGCAGGCCAAAGTCACCTACGACAGTAATACCACCG

GACAC

CGCCTTAGCTACAAGTTGCCAACCAAGCGTCAGAAATTGGTGGTCATGGTGGGAG

AAAAG

CCCATTACCATAACTCAGCACTCGGTAGAAACCGAAGGCTGCATTCACTCACCTT

GTCAA

GGACCTGAGGATCTCTGCACCCTTATTAAGACCCTGTGCGGTCTCAAAGATCTTAT

TCCC

TTTAACTAATAAAAAAAAATAATAAAGCATCACTTACTTAAAATCAGTTAGCAAA

TTTCT

GTCCAGTTTATTCAGCAGCACCTCCTTGCCCTCCTCCCAGCTCTGGTATTGCAGCT

TCCT

CCTGGCTGCAAACTTTCTCCACAATCTAAATGGAATGTCAGTTTCCTCCTGTTCCT

GTCC

ATCCGCACCCACTATCTTCATGTTGTTGCAGATGAAGCGCGCAAGACCGTCTGAA

GATAC

CTTCAACCCCGTGTATCCATATGACACGGAAACCGGTCCTCCAACTGTGCCTTTTC

TTAC

TCCTCCCTTTGTATCCCCCAATGGGTTTCAAGAGAGTCCCCCTGGGGTACTCTCTT

TGCG

CCTATCCGAACCTCTAGTTACCTCCAATGGCATGCTTGCGCTCAAAATGGGCAAC GGCCT

CTCTCTGGACGAGGCCGGCAACCTTACCTCCCAAAATGTAACCACTGTGAGCCCA CCTCT

CAAAAAAACCAAGTCAAACATAAACCTGGAAATATCTGCACCCCTCACAGTTACC TCAGA

AGCCCTAACTGTGGCTGCCGCCGCACCTCTAATGGTCGCGGGCAACACACTCACC ATGCA

ATCACAGGCCCCGCTAACCGTGCACGACTCCAAACTTAGCATTGCCACCCAAGGA CCCCT

CACAGTGTCAGAAGGAAAGCTAGCCCTGCAAACATCAGGCCCCCTCACCACCACC GATAG

CAGTACCCTTACTATCACTGCCTCACCCCCTCTAACTACTGCCACTGGTAGCTTGG GCAT

TGACTTGAAAGAGCCCATTTATACACAAAATGGAAAACTAGGACTAAAGTACGG GGCTCC

TTTGCATGTAACAGACGACCTAAACACTTTGACCGTAGCAACTGGTCCAGGTGTG ACTAT

TAATAATACTTCCTTGCAAACTAAAGTTACTGGAGCCTTGGGTTTTGATTCACAAG GCAA

TATGCAACTTAATGTAGCAGGAGGACTAAGGATTGATTCTCAAAACAGACGCCTT ATACT

TGATGTTAGTTATCCGTTTGATGCTCAAAACCAACTAAATCTAAGACTAGGACAG GGCCC

TCTTTTTATAAACTCAGCCCACAACTTGGATATTAACTACAACAAAGGCCTTTACT

TGTT

TACAGCTTCAAACAATTCCAAAAAGCTTGAGGTTAACCTAAGCACTGCCAAGGGG

TTGAT

GTTTGACGCTACAGCCATAGCCATTAATGCAGGAGATGGGCTTGAATTTGGTTCA

CCTAA

TGCACCAAACACAAATCCCCTCAAAACAAAAATTGGCCATGGCCTAGAATTTGAT

TCAAA

CAAGGCTATGGTTCCTAAACTAGGAACTGGCCTTAGTTTTGACAGCACAGGTGCC

ATTAC

AGTAGGAAACAAAAATAATGATAAGCTAACTTTGTGGACCACACCAGCTCCATCT

CCTAA

CTGTAGACTAAATGCAGAGAAAGATGCTAAACTCACTTTGGTCTTAACAAAATGT

GGCAG

TCAAATACTTGCTACAGTTTCAGTTTTGGCTGTTAAAGGCAGTTTGGCTCCAATAT

CTGG

AACAGTTCAAAGTGCTCATCTTATTATAAGATTTGACGAAAATGGAGTGCTACTA

AACAA

TTCCTTCCTGGACCCAGAATATTGGAACTTTAGAAATGGAGATCTTACTGAAGGC

ACAGC

CTATACAAACGCTGTTGGATTTATGCCTAACCTATCAGCTTATCCAAAATCTCACG

GTAA

AACTGCCAAAAGTAACATTGTCAGTCAAGTTTACTTAAACGGAGACAAAACTAAA

CCTGT

AACACTAACCATTACACTAAACGGTACACAGGAAACAGGAGACACAACTCCAAG

TGCATA

CTCTATGTCATTTTCATGGGACTGGTCTGGCCACAACTACATTAATGAAATATTTG

CCAC

ATCCTCTTACACTTTTTCATACATTGCCCAAGAATAAAGAATCGTTTGTGTTATGT

TTCA

ACGTGTTTATTTTTCAATTGCAGAAAATTTCAAGTCATTTTTCATTCAGTAGTATA

GCCC

CACCACCACATAGCTTATACAGATCACCGTACCTTAATCAAACTCACAGAACCCT

AGTAT

TCAACCTGCCACCTCCCTCCCAACACACAGAGTACACAGTCCTTTCTCCCCGGCTG

GCCT

TAAAAAGCATCATATCATGGGTAACAGACATATTCTTAGGTGTTATATTCCACAC

GGTTT

CCTGTCGAGCCAAACGCTCATCAGTGATATTAATAAACTCCCCGGGCAGCTCACT

TAAGT

TCATGTCGCTGTCCAGCTGCTGAGCCACAGGCTGCTGTCCAACTTGCGGTTGCTTA

ACGG

GCGGCGAAGGAGAAGTCCACGCCTACATGGGGGTAGAGTCATAATCGTGCATCA

GGATAG

GGCGGTGGTGCTGCAGCAGCGCGCGAATAAACTGCTGCCGCCGCCGCTCCGTCCT

GCAGG

AATACAACATGGCAGTGGTCTCCTCAGCGATGATTCGCACCGCCCGCAGCATAAG

GCGCC

TTGTCCTCCGGGCACAGCAGCGCACCCTGATCTCACTTAAATCAGCACAGTAACT
GCAGC

ACAGCACCACAATATTGTTCAAAATCCCACAGTGCAAGGCGCTGTATCCAAAGCT
CATGG

CGGGGACCACAGAACCCACGTGGCCATCATACCACAAGCGCAGGTAGATTAAGT
GGCGAC

CCCTCATAAACACGCTGGACATAAACATTACCTCTTTTGGCATGTTGTAATTCACC
ACCT

CCCGGTACCATATAAACCTCTGATTAAACATGGCGCCATCCACCACCATCCTAAA
CCAGC

TGGCCAAAACCTGCCCGCCGGCTATACACTGCAGGGAACCGGGACTGGAACAAT
GACAGT

GGAGAGCCCAGGACTCGTAACCATGGATCATCATGCTCGTCATGATATCAATGTT
GGCAC

AACACAGGCACACGTGCATACACTTCCTCAGGATTACAAGCTCCTCCCGCGTTAG
AACCA

TATCCCAGGGAACAACCCATTCCTGAATCAGCGTAAATCCCACACTGCAGGGAAG
ACCTC

GCACGTAACTCACGTTGTGCATTGTCAAAGTGTTACATTCGGGCAGCAGCGGATG
ATCCT

CCAGTATGGTAGCGCGGGTTTCTGTCTCAAAAGGAGGTAGACGATCCCTACTGTA
CGGAG

TGCGCCGAGACAACCGAGATCGTGTTGGTCGTAGTGTCATGCCAAATGGAACGCC
GGACG

TAGTCATATTTCCTGAAGCAAAACCAGGTGCGGGCGTGACAAACAGATCTGCGTC

TCCGG

TCTCGCCGCTTAGATCGCTCTGTGTAGTAGTTGTAGTATATCCACTCTCTCAAAGC

ATCC

AGGCGCCCCCTGGCTTCGGGTTCTATGTAAACTCCTTCATGCGCCGCTGCCCTGAT

AACA

TCCACCACCGCAGAATAAGCCACACCCAGCCAACCTACACATTCGTTCTGCGAGT

CACAC

ACGGGAGGAGCGGGAAGAGCTGGAAGAACCATGTTTTTTTTTTATTCCAAAAGA

TTATC

CAAAACCTCAAAATGAAGATCTATTAAGTGAACGCGCTCCCCTCCGGTGGCGTGG

TCAAA

CTCTACAGCCAAAGAACAGATAATGGCATTTGTAAGATGTTGCACAATGGCTTCC

AAAAG

GCAAACGGCCCTCACGTCCAAGTGGACGTAAAGGCTAAACCCTTCAGGGTGAATC

TCCTC

TATAAACATTCCAGCACCTTCAACCATGCCCAAATAATTCTCATCTCGCCACCTTC

TCAA

TATATCTCTAAGCAAATCCCGAATATTAAGTCCGGCCATTGTAAAAATCTGCTCCA

GAGC

GCCCTCCACCTTCAGCCTCAAGCAGCGAATCATGATTGCAAAAATTCAGGTTCCTC

ACAG

ACCTGTATAAGATTCAAAAGCGGAACATTAACAAAAATACCGCGATCCCGTAGGT

CCCTT

CGCAGGGCCAGCTGAACATAATCGTGCAGGTCTGCACGGACCAGCGCGGCCACTT
CCCCG

CCAGGAACCTTGACAAAAGAACCCACACTGATTATGACACGCATACTCGGAGCTA
TGCTA

ACCAGCGTAGCCCCGATGTAAGCTTTGTTGCATGGGCGGCGATATAAAATGCAAG
GTGCT

GCTCAAAAAATCAGGCAAAGCCTCGCGCAAAAAAGAAAGCACATCGTAGTCATG
CTCATG

CAGATAAAGGCAGGTAAGCTCCGGAACCACCACAGAAAAAGACACCATTTTTCTC
TCAAA

CATGTCTGCGGGTTTCTGCATAAACACAAAATAAAATAACAAAAAAACATTTAAA
CATTA

GAAGCCTGTCTTACAACAGGAAAAACAACCCTTATAAGCATAAGACGGACTACG
GCCATG

CCGGCGTGACCGTAAAAAAACTGGTCACCGTGATTAAAAAGCACCACCGACAGCT
CCTCG

GTCATGTCCGGAGTCATAATGTAAGACTCGGTAAACACATCAGGTTGATTCATCG
GTCAG

TGCTAAAAAGCGACCGAAATAGCCCGGGGGAATACATACCCGCAGGCGTAGAGA
CAACAT

TACAGCCCCCATAGGAGGTATAACAAAATTAATAGGAGAGAAAAACACATAAAC
ACCTGA

AAAACCCTCCTGCCTAGGCAAAATAGCACCCTCCCGCTCCAGAACAACATACAGC
GCTTC

CACAGCGGCAGCCATAACAGTCAGCCTTACCAGTAAAAAAGAAAACCTATTAAA

AAAACA

CCACTCGACACGGCACCAGCTCAATCAGTCACAGTGTAAAAAAGGGCCAAGTGC

AGAGCG

AGTATATATAGGACTAAAAAATGACGTAACGGTTAAAGTCCACAAAAAACACCC

AGAAAA

CCGCACGCGAACCTACGCCCAGAAACGAAAGCCAAAAAACCCACAACTTCCTCA

AATCGT

CACTTCCGTTTTCCCACGTTACGTCACTTCCCATTTTAATTAAGAAAACTACAATT

CCCA

ACACATACAAGTTACTCCGCCCTAAAACCTACGTCACCCGCCCCGTTCCCACGCCC

CGCG

CCACGTCACAAACTCCACCCCCTCATTATCATATTGGCTTCAATCCAAAATAAGGT

ATAT

TATTGATGATGATTACCCTGTTAT

SEQ ID NO:50 is the OV1164 sequence

CAGGGTAATCATCATCAATAATATACCTTATTTTGGATTGAAGCCAATATGATAAT

GAGG

GGGTGGAGTTTGTGACGTGGCGCGGGGCGTGGGAACGGGGCGGGTGACGTAGTA

GTGTGG

CGGAAGTGTGATGTTGCAAGTGTGGCGGAACACATGTAAGCGACGGATGTGGCA

AAAGTG

ACGTTTTTGGTGTGCGCCGGTGTACACAGGAAGTGACAATTTTCGCGCGGTTTTAG

GCGG

ATGTTGTAGTAAATTTGGGCGTAACCGAGTAAGATTTGGCCATTTTCGCGGGAAA

ACTGA

ATAAGAGGAAGTGAAATCTGAATAATTTTGTGTTACTCATAGCGCGTAATATTTGT

CTAG

GGCCGGGATCTCTGCAGGAATTTGATATCAAGCTTATCGATACCGTCGAAACTTG

TTTAT

TGCAGCTTATAATGGTTACAAATAAAGCAATAGCATCACAAATTTCACAAATAAA

GCATT

TTTTTCACTGCATTCTAGTTGTGGTTTGTCCAAACTCATCAATGTATCTTATCATGT

CTG

GATCCGCTAGCGGCGCGCCGTTTCATCCGGACAAAGCCTGCGCGCGCCCCGCCCC

GCCAT

TGGCCGTACCGCCCCGCGCCGCCGCCCCATCTCGCCCCTCGCCGCCGGGTCCGGC

GCGTT

AAAGCCAATAGGAACCGCCGCCGTTGTTCCCGTCACGGCCGGGGCAGCCAATTGT

GGCGG

CGCTCGGCGGCTCGTGGCTCTTTCGCGGCAAAAAGGATTTGGCGCGTAAAAGTGG

CCGGG

ACTTTGCAGGCAGCGGCGGCCGGGGGCGGAGCGGGATCGAGCCCTCGATGATAT

CAGATC

AAACGATATCACCGGTCGACTGAAAATGAGACATATTATCTGCCACGGAGGTGTT

ATTAC

CGAAGAAATGGCCGCCAGTCTTTTGGACCAGCTGATCGAAGAGGTACTGGCTGAT

AATCT

TCCACCTCCTAGCCATTTTGAACCACCTACCCTTCACGAACTGTATGATTTAGACG

TGAC

GGCCCCCGAAGATCCCAACGAGGAGGCGGTTTCGCAGATTTTTCCCGACTCTGTA

ATGTT

GGCGGTGCAGGAAGGGATTGACTTACTCACTTTTCCGCCGGCGCCCGGTTCTCCG

GAGCC

GCCTCACCTTTCCCGGCAGCCCGAGCAGCCGGAGCAGAGAGCCTTGGGTCCGGTT

TCTAT

GCCAAACCTTGTACCGGAGGTGATCGATCTTACCTGCCACGAGGCTGGCTTTCCA

CCCAG

TGACGACGAGGATGAAGAGGGTGAGGAGTTTGTGTTAGATTATGTGGAGCACCCC

GGGCA

CGGTTGCAGGTCTTGTCATTATCACCGGAGGAATACGGGGGACCCAGATATTATG

TGTTC

GCTTTGCTATATGAGGACCTGTGGCATGTTTGTCTACAGTAAGTGAAAATTATGGG

CAGT

GGGTGATAGAGTGGTGGGTTTGGTGTGGTAATTTTTTTTTAATTTTTACAGTTTTG

TGG

TTTAAAGAATTTTGTATTGTGATTTTTTTAAAAGGTCCTGTGTCTGAACCTGAGCC

TGAG

CCCGAGCCAGAACCGGAGCCTGCAAGACCTACCCGCCGTCCTAAAATGGCGCCTG

CTATC

CTGAGACGCCCGACATCACCTGTGTCTAGAGAATGCAATAGTAGTACGGATAGCT

GTGAC

TCCGGTCCTTCTAACACACCTCCTGAGATACACCCGGTGGTCCCGCTGTGCCCCAT TAAA

CCAGTTGCCGTGAGAGTTGGTGGGCGTCGCCAGGCTGTGGAATGTATCGAGGACT TGCTT

AACGAGCCTGGGCAACCTTTGGACTTGAGCTGTAAACGCCCCAGGCCATAAGGTG TAAAC

CTGTGATTGCGTGTGTGGTTAACGCCTTTGTTTGCTGAATGAGTTGATGTAAGTTT AATA

AAGGGTGAGATAATGTTTAACTTGCATGGCGTGTTAAATGGGGCGGGGCTTAAAG GGTAT

ATAATGCGCCGTGGGCTAATCTTGGTTACATCTGACCTCATGGAGGCTTGGGAGT GTTTG

GAAGATTTTTCTGCTGTGCGTAACTTGCTGGAACAGAGCTCTAACAGTACCTCTTG GTTT

TGGAGGTTTCTGTGGGGCTCATCCCAGGCAAAGTTAGTCTGCAGAATTAAGGAGG ATTAC

AAGTGGGAATTTGAAGAGCTTTTGAAATCCTGTGGTGAGCTGTTTGATTCTTTGAA TCTG

GGTCACCAGGCGCTTTTCCAAGAGAAGGTCATCAAGACTTTGGATTTTTCCACACC GGGG

CGCGCTGCGGCTGCTGTTGCTTTTTTGAGTTTTATAAAGGATAAATGGAGCGAAG AAACC

CATCTGAGCGGGGGGTACCTGCTGGATTTTCTGGCCATGCATCTGTGGAGAGCGG TTGTG

AGACACAAGAATCGCCTGCTACTGTTGTCTTCCGTCCGCCCGGCGATAATACCGA

CGGAG

GAGCAGCAGCAGCAGCAGGAGGAAGCCAGGCGGCGGCGGCAGGAGCAGAGCCC

ATGGAAC

CCGAGAGCCGGCCTGGACCCTCGGGAATGAATGTTGTACAGGTGGCTGAACTGTA

TCCAG

AACTGAGACGCATTTTGACAATTACAGAGGATGGGCAGGGGCTAAAGGGGGTAA

AGAGGG

AGCGGGGGGCTTGTGAGGCTACAGAGGAGGCTAGGAATCTAGCTTTTAGCTTAAT

GACCA

GACACCGTCCTGAGTGTATTACTTTTCAACAGATCAAGGATAATTGCGCTAATGA

GCTTG

ATCTGCTGGCGCAGAAGTATTCCATAGAGCAGCTGACCACTTACTGGCTGCAGCC

AGGGG

ATGATTTTGAGGAGGCTATTAGGGTATATGCAAAGGTGGCACTTAGGCCAGATTG

CAAGT

ACAAGATCAGCAAACTTGTAAATATCAGGAATTGTTGCTACATTTCTGGGAACGG

GGCCG

AGGTGGAGATAGATACGGAGGATAGGGTGGCCTTTAGATGTAGCATGATAAATAT

GTGGC

CGGGGGTGCTTGGCATGGACGGGGTGGTTATTATGAATGTAAGGTTTACTGGCCC

CAATT

TTAGCGGTACGGTTTTCCTGGCCAATACCAACCTTATCCTACACGGTGTAAGCTTC

TATG

GGTTTAACAATACCTGTGTGGAAGCCTGGACCGATGTAAGGGTTCGGGGCTGTGC CTTTT

ACTGCTGCTGGAAGGGGGTGGTGTGTCGCCCCAAAAGCAGGGCTTCAATTAAGAA ATGCC

TCTTTGAAAGGTGTACCTTGGGTATCCTGTCTGAGGGTAACTCCAGGGTGCGCCAC AATG

TGGCCTCCGACTGTGGTTGCTTCATGCTAGTGAAAAGCGTGGCTGTGATTAAGCAT AACA

TGGTATGTGGCAACTGCGAGGACAGGGCCTCTCAGATGCTGACCTGCTCGGACGG CAACT

GTCACCTGCTGAAGACCATTCACGTAGCCAGCCACTCTCGCAAGGCCTGGCCAGT GTTTG

AGCATAACATACTGACCCGCTGTTCCTTGCATTTGGGTAACAGGAGGGGGGTGTT CCTAC

CTTACCAATGCAATTTGAGTCACACTAAGATATTGCTTGAGCCCGAGAGCATGTC CAAGG

TGAACCTGAACGGGGTGTTTGACATGACCATGAAGATCTGGAAGGTGCTGAGGTA CGATG

AGACCCGCACCAGGTGCAGACCCTGCGAGTGTGGCGGTAAACATATTAGGAACC AGCCTG

TGATGCTGGATGTGACCGAGGAGCTGAGGCCCGATCACTTGGTGCTGGCCTGCAC CCGCG

CTGAGTTTGGCTCTAGCGATGAAGATACAGATTGAGGTACTGAAATGTGTGGGCG TGGCT

TAAGGGTGGGAAAGAATATATAAGGTGGGGGTCTTATGTAGTTTTGTATCTGTTTT

GCAG

CAGCCGCCGCCGCCATGAGCACCAACTCGTTTGATGGAAGCATTGTGAGCTCATA

TTTGA

CAACGCGCATGCCCCCATGGGCCGGGGTGCGTCAGAATGTGATGGGCTCCAGCAT

TGATG

GTCGCCCCGTCCTGCCCGCAAACTCTACTACCTTGACCTACGAGACCGTGTCTGGA

ACGC

CGTTGGAGACTGCAGCCTCCGCCGCCGCTTCAGCCGCTGCAGCCACCGCCCGCGG

GATTG

TGACTGACTTTGCTTTCCTGAGCCCGCTTGCAAGCAGTGCAGCTTCCCGTTCATCC

GCCC

GCGATGACAAGTTGACGGCTCTTTTGGCACAATTGGATTCTTTGACCCGGGAACTT

AATG

TCGTTTCTCAGCAGCTGTTGGATCTGCGCCAGCAGGTTTCTGCCCTGAAGGCTTCC

TCCC

CTCCCAATGCGGTTTAAAACATAAATAAAAAACCAGACTCTGTTTGGATTTGGAT

CAAGC

AAGTGTCTTGCTGTCTTTATTTAGGGGTTTTGCGCGCGCGGTAGGCCCGGGACCAG

CGGT

CTCGGTCGTTGAGGGTCCTGTGTATTTTTTCCAGGACGTGGTAAAGGTGACTCTGG

ATGT

TCAGATACATGGGCATAAGCCCGTCTCTGGGGTGGAGGTAGCACCACTGCAGAGC

TTCAT

GCTGCGGGGTGGTGTTGTAGATGATCCAGTCGTAGCAGGAGCGCTGGGCGTGGTG

CCTAA

AAATGTCTTTCAGTAGCAAGCTGATTGCCAGGGGCAGGCCCTTGGTGTAAGTGTT

TACAA

AGCGGTTAAGCTGGGATGGGTGCATACGTGGGGATATGAGATGCATCTTGGACTG

TATTT

TTAGGTTGGCTATGTTCCCAGCCATATCCCTCCGGGGATTCATGTTGTGCAGAACC

ACCA

GCACAGTGTATCCGGTGCACTTGGGAAATTTGTCATGTAGCTTAGAAGGAAATGC

GTGGA

AGAACTTGGAGACGCCCTTGTGACCTCCAAGATTTTCCATGCATTCGTCCATAATG

ATGG

CAATGGGCCCACGGGCGGCGGCCTGGGCGAAGATATTTCTGGGATCACTAACGTC

ATAGT

TGTGTTCCAGGATGAGATCGTCATAGGCCATTTTTACAAAGCGCGGGCGGAGGGT

GCCAG

ACTGCGGTATAATGGTTCCATCCGGCCCAGGGGCGTAGTTACCCTCACAGATTTG

CATTT

CCCACGCTTTGAGTTCAGATGGGGGGATCATGTCTACCTGCGGGGCGATGAAGAA

AACGG

TTTCCGGGGTAGGGGAGATCAGCTGGGAAGAAAGCAGGTTCCTGAGCAGCTGCG

ACTTAC

CGCAGCCGGTGGGCCCGTAAATCACACCTATTACCGGGTGCAACTGGTAGTTAAG

AGAGC

TGCAGCTGCCGTCATCCCTGAGCAGGGGGGCCACTTCGTTAAGCATGTCCCTGAC

TCGCA

TGTTTTCCCTGACCAAATCCGCCAGAAGGCGCTCGCCGCCCAGCGATAGCAGTTC

TTGCA

AGGAAGCAAAGTTTTTCAACGGTTTGAGACCGTCCGCCGTAGGCATGCTTTTGAG

CGTTT

GACCAAGCAGTTCCAGGCGGTCCCACAGCTCGGTCACCTGCTCTACGGCATCTCG

ATCCA

GCATATCTCCTCGTTTCGCGGGTTGGGGCGGCTTTCGCTGTACGGCAGTAGTCGGT

GCTC

GTCCAGACGGGCCAGGGTCATGTCTTTCCACGGGCGCAGGGTCCTCGTCAGCGTA

GTCTG

GGTCACGGTGAAGGGGTGCGCTCCGGGCTGCGCGCTGGCCAGGGTGCGCTTGAGG

CTGGT

CCTGCTGGTGCTGAAGCGCTGCCGGTCTTCGCCCTGCGCGTCGGCCAGGTAGCATT

TGAC

CATGGTGTCATAGTCCAGCCCCTCCGCGGCGTGGCCCTTGGCGCGCAGCTTGCCCT

TGGA

GGAGGCGCCGCACGAGGGGCAGTGCAGACTTTTGAGGGCGTAGAGCTTGGGCGC

GAGAAA

TACCGATTCCGGGGAGTAGGCATCCGCGCCGCAGGCCCCGCAGACGGTCTCGCAT

TCCAC

GAGCCAGGTGAGCTCTGGCCGTTCGGGGTCAAAAACCAGGTTTCCCCCATGCTTT

TTGAT

GCGTTTCTTACCTCTGGTTTCCATGAGCCGGTGTCCACGCTCGGTGACGAAAAGGC TGTC
CGTGTCCCCGTATACAGACTTGAGAGGCCTGTCCTCGAGCGGTGTTCCGCGGTCCT CCTC
GTATAGAAACTCGGACCACTCTGAGACAAAGGCTCGCGTCCAGGCCAGCACGAA GGAGGC
TAAGTGGGAGGGGTAGCGGTCGTTGTCCACTAGGGGGTCCACTCGCTCCAGGGTG TGAAG
ACACATGTCGCCCTCTTCGGCATCAAGGAAGGTGATTGGTTTGTAGGTGTAGGCC ACGTG
ACCGGGTGTTCCTGAAGGGGGGCTATAAAAGGGGGTGGGGGCGCGTTCGTCCTCA CTCTC
TTCCGCATCGCTGTCTGCGAGGGCCAGCTGTTGGGGTGAGTACTCCCTCTGAAAA GCGGG
CATGACTTCTGCGCTAAGATTGTCAGTTTCCAAAAACGAGGAGGATTTGATATTC ACCTG
GCCCGCGGTGATGCCTTTGAGGGTGGCCGCATCCATCTGGTCAGAAAAGACAATC TTTTT
GTTGTCAAGCTTGGTGGCAAACGACCCGTAGAGGGCGTTGGACAGCAACTTGGCG ATGGA
GCGCAGGGTTTGGTTTTTGTCGCGATCGGCGCGCTCCTTGGCCGCGATGTTTAGCT GCAC
GTATTCGCGCGCAACGCACCGCCATTCGGGAAAGACGGTGGTGCGCTCGTCGGGC ACCAG

GTGCACGCGCCAACCGCGGTTGTGCAGGGTGACAAGGTCAACGCTGGTGGCTACC

TCTCC

GCGTAGGCGCTCGTTGGTCCAGCAGAGGCGGCCGCCCTTGCGCGAGCAGAATGGC

GGTAG

GGGGTCTAGCTGCGTCTCGTCCGGGGGGTCTGCGTCCACGGTAAAGACCCCGGGC

AGCAG

GCGCGCGTCGAAGTAGTCTATCTTGCATCCTTGCAAGTCTAGCGCCTGCTGCCATG

CGCG

GGCGGCAAGCGCGCGCTCGTATGGGTTGAGTGGGGGACCCCATGGCATGGGGTG

GGTGAG

CGCGGAGGCGTACATGCCGCAAATGTCGTAAACGTAGAGGGGCTCTCTGAGTATT

CCAAG

ATATGTAGGGTAGCATCTTCCACCGCGGATGCTGGCGCGCACGTAATCGTATAGT

TCGTG

CGAGGGAGCGAGGAGGTCGGGACCGAGGTTGCTACGGGCGGGCTGCTCTGCTCG

GAAGAC

TATCTGCCTGAAGATGGCATGTGAGTTGGATGATATGGTTGGACGCTGGAAGACG

TTGAA

GCTGGCGTCTGTGAGACCTACCGCGTCACGCACGAAGGAGGCGTAGGAGTCGCGC

AGCTT

GTTGACCAGCTCGGCGGTGACCTGCACGTCTAGGGCGCAGTAGTCCAGGGTTTCC

TTGAT

GATGTCATACTTATCCTGTCCCTTTTTTTCCACAGCTCGCGGTTGAGGACAAACT

CTTC

GCGGTCTTTCCAGTACTCTTGGATCGGAAACCCGTCGGCCTCCGAACGGTAAGAG

CCTAG

CATGTAGAACTGGTTGACGGCCTGGTAGGCGCAGCATCCCTTTTCTACGGGTAGC

GCGTA

TGCCTGCGCGGCCTTCCGGAGCGAGGTGTGGGTGAGCGCAAAGGTGTCCCTGACC

ATGAC

TTTGAGGTACTGGTATTTGAAGTCAGTGTCGTCGCATCCGCCCTGCTCCCAGAGCA

AAAA

GTCCGTGCGCTTTTTGGAACGCGGATTTGGCAGGGCGAAGGTGACATCGTTGAAG

AGTAT

CTTTCCCGCGCGAGGCATAAAGTTGCGTGTGATGCGGAAGGGTCCCGGCACCTCG

GAACG

GTTGTTAATTACCTGGGCGGCGAGCACGATCTCGTCAAAGCCGTTGATGTTGTGG

CCCAC

AATGTAAAGTTCCAAGAAGCGCGGGATGCCCTTGATGGAAGGCAATTTTTTAAGT

TCCTC

GTAGGTGAGCTCTTCAGGGGAGCTGAGCCCGTGCTCTGAAAGGGCCCAGTCTGCA

AGATG

AGGGTTGGAAGCGACGAATGAGCTCCACAGGTCACGGGCCATTAGCATTTGCAGG

TGGTC

GCGAAAGGTCCTAAACTGGCGACCTATGGCCATTTTTTCTGGGGTGATGCAGTAG

AAGGT

AAGCGGGTCTTGTTCCCAGCGGTCCCATCCAAGGTTCGCGGCTAGGTCTCGCGCG

GCAGT

CACTAGAGGCTCATCTCCGCCGAACTTCATGACCAGCATGAAGGGCACGAGCTGC

TTCCC

AAAGGCCCCCATCCAAGTATAGGTCTCTACATCGTAGGTGACAAAGAGACGCTCG

GTGCG

AGGATGCGAGCCGATCGGGAAGAACTGGATCTCCCGCCACCAATTGGAGGAGTG

GCTATT

GATGTGGTGAAAGTAGAAGTCCCTGCGACGGGCCGAACACTCGTGCTGGCTTTTG

TAAAA

ACGTGCGCAGTACTGGCAGCGGTGCACGGGCTGTACATCCTGCACGAGGTTGACC

TGACG

ACCGCGCACAAGGAAGCAGAGTGGGAATTTGAGCCCCTCGCCTGGCGGGTTTGGC

TGGTG

GTCTTCTACTTCGGCTGCTTGTCCTTGACCGTCTGGCTGCTCGAGGGGAGTTACGG

TGGA

TCGGACCACCACGCCGCGCGAGCCCAAAGTCCAGATGTCCGCGCGCGGCGGTCGG

AGCTT

GATGACAACATCGCGCAGATGGGAGCTGTCCATGGTCTGGAGCTCCCGCGGCGTC

AGGTC

AGGCGGGAGCTCCTGCAGGTTTACCTCGCATAGACGGGTCAGGGCGCGGGCTAGA

TCCAG

GTGATACCTAATTTCCAGGGGCTGGTTGGTGGCGGCGTCGATGGCTTGCAAGAGG

CCGCA

TCCCCGCGGCGCGACTACGGTACCGCGCGGCGGGCGGTGGGCCGCGGGGGTGTCC

TTGGA

TGATGCATCTAAAAGCGGTGACGCGGGCGAGCCCCCGGAGGTAGGGGGGGCTCC

GGACCC

GCCGGGAGAGGGGGCAGGGGCACGTCGGCGCCGCGCGGGCAGGAGCTGGTGC

TGCGCG

CGTAGGTTGCTGGCGAACGCGACGACGCGGCGGTTGATCTCCTGAATCTGGCGCC

TCTGC

GTGAAGACGACGGGCCCGGTGAGCTTGAGCCTGAAAGAGAGTTCGACAGAATCA

ATTTCG

GTGTCGTTGACGGCGGCCTGGCGCAAAATCTCCTGCACGTCTCCTGAGTTGTCTTG

ATAG

GCGATCTCGGCCATGAACTGCTCGATCTCTTCCTCCTGGAGATCTCCGCGTCCGGC

TCGC

TCCACGGTGGCGGCGAGGTCGTTGGAAATGCGGGCCATGAGCTGCGAGAAGGCG

TTGAGG

CCTCCCTCGTTCCAGACGCGGCTGTAGACCACGCCCCCTTCGGCATCGCGGGCGC

GCATG

ACCACCTGCGCGAGATTGAGCTCCACGTGCCGGGCGAAGACGGCGTAGTTTCGCA

GGCGC

TGAAAGAGGTAGTTGAGGGTGGTGGCGGTGTGTTCTGCCACGAAGAAGTACATAA

CCCAG

CGTCGCAACGTGGATTCGTTGATATCCCCCAAGGCCTCAAGGCGCTCCATGGCCT

CGTAG

AAGTCCACGGCGAAGTTGAAAAACTGGGAGTTGCGCGCCGACACGGTTAACTCCT

CCTCC

AGAAGACGGATGAGCTCGGCGACAGTGTCGCGCACCTCGCGCTCAAAGGCTACA
GGGGCC
TCTTCTTCTTCTTCAATCTCCTCTTCCATAAGGGCCTCCCCTTCTTCTTCTTCTGGCG
GC
GGTGGGGGAGGGGGGACACGGCGGCGACGACGGCGCACCGGGAGGCGGTCGAC
AAAGCGC
TCGATCATCTCCCCGCGGCGACGGCGCATGGTCTCGGTGACGGCGCGGCCGTTCT
CGCGG
GGGCGCAGTTGGAAGACGCCGCCCGTCATGTCCCGGTTATGGGTTGGCGGGGGGC
TGCCA
TGCGGCAGGGATACGGCGCTAACGATGCATCTCAACAATTGTTGTGTAGGTACTC
CGCCG
CCGAGGGACCTGAGCGAGTCCGCATCGACCGGATCGGAAAACCTCTCGAGAAAG
GCGTCT
AACCAGTCACAGTCGCAAGGTAGGCTGAGCACCGTGGCGGGCGGCAGCGGGCGG
CGGTCG
GGGTTGTTTCTGGCGGAGGTGCTGCTGATGATGTAATTAAAGTAGGCGGTCTTGA
GACGG
CGGATGGTCGACAGAAGCACCATGTCCTTGGGTCCGGCCTGCTGAATGCGCAGGC
GGTCG
GCCATGCCCCAGGCTTCGTTTTGACATCGGCGCAGGTCTTTGTAGTAGTCTTGCAT
GAGC
CTTTCTACCGGCACTTCTTCTTCTCCTTCCTCTTGTCCTGCATCTCTTGCATCTATCG
CT

GCGGCGGCGGCGGAGTTTGGCCGTAGGTGGCGCCCTCTTCCTCCCATGCGTGTGA
CCCCG
AAGCCCCTCATCGGCTGAAGCAGGGCTAGGTCGGCGACAACGCGCTCGGCTAATA
TGGCC
TGCTGCACCTGCGTGAGGGTAGACTGGAAGTCATCCATGTCCACAAAGCGGTGGT
ATGCG
CCCGTGTTGATGGTGTAAGTGCAGTTGGCCATAACGGACCAGTTAACGGTCTGGT
GACCC
GGCTGCGAGAGCTCGGTGTACCTGAGACGCGAGTAAGCCCTCGAGTCAAATACGT
AGTCG
TTGCAAGTCCGCACCAGGTACTGGTATCCCACCAAAAAGTGCGGCGGCGGCTGGC
GGTAG
AGGGGCCAGCGTAGGGTGGCCGGGGCTCCGGGGGCGAGATCTTCCAACATAAGG
CGATGA
TATCCGTAGATGTACCTGGACATCCAGGTGATGCCGGCGGCGGTGGTGGAGGCGC
GCGGA
AAGTCGCGGACGCGGTTCCAGATGTTGCGCAGCGGCAAAAAGTGCTCCATGGTCG
GGACG
CTCTGGCCGGTCAGGCGCGCGCAATCGTTGACGCTCTAGACCGTGCAAAAGGAGA
GCCTG
TAAGCGGGCACTCTTCCGTGGTCTGGTGGATAAATTCGCAAGGGTATCATGGCGG
ACGAC
CGGGGTTCGAGCCCCGTATCCGGCCGTCCGCCGTGATCCATGCGGTTACCGCCCG
CGTGT

CGAACCCAGGTGTGCGACGTCAGACAACGGGGGAGTGCTCCTTTTGGCTTCCTTC

CAGGC

GCGGCGGCTGCTGCGCTAGCTTTTTTGGCCACTGGCCGCGCGCAGCGTAAGCGGT

TAGGC

TGGAAAGCGAAAGCATTAAGTGGCTCGCTCCCTGTAGCCGGAGGGTTATTTTCCA

AGGGT

TGAGTCGCGGGACCCCCGGTTCGAGTCTCGGACCGGCCGGACTGCGGCGAACGGG

GGTTT

GCCTCCCCGTCATGCAAGACCCCGCTTGCAAATTCCTCCGGAAACAGGGACGAGC

CCCTT

TTTTGCTTTTCCCAGATGCATCCGGTGCTGCGGCAGATGCGCCCCCCTCCTCAGCA

GCGG

CAAGAGCAAGAGCAGCGGCAGACATGCAGGGCACCCTCCCCTCCTCCTACCGCGT

CAGGA

GGGGCGACATCCGCGGTTGACGCGGCAGCAGATGGTGATTACGAACCCCCGCGG

CGCCGG

GCCCGGCACTACCTGGACTTGGAGGAGGGCGAGGGCCTGGCGCGGCTAGGAGCG

CCCTCT

CCTGAGCGGTACCCAAGGGTGCAGCTGAAGCGTGATACGCGTGAGGCGTACGTGC

CGCGG

CAGAACCTGTTTCGCGACCGCGAGGGAGAGGAGCCCGAGGAGATGCGGGATCGA

AAGTTC

CACGCAGGGCGCGAGCTGCGGCATGGCCTGAATCGCGAGCGGTTGCTGCGCGAG

GAGGAC

TTTGAGCCCGACGCGCGAACCGGGATTAGTCCCGCGCGCGCACACGTGGCGGCCG

CCGAC

CTGGTAACCGCATACGAGCAGACGGTGAACCAGGAGATTAACTTTCAAAAAAGCT

TTAAC

AACCACGTGCGTACGCTTGTGGCGCGCGAGGAGGTGGCTATAGGACTGATGCATC

TGTGG

GACTTTGTAAGCGCGCTGGAGCAAAACCCAAATAGCAAGCCGCTCATGGCGCAGC

TGTTC

CTTATAGTGCAGCACAGCAGGGACAACGAGGCATTCAGGGATGCGCTGCTAAAC

ATAGTA

GAGCCCGAGGGCCGCTGGCTGCTCGATTTGATAAACATCCTGCAGAGCATAGTGG

TGCAG

GAGCGCAGCTTGAGCCTGGCTGACAAGGTGGCCGCCATCAACTATTCCATGCTTA

GCCTG

GGCAAGTTTTACGCCCGCAAGATATACCATACCCCTTACGTTCCCATAGACAAGG

AGGTA

AAGATCGAGGGGTTCTACATGCGCATGGCGCTGAAGGTGCTTACCTTGAGCGACG

ACCTG

GGCGTTTATCGCAACGAGCGCATCCACAAGGCCGTGAGCGTGAGCCGGCGGCGC

GAGCTC

AGCGACCGCGAGCTGATGCACAGCCTGCAAAGGGCCCTGGCTGGCACGGGCAGC

GGCGAT

AGAGAGGCCGAGTCCTACTTTGACGCGGGCGCTGACCTGCGCTGGGCCCCAAGCC

GACGC

GCCCTGGAGGCAGCTGGGGCCGGACCTGGGCTGGCGGTGGCACCCGCGCGCGCT
GGCAAC
GTCGGCGGCGTGGAGGAATATGACGAGGACGATGAGTACGAGCCAGAGGACGGC
GAGTAC
TAAGCGGTGATGTTTCTGATCAGATGATGCAAGACGCAACGGACCCGGCGGTGCG
GGCGG
CGCTGCAGAGCCAGCCGTCCGGCCTTAACTCCACGGACGACTGGCGCCAGGTCAT
GGACC
GCATCATGTCGCTGACTGCGCGCAATCCTGACGCGTTCCGGCAGCAGCCGCAGGC
CAACC
GGCTCTCCGCAATTCTGGAAGCGGTGGTCCCGGCGCGCGCAAACCCCACGCACGA
GAAGG
TGCTGGCGATCGTAAACGCGCTGGCCGAAAACAGGGCCATCCGGCCCGACGAGG
CCGGCC
TGGTCTACGACGCGCTGCTTCAGCGCGTGGCTCGTTACAACAGCGGCAACGTGCA
GACCA
ACCTGGACCGGCTGGTGGGGGATGTGCGCGAGGCCGTGGCGCAGCGTGAGCGCG
CGCAGC
AGCAGGGCAACCTGGGCTCCATGGTTGCACTAAACGCCTTCCTGAGTACACAGCC
CGCCA
ACGTGCCGCGGGGACAGGAGGACTACACCAACTTTGTGAGCGCACTGCGGCTAAT
GGTGA
CTGAGACACCGCAAAGTGAGGTGTACCAGTCTGGGCCAGACTATTTTTCCAGAC
CAGTA

GACAAGGCCTGCAGACCGTAAACCTGAGCCAGGCTTTCAAAAACTTGCAGGGGCT

GTGGG

GGGTGCGGGCTCCCACAGGCGACCGCGACCGTGTCTAGCTTGCTGACGCCCAA

CTCGC

GCCTGTTGCTGCTGCTAATAGCGCCCTTCACGGACAGTGGCAGCGTGTCCCGGGA

CACAT

ACCTAGGTCACTTGCTGACACTGTACCGCGAGGCCATAGGTCAGGCGCATGTGGA

CGAGC

ATACTTTCCAGGAGATTACAAGTGTCAGCCGCGCGCTGGGGCAGGAGGACACGG

GCAGCC

TGGAGGCAACCCTAAACTACCTGCTGACCAACCGGCGGCAGAAGATCCCCTCGTT

GCACA

GTTTAAACAGCGAGGAGGAGCGCATTTTGCGCTACGTGCAGCAGAGCGTGAGCCT

TAACC

TGATGCGCGACGGGGTAACGCCCAGCGTGGCGCTGGACATGACCGCGCGCAACA

TGGAAC

CGGGCATGTATGCCTCAAACCGGCCGTTTATCAACCGCCTAATGGACTACTTGCAT

CGCG

CGGCCGCCGTGAACCCCGAGTATTTCACCAATGCCATCTTGAACCCGCACTGGCT

ACCGC

CCCCTGGTTTCTACACCGGGGGATTCGAGGTGCCCGAGGGTAACGATGGATTCCT

CTGGG

ACGACATAGACGACAGCGTGTTTTCCCCGCAACCGCAGACCCTGCTAGAGTTGCA

ACAGC

GCGAGCAGGCAGAGGCGGCGCTGCGAAAGGAAAGCTTCCGCAGGCCAAGCAGCT
TGTCCG
ATCTAGGCGCTGCGGCCCCGCGGTCAGATGCTAGTAGCCCATTTCCAAGCTTGAT
AGGGT
CTCTTACCAGCACTCGCACCACCCGCCCGCGCCTGCTGGGCGAGGAGGAGTACCT
AAACA
ACTCGCTGCTGCAGCCGCAGCGCGAAAAAAACCTGCCTCCGGCATTTCCCAACAA
CGGGA
TAGAGAGCCTAGTGGACAAGATGAGTAGATGGAAGACGTACGCGCAGGAGCACA
GGGACG
TGCCAGGCCCGCGCCCGCCCACCCGTCGTCAAAGGCACGACCGTCAGCGGGGTCT
GGTGT
GGGAGGACGATGACTCGGCAGACGACAGCAGCGTCCTGGATTTGGGAGGGAGTG
GCAACC
CGTTTGCGCACCTTCGCCCCAGGCTGGGGAGAATGTTTTAAAAAAAAAAAAGCAT
GATGC
AAAATAAAAAACTCACCAAGGCCATGGCACCGAGCGTTGGTTTCTTGTATTCCC
CTTAG
TATGCGGCGCGCGGCGATGTATGAGGAAGGTCCTCCTCCCTCCTACGAGAGTGTG
GTGAG
CGCGGCGCCAGTGGCGGCGGCGCTGGGTTCTCCCTTCGATGCTCCCCTGGACCCG
CCGTT
TGTGCCTCCGCGGTACCTGCGGCCTACCGGGGGGAGAAACAGCATCCGTTACTCT
GAGTT

GGCACCCCTATTCGACACCACCCGTGTGTACCTGGTGGACAACAAGTCAACGGAT

GTGGC

ATCCCTGAACTACCAGAACGACCACAGCAACTTTCTGACCACGGTCATTCAAAAC

AATGA

CTACAGCCCGGGGGAGGCAAGCACACAGACCATCAATCTTGACGACCGGTCGCA

CTGGGG

CGGCGACCTGAAAACCATCCTGCATACCAACATGCCAAATGTGAACGAGTTCATG

TTTAC

CAATAAGTTTAAGGCGCGGGTGATGGTGTCGCGCTTGCCTACTAAGGACAATCAG

GTGGA

GCTGAAATACGAGTGGGTGGAGTTCACGCTGCCCGAGGGCAACTACTCCGAGACC

ATGAC

CATAGACCTTATGAACAACGCGATCGTGGAGCACTACTTGAAAGTGGGCAGACAG

AACGG

GGTTCTGGAAAGCGACATCGGGGTAAAGTTTGACACCCGCAACTTCAGACTGGGG

TTTGA

CCCCGTCACTGGTCTTGTCATGCCTGGGGTATATACAAACGAAGCCTTCCATCCAG

ACAT

CATTTTGCTGCCAGGATGCGGGGTGGACTTCACCCACAGCCGCCTGAGCAACTTG

TTGGG

CATCCGCAAGCGGCAACCCTTCCAGGAGGGCTTTAGGATCACCTACGATGATCTG

GAGGG

TGGTAACATTCCCGCACTGTTGGATGTGGACGCCTACCAGGCGAGCTTGAAAGAT

GACAC

CGAACAGGGCGGGGGTGGCGCAGGCGGCAGCAACAGCAGTGGCAGCGGCGCGG

AAGAGAA

CTCCAACGCGGCAGCCGCGGCAATGCAGCCGGTGGAGGACATGAACGATCATGC

CATTCG

CGGCGACACCTTTGCCACACGGGCTGAGGAGAAGCGCGCTGAGGCCGAAGCAGC

GGCCGA

AGCTGCCGCCCCCGCTGCGCAACCCGAGGTCGAGAAGCCTCAGAAGAAACCGGT

GATCAA

ACCCCTGACAGAGGACAGCAAGAAACGCAGTTACAACCTAATAAGCAATGACAG

CACCTT

CACCCAGTACCGCAGCTGGTACCTTGCATACAACTACGGCGACCCTCAGACCGGA

ATCCG

CTCATGGACCCTGCTTTGCACTCCTGACGTAACCTGCGGCTCGGAGCAGGTCTACT

GGTC

GTTGCCAGACATGATGCAAGACCCCGTGACCTTCCGCTCCACGCGCCAGATCAGC

AACTT

TCCGGTGGTGGGCGCCGAGCTGTTGCCCGTGCACTCCAAGAGCTTCTACAACGAC

CAGGC

CGTCTACTCCCAACTCATCCGCCAGTTTACCTCTCTGACCCACGTGTTCAATCGCT

TTCC

CGAGAACCAGATTTTGGCGCGCCCGCCAGCCCCCACCATCACCACCGTCAGTGAA

AACGT

TCCTGCTCTCACAGATCACGGGACGCTACCGCTGCGCAACAGCATCGGAGGAGTC

CAGCG

AGTGACCATTACTGACGCCAGACGCCGCACCTGCCCCTACGTTTACAAGGCCCTG
GGCAT

AGTCTCGCCGCGCGTCCTATCGAGCCGCACTTTTTGAGCAAGCATGTCCATCCTTA
TATC

GCCCAGCAATAACACAGGCTGGGGCCTGCGCTTCCCAAGCAAGATGTTTGGCGGG
GCCAA

GAAGCGCTCCGACCAACACCCAGTGCGCGTGCGCGGGCACTACCGCGCGCCCTGG
GGCGC

GCACAAACGCGGCCGCACTGGGCGCACCACCGTCGATGACGCCATCGACGCGGT
GGTGGA

GGAGGCGCGCAACTACACGCCCACGCCGCCACCAGTGTCCACAGTGGACGCGGC
CATTCA

GACCGTGGTGCGCGGAGCCCGGCGCTATGCTAAAATGAAGAGACGGCGGAGGCG
CGTAGC

ACGTCGCCACCGCCGCCGACCCGGCACTGCCGCCCAACGCGCGGCGGCGGCCCTG
CTTAA

CCGCGCACGTCGCACCGGCCGACGGGCGGCCATGCGGGCCGCTCGAAGGCTGGC
CGCGGG

TATTGTCACTGTGCCCCCCAGGTCCAGGCGACGAGCGGCCGCCGCAGCAGCCGCG
GCCAT

TAGTGCTATGACTCAGGGTCGCAGGGGCAACGTGTATTGGGTGCGCGACTCGGTT
AGCGG

CCTGCGCGTGCCCGTGCGCACCCGCCCCCCGCGCAACTAGATTGCAAGAAAAAAC
TACTT

AGACTCGTACTGTTGTATGTATCCAGCGGCGGCGGCGCGCAACGAAGCTATGTCC

AAGCG

CAAAATCAAAGAAGAGATGCTCCAGGTCATCGCGCCGGAGATCTATGGCCCCCCG

AAGAA

GGAAGAGCAGGATTACAAGCCCCGAAAGCTAAAGCGGGTCAAAAAGAAAAAGA

AAGATGA

TGATGATGAACTTGACGACGAGGTGGAACTGCTGCACGCTACCGCGCCCAGGCGA

CGGGT

ACAGTGGAAAGGTCGACGCGTAAAACGTGTTTTGCGACCCGGCACCACCGTAGTC

TTTAC

GCCCGGTGAGCGCTCCACCCGCACCTACAAGCGCGTGTATGATGAGGTGTACGGC

GACGA

GGACCTGCTTGAGCAGGCCAACGAGCGCCTCGGGGAGTTTGCCTACGGAAAGCG

GCATAA

GGACATGCTGGCGTTGCCGCTGGACGAGGGCAACCCAACACCTAGCCTAAAGCCC

GTAAC

ACTGCAGCAGGTGCTGCCCGCGCTTGCACCGTCCGAAGAAAAGCGCGGCCTAAAG

CGCGA

GTCTGGTGACTTGGCACCCACCGTGCAGCTGATGGTACCCAAGCGCCAGCGACTG

GAAGA

TGTCTTGGAAAAAATGACCGTGGAACCTGGGCTGGAGCCCGAGGTCCGCGTGCGG

CCAAT

CAAGCAGGTGGCGCCGGGACTGGGCGTGCAGACCGTGGACGTTCAGATACCCACT

ACCAG

TAGCACCAGTATTGCCACCGCCACAGAGGGCATGGAGACACAAACGTCCCCGGTT GCCTC

AGCGGTGGCGGATGCCGCGGTGCAGGCGGTCGCTGCGGCCGCGTCCAAGACCTCT ACGGA

GGTGCAAACGGACCCGTGGATGTTTCGCGTTTCAGCCCCCCGGCGCCCGCGCGGT TCGAG

GAAGTACGGCGCCGCCAGCGCGCTACTGCCCGAATATGCCCTACATCCTTCCATT GCGCC

TACCCCCGGCTATCGTGGCTACACCTACCGCCCCAGAAGACGAGCAACTACCCGA CGCCG

AACCACCACTGGAACCCGCCGCCGCCGTCGCCGTCGCCAGCCCGTGCTGGCCCCG ATTTC

CGTGCGCAGGGTGGCTCGCGAAGGAGGCAGGACCCTGGTGCTGCCAACAGCGCG CTACCA

CCCCAGCATCGTTTAAAAGCCGGTCTTTGTGGTTCTTGCAGATATGGCCCTCACCT GCCG

CCTCCGTTTCCCGGTGCCGGGATTCCGAGGAAGAATGCACCGTAGGAGGGGCATG GCCGG

CCACGGCCTGACGGGCGGCATGCGTCGTGCGCACCACCGGCGGCGGCGCGCGTCG CACCG

TCGCATGCGCGGCGGTATCCTGCCCCTCCTTATTCCACTGATCGCCGCGGCGATTG GCGC

CGTGCCCGGAATTGCATCCGTGGCCTTGCAGGCGCAGAGACACTGATTAAAAACA AGTTG

CATGTGGAAAAATCAAAATAAAAAGTCTGGACTCTCACGCTCGCTTGGTCCTGTA ACTAT

TTTGTAGAATGGAAGACATCAACTTTGCGTCTCTGGCCCCGCGACACGGCTCGCG CCCGT

TCATGGGAAACTGGCAAGATATCGGCACCAGCAATATGAGCGGTGGCGCCTTCAG CTGGG

GCTCGCTGTGGAGCGGCATTAAAAATTTCGGTTCCACCGTTAAGAACTATGGCAG CAAGG

CCTGGAACAGCAGCACAGGCCAGATGCTGAGGGATAAGTTGAAAGAGCAAAATT TCCAAC

AAAAGGTGGTAGATGGCCTGGCCTCTGGCATTAGCGGGGTGGTGGACCTGGCCAA CCAGG

CAGTGCAAAATAAGATTAACAGTAAGCTTGATCCCCGCCCTCCCGTAGAGGAGCC TCCAC

CGGCCGTGGAGACAGTGTCTCCAGAGGGGCGTGGCGAAAAGCGTCCGCGCCCCG ACAGGG

AAGAAACTCTGGTGACGCAAATAGACGAGCCTCCCTCGTACGAGGAGGCACTAA AGCAAG

GCCTGCCCACCACCCGTCCCATCGCGCCCATGGCTACCGGAGTGCTGGGCCAGCA CACAC

CCGTAACGCTGGACCTGCCTCCCCCCGCCGACACCCAGCAGAAACCTGTGCTGCC AGGCC

CGACCGCCGTTGTTGTAACCCGTCCTAGCCGCGCGTCCCTGCGCCGCGCCGCCAG CGGTC

CGCGATCGTTGCGGCCCGTAGCCAGTGGCAACTGGCAAAGCACACTGAACAGCAT

CGTGG

GTCTGGGGGTGCAATCCCTGAAGCGCCGACGATGCTTCTGAATAGCTAACGTGTC

GTATG

TGTGTCATGTATGCGTCCATGTCGCCGCCAGAGGAGCTGCTGAGCCGCCGCGCGC

CCGCT

TTCCAAGATGGCTACCCCTTCGATGATGCCGCAGTGGTCTTACATGCACATCTCGG

GCCA

GGACGCCTCGGAGTACCTGAGCCCCGGGCTGGTGCAGTTTGCCCGCGCCACCGAG

ACGTA

CTTCAGCCTGAATAACAAGTTTAGAAACCCCACGGTGGCGCCTACGCACGACGTG

ACCAC

AGACCGGTCCCAGCGTTTGACGCTGCGGTTCATCCCTGTGGACCGTGAGGATACT

GCGTA

CTCGTACAAGGCGCGGTTCACCCTAGCTGTGGGTGATAACCGTGTGCTGGACATG

GCTTC

CACGTACTTTGACATCCGCGGCGTGCTGGACAGGGGCCCTACTTTTAAGCCCTACT

CTGG

CACTGCCTACAACGCCCTGGCTCCCAAGGGTGCCCCAAATCCTTGCGAATGGGAT

GAAGC

TGCTACTGCTCTTGAAATAAACCTAGAAGAAGAGGACGATGACAACGAAGACGA

AGTAGA

CGAGCAAGCTGAGCAGCAAAAAACTCACGTATTTGGGCAGGCGCCTTATTCTGGT

ATAAA

TATTACAAAGGAGGGTATTCAAATAGGTGTCGAAGGTCAAACACCTAAATATGCC

GATAA

AACATTTCAACCTGAACCTCAAATAGGAGAATCTCAGTGGTACGAAACTGAAATT

AATCA

TGCAGCTGGGAGAGTCCTTAAAAAGACTACCCCAATGAAACCATGTTACGGTTCA

TATGC

AAAACCCACAAATGAAAATGGAGGGCAAGGCATTCTTGTAAAGCAACAAAATGG

AAAGCT

AGAAAGTCAAGTGGAAATGCAATTTTTCTCAACTACTGAGGCGACCGCAGGCAAT

GGTGA

TAACTTGACTCCTAAAGTGGTATTGTACAGTGAAGATGTAGATATAGAAACCCCA

GACAC

TCATATTTCTTACATGCCCACTATTAAGGAAGGTAACTCACGAGAACTAATGGGC

CAACA

ATCTATGCCCAACAGGCCTAATTACATTGCTTTTAGGGACAATTTTATTGGTCTAA

TGTA

TTACAACAGCACGGGTAATATGGGTGTTCTGGCGGGCCAAGCATCGCAGTTGAAT

GCTGT

TGTAGATTTGCAAGACAGAAACACAGAGCTTTCATACCAGCTTTTGCTTGATTCCA

TTGG

TGATAGAACCAGGTACTTTTCTATGTGGAATCAGGCTGTTGACAGCTATGATCCA

GATGT

TAGAATTATTGAAAATCATGGAACTGAAGATGAACTTCCAAATTACTGCTTTCCA

CTGGG

AGGTGTGATTAATACAGAGACTCTTACCAAGGTAAAACCTAAAACAGGTCAGGA
AAATGG
ATGGGAAAAAGATGCTACAGAATTTTCAGATAAAAATGAAATAAGAGTTGGAAA
TAATTT
TGCCATGGAAATCAATCTAAATGCCAACCTGTGGAGAAATTTCCTGTACTCCAAC
ATAGC
GCTGTATTTGCCCGACAAGCTAAAGTACAGTCCTTCCAACGTAAAAATTTCTGATA
ACCC
AAACACCTACGACTACATGAACAAGCGAGTGGTGGCTCCCGGGTTAGTGGACTGC
TACAT
TAACCTTGGAGCACGCTGGTCCCTTGACTATATGGACAACGTCAACCCATTTAACC
ACCA
CCGCAATGCTGGCCTGCGCTACCGCTCAATGTTGCTGGGCAATGGTCGCTATGTGC
CCTT
CCACATCCAGGTGCCTCAGAAGTTCTTTGCCATTAAAAACCTCCTTCTCCTGCCGG
GCTC
ATACACCTACGAGTGGAACTTCAGGAAGGATGTTAACATGGTTCTGCAGAGCTCC
CTAGG
AAATGACCTAAGGGTTGACGGAGCCAGCATTAAGTTTGATAGCATTTGCCTTTAC
GCCAC
CTTCTTCCCCATGGCCCACAACACCGCCTCCACGCTTGAGGCCATGCTTAGAAACG
ACAC
CAACGACCAGTCCTTTAACGACTATCTCTCCGCCGCCAACATGCTCTACCCTATAC
CCGC

CAACGCTACCAACGTGCCCATATCCATCCCCTCCCGCAACTGGGCGGCTTTCCGCG

GCTG

GGCCTTCACGCGCCTTAAGACTAAGGAAACCCCATCACTGGGCTCGGGCTACGAC

CCTTA

TTACACCTACTCTGGCTCTATACCCTACCTAGATGGAACCTTTTACCTCAACCACA

CCTT

TAAGAAGGTGGCCATTACCTTTGACTCTTCTGTCAGCTGGCCTGGCAATGACCGCC

TGCT

TACCCCCAACGAGTTTGAAATTAAGCGCTCAGTTGACGGGGAGGGTTACAACGTT

GCCCA

GTGTAACATGACCAAAGACTGGTTCCTGGTACAAATGCTAGCTAACTACAACATT

GGCTA

CCAGGGCTTCTATATCCCAGAGAGCTACAAGGACCGCATGTACTCCTTCTTTAGA

AACTT

CCAGCCCATGAGCCGTCAGGTGGTGGATGATACTAAATACAAGGACTACCAACAG

GTGGG

CATCCTACACCAACACAACAACTCTGGATTTGTTGGCTACCTTGCCCCCACCATGC

GCGA

AGGACAGGCCTACCCTGCTAACTTCCCCTATCCGCTTATAGGCAAGACCGCAGTT

GACAG

CATTACCCAGAAAAAGTTTCTTTGCGATCGCACCCTTTGGCGCATCCCATTCTCCA

GTAA

CTTTATGTCCATGGGCGCACTCACAGACCTGGGCCAAAACCTTCTCTACGCCAACT

CCGC

CCACGCGCTAGACATGACTTTTGAGGTGGATCCCATGGACGAGCCCACCCTTCTTT
ATGT
TTTGTTTGAAGTCTTTGACGTGGTCCGTGTGCACCGGCCGCACCGCGGCGTCATCG
AAAC
CGTGTACCTGCGCACGCCCTTCTCGGCCGGCAACGCCACAACATAAGCGATCGCA
GCAGG
TTTCCCCAACTGACACAAAACGTGCAACTTGAAACTCCGCCTGGTCTTTCCAGGTC
TAGA
GGGGTAACACTTTGTACTGCGTTTGGCTCCACGCTCGATCCACTGGCGAGTGTTAG
TAAC
AGCACTGTTGCTTCGTAGCGGAGCATGACGGCCGTGGGAACTCCTCCTTGGTAAC
AAGGA
CCCACGGGGCCAAAAGCCACGCCCACACGGGCCCGTCATGTGTGCAACCCCAGCA
CGGCG
ACTTTACTGCGAAACCCACTTTAAAGTGACATTGAAACTGGTACCCACACACTGG
TGACA
GGCTAAGGATGCCCTTCAGGTACCCCGAGGTAACACGCGACACTCGGGATCTGAG
AAGGG
GACTGGGGCTTCTATAAAAGCGCTCGGTTTAAAAAGCTTCTATGCCTGAATANGT
GACCG
GANGTCGGCACCTTTCCTTTGCAATTAATGACCCTGTATACGCCACCATGGCTATG
ATGG
AGGTCCAGGGGGGACCCAGCCTGGGACAGACCTGCGTGCTGATCGTGATCTTTAC
AGTGC

TCCTGCAGTCTCTCTGTGTGGCTGTAACTTACGTGTACTTTACCAACGAGCTGAAG
CAGA

TGCAGGACAAGTACTCCAAAAGTGGCATTGCTTGTTTCTTAAAAGAAGATGACAG
TTATT

GGGACCCCAATGACGAAGAGAGTATGAACAGCCCCTGCTGGCAAGTCAAGTGGC
AACTCC

GTCAGCTCGTTAGAAAGATGATTTTGAGAACCTCTGAGGAAACCATTTCTACAGT
TCAAG

AAAAGCAACAAAATATTTCTCCCCTAGTGAGAGAAAGAGGTCCTCAGAGAGTAG
CAGCTC

ACATAACTGGGACCAGAGGAAGAAGCAACACATTGTCTTCTCCAAACTCCAAGAA
TGAAA

AGGCTCTGGGCCGCAAAATAAACTCCTGGGAATCATCAAGGAGTGGGCATTCATT
CCTGA

GCAACTTGCACTTGAGGAATGGTGAACTGGTCATCCATGAAAAAGGGTTTTACTA
CATCT

ATTCCCAAACATACTTTCGATTTCAGGAGGAAATAAAAGAAAACACAAAGAACG
ACAAAC

AAATGGTCCAATATATTTACAAATACACAAGTTATCCTGACCCTATATTGTTGATG
AAAA

GTGCTAGAAATAGTTGTTGGTCTAAAGATGCAGAATATGGACTCTATTCCATCTAT
CAAG

GGGGAATATTTGAGCTTAAGGAAAATGACAGAATTTTTGTTTCTGTAACAAATGA
GCACT

TAATAGACATGGACCATGAAGCCAGTTTTTCGGGGCCTTTTAGTTGGCTAAGTA

TACT

TCGAATGCATGCGATCGCAGAAGCAAGCAACATCAACAACAGCTGCCGCCATGG

GCTCCA

GTGAGCAGGAACTGAAAGCCATTGTCAAAGATCTTGGTTGTGGGCCATATTTTTT

GGGCA

CCTATGACAAGCGCTTTCCAGGCTTTGTTTCTCCACACAAGCTCGCCTGCGCCATA

GTCA

ATACGGCCGGTCGCGAGACTGGGGGCGTACACTGGATGGCCTTTGCCTGGAACCC

GCACT

CAAAAACATGCTACCTCTTTGAGCCCTTTGGCTTTTCTGACCAGCGACTCAAGCAG

GTTT

ACCAGTTTGAGTACGAGTCACTCCTGCGCCGTAGCGCCATTGCTTCTTCCCCCGAC

CGCT

GTATAACGCTGGAAAAGTCCACCCAAAGCGTACAGGGGCCCAACTCGGCCGCCTG

TGGAC

TATTCTGCTGCATGTTTCTCCACGCCTTTGCCAACTGGCCCCAAACTCCCATGGAT

CACA

ACCCCACCATGAACCTTATTACCGGGGTACCCAACTCCATGCTCAACAGTCCCCA

GGTAC

AGCCCACCCTGCGTCGCAACCAGGAACAGCTCTACAGCTTCCTGGAGCGCCACTC

GCCCT

ACTTCCGCAGCCACAGTGCGCAGATTAGGAGCGCCACTTCTTTTTGTCACTTGAAA

AACA

TGTAAAAAATAATGTACTAGAGACACTTTCAATAAAGGCAAATGCTTTTATTTGT ACACT

CTCGGGTGATTATTTACCCCCACCCTTGCCGTCTGCGCCGTTTAAAAATCAAAGGG GTTC

TGCCGCGCATCGCTATGCGCCACTGGCAGGGACACGTTGCGATACTGGTGTTTAG TGCTC

CACTTAAACTCAGGCACAACCATCCGCGGCAGCTCGGTGAAGTTTTCACTCCACA GGCTG

CGCACCATCACCAACGCGTTTAGCAGGTCGGGCGCCGATATCTTGAAGTCGCAGT TGGGG

CCTCCGCCCTGCGCGCGCGAGTTGCGATACACAGGGTTGCAGCACTGGAACACTA TCAGC

GCCGGGTGGTGCACGCTGGCCAGCACGCTCTTGTCGGAGATCAGATCCGCGTCCA GGTCC

TCCGCGTTGCTCAGGGCGAACGGAGTCAACTTTGGTAGCTGCCTTCCCAAAAAGG GCGCG

TGCCCAGGCTTTGAGTTGCACTCGCACCGTAGTGGCATCAAAAGGTGACCGTGCC CGGTC

TGGGCGTTAGGATACAGCGCCTGCATAAAAGCCTTGATCTGCTTAAAAGCCACCT GAGCC

TTTGCGCCTTCAGAGAAGAACATGCCGCAAGACTTGCCGGAAAACTGATTGGCCG GACAG

GCCGCGTCGTGCACGCAGCACCTTGCGTCGGTGTTGGAGATCTGCACCACATTTC GGCCC

CACCGGTTCTTCACGATCTTGGCCTTGCTAGACTGCTCCTTCAGCGCGCGCTGCCC

GTTT

TCGCTCGTCACATCCATTTCAATCACGTGCTCCTTATTTATCATAATGCTTCCGTGT

AGA

CACTTAAGCTCGCCTTCGATCTCAGCGCAGCGGTGCAGCCACAACGCGCAGCCCG

TGGGC

TCGTGATGCTTGTAGGTCACCTCTGCAAACGACTGCAGGTACGCCTGCAGGAATC

GCCCC

ATCATCGTCACAAAGGTCTTGTTGCTGGTGAAGGTCAGCTGCAACCCGCGGTGCT

CCTCG

TTCAGCCAGGTCTTGCATACGGCCGCCAGAGCTTCCACTTGGTCAGGCAGTAGTTT

GAAG

TTCGCCTTTAGATCGTTATCCACGTGGTACTTGTCCATCAGCGCGCGCGCAGCCTC

CATG

CCCTTCTCCCACGCAGACACGATCGGCACACTCAGCGGGTTCATCACCGTAATTTC

ACTT

TCCGCTTCGCTGGGCTCTTCCTCTTCCTCTTGCGTCCGCATACCACGCGCCACTGG

GTCG

TCTTCATTCAGCCGCCGCACTGTGCGCTTACCTCCTTTGCCATGCTTGATTAGCAC

CGGT

GGGTTGCTGAAACCCACCATTTGTAGCGCCACATCTTCTCTTTCTTCCTCGCTGTCC

ACG

ATTACCTCTGGTGATGGCGGGCGCTCGGGCTTGGGAGAAGGGCGCTTCTTTTTCTT

CTTG

GGCGCAATGGCCAAATCCGCCGCCGAGGTCGATGGCCGCGGGCTGGGTGTGCGC

GGCACC

AGCGCGTCTTGTGATGAGTCTTCCTCGTCCTCGGACTCGATACGCCGCCTCATCCG

CTTT

TTTGGGGGCGCCCGGGGAGGCGGCGGCGACGGGGACGGGGACGACACGTCCTCC

ATGGTT

GGGGGACGTCGCGCCGCACCGCGTCCGCGCTCGGGGGTGGTTTCGCGCTGCTCCT

CTTCC

CGACTGGCCATTTCCTTCTCCTATAGGCAGAAAAAGATCATGGAGTCAGTCGAGA

AGAAG

GACAGCCTAACCGCCCCCTCTGAGTTCGCCACCACCGCCTCCACCGATGCCGCCA

ACGCG

CCTACCACCTTCCCCGTCGAGGCACCCCCGCTTGAGGAGGAGGAAGTGATTATCG

AGCAG

GACCCAGGTTTTGTAAGCGAAGACGACGAGGACCGCTCAGTACCAACAGAGGAT

AAAAAG

CAAGACCAGGACAACGCAGAGGCAAACGAGGAACAAGTCGGGCGGGGGGACGA

AAGGCAT

GGCGACTACCTAGATGTGGGAGACGACGTGCTGTTGAAGCATCTGCAGCGCCAGT

GCGCC

ATTATCTGCGACGCGTTGCAAGAGCGCAGCGATGTGCCCCTCGCCATAGCGGATG

TCAGC

CTTGCCTACGAACGCCACCTATTCTCACCGCGCGTACCCCCCAAACGCCAAGAAA

ACGGC

ACATGCGAGCCCAACCCGCGCCTCAACTTCTACCCCGTATTTGCCGTGCCAGAGG

TGCTT

GCCACCTATCACATCTTTTTCCAAAACTGCAAGATACCCCTATCCTGCCGTGCCAA

CCGC

AGCCGAGCGGACAAGCAGCTGGCCTTGCGGCAGGGCGCTGTCATACCTGATATCG

CCTCG

CTCAACGAAGTGCCAAAAATCTTTGAGGGTCTTGGACGCGACGAGAAGCGCGCG

GCAAAC

GCTCTGCAACAGGAAAACAGCGAAAATGAAAGTCACTCTGGAGTGTTGGTGGAA

CTCGAG

GGTGACAACGCGCGCCTAGCCGTACTAAAACGCAGCATCGAGGTCACCCACTTTG

CCTAC

CCGGCACTTAACCTACCCCCCAAGGTCATGAGCACAGTCATGAGTGAGCTGATCG

TGCGC

CGTGCGCAGCCCCTGGAGAGGGATGCAAATTTGCAAGAACAAACAGAGGAGGGC

CTACCC

GCAGTTGGCGACGAGCAGCTAGCGCGCTGGCTTCAAACGCGCGAGCCTGCCGACT

TGGAG

GAGCGACGCAAACTAATGATGGCCGCAGTGCTCGTTACCGTGGAGCTTGAGTGCA

TGCAG

CGGTTCTTTGCTGACCCGGAGATGCAGCGCAAGCTAGAGGAAACATTGCACTACA

CCTTT

CGACAGGGCTACGTACGCCAGGCCTGCAAGATCTCCAACGTGGAGCTCTGCAACC

TGGTC

TCCTACCTTGGAATTTTGCACGAAAACCGCCTTGGGCAAAACGTGCTTCATTCCAC

GCTC

AAGGGCGAGGCGCGCCGCGACTACGTCCGCGACTGCGTTTACTTATTTCTATGCT

ACACC

TGGCAGACGGCCATGGGCGTTTGGCAGCAGTGCTTGGAGGAGTGCAACCTCAAGG

AGCTG

CAGAAACTGCTAAAGCAAAACTTGAAGGACCTATGGACGGCCTTCAACGAGCGCT

CCGTG

GCCGCGCACCTGGCGGACATCATTTTCCCCGAACGCCTGCTTAAAACCCTGCAAC

AGGGT

CTGCCAGACTTCACCAGTCAAAGCATGTTGCAGAACTTTAGGAACTTTATCCTAG

AGCGC

TCAGGAATCTTGCCCGCCACCTGCTGTGCACTTCCTAGCGACTTTGTGCCCATTAA

GTAC

CGCGAATGCCCTCCGCCGCTTTGGGGCCACTGCTACCTTCTGCAGCTAGCCAACTA

CCTT

GCCTACCACTCTGACATAATGGAAGACGTGAGCGGTGACGGTCTACTGGAGTGTC

ACTGT

CGCTGCAACCTATGCACCCCGCACCGCTCCCTGGTTTGCAATTCGCAGCTGCTTAA

CGAA

AGTCAAATTATCGGTACCTTTGAGCTGCAGGGTCCCTCGCCTGACGAAAAGTCCG

CGGCT

CCGGGGTTGAAACTCACTCCGGGGCTGTGGACGTCGGCTTACCTTCGCAAATTTGT

ACCT

GAGGACTACCACGCCCACGAGATTAGGTTCTACGAAGACCAATCCCGCCCGCCAA ATGCG

GAGCTTACCGCCTGCGTCATTACCCAGGGCCACATTCTTGGCCAATTGCAAGCCAT CAAC

AAAGCCCGCCAAGAGTTTCTGCTACGAAAGGGACGGGGGTTTACTTGGACCCCC AGTCC

GGCGAGGAGCTCAACCCAATCCCCCCGCCGCCGCAGCCCTATCAGCAGCAGCCGC GGGCC

CTTGCTTCCCAGGATGGCACCCAAAAAGAAGCTGCAGCTGCCGCCGCCACCCACG GACGA

GGAGGAATACTGGGACAGTCAGGCAGAGGAGGTTTTGGACGAGGAGGAGGAGGA CATGAT

GGAAGACTGGGAGAGCCTAGACGAGGAAGCTTCCGAGGTCGAAGAGGTGTCAGA CGAAAC

ACCGTCACCCTCGGTCGCATTCCCCTCGCCGGCGCCCCAGAAATCGGCAACCGGT TCCAG

CATGGCTACAACCTCCGCTCCTCAGGCGCCGCCGGCACTGCCCGTTCGCCGACCC AACCG

TAGATGGGACACCACTGGAACCAGGGCCGGTAAGTCCAAGCAGCCGCCGCCGTT AGCCCA

AGAGCAACAACAGCGCCAAGGCTACCGCTCATGGCGCGGGCACAAGAACGCCAT AGTTGC

TTGCTTGCAAGACTGTGGGGGCAACATCTCCTTCGCCCGCCGCTTTCTTCTCTACC ATCA

CGGCGTGGCCTTCCCCCGTAACATCCTGCATTACTACCGTCATCTCTACAGCCCAT ACTG

CACCGGCGGCAGCGGCAGCGGCAGCAACAGCAGCGGCCACACAGAAGCAAAGGC GACCGG

ATAGCAAGACTCTGACAAAGCCCAAGAAATCCACAGCGGCGGCAGCAGCAGGAG GAGGAG

CGCTGCGTCTGGCGCCCAACGAACCCGTATCGACCCGCGAGCTTAGAAACAGGAT TTTTC

CCACTCTGTATGCTATATTTCAACAGAGCAGGGGCCAAGAACAAGAGCTGAAAAT AAAAA

ACAGGTCTCTGCGATCCCTCACCCGCAGCTGCCTGTATCACAAAAGCGAAGATCA GCTTC

GGCGCACGCTGGAAGACGCGGAGGCTCTCTTCAGTAAATACTGCGCGCTGACTCT TAAGG

ACTAGTTTCGCGCCCTTTCTCAAATTTAAGCGCGAAAACTACGTCATCTCCAGCGG CCAC

ACCCGGCGCCAGCACCTGTCGTCAGCGCCATTATGAGCAAGGAAATTCCCACGCC CTACA

TGTGGAGTTACCAGCCACAAATGGGACTTGCGGCTGGAGCTGCCCAAGACTACTC AACCC

GAATAAACTACATGAGCGCGGACCCCACATGATATCCCGGGTCAACGGAATCCG CGCCC

ACCGAAACCGAATTCTCTTGGAACAGGCGGCTATTACCACCACACCTCGTAATAA CCTTA

ATCCCCGTAGTTGGCCCGCTGCCCTGGTGTACCAGGAAAGTCCCGCTCCCACCACT GTGG

TACTTCCCAGAGACGCCCAGGCCGAAGTTCAGATGACTAACTCAGGGGCGCAGCT TGCGG

GCGGCTTTCGTCACAGGGTGCGGTCGCCCGGGCAGGGTATAACTCACCTGACAAT CAGAG

GGCGAGGTATTCAGCTCAACGACGAGTCGGTGAGCTCCTCGCTTGGTCTCCGTCC GGACG

GGACATTTCAGATCGGCGGCGCCGGCCGTCCTTCATTCACGCCTCGTCAGGCAAT CCTAA

CTCTGCAGACCTCGTCCTCTGAGCCGCGCTCTGGAGGCATTGGAACTCTGCAATTT ATTG

AGGAGTTTGTGCCATCGGTCTACTTTAACCCCTTCTCGGGACCTCCCGGCCACTAT CCGG

ATCAATTTATTCCTAACTTTGACGCGGTAAAGGACTCGGCGGACGGCTACGACTG AATGT

TAAGTGGAGAGGCAGAGCAACTGCGCCTGAAACACCTGGTCCACTGTCGCCGCCA CAAGT

GCTTTGCCCGCGACTCCGGTGAGTTTTGCTACTTTGAATTGCCCGAGGATCATATC GAGG

GCCCGGCGCACGGCGTCCGGCTTACCGCCCAGGGAGAGCTTGCCCGTAGCCTGAT TCGGG

AGTTTACCCAGCGCCCCCTGCTAGTTGAGCGGGACAGGGGACCCTGTGTTCTCAC TGTGA

TTTGCAACTGTCCTAACCTTGGATTACATCAAGATCTTTGTTGCCATCTCTGTGCTG

AGT

ATAATAAATACAGAAATTAAAATATACTGGGGCTCCTATCGCCATCCTGTAAACG

CCACC

GTCTTCACCCGCCCAAGCAAACCAAGGCGAACCTTACCTGGTACTTTTAACATCTC

TCCC

TCTGTGATTTACAACAGTTTCAACCCAGACGGAGTGAGTCTACGAGAGAACCTCT

CCGAG

CTCAGCTACTCCATCAGAAAAAACACCACCCTCCTTACCTGCCGGGAACGTACGA

GTGCG

TCACCGGCCGCTGCACCACACCTACCGCCTGACCGTAAACCAGACTTTTTCCGGA

CAGAC

CTCAATAACTCTGTTTACCAGAACAGGAGGTGAGCTTAGAAAACCCTTAGGGTAT

TAGGC

CAAAGGCGCAGCTACTGTGGGGTTTATGAACAATTCAAGCAACTCTACGGGCTAT

TCTAA

TTCAGGTTTCTCTAGAATCGGGGTTGGGGTTATTCTCTGTCTTGTGATTCTCTTTAT

TCT

TATACTAACGCTTCTCTGCCTAAGGCTCGCCGCCTGCTGTGTGCACATTTGCATTT

ATTG

TCAGCTTTTTAAACGCTGGGGTCGCCACCCAAGATGATTAGGTACATAATCCTAG

GTTTA

CTCACCCTTGCGTCAGCCCACGGTACCACCCAAAAGGTGGATTTTAAGGAGCCAG

CCTGT

AATGTTACATTCGCAGCTGAAGCTAATGAGTGCACCACTCTTATAAAATGCACCA CAGAA

CATGAAAAGCTGCTTATTCGCCACAAAAACAAAATTGGCAAGTATGCTGTTTATG CTATT

TGGCAGCCAGGTGACACTACAGAGTATAATGTTACAGTTTTCCAGGGTAAAAGTC ATAAA

ACTTTTATGTATACTTTTCCATTTTATGAAATGTGCGACATTACCATGTACATGAG CAAA

CAGTATAAGTTGTGGCCCCCACAAAATTGTGTGGAAAACACTGGCACTTTCTGCT GCACT

GCTATGCTAATTACAGTGCTCGCTTTGGTCTGTACCCTACTCTATATTAAATACAA AAGC

AGACGCAGCTTTATTGAGGAAAAGAAAATGCCTTAATTTACTAAGTTACAAAGCT AATGT

CACCACTAACTGCTTTACTCGCTGCTTGCAAAACAAATTCAAAAAGTTAGCATTAT AATT

AGAATAGGATTTAAACCCCCCGGTCATTTCCTGCTCAATACCATTCCCCTGAACAA TTGA

CTCTATGTGGGATATGCTCCAGCGCTACAACCTTGAAGTCAGGCTTCCTGGATGTC AGCA

TCTGACTTTGGCCAGCACCTGTCCCGCGGATTTGTTCCAGTCCAACTACAGCGACC CACC

CTAACAGAGATGACCAACACAACCAACGCGGCCGCCGCTACCGGACTTACATCTA CCACA

AATACACCCCAAGTTTCTGCCTTTGTCAATAACTGGGATAACTTGGGCATGTGGTG GTTC

TCCATAGCGCTTATGTTTGTATGCCTTATTATTATGTGGCTCATCTGCTGCCTAAAG CGC

AAACGCGCCCGACCACCCATCTATAGTCCCATCATTGTGCTACACCCAAACAATG ATGGA

ATCCATAGATTGGACGGACTGAAACACATGTTCTTTTCTCTTACAGTATGATTAAA TGAG

ACATGATTCCTCGAGTTTTTATATTACTGACCCTTGTTGCGCTTTTTTGTGCGTGCT CCA

CATTGGCTGCGGTTTCTCACATCGAAGTAGACTGCATTCCAGCCTTCACAGTCTAT TTGC

TTTACGGATTTGTCACCCTCACGCTCATCTGCAGCCTCATCACTGTGGTCATCGCC TTTA

TCCAGTGCATTGACTGGGTCTGTGTGCGCTTTGCATATCTCAGACACCATCCCCAG TACA

GGGACAGGACTATAGCTGAGCTTCTTAGAATTCTTTAATTATGAAATTTACTGTGA CTTT

TCTGCTGATTATTTGCACCCTATCTGCGTTTTGTTCCCCGACCTCCAAGCCTCAAA GACA

TATATCATGCAGATTCACTCGTATATGGAATATTCCAAGTTGCTACAATGAAAAA AGCGA

TCTTTCCGAAGCCTGGTTATATGCAATCATCTCTGTTATGGTGTTCTGCAGTACCA TCTT

AGCCCTAGCTATATATCCCTACCTTGACATTGGCTGGAACGCAATAGATGCCATG AACCA

CCCAACTTTCCCCGCGCCCGCTATGCTTCCACTGCAACAAGTTGTTGCCGGCGGCT TTGT

CCCAGCCAATCAGCCTCGCCCACCTTCTCCCACCCCCACTGAAATCAGCTACTTTA ATCT

AACAGGAGGAGATGACTGACACCCTAGATCTAGAAATGGACGGAATTATTACAG AGCAGC

GCCTGCTAGAAAGACGCAGGGCAGCGGCCGAGCAACAGCGCATGAATCAAGAGC TCCAAG

ACATGGTTAACTTGCACCAGTGCAAAAGGGGTATCTTTTGTCTGGTAAAGCAGGC CAAAG

TCACCTACGACAGTAATACCACCGGACACCGCCTTAGCTACAAGTTGCCAACCAA GCGTC

AGAAATTGGTGGTCATGGTGGGAGAAAAGCCCATTACCATAACTCAGCACTCGGT AGAAA

CCGAAGGCTGCATTCACTCACCTTGTCAAGGACCTGAGGATCTCTGCACCCTTATT AAGA

CCCTGTGCGGTCTCAAAGATCTTATTCCCTTTAACTAATAAAAAAAAATAATAAA GCATC

ACTTACTTAAAATCAGTTAGCAAATTTCTGTCCAGTTTATTCAGCAGCACCTCCTT GCCC

TCCTCCCAGCTCTGGTATTGCAGCTTCCTCCTGGCTGCAAACTTTCTCCACAATCT AAAT

GGAATGTCAGTTTCCTCCTGTTCCTGTCCATCCGCACCCACTATCTTCATGTTGTTG

CAG

ATGAAGCGCGCAAGACCGTCTGAAGATACCTTCAACCCCGTGTATCCATATGACA

CGGAA

ACCGGTCCTCCAACTGTGCCTTTTCTTACTCCTCCCTTTGTATCCCCCAATGGGTTT

CAA

GAGAGTCCCCCTGGGGTACTCTCTTTGCGCCTATCCGAACCTCTAGTTACCTCCAA

TGGC

ATGCTTGCGCTCAAAATGGGCAACGGCCTCTCTCTGGACGAGGCCGGCAACCTTA

CCTCC

CAAAATGTAACCACTGTGAGCCCACCTCTCAAAAAAACCAAGTCAAACATAAACC

TGGAA

ATATCTGCACCCCTCACAGTTACCTCAGAAGCCCTAACTGTGGCTGCCGCCGCACC

TCTA

ATGGTCGCGGGCAACACACTCACCATGCAATCACAGGCCCCGCTAACCGTGCACG

ACTCC

AAACTTAGCATTGCCACCCAAGGACCCCTCACAGTGTCAGAAGGAAAGCTAGCCC

TGCAA

ACATCAGGCCCCCTCACCACCACCGATAGCAGTACCCTTACTATCACTGCCTCACC

CCCT

CTAACTACTGCCACTGGTAGCTTGGGCATTGACTTGAAAGAGCCCATTTATACAC

AAAAT

GGAAAACTAGGACTAAAGTACGGGGCTCCTTTGCATGTAACAGACGACCTAAACA

CTTTG

ACCGTAGCAACTGGTCCAGGTGTGACTATTAATAATACTTCCTTGCAAACTAAAG

TTACT

GGAGCCTTGGGTTTTGATTCACAAGGCAATATGCAACTTAATGTAGCAGGAGGAC

TAAGG

ATTGATTCTCAAAACAGACGCCTTATACTTGATGTTAGTTATCCGTTTGATGCTCA

AAAC

CAACTAAATCTAAGACTAGGACAGGGCCCTCTTTTTATAAACTCAGCCCACAACT

TGGAT

ATTAACTACAACAAAGGCCTTTACTTGTTTACAGCTTCAAACAATTCCAAAAAGCT

TGAG

GTTAACCTAAGCACTGCCAAGGGGTTGATGTTTGACGCTACAGCCATAGCCATTA

ATGCA

GGAGATGGGCTTGAATTTGGTTCACCTAATGCACCAAACACAAATCCCCTCAAAA

CAAAA

ATTGGCCATGGCCTAGAATTTGATTCAAACAAGGCTATGGTTCCTAAACTAGGAA

CTGGC

CTTAGTTTTGACAGCACAGGTGCCATTACAGTAGGAAACAAAAATAATGATAAGC

TAACT

TTGTGGACCACACCAGCTCCATCTCCTAACTGTAGACTAAATGCAGAGAAAGATG

CTAAA

CTCACTTTGGTCTTAACAAAATGTGGCAGTCAAATACTTGCTACAGTTTCAGTTTT

GGCT

GTTAAAGGCAGTTTGGCTCCAATATCTGGAACAGTTCAAAGTGCTCATCTTATTAT

AAGA

TTTGACGAAAATGGAGTGCTACTAAACAATTCCTTCCTGGACCCAGAATATTGGA

ACTTT

AGAAATGGAGATCTTACTGAAGGCACAGCCTATACAAACGCTGTTGGATTTATGC

CTAAC

CTATCAGCTTATCCAAAATCTCACGGTAAAACTGCCAAAAGTAACATTGTCAGTC

AAGTT

TACTTAAACGGAGACAAAACTAAACCTGTAACACTAACCATTACACTAAACGGTA

CACAG

GAAACAGGAGACACAACTCCAAGTGCATACTCTATGTCATTTTCATGGGACTGGT

CTGGC

CACAACTACATTAATGAAATATTTGCCACATCCTCTTACACTTTTTCATACATTGC

CCAA

GAATAAAGAATCGTTTGTGTTATGTTTCAACGTGTTTATTTTTCAATTGCAGAAAA

TTTC

AAGTCATTTTTCATTCAGTAGTATAGCCCCACCACCACATAGCTTATACAGATCAC

CGTA

CCTTAATCAAACTCACAGAACCCTAGTATTCAACCTGCCACCTCCCTCCCAACACA

CAGA

GTACACAGTCCTTTCTCCCCGGCTGGCCTTAAAAAGCATCATATCATGGGTAACA

GACAT

ATTCTTAGGTGTTATATTCCACACGGTTTCCTGTCGAGCCAAACGCTCATCAGTGA

TATT

AATAAACTCCCCGGGCAGCTCACTTAAGTTCATGTCGCTGTCCAGCTGCTGAGCC

ACAGG

CTGCTGTCCAACTTGCGGTTGCTTAACGGGCGGCGAAGGAGAAGTCCACGCCTAC

ATGGG

GGTAGAGTCATAATCGTGCATCAGGATAGGGCGGTGGTGCTGCAGCAGCGCGCG

AATAAA

CTGCTGCCGCCGCCGCTCCGTCCTGCAGGAATACAACATGGCAGTGGTCTCCTCA

GCGAT

GATTCGCACCGCCCGCAGCATAAGGCGCCTTGTCCTCCGGGCACAGCAGCGCACC

CTGAT

CTCACTTAAATCAGCACAGTAACTGCAGCACAGCACCACAATATTGTTCAAAATC

CCACA

GTGCAAGGCGCTGTATCCAAAGCTCATGGCGGGGACCACAGAACCCACGTGGCC

ATCATA

CCACAAGCGCAGGTAGATTAAGTGGCGACCCCTCATAAACACGCTGGACATAAAC

ATTAC

CTCTTTTGGCATGTTGTAATTCACCACCTCCCGGTACCATATAAACCTCTGATTAA

ACAT

GGCGCCATCCACCACCATCCTAAACCAGCTGGCCAAAACCTGCCCGCCGGCTATA

CACTG

CAGGGAACCGGGACTGGAACAATGACAGTGGAGAGCCCAGGACTCGTAACCATG

GATCAT

CATGCTCGTCATGATATCAATGTTGGCACAACACAGGCACACGTGCATACACTTC

CTCAG

GATTACAAGCTCCTCCCGCGTTAGAACCATATCCCAGGGAACAACCCATTCCTGA

ATCAG

CGTAAATCCCACACTGCAGGGAAGACCTCGCACGTAACTCACGTTGTGCATTGTC
AAAGT

GTTACATTCGGGCAGCAGCGGATGATCCTCCAGTATGGTAGCGCGGGTTTCTGTCT
CAAA

AGGAGGTAGACGATCCCTACTGTACGGAGTGCGCCGAGACAACCGAGATCGTGTT
GGTCG

TAGTGTCATGCCAAATGGAACGCCGGACGTAGTCATATTTCCTGAAGCAAAACCA
GGTGC

GGGCGTGACAAACAGATCTGCGTCTCCGGTCTCGCCGCTTAGATCGCTCTGTGTA
GTAGT

TGTAGTATATCCACTCTCTCAAAGCATCCAGGCGCCCCCTGGCTTCGGGTTCTATG
TAAA

CTCCTTCATGCGCCGCTGCCCTGATAACATCCACCACCGCAGAATAAGCCACACC
CAGCC

AACCTACACATTCGTTCTGCGAGTCACACACGGGAGGAGCGGGAAGAGCTGGAA
GAACCA

TGTTTTTTTTTTTATTCCAAAAGATTATCCAAAACCTCAAAATGAAGATCTATTAA
GTGA

ACGCGCTCCCCTCCGGTGGCGTGGTCAAACTCTACAGCCAAAGAACAGATAATGG
CATTT

GTAAGATGTTGCACAATGGCTTCCAAAAGGCAAACGGCCCTCACGTCCAAGTGGA
CGTAA

AGGCTAAACCCTTCAGGGTGAATCTCCTCTATAAACATTCCAGCACCTTCAACCAT
GCCC

AAATAATTCTCATCTCGCCACCTTCTCAATATATCTCTAAGCAAATCCCGAATATT AAGT

CCGGCCATTGTAAAAATCTGCTCCAGAGCGCCCTCCACCTTCAGCCTCAAGCAGC GAATC

ATGATTGCAAAAATTCAGGTTCCTCACAGACCTGTATAAGATTCAAAAGCGGAAC ATTAA

CAAAAATACCGCGATCCCGTAGGTCCCTTCGCAGGGCCAGCTGAACATAATCGTG CAGGT

CTGCACGGACCAGCGCGGCCACTTCCCCGCCAGGAACCTTGACAAAAGAACCCAC ACTGA

TTATGACACGCATACTCGGAGCTATGCTAACCAGCGTAGCCCCGATGTAAGCTTT GTTGC

ATGGGCGGCGATATAAAATGCAAGGTGCTGCTCAAAAAATCAGGCAAAGCCTCG CGCAAA

AAAGAAAGCACATCGTAGTCATGCTCATGCAGATAAAGGCAGGTAAGCTCCGGA ACCACC

ACAGAAAAAGACACCATTTTTCTCTCAAACATGTCTGCGGGTTTCTGCATAAACA CAAAA

TAAAATAACAAAAAAACATTTAAACATTAGAAGCCTGTCTTACAACAGGAAAAA CAACCC

TTATAAGCATAAGACGGACTACGGCCATGCCGGCGTGACCGTAAAAAAACTGGTC ACCGT

GATTAAAAAGCACCACCGACAGCTCCTCGGTCATGTCCGGAGTCATAATGTAAGA CTCGG

TAAACACATCAGGTTGATTCATCGGTCAGTGCTAAAAAGCGACCGAAATAGCCCG

GGGGA

ATACATACCCGCAGGCGTAGAGACAACATTACAGCCCCCATAGGAGGTATAACA

AAATTA

ATAGGAGAGAAAAACACATAAACACCTGAAAAACCCTCCTGCCTAGGCAAAATA

GCACCC

TCCCGCTCCAGAACAACATACAGCGCTTCCACAGCGGCAGCCATAACAGTCAGCC

TTACC

AGTAAAAAAGAAAACCTATTAAAAAAACACCACTCGACACGGCACCAGCTCAAT

CAGTCA

CAGTGTAAAAAAGGGCCAAGTGCAGAGCGAGTATATATAGGACTAAAAAATGAC

GTAACG

GTTAAAGTCCACAAAAAACACCCAGAAAACCGCACGCGAACCTACGCCCAGAAA

CGAAAG

CCAAAAAACCCACAACTTCCTCAAATCGTCACTTCCGTTTTCCCACGTTACGTCAC

TTCC

CATTTTAATTAAGAAAACTACAATTCCCAACACATACAAGTTACTCCGCCCTAAA

ACCTA

CGTCACCCGCCCCGTTCCCACGCCCCGCGCCACGTCACAAACTCCACCCCCTCATT

ATCA

TATTGGCTTCAATCCAAAATAAGGTATATTATTGATGATGATTACCCTGTTAT

SEQ ID NO:51 is the OV1160 sequence

AGGGTAATCATCATCAATAATATACCTTATTTTGGATTGAAGCCAATATGATAATG

AGGG

GGTGGAGTTTGTGACGTGGCGCGGGGCGTGGGAACGGGGCGGGTGACGTAGTAG

TGTGGC

GGAAGTGTGATGTTGCAAGTGTGGCGGAACACATGTAAGCGACGGATGTGGCAA

AAGTGA

CGTTTTTGGTGTGCGCCGGTGTACACAGGAAGTGACAATTTTCGCGCGGTTTTAGG

CGGA

TGTTGTAGTAAATTTGGGCGTAACCGAGTAAGATTTGGCCATTTTCGCGGGAAAA

CTGAA

TAAGAGGAAGTGAAATCTGAATAATTTTGTGTTACTCATAGCGCGTAATATTTGTC

TAGG

GCCGGGATCTCTGCAGGAATTTGATATCAAGCTTATCGATACCGTCGAAACTTGTT

TATT

GCAGCTTATAATGGTTACAAATAAAGCAATAGCATCACAAATTTCACAAATAAAG

CATTT

TTTTCACTGCATTCTAGTTGTGGTTTGTCCAAACTCATCAATGTATCTTATCATGTC

TGG

ATCCGCTAGCGGCGCGCCGTTTCATCCGGACAAAGCCTGCGCGCGCCCCGCCCCG

CCATT

GGCCGTACCGCCCCGCGCCGCCGCCCCATCTCGCCCCTCGCCGCCGGGTCCGGCG

CGTTA

AAGCCAATAGGAACCGCCGCCGTTGTTCCCGTCACGGCCGGGGCAGCCAATTGTG

GCGGC

GCTCGGCGGCTCGTGGCTCTTTCGCGGCAAAAAGGATTTGGCGCGTAAAAGTGGC

CGGGA

CTTTGCAGGCAGCGGCGGCCGGGGGCGGAGCGGGATCGAGCCCTCGATGATATC

AGATCA

AACGATATCACCGGTCGACTGAAAATGAGACATATTATCTGCCACGGAGGTGTTA

TTACC

GAAGAAATGGCCGCCAGTCTTTTGGACCAGCTGATCGAAGAGGTACTGGCTGATA

ATCTT

CCACCTCCTAGCCATTTTGAACCACCTACCCTTCACGAACTGTATGATTTAGACGT

GACG

GCCCCCGAAGATCCCAACGAGGAGGCGGTTTCGCAGATTTTTCCCGACTCTGTAA

TGTTG

GCGGTGCAGGAAGGGATTGACTTACTCACTTTTCCGCCGGCGCCCGGTTCTCCGG

AGCCG

CCTCACCTTTCCCGGCAGCCCGAGCAGCCGGAGCAGAGAGCCTTGGGTCCGGTTT

CTATG

CCAAACCTTGTACCGGAGGTGATCGATCTTACCTGCCACGAGGCTGGCTTTCCACC

CAGT

GACGACGAGGATGAAGAGGGTGAGGAGTTTGTGTTAGATTATGTGGAGCACCCC

GGGCAC

GGTTGCAGGTCTTGTCATTATCACCGGAGGAATACGGGGGACCCAGATATTATGT

GTTCG

CTTTGCTATATGAGGACCTGTGGCATGTTTGTCTACAGTAAGTGAAAATTATGGGC

AGTG

GGTGATAGAGTGGTGGGTTTGGTGTGGTAATTTTTTTTTAATTTTTACAGTTTTGT

GGT

TTAAAGAATTTTGTATTGTGATTTTTTTAAAAGGTCCTGTGTCTGAACCTGAGCCT

GAGC

CCGAGCCAGAACCGGAGCCTGCAAGACCTACCCGCCGTCCTAAAATGGCGCCTGC

TATCC

TGAGACGCCCGACATCACCTGTGTCTAGAGAATGCAATAGTAGTACGGATAGCTG

TGACT

CCGGTCCTTCTAACACACCTCCTGAGATACACCCGGTGGTCCCGCTGTGCCCCATT

AAAC

CAGTTGCCGTGAGAGTTGGTGGGCGTCGCCAGGCTGTGGAATGTATCGAGGACTT

GCTTA

ACGAGCCTGGGCAACCTTTGGACTTGAGCTGTAAACGCCCCAGGCCATAAGGTGT

AAACC

TGTGATTGCGTGTGTGGTTAACGCCTTTGTTTGCTGAATGAGTTGATGTAAGTTTA

ATAA

AGGGTGAGATAATGTTTAACTTGCATGGCGTGTTAAATGGGGCGGGGCTTAAAGG

GTATA

TAATGCGCCGTGGGCTAATCTTGGTTACATCTGACCTCATGGAGGCTTGGGAGTGT

TTGG

AAGATTTTTCTGCTGTGCGTAACTTGCTGGAACAGAGCTCTAACAGTACCTCTTGG

TTTT

GGAGGTTTCTGTGGGGCTCATCCCAGGCAAAGTTAGTCTGCAGAATTAAGGAGGA

TTACA

AGTGGGAATTTGAAGAGCTTTTGAAATCCTGTGGTGAGCTGTTTGATTCTTTGAAT

CTGG

GTCACCAGGCGCTTTTCCAAGAGAAGGTCATCAAGACTTTGGATTTTTCCACACCG

GGGC

GCGCTGCGGCTGCTGTTGCTTTTTTGAGTTTTATAAAGGATAAATGGAGCGAAGA

AACCC

ATCTGAGCGGGGGGTACCTGCTGGATTTTCTGGCCATGCATCTGTGGAGAGCGGT

TGTGA

GACACAAGAATCGCCTGCTACTGTTGTCTTCCGTCCGCCCGGCGATAATACCGAC

GGAGG

AGCAGCAGCAGCAGCAGGAGGAAGCCAGGCGGCGGCGGCAGGAGCAGAGCCCA

TGGAACC

CGAGAGCCGGCCTGGACCCTCGGGAATGAATGTTGTACAGGTGGCTGAACTGTAT

CCAGA

ACTGAGACGCATTTTGACAATTACAGAGGATGGGCAGGGGCTAAAGGGGGTAAA

GAGGGA

GCGGGGGGCTTGTGAGGCTACAGAGGAGGCTAGGAATCTAGCTTTTAGCTTAATG

ACCAG

ACACCGTCCTGAGTGTATTACTTTTCAACAGATCAAGGATAATTGCGCTAATGAG

CTTGA

TCTGCTGGCGCAGAAGTATTCCATAGAGCAGCTGACCACTTACTGGCTGCAGCCA

GGGGA

TGATTTTGAGGAGGCTATTAGGGTATATGCAAAGGTGGCACTTAGGCCAGATTGC

AAGTA

CAAGATCAGCAAACTTGTAAATATCAGGAATTGTTGCTACATTTCTGGGAACGGG

GCCGA

GGTGGAGATAGATACGGAGGATAGGGTGGCCTTTAGATGTAGCATGATAAATATG TGGCC

GGGGGTGCTTGGCATGGACGGGGTGGTTATTATGAATGTAAGGTTTACTGGCCCC AATTT

TAGCGGTACGGTTTTCCTGGCCAATACCAACCTTATCCTACACGGTGTAAGCTTCT ATGG

GTTTAACAATACCTGTGTGGAAGCCTGGACCGATGTAAGGGTTCGGGGCTGTGCC TTTTA

CTGCTGCTGGAAGGGGGTGGTGTGTCGCCCCAAAAGCAGGGCTTCAATTAAGAAA TGCCT

CTTTGAAAGGTGTACCTTGGGTATCCTGTCTGAGGGTAACTCCAGGGTGCGCCAC AATGT

GGCCTCCGACTGTGGTTGCTTCATGCTAGTGAAAAGCGTGGCTGTGATTAAGCAT AACAT

GGTATGTGGCAACTGCGAGGACAGGGCCTCTCAGATGCTGACCTGCTCGGACGGC AACTG

TCACCTGCTGAAGACCATTCACGTAGCCAGCCACTCTCGCAAGGCCTGGCCAGTG TTTGA

GCATAACATACTGACCCGCTGTTCCTTGCATTTGGGTAACAGGAGGGGGGTGTTC CTACC

TTACCAATGCAATTTGAGTCACACTAAGATATTGCTTGAGCCCGAGAGCATGTCC AAGGT

GAACCTGAACGGGGTGTTTGACATGACCATGAAGATCTGGAAGGTGCTGAGGTAC GATGA

GACCCGCACCAGGTGCAGACCCTGCGAGTGTGGCGGTAAACATATTAGGAACCA

GCCTGT

GATGCTGGATGTGACCGAGGAGCTGAGGCCCGATCACTTGGTGCTGGCCTGCACC

CGCGC

TGAGTTTGGCTCTAGCGATGAAGATACAGATTGAGGTACTGAAATGTGTGGGCGT

GGCTT

AAGGGTGGGAAAGAATATATAAGGTGGGGGTCTTATGTAGTTTTGTATCTGTTTT

GCAGC

AGCCGCCGCCGCCATGAGCACCAACTCGTTTGATGGAAGCATTGTGAGCTCATAT

TTGAC

AACGCGCATGCCCCCATGGGCCGGGGTGCGTCAGAATGTGATGGGCTCCAGCATT

GATGG

TCGCCCCGTCCTGCCCGCAAACTCTACTACCTTGACCTACGAGACCGTGTCTGGAA

CGCC

GTTGGAGACTGCAGCCTCCGCCGCCGCTTCAGCCGCTGCAGCCACCGCCCGCGGG

ATTGT

GACTGACTTTGCTTTCCTGAGCCCGCTTGCAAGCAGTGCAGCTTCCCGTTCATCCG

CCCG

CGATGACAAGTTGACGGCTCTTTTGGCACAATTGGATTCTTTGACCCGGGAACTTA

ATGT

CGTTTCTCAGCAGCTGTTGGATCTGCGCCAGCAGGTTTCTGCCCTGAAGGCTTCCT

CCCC

TCCCAATGCGGTTTAAAACATAAATAAAAAACCAGACTCTGTTTGGATTTGGATC

AAGCA

AGTGTCTTGCTGTCTTTATTTAGGGGTTTTGCGCGCGCGGTAGGCCCGGGACCAGC GGTC

TCGGTCGTTGAGGGTCCTGTGTATTTTTTCCAGGACGTGGTAAAGGTGACTCTGGA TGTT

CAGATACATGGGCATAAGCCCGTCTCTGGGGTGGAGGTAGCACCACTGCAGAGCT TCATG

CTGCGGGGTGGTGTTGTAGATGATCCAGTCGTAGCAGGAGCGCTGGGCGTGGTGC CTAAA

AATGTCTTTCAGTAGCAAGCTGATTGCCAGGGGCAGGCCCTTGGTGTAAGTGTTT ACAAA

GCGGTTAAGCTGGGATGGGTGCATACGTGGGGATATGAGATGCATCTTGGACTGT ATTTT

TAGGTTGGCTATGTTCCCAGCCATATCCCTCCGGGGATTCATGTTGTGCAGAACCA CCAG

CACAGTGTATCCGGTGCACTTGGGAAATTTGTCATGTAGCTTAGAAGGAAATGCG TGGAA

GAACTTGGAGACGCCCTTGTGACCTCCAAGATTTTCCATGCATTCGTCCATAATGA TGGC

AATGGGCCCACGGGCGGCGGCCTGGGCGAAGATATTTCTGGGATCACTAACGTCA TAGTT

GTGTTCCAGGATGAGATCGTCATAGGCCATTTTTACAAAGCGCGGGCGGAGGGTG CCAGA

CTGCGGTATAATGGTTCCATCCGGCCCAGGGGCGTAGTTACCCTCACAGATTTGC ATTTC

CCACGCTTTGAGTTCAGATGGGGGGATCATGTCTACCTGCGGGGCGATGAAGAAA
ACGGT
TTCCGGGGTAGGGGAGATCAGCTGGGAAGAAAGCAGGTTCCTGAGCAGCTGCGA
CTTACC
GCAGCCGGTGGGCCCGTAAATCACACCTATTACCGGGTGCAACTGGTAGTTAAGA
GAGCT
GCAGCTGCCGTCATCCCTGAGCAGGGGGGCCACTTCGTTAAGCATGTCCCTGACT
CGCAT
GTTTTCCCTGACCAAATCCGCCAGAAGGCGCTCGCCGCCCAGCGATAGCAGTTCT
TGCAA
GGAAGCAAAGTTTTTCAACGGTTTGAGACCGTCCGCCGTAGGCATGCTTTTGAGC
GTTTG
ACCAAGCAGTTCCAGGCGGTCCCACAGCTCGGTCACCTGCTCTACGGCATCTCGA
TCCAG
CATATCTCCTCGTTTCGCGGGTTGGGGCGGCTTTCGCTGTACGGCAGTAGTCGGTG
CTCG
TCCAGACGGGCCAGGGTCATGTCTTTCCACGGGCGCAGGGTCCTCGTCAGCGTAG
TCTGG
GTCACGGTGAAGGGGTGCGCTCCGGGCTGCGCGCTGGCCAGGGTGCGCTTGAGGC
TGGTC
CTGCTGGTGCTGAAGCGCTGCCGGTCTTCGCCCTGCGCGTCGGCCAGGTAGCATTT
GACC
ATGGTGTCATAGTCCAGCCCCTCCGCGGCGTGGCCCTTGGCGCGCAGCTTGCCCTT
GGAG

GAGGCGCCGCACGAGGGGCAGTGCAGACTTTTGAGGGCGTAGAGCTTGGGCGCG
AGAAAT
ACCGATTCCGGGGAGTAGGCATCCGCGCCGCAGGCCCCGCAGACGGTCTCGCATT
CCACG
AGCCAGGTGAGCTCTGGCCGTTCGGGGTCAAAAACCAGGTTTCCCCCATGCTTTTT
GATG
CGTTTCTTACCTCTGGTTTCCATGAGCCGGTGTCCACGCTCGGTGACGAAAAGGCT
GTCC
GTGTCCCCGTATACAGACTTGAGAGGCCTGTCCTCGAGCGGTGTTCCGCGGTCCTC
CTCG
TATAGAAACTCGGACCACTCTGAGACAAAGGCTCGCGTCCAGGCCAGCACGAAG
GAGGCT
AAGTGGGAGGGGTAGCGGTCGTTGTCCACTAGGGGGTCCACTCGCTCCAGGGTGT
GAAGA
CACATGTCGCCCTCTTCGGCATCAAGGAAGGTGATTGGTTTGTAGGTGTAGGCCA
CGTGA
CCGGGTGTTCCTGAAGGGGGGCTATAAAAGGGGGTGGGGGCGCGTTCGTCCTCAC
TCTCT
TCCGCATCGCTGTCTGCGAGGGCCAGCTGTTGGGGTGAGTACTCCCTCTGAAAAG
CGGGC
ATGACTTCTGCGCTAAGATTGTCAGTTTCCAAAAACGAGGAGGATTTGATATTCA
CCTGG
CCCGCGGTGATGCCTTTGAGGGTGGCCGCATCCATCTGGTCAGAAAAGACAATCT
TTTTG

TTGTCAAGCTTGGTGGCAAACGACCCGTAGAGGGCGTTGGACAGCAACTTGGCGA
TGGAG

CGCAGGGTTTGGTTTTTGTCGCGATCGGCGCGCTCCTTGGCCGCGATGTTTAGCTG
CACG

TATTCGCGCGCAACGCACCGCCATTCGGGAAAGACGGTGGTGCGCTCGTCGGGCA
CCAGG

TGCACGCGCCAACCGCGGTTGTGCAGGGTGACAAGGTCAACGCTGGTGGCTACCT
CTCCG

CGTAGGCGCTCGTTGGTCCAGCAGAGGCGGCCGCCCTTGCGCGAGCAGAATGGCG
GTAGG

GGGTCTAGCTGCGTCTCGTCCGGGGGGTCTGCGTCCACGGTAAAGACCCCGGGCA
GCAGG

CGCGCGTCGAAGTAGTCTATCTTGCATCCTTGCAAGTCTAGCGCCTGCTGCCATGC
GCGG

GCGGCAAGCGCGCGCTCGTATGGGTTGAGTGGGGGACCCCATGGCATGGGGTGG
GTGAGC

GCGGAGGCGTACATGCCGCAAATGTCGTAAACGTAGAGGGGCTCTCTGAGTATTC
CAAGA

TATGTAGGGTAGCATCTTCCACCGCGGATGCTGGCGCGCACGTAATCGTATAGTT
CGTGC

GAGGGAGCGAGGAGGTCGGGACCGAGGTTGCTACGGGCGGGCTGCTCTGCTCGG
AAGACT

ATCTGCCTGAAGATGGCATGTGAGTTGGATGATATGGTTGGACGCTGGAAGACGT
TGAAG

CTGGCGTCTGTGAGACCTACCGCGTCACGCACGAAGGAGGCGTAGGAGTCGCGCA GCTTG

TTGACCAGCTCGGCGGTGACCTGCACGTCTAGGGCGCAGTAGTCCAGGGTTTCCT TGATG

ATGTCATACTTATCCTGTCCCTTTTTTTCCACAGCTCGCGGTTGAGGACAAACTCT TCG

CGGTCTTTCCAGTACTCTTGGATCGGAAACCCGTCGGCCTCCGAACGGTAAGAGC CTAGC

ATGTAGAACTGGTTGACGGCCTGGTAGGCGCAGCATCCCTTTTCTACGGGTAGCG CGTAT

GCCTGCGCGGCCTTCCGGAGCGAGGTGTGGGTGAGCGCAAAGGTGTCCCTGACCA TGACT

TTGAGGTACTGGTATTTGAAGTCAGTGTCGTCGCATCCGCCCTGCTCCCAGAGCAA AAAG

TCCGTGCGCTTTTTGGAACGCGGATTTGGCAGGGCGAAGGTGACATCGTTGAAGA GTATC

TTTCCCGCGCGAGGCATAAAGTTGCGTGTGATGCGGAAGGGTCCCGGCACCTCGG AACGG

TTGTTAATTACCTGGGCGGCGAGCACGATCTCGTCAAAGCCGTTGATGTTGTGGCC CACA

ATGTAAAGTTCCAAGAAGCGCGGGATGCCCTTGATGGAAGGCAATTTTTTAAGTT CCTCG

TAGGTGAGCTCTTCAGGGGAGCTGAGCCCGTGCTCTGAAAGGGCCCAGTCTGCAA GATGA

GGGTTGGAAGCGACGAATGAGCTCCACAGGTCACGGGCCATTAGCATTTGCAGGT GGTCG

CGAAAGGTCCTAAACTGGCGACCTATGGCCATTTTTTCTGGGGTGATGCAGTAGA AGGTA

AGCGGGTCTTGTTCCCAGCGGTCCCATCCAAGGTTCGCGGCTAGGTCTCGCGCGG CAGTC

ACTAGAGGCTCATCTCCGCCGAACTTCATGACCAGCATGAAGGGCACGAGCTGCT TCCCA

AAGGCCCCCATCCAAGTATAGGTCTCTACATCGTAGGTGACAAAGAGACGCTCGG TGCGA

GGATGCGAGCCGATCGGGAAGAACTGGATCTCCCGCCACCAATTGGAGGAGTGG CTATTG

ATGTGGTGAAAGTAGAAGTCCCTGCGACGGGCCGAACACTCGTGCTGGCTTTTGT AAAAA

CGTGCGCAGTACTGGCAGCGGTGCACGGGCTGTACATCCTGCACGAGGTTGACCT GACGA

CCGCGCACAAGGAAGCAGAGTGGGAATTTGAGCCCCTCGCCTGGCGGGTTTGGCT GGTGG

TCTTCTACTTCGGCTGCTTGTCCTTGACCGTCTGGCTGCTCGAGGGGAGTTACGGT GGAT

CGGACCACCACGCCGCGCGAGCCCAAAGTCCAGATGTCCGCGCGCGGCGGTCGG AGCTTG

ATGACAACATCGCGCAGATGGGAGCTGTCCATGGTCTGGAGCTCCCGCGGCGTCA GGTCA

GGCGGGAGCTCCTGCAGGTTTACCTCGCATAGACGGGTCAGGGCGCGGGCTAGAT
CCAGG

TGATACCTAATTTCCAGGGGCTGGTTGGTGGCGGCGTCGATGGCTTGCAAGAGGC
CGCAT

CCCCGCGGCGCGACTACGGTACCGCGCGGCGGGCGGTGGGCCGCGGGGGTGTCCT
TGGAT

GATGCATCTAAAAGCGGTGACGCGGGCGAGCCCCCGGAGGTAGGGGGGGCTCCG
GACCCG

CCGGGAGAGGGGGCAGGGGCACGTCGGCGCCGCGCGCGGGCAGGAGCTGGTGCT
GCGCGC

GTAGGTTGCTGGCGAACGCGACGACGCGGCGGTTGATCTCCTGAATCTGGCGCCT
CTGCG

TGAAGACGACGGGCCCGGTGAGCTTGAGCCTGAAAGAGAGTTCGACAGAATCAA
TTTCGG

TGTCGTTGACGGCGGCCTGGCGCAAAATCTCCTGCACGTCTCCTGAGTTGTCTTGA
TAGG

CGATCTCGGCCATGAACTGCTCGATCTCTTCCTCCTGGAGATCTCCGCGTCCGGCT
CGCT

CCACGGTGGCGGCGAGGTCGTTGGAAATGCGGGCCATGAGCTGCGAGAAGGCGT
TGAGGC

CTCCCTCGTTCCAGACGCGGCTGTAGACCACGCCCCCTTCGGCATCGCGGGCGCG
CATGA

CCACCTGCGCGAGATTGAGCTCCACGTGCCGGGCGAAGACGGCGTAGTTTCGCAG
GCGCT

GAAAGAGGTAGTTGAGGGTGGTGGCGGTGTGTTCTGCCACGAAGAAGTACATAA

CCCAGC

GTCGCAACGTGGATTCGTTGATATCCCCCAAGGCCTCAAGGCGCTCCATGGCCTC

GTAGA

AGTCCACGGCGAAGTTGAAAAACTGGGAGTTGCGCGCCGACACGGTTAACTCCTC

CTCCA

GAAGACGGATGAGCTCGGCGACAGTGTCGCGCACCTCGCGCTCAAAGGCTACAG

GGGCCT

CTTCTTCTTCTTCAATCTCCTCTTCCATAAGGGCCTCCCCTTCTTCTTCTTCTGGCG

GCG

GTGGGGGAGGGGGGACACGGCGGCGACGACGGCGCACCGGGAGGCGGTCGACA

AAGCGCT

CGATCATCTCCCCGCGGCGACGGCGCATGGTCTCGGTGACGGCGCGGCCGTTCTC

GCGGG

GGCGCAGTTGGAAGACGCCGCCCGTCATGTCCCGGTTATGGGTTGGCGGGGGGCT

GCCAT

GCGGCAGGGATACGGCGCTAACGATGCATCTCAACAATTGTTGTGTAGGTACTCC

GCCGC

CGAGGGACCTGAGCGAGTCCGCATCGACCGGATCGGAAAACCTCTCGAGAAAGG

CGTCTA

ACCAGTCACAGTCGCAAGGTAGGCTGAGCACCGTGGCGGGCGGCAGCGGGCGGC

GGTCGG

GGTTGTTTCTGGCGGAGGTGCTGCTGATGATGTAATTAAAGTAGGCGGTCTTGAG

ACGGC

GGATGGTCGACAGAAGCACCATGTCCTTGGGTCCGGCCTGCTGAATGCGCAGGCG GTCGG

CCATGCCCCAGGCTTCGTTTTGACATCGGCGCAGGTCTTTGTAGTAGTCTTGCATG AGCC

TTTCTACCGGCACTTCTTCTTCTCCTTCCTCTTGTCCTGCATCTCTTGCATCTATCGC TG

CGGCGGCGGCGGAGTTTGGCCGTAGGTGGCGCCCTCTTCCTCCCATGCGTGTGAC CCCGA

AGCCCCTCATCGGCTGAAGCAGGGCTAGGTCGGCGACAACGCGCTCGGCTAATAT GGCCT

GCTGCACCTGCGTGAGGGTAGACTGGAAGTCATCCATGTCCACAAAGCGGTGGTA TGCGC

CCGTGTTGATGGTGTAAGTGCAGTTGGCCATAACGGACCAGTTAACGGTCTGGTG ACCCG

GCTGCGAGAGCTCGGTGTACCTGAGACGCGAGTAAGCCCTCGAGTCAAATACGTA GTCGT

TGCAAGTCCGCACCAGGTACTGGTATCCCACCAAAAAGTGCGGCGGCGGCTGGCG GTAGA

GGGGCCAGCGTAGGGTGGCCGGGGCTCCGGGGGCGAGATCTTCCAACATAAGGC GATGAT

ATCCGTAGATGTACCTGGACATCCAGGTGATGCCGGCGGCGGTGGTGGAGGCGCG CGGAA

AGTCGCGGACGCGGTTCCAGATGTTGCGCAGCGGCAAAAAGTGCTCCATGGTCGG GACGC

TCTGGCCGGTCAGGCGCGCAATCGTTGACGCTCTAGACCGTGCAAAAGGAGAG

CCTGT

AAGCGGGCACTCTTCCGTGGTCTGGTGGATAAATTCGCAAGGGTATCATGGCGGA

CGACC

GGGGTTCGAGCCCCGTATCCGGCCGTCCGCCGTGATCCATGCGGTTACCGCCCGC

GTGTC

GAACCCAGGTGTGCGACGTCAGACAACGGGGGAGTGCTCCTTTTGGCTTCCTTCC

AGGCG

CGGCGGCTGCTGCGCTAGCTTTTTTGGCCACTGGCCGCGCGCAGCGTAAGCGGTT

AGGCT

GGAAAGCGAAAGCATTAAGTGGCTCGCTCCCTGTAGCCGGAGGGTTATTTTCCAA

GGGTT

GAGTCGCGGGACCCCCGGTTCGAGTCTCGGACCGGCCGGACTGCGGCGAACGGG

GGTTTG

CCTCCCCGTCATGCAAGACCCCGCTTGCAAATTCCTCCGGAAACAGGGACGAGCC

CCTTT

TTTGCTTTTCCCAGATGCATCCGGTGCTGCGGCAGATGCGCCCCCCTCCTCAGCAG

CGGC

AAGAGCAAGAGCAGCGGCAGACATGCAGGGCACCCTCCCCTCCTCCTACCGCGTC

AGGAG

GGGCGACATCCGCGGTTGACGCGGCAGCAGATGGTGATTACGAACCCCCGCGGC

GCCGGG

CCCGGCACTACCTGGACTTGGAGGAGGGCGAGGGCCTGGCGCGGCTAGGAGCGC

CCTCTC

CTGAGCGGTACCCAAGGGTGCAGCTGAAGCGTGATACGCGTGAGGCGTACGTGCC GCGGC

AGAACCTGTTTCGCGACCGCGAGGGAGAGGAGCCCGAGGAGATGCGGGATCGAA AGTTCC

ACGCAGGGCGCGAGCTGCGGCATGGCCTGAATCGCGAGCGGTTGCTGCGCGAGG AGGACT

TTGAGCCCGACGCGCGAACCGGGATTAGTCCCGCGCGCACACGTGGCGGCCGC CGACC

TGGTAACCGCATACGAGCAGACGGTGAACCAGGAGATTAACTTTCAAAAAAGCTT TAACA

ACCACGTGCGTACGCTTGTGGCGCGCGAGGAGGTGGCTATAGGACTGATGCATCT GTGGG

ACTTTGTAAGCGCGCTGGAGCAAAACCCAAATAGCAAGCCGCTCATGGCGCAGCT GTTCC

TTATAGTGCAGCACAGCAGGGACAACGAGGCATTCAGGGATGCGCTGCTAAACAT AGTAG

AGCCCGAGGGCCGCTGGCTGCTCGATTTGATAAACATCCTGCAGAGCATAGTGGT GCAGG

AGCGCAGCTTGAGCCTGGCTGACAAGGTGGCCGCCATCAACTATTCCATGCTTAG CCTGG

GCAAGTTTTACGCCCGCAAGATATACCATACCCCTTACGTTCCCATAGACAAGGA GGTAA

AGATCGAGGGGTTCTACATGCGCATGGCGCTGAAGGTGCTTACCTTGAGCGACGA CCTGG

GCGTTTATCGCAACGAGCGCATCCACAAGGCCGTGAGCGTGAGCCGGCGGCGCG

AGCTCA

GCGACCGCGAGCTGATGCACAGCCTGCAAAGGGCCCTGGCTGGCACGGGCAGCG

GCGATA

GAGAGGCCGAGTCCTACTTTGACGCGGGCGCTGACCTGCGCTGGGCCCCAAGCCG

ACGCG

CCCTGGAGGCAGCTGGGGCCGGACCTGGGCTGGCGGTGGCACCCGCGCGCGCTG

GCAACG

TCGGCGGCGTGGAGGAATATGACGAGGACGATGAGTACGAGCCAGAGGACGGCG

AGTACT

AAGCGGTGATGTTTCTGATCAGATGATGCAAGACGCAACGGACCCGGCGGTGCGG

GCGGC

GCTGCAGAGCCAGCCGTCCGGCCTTAACTCCACGGACGACTGGCGCCAGGTCATG

GACCG

CATCATGTCGCTGACTGCGCGCAATCCTGACGCGTTCCGGCAGCAGCCGCAGGCC

AACCG

GCTCTCCGCAATTCTGGAAGCGGTGGTCCCGGCGCGCGCAAACCCCACGCACGAG

AAGGT

GCTGGCGATCGTAAACGCGCTGGCCGAAAACAGGGCCATCCGGCCCGACGAGGC

CGGCCT

GGTCTACGACGCGCTGCTTCAGCGCGTGGCTCGTTACAACAGCGGCAACGTGCAG

ACCAA

CCTGGACCGGCTGGTGGGGGATGTGCGCGAGGCCGTGGCGCAGCGTGAGCGCGC

GCAGCA

GCAGGGCAACCTGGGCTCCATGGTTGCACTAAACGCCTTCCTGAGTACACAGCCC GCCAA

CGTGCCGCGGGGACAGGAGGACTACACCAACTTTGTGAGCGCACTGCGGCTAATG GTGAC

TGAGACACCGCAAAGTGAGGTGTACCAGTCTGGGCCAGACTATTTTTCCAGACC AGTAG

ACAAGGCCTGCAGACCGTAAACCTGAGCCAGGCTTTCAAAAACTTGCAGGGGCTG TGGGG

GGTGCGGGCTCCCACAGGCGACCGCGACCGTGTCTAGCTTGCTGACGCCCAAC TCGCG

CCTGTTGCTGCTGCTAATAGCGCCCTTCACGGACAGTGGCAGCGTGTCCCGGGAC ACATA

CCTAGGTCACTTGCTGACACTGTACCGCGAGGCCATAGGTCAGGCGCATGTGGAC GAGCA

TACTTTCCAGGAGATTACAAGTGTCAGCCGCGCGCTGGGGCAGGAGGACACGGGC AGCCT

GGAGGCAACCCTAAACTACCTGCTGACCAACCGGCGGCAGAAGATCCCCTCGTTG CACAG

TTTAAACAGCGAGGAGGAGCGCATTTTGCGCTACGTGCAGCAGAGCGTGAGCCTT AACCT

GATGCGCGACGGGGTAACGCCCAGCGTGGCGCTGGACATGACCGCGCGCAACAT GGAACC

GGGCATGTATGCCTCAAACCGGCCGTTTATCAACCGCCTAATGGACTACTTGCATC GCGC

GGCCGCCGTGAACCCCGAGTATTTCACCAATGCCATCTTGAACCCGCACTGGCTA
CCGCC

CCCTGGTTTCTACACCGGGGGATTCGAGGTGCCCGAGGGTAACGATGGATTCCTC
TGGGA

CGACATAGACGACAGCGTGTTTCCCCGCAACCGCAGACCCTGCTAGAGTTGCAA
CAGCG

CGAGCAGGCAGAGGCGGCGCTGCGAAAGGAAAGCTTCCGCAGGCCAAGCAGCTT
GTCCGA

TCTAGGCGCTGCGGCCCCGCGGTCAGATGCTAGTAGCCCATTTCCAAGCTTGATA
GGGTC

TCTTACCAGCACTCGCACCACCCGCCCGCGCCTGCTGGGCGAGGAGGAGTACCTA
AACAA

CTCGCTGCTGCAGCCGCAGCGCGAAAAAAACCTGCCTCCGGCATTTCCCAACAAC
GGGAT

AGAGAGCCTAGTGGACAAGATGAGTAGATGGAAGACGTACGCGCAGGAGCACAG
GGACGT

GCCAGGCCCGCGCCCGCCCACCCGTCGTCAAAGGCACGACCGTCAGCGGGGTCTG
GTGTG

GGAGGACGATGACTCGGCAGACGACAGCAGCGTCCTGGATTTGGGAGGGAGTGG
CAACCC

GTTTGCGCACCTTCGCCCCAGGCTGGGGAGAATGTTTTAAAAAAAAAAAAGCATG
ATGCA

AAATAAAAAACTCACCAAGGCCATGGCACCGAGCGTTGGTTTTCTTGTATTCCCCT
TAGT

ATGCGGCGCGCGGCGATGTATGAGGAAGGTCCTCCTCCCTCCTACGAGAGTGTGG
TGAGC

GCGGCGCCAGTGGCGGCGGCGCTGGGTTCTCCCTTCGATGCTCCCCTGGACCCGC
CGTTT

GTGCCTCCGCGGTACCTGCGGCCTACCGGGGGGAGAAACAGCATCCGTTACTCTG
AGTTG

GCACCCCTATTCGACACCACCCGTGTGTACCTGGTGGACAACAAGTCAACGGATG
TGGCA

TCCCTGAACTACCAGAACGACCACAGCAACTTTCTGACCACGGTCATTCAAAACA
ATGAC

TACAGCCCGGGGGAGGCAAGCACACAGACCATCAATCTTGACGACCGGTCGCACT
GGGGC

GGCGACCTGAAAACCATCCTGCATACCAACATGCCAAATGTGAACGAGTTCATGT
TTACC

AATAAGTTTAAGGCGCGGGTGATGGTGTCGCGCTTGCCTACTAAGGACAATCAGG
TGGAG

CTGAAATACGAGTGGGTGGAGTTCACGCTGCCCGAGGGCAACTACTCCGAGACCA
TGACC

ATAGACCTTATGAACAACGCGATCGTGGAGCACTACTTGAAAGTGGGCAGACAG
AACGGG

GTTCTGGAAAGCGACATCGGGGTAAAGTTTGACACCCGCAACTTCAGACTGGGGT
TTGAC

CCCGTCACTGGTCTTGTCATGCCTGGGGTATATACAAACGAAGCCTTCCATCCAGA
CATC

ATTTTGCTGCCAGGATGCGGGGTGGACTTCACCCACAGCCGCCTGAGCAACTTGT

TGGGC

ATCCGCAAGCGGCAACCCTTCCAGGAGGGCTTTAGGATCACCTACGATGATCTGG

AGGGT

GGTAACATTCCCGCACTGTTGGATGTGGACGCCTACCAGGCGAGCTTGAAAGATG

ACACC

GAACAGGGCGGGGGTGGCGCAGGCGGCAGCAACAGCAGTGGCAGCGGCGCGGA

AGAGAAC

TCCAACGCGGCAGCCGCGGCAATGCAGCCGGTGGAGGACATGAACGATCATGCC

ATTCGC

GGCGACACCTTTGCCACACGGGCTGAGGAGAAGCGCGCTGAGGCCGAAGCAGCG

GCCGAA

GCTGCCGCCCCCGCTGCGCAACCCGAGGTCGAGAAGCCTCAGAAGAAACCGGTG

ATCAAA

CCCCTGACAGAGGACAGCAAGAAACGCAGTTACAACCTAATAAGCAATGACAGC

ACCTTC

ACCCAGTACCGCAGCTGGTACCTTGCATACAACTACGGCGACCCTCAGACCGGAA

TCCGC

TCATGGACCCTGCTTTGCACTCCTGACGTAACCTGCGGCTCGGAGCAGGTCTACTG

GTCG

TTGCCAGACATGATGCAAGACCCCGTGACCTTCCGCTCCACGCGCCAGATCAGCA

ACTTT

CCGGTGGTGGGCGCCGAGCTGTTGCCCGTGCACTCCAAGAGCTTCTACAACGACC

AGGCC

GTCTACTCCCAACTCATCCGCCAGTTTACCTCTCTGACCCACGTGTTCAATCGCTTT

CCC

GAGAACCAGATTTTGGCGCGCCCGCCAGCCCCCACCATCACCACCGTCAGTGAAA

ACGTT

CCTGCTCTCACAGATCACGGGACGCTACCGCTGCGCAACAGCATCGGAGGAGTCC

AGCGA

GTGACCATTACTGACGCCAGACGCCGCACCTGCCCCTACGTTTACAAGGCCCTGG

GCATA

GTCTCGCCGCGCGTCCTATCGAGCCGCACTTTTTGAGCAAGCATGTCCATCCTTAT

ATCG

CCCAGCAATAACACAGGCTGGGGCCTGCGCTTCCCAAGCAAGATGTTTGGCGGGG

CCAAG

AAGCGCTCCGACCAACACCCAGTGCGCGTGCGCGGGCACTACCGCGCGCCCTGGG

GCGCG

CACAAACGCGGCCGCACTGGGCGCACCACCGTCGATGACGCCATCGACGCGGTG

GTGGAG

GAGGCGCGCAACTACACGCCCACGCCGCCACCAGTGTCCACAGTGGACGCGGCC

ATTCAG

ACCGTGGTGCGCGGAGCCCGGCGCTATGCTAAAATGAAGAGACGGCGGAGGCGC

GTAGCA

CGTCGCCACCGCCGCCGACCCGGCACTGCCGCCCAACGCGCGGCGGCGGCCCTGC

TTAAC

CGCGCACGTCGCACCGGCCGACGGGCGGCCATGCGGGCCGCTCGAAGGCTGGCC

GCGGGT

ATTGTCACTGTGCCCCCCAGGTCCAGGCGACGAGCGGCCGCCGCAGCAGCCGCGG

CCATT

AGTGCTATGACTCAGGGTCGCAGGGGCAACGTGTATTGGGTGCGCGACTCGGTTA

GCGGC

CTGCGCGTGCCCGTGCGCACCCGCCCCCCGCGCAACTAGATTGCAAGAAAAAACT

ACTTA

GACTCGTACTGTTGTATGTATCCAGCGGCGGCGGCGCGCAACGAAGCTATGTCCA

AGCGC

AAAATCAAAGAAGAGATGCTCCAGGTCATCGCGCCGGAGATCTATGGCCCCCCGA

AGAAG

GAAGAGCAGGATTACAAGCCCCGAAAGCTAAAGCGGGTCAAAAAGAAAAAGAA

AGATGAT

GATGATGAACTTGACGACGAGGTGGAACTGCTGCACGCTACCGCGCCCAGGCGAC

GGGTA

CAGTGGAAAGGTCGACGCGTAAAACGTGTTTTGCGACCCGGCACCACCGTAGTCT

TTACG

CCCGGTGAGCGCTCCACCCGCACCTACAAGCGCGTGTATGATGAGGTGTACGGCG

ACGAG

GACCTGCTTGAGCAGGCCAACGAGCGCCTCGGGGAGTTTGCCTACGGAAAGCGGC

ATAAG

GACATGCTGGCGTTGCCGCTGGACGAGGGCAACCCAACACCTAGCCTAAAGCCCG

TAACA

CTGCAGCAGGTGCTGCCCGCGCTTGCACCGTCCGAAGAAAAGCGCGGCCTAAAGC

GCGAG

TCTGGTGACTTGGCACCCACCGTGCAGCTGATGGTACCCAAGCGCCAGCGACTGG
AAGAT

GTCTTGGAAAAAATGACCGTGGAACCTGGGCTGGAGCCCGAGGTCCGCGTGCGGC
CAATC

AAGCAGGTGGCGCCGGGACTGGGCGTGCAGACCGTGGACGTTCAGATACCCACT
ACCAGT

AGCACCAGTATTGCCACCGCCACAGAGGGCATGGAGACACAAACGTCCCCGGTTG
CCTCA

GCGGTGGCGGATGCCGCGGTGCAGGCGGTCGCTGCGGCCGCGTCCAAGACCTCTA
CGGAG

GTGCAAACGGACCCGTGGATGTTTCGCGTTTCAGCCCCCCGGCGCCCGCGCGGTT
CGAGG

AAGTACGGCGCCGCCAGCGCGCTACTGCCCGAATATGCCCTACATCCTTCCATTG
CGCCT

ACCCCCGGCTATCGTGGCTACACCTACCGCCCCAGAAGACGAGCAACTACCCGAC
GCCGA

ACCACCACTGGAACCCGCCGCCGCCGTCGCCGTCGCCAGCCCGTGCTGGCCCCGA
TTTCC

GTGCGCAGGGTGGCTCGCGAAGGAGGCAGGACCCTGGTGCTGCCAACAGCGCGC
TACCAC

CCCAGCATCGTTTAAAAGCCGGTCTTTGTGGTTCTTGCAGATATGGCCCTCACCTG
CCGC

CTCCGTTTCCCGGTGCCGGGATTCCGAGGAAGAATGCACCGTAGGAGGGGCATGG
CCGGC

CACGGCCTGACGGGCGGCATGCGTCGTGCGCACCACCGGCGGCGGCGCGTCGC ACCGT

CGCATGCGCGGCGGTATCCTGCCCCTCCTTATTCCACTGATCGCCGCGGCGATTGG CGCC

GTGCCCGGAATTGCATCCGTGGCCTTGCAGGCGCAGAGACACTGATTAAAAACAA GTTGC

ATGTGGAAAAATCAAAATAAAAAGTCTGGACTCTCACGCTCGCTTGGTCCTGTAA CTATT

TTGTAGAATGGAAGACATCAACTTTGCGTCTCTGGCCCCGCGACACGGCTCGCGC CCGTT

CATGGGAAACTGGCAAGATATCGGCACCAGCAATATGAGCGGTGGCGCCTTCAGC TGGGG

CTCGCTGTGGAGCGGCATTAAAAATTTCGGTTCCACCGTTAAGAACTATGGCAGC AAGGC

CTGGAACAGCAGCACAGGCCAGATGCTGAGGGATAAGTTGAAAGAGCAAAATTT CCAACA

AAAGGTGGTAGATGGCCTGGCCTCTGGCATTAGCGGGGTGGTGGACCTGGCCAAC CAGGC

AGTGCAAAATAAGATTAACAGTAAGCTTGATCCCCGCCCTCCCGTAGAGGAGCCT CCACC

GGCCGTGGAGACAGTGTCTCCAGAGGGGCGTGGCGAAAAGCGTCCGCGCCCCGA CAGGGA

AGAAACTCTGGTGACGCAAATAGACGAGCCTCCCTCGTACGAGGAGGCACTAAA GCAAGG

CCTGCCCACCACCCGTCCCATCGCGCCCATGGCTACCGGAGTGCTGGGCCAGCAC

ACACC

CGTAACGCTGGACCTGCCTCCCCCCGCCGACACCCAGCAGAAACCTGTGCTGCCA

GGCCC

GACCGCCGTTGTTGTAACCCGTCCTAGCCGCGCGTCCCTGCGCCGCGCCGCCAGC

GGTCC

GCGATCGTTGCGGCCCGTAGCCAGTGGCAACTGGCAAAGCACACTGAACAGCATC

GTGGG

TCTGGGGGTGCAATCCCTGAAGCGCCGACGATGCTTCTGAATAGCTAACGTGTCG

TATGT

GTGTCATGTATGCGTCCATGTCGCCGCCAGAGGAGCTGCTGAGCCGCCGCGCGCC

CGCTT

TCCAAGATGGCTACCCCTTCGATGATGCCGCAGTGGTCTTACATGCACATCTCGGG

CCAG

GACGCCTCGGAGTACCTGAGCCCCGGGCTGGTGCAGTTTGCCCGCGCCACCGAGA

CGTAC

TTCAGCCTGAATAACAAGTTTAGAAACCCCACGGTGGCGCCTACGCACGACGTGA

CCACA

GACCGGTCCCAGCGTTTGACGCTGCGGTTCATCCCTGTGGACCGTGAGGATACTG

CGTAC

TCGTACAAGGCGCGGTTCACCCTAGCTGTGGGTGATAACCGTGTGCTGGACATGG

CTTCC

ACGTACTTTGACATCCGCGGCGTGCTGGACAGGGGCCCTACTTTTAAGCCCTACTC

TGGC

ACTGCCTACAACGCCCTGGCTCCCAAGGGTGCCCCAAATCCTTGCGAATGGGATG AAGCT

GCTACTGCTCTTGAAATAAACCTAGAAGAAGAGGACGATGACAACGAAGACGAA GTAGAC

GAGCAAGCTGAGCAGCAAAAAACTCACGTATTTGGGCAGGCGCCTTATTCTGGTA TAAAT

ATTACAAAGGAGGGTATTCAAATAGGTGTCGAAGGTCAAACACCTAAATATGCCG ATAAA

ACATTTCAACCTGAACCTCAAATAGGAGAATCTCAGTGGTACGAAACTGAAATTA ATCAT

GCAGCTGGGAGAGTCCTTAAAAAGACTACCCCAATGAAACCATGTTACGGTTCAT ATGCA

AAACCCACAAATGAAAATGGAGGGCAAGGCATTCTTGTAAAGCAACAAAATGGA AAGCTA

GAAAGTCAAGTGGAAATGCAATTTTTCTCAACTACTGAGGCGACCGCAGGCAATG GTGAT

AACTTGACTCCTAAAGTGGTATTGTACAGTGAAGATGTAGATATAGAAACCCCAG ACACT

CATATTTCTTACATGCCCACTATTAAGGAAGGTAACTCACGAGAACTAATGGGCC AACAA

TCTATGCCCAACAGGCCTAATTACATTGCTTTTAGGGACAATTTTATTGGTCTAAT GTAT

TACAACAGCACGGGTAATATGGGTGTTCTGGCGGGCCAAGCATCGCAGTTGAATG CTGTT

GTAGATTTGCAAGACAGAAACACAGAGCTTTCATACCAGCTTTTGCTTGATTCCAT

TGGT

GATAGAACCAGGTACTTTTCTATGTGGAATCAGGCTGTTGACAGCTATGATCCAG

ATGTT

AGAATTATTGAAAATCATGGAACTGAAGATGAACTTCCAAATTACTGCTTTCCAC

TGGGA

GGTGTGATTAATACAGAGACTCTTACCAAGGTAAAACCTAAAACAGGTCAGGAA

AATGGA

TGGGAAAAAGATGCTACAGAATTTTCAGATAAAAATGAAATAAGAGTTGGAAAT

AATTTT

GCCATGGAAATCAATCTAAATGCCAACCTGTGGAGAAATTTCCTGTACTCCAACA

TAGCG

CTGTATTTGCCCGACAAGCTAAAGTACAGTCCTTCCAACGTAAAAATTTCTGATAA

CCCA

AACACCTACGACTACATGAACAAGCGAGTGGTGGCTCCCGGGTTAGTGGACTGCT

ACATT

AACCTTGGAGCACGCTGGTCCCTTGACTATATGGACAACGTCAACCCATTTAACC

ACCAC

CGCAATGCTGGCCTGCGCTACCGCTCAATGTTGCTGGGCAATGGTCGCTATGTGCC

CTTC

CACATCCAGGTGCCTCAGAAGTTCTTTGCCATTAAAAACCTCCTTCTCCTGCCGGG

CTCA

TACACCTACGAGTGGAACTTCAGGAAGGATGTTAACATGGTTCTGCAGAGCTCCC

TAGGA

AATGACCTAAGGGTTGACGGAGCCAGCATTAAGTTTGATAGCATTTGCCTTTACG CCACC

TTCTTCCCCATGGCCCACAACACCGCCTCCACGCTTGAGGCCATGCTTAGAAACG ACACC

AACGACCAGTCCTTTAACGACTATCTCTCCGCCGCCAACATGCTCTACCCTATACC CGCC

AACGCTACCAACGTGCCCATATCCATCCCCTCCCGCAACTGGGCGGCTTTCCGCG GCTGG

GCCTTCACGCGCCTTAAGACTAAGGAAACCCCATCACTGGGCTCGGGCTACGACC CTTAT

TACACCTACTCTGGCTCTATACCCTACCTAGATGGAACCTTTTACCTCAACCACAC CTTT

AAGAAGGTGGCCATTACCTTTGACTCTTCTGTCAGCTGGCCTGGCAATGACCGCCT GCTT

ACCCCCAACGAGTTTGAAATTAAGCGCTCAGTTGACGGGGAGGGTTACAACGTTG CCCAG

TGTAACATGACCAAAGACTGGTTCCTGGTACAAATGCTAGCTAACTACAACATTG GCTAC

CAGGGCTTCTATATCCCAGAGAGCTACAAGGACCGCATGTACTCCTTCTTTAGAA ACTTC

CAGCCCATGAGCCGTCAGGTGGTGGATGATACTAAATACAAGGACTACCAACAG GTGGGC

ATCCTACACCAACACAACAACTCTGGATTTGTTGGCTACCTTGCCCCCACCATGCG CGAA

GGACAGGCCTACCCTGCTAACTTCCCCTATCCGCTTATAGGCAAGACCGCAGTTG
ACAGC

ATTACCCAGAAAAAGTTTCTTTGCGATCGCACCCTTTGGCGCATCCCATTCTCCAG
TAAC

TTTATGTCCATGGGCGCACTCACAGACCTGGGCCAAAACCTTCTCTACGCCAACTC
CGCC

CACGCGCTAGACATGACTTTTGAGGTGGATCCCATGGACGAGCCCACCCTTCTTTA
TGTT

TTGTTTGAAGTCTTTGACGTGGTCCGTGTGCACCGGCCGCACCGCGGCGTCATCGA
AACC

GTGTACCTGCGCACGCCCTTCTCGGCCGGCAACGCCACAACATAAAGAAGCAAGC
AACAT

CAACAACAGCTGCCGCCATGGGCTCCAGTGAGCAGGAACTGAAAGCCATTGTCAA
AGATC

TTGGTTGTGGGCCATATTTTTTGGGCACCTATGACAAGCGCTTTCCAGGCTTTGTT
TCTC

CACACAAGCTCGCCTGCGCCATAGTCAATACGGCCGGTCGCGAGACTGGGGGCGT
ACACT

GGATGGCCTTTGCCTGGAACCCGCACTCAAAAACATGCTACCTCTTTGAGCCCTTT
GGCT

TTTCTGACCAGCGACTCAAGCAGGTTTACCAGTTTGAGTACGAGTCACTCCTGCGC
CGTA

GCGCCATTGCTTCTTCCCCCGACCGCTGTATAACGCTGGAAAAGTCCACCCAAAG
CGTAC

AGGGGCCCAACTCGGCCGCCTGTGGACTATTCTGCTGCATGTTTCTCCACGCCTTT

GCCA

ACTGGCCCCAAACTCCCATGGATCACAACCCCACCATGAACCTTATTACCGGGGT

ACCCA

ACTCCATGCTCAACAGTCCCCAGGTACAGCCCACCCTGCGTCGCAACCAGGAACA

GCTCT

ACAGCTTCCTGGAGCGCCACTCGCCCTACTTCCGCAGCCACAGTGCGCAGATTAG

GAGCG

CCACTTCTTTTTGTCACTTGAAAAACATGTAAAAATAATTACTTATGACTCGTACT

ATTG

TTATTCATCCAGGCGGTAGGAGGGCCATCATGGCTATGATGGAGGTCCAGGGGGG

ACCCA

GCCTGGGACAGACCTGCGTGCTGATCGTGATCTTTACAGTGCTCCTGCAGTCTCTC

TGTG

TGGCTGTAACTTACGTGTACTTTACCAACGAGCTGAAGCAGATGCAGGACAAGTA

CTCCA

AAAGTGGCATTGCTTGTTTCTTAAAAGAAGATGACAGTTATTGGGACCCCAATGA

CGAAG

AGAGTATGAACAGCCCCTGCTGGCAAGTCAAGTGGCAACTCCGTCAGCTCGTTAG

AAAGA

TGATTTTGAGAACCTCTGAGGAAACCATTTCTACAGTTCAAGAAAAGCAACAAAA

TATTT

CTCCCCTAGTGAGAGAAAGAGGTCCTCAGAGAGTAGCAGCTCACATAACTGGGAC

CAGAG

GAAGAAGCAACACATTGTCTTCTCCAAACTCCAAGAATGAAAAGGCTCTGGGCCG

CAAAA

TAAACTCCTGGGAATCATCAAGGAGTGGGCATTCATTCCTGAGCAACTTGCACTT

GAGGA

ATGGTGAACTGGTCATCCATGAAAAAGGGTTTACTACATCTATTCCCAAACATA

CTTTC

GATTTCAGGAGGAAATAAAAGAAAACACAAAGAACGACAAACAAATGGTCCAAT

ATATTT

ACAAATACACAAGTTATCCTGACCCTATATTGTTGATGAAAAGTGCTAGAAATAG

TTGTT

GGTCTAAAGATGCAGAATATGGACTCTATTCCATCTATCAAGGGGGAATATTTGA

GCTTA

AGGAAAATGACAGAATTTTTGTTTCTGTAACAAATGAGCACTTAATAGACATGGA

CCATG

AAGCCAGTTTTTTCGGGGCCTTTTTAGTTGGCTAAGCTAGCTACTAGAGACACTTT

CAAT

AAAGGCAAATGCTTTTATTTGTACACTCTCGGGTGATTATTTACCCCCACCCTTGC

CGTC

TGCGCCGTTTAAAAATCAAAGGGGTTCTGCCGCGCATCGCTATGCGCCACTGGCA

GGGAC

ACGTTGCGATACTGGTGTTTAGTGCTCCACTTAAACTCAGGCACAACCATCCGCG

GCAGC

TCGGTGAAGTTTTCACTCCACAGGCTGCGCACCATCACCAACGCGTTTAGCAGGT

CGGGC

GCCGATATCTTGAAGTCGCAGTTGGGGCCTCCGCCCTGCGCGCGCGAGTTGCGAT

ACACA

GGGTTGCAGCACTGGAACACTATCAGCGCCGGGTGGTGCACGCTGGCCAGCACGC

TCTTG

TCGGAGATCAGATCCGCGTCCAGGTCCTCCGCGTTGCTCAGGGCGAACGGAGTCA

ACTTT

GGTAGCTGCCTTCCCAAAAAGGGCGCGTGCCCAGGCTTTGAGTTGCACTCGCACC

GTAGT

GGCATCAAAAGGTGACCGTGCCCGGTCTGGGCGTTAGGATACAGCGCCTGCATAA

AAGCC

TTGATCTGCTTAAAAGCCACCTGAGCCTTTGCGCCTTCAGAGAAGAACATGCCGC

AAGAC

TTGCCGGAAAACTGATTGGCCGGACAGGCCGCGTCGTGCACGCAGCACCTTGCGT

CGGTG

TTGGAGATCTGCACCACATTTCGGCCCCACCGGTTCTTCACGATCTTGGCCTTGCT

AGAC

TGCTCCTTCAGCGCGCGCTGCCCGTTTTCGCTCGTCACATCCATTTCAATCACGTG

CTCC

TTATTTATCATAATGCTTCCGTGTAGACACTTAAGCTCGCCTTCGATCTCAGCGCA

GCGG

TGCAGCCACAACGCGCAGCCCGTGGGCTCGTGATGCTTGTAGGTCACCTCTGCAA

ACGAC

TGCAGGTACGCCTGCAGGAATCGCCCCATCATCGTCACAAAGGTCTTGTTGCTGG

TGAAG

GTCAGCTGCAACCCGCGGTGCTCCTCGTTCAGCCAGGTCTTGCATACGGCCGCCA

GAGCT

TCCACTTGGTCAGGCAGTAGTTTGAAGTTCGCCTTTAGATCGTTATCCACGTGGTA

CTTG

TCCATCAGCGCGCGCGCAGCCTCCATGCCCTTCTCCCACGCAGACACGATCGGCA

CACTC

AGCGGGTTCATCACCGTAATTTCACTTTCCGCTTCGCTGGGCTCTTCCTCTTCCTCT

TGC

GTCCGCATACCACGCGCCACTGGGTCGTCTTCATTCAGCCGCCGCACTGTGCGCTT

ACCT

CCTTTGCCATGCTTGATTAGCACCGGTGGGTTGCTGAAACCCACCATTTGTAGCGC

CACA

TCTTCTCTTTCTTCCTCGCTGTCCACGATTACCTCTGGTGATGGCGGGCGCTCGGG

CTTG

GGAGAAGGGCGCTTCTTTTTCTTCTTGGGCGCAATGGCCAAATCCGCCGCCGAGG

TCGAT

GGCCGCGGGCTGGGTGTGCGCGGCACCAGCGCGTCTTGTGATGAGTCTTCCTCGT

CCTCG

GACTCGATACGCCGCCTCATCCGCTTTTTTGGGGGCGCCCGGGGAGGCGGCGGCG

ACGGG

GACGGGGACGACACGTCCTCCATGGTTGGGGGACGTCGCGCCGCACCGCGTCCGC

GCTCG

GGGGTGGTTTCGCGCTGCTCCTCTTCCCGACTGGCCATTTCCTTCTCCTATAGGCA

GAAA

AAGATCATGGAGTCAGTCGAGAAGAAGGACAGCCTAACCGCCCCCTCTGAGTTCG

CCACC

ACCGCCTCCACCGATGCCGCCAACGCGCCTACCACCTTCCCCGTCGAGGCACCCC

CGCTT

GAGGAGGAGGAAGTGATTATCGAGCAGGACCCAGGTTTTGTAAGCGAAGACGAC

GAGGAC

CGCTCAGTACCAACAGAGGATAAAAAGCAAGACCAGGACAACGCAGAGGCAAAC

GAGGAA

CAAGTCGGGCGGGGGGACGAAAGGCATGGCGACTACCTAGATGTGGGAGACGAC

GTGCTG

TTGAAGCATCTGCAGCGCCAGTGCGCCATTATCTGCGACGCGTTGCAAGAGCGCA

GCGAT

GTGCCCCTCGCCATAGCGGATGTCAGCCTTGCCTACGAACGCCACCTATTCTCACC

GCGC

GTACCCCCCAAACGCCAAGAAAACGGCACATGCGAGCCCAACCCGCGCCTCAACT

TCTAC

CCCGTATTTGCCGTGCCAGAGGTGCTTGCCACCTATCACATCTTTTTCCAAAACTG

CAAG

ATACCCCTATCCTGCCGTGCCAACCGCAGCCGAGCGGACAAGCAGCTGGCCTTGC

GGCAG

GGCGCTGTCATACCTGATATCGCCTCGCTCAACGAAGTGCCAAAAATCTTTGAGG

GTCTT

GGACGCGACGAGAAGCGCGCGGCAAACGCTCTGCAACAGGAAAACAGCGAAAAT

GAAAGT

CACTCTGGAGTGTTGGTGGAACTCGAGGGTGACAACGCGCGCCTAGCCGTACTAA

AACGC

AGCATCGAGGTCACCCACTTTGCCTACCCGGCACTTAACCTACCCCCCAAGGTCAT

GAGC

ACAGTCATGAGTGAGCTGATCGTGCGCCGTGCGCAGCCCCTGGAGAGGGATGCAA

ATTTG

CAAGAACAAACAGAGGAGGGCCTACCCGCAGTTGGCGACGAGCAGCTAGCGCGC

TGGCTT

CAAACGCGCGAGCCTGCCGACTTGGAGGAGCGACGCAAACTAATGATGGCCGCA

GTGCTC

GTTACCGTGGAGCTTGAGTGCATGCAGCGGTTCTTTGCTGACCCGGAGATGCAGC

GCAAG

CTAGAGGAAACATTGCACTACACCTTTCGACAGGGCTACGTACGCCAGGCCTGCA

AGATC

TCCAACGTGGAGCTCTGCAACCTGGTCTCCTACCTTGGAATTTTGCACGAAAACCG

CCTT

GGGCAAAACGTGCTTCATTCCACGCTCAAGGGCGAGGCGCGCCGCGACTACGTCC

GCGAC

TGCGTTTACTTATTTCTATGCTACACCTGGCAGACGGCCATGGGCGTTTGGCAGCA

GTGC

TTGGAGGAGTGCAACCTCAAGGAGCTGCAGAAACTGCTAAAGCAAAACTTGAAG

GACCTA

TGGACGGCCTTCAACGAGCGCTCCGTGGCCGCGCACCTGGCGGACATCATTTTCC

CCGAA

CGCCTGCTTAAAACCCTGCAACAGGGTCTGCCAGACTTCACCAGTCAAAGCATGT
TGCAG
AACTTTAGGAACTTTATCCTAGAGCGCTCAGGAATCTTGCCCGCCACCTGCTGTGC
ACTT
CCTAGCGACTTTGTGCCCATTAAGTACCGCGAATGCCCTCCGCCGCTTTGGGGCCA
CTGC
TACCTTCTGCAGCTAGCCAACTACCTTGCCTACCACTCTGACATAATGGAAGACGT
GAGC
GGTGACGGTCTACTGGAGTGTCACTGTCGCTGCAACCTATGCACCCCGCACCGCT
CCCTG
GTTTGCAATTCGCAGCTGCTTAACGAAAGTCAAATTATCGGTACCTTTGAGCTGCA
GGGT
CCCTCGCCTGACGAAAAGTCCGCGGCTCCGGGGTTGAAACTCACTCCGGGGCTGT
GGACG
TCGGCTTACCTTCGCAAATTTGTACCTGAGGACTACCACGCCCACGAGATTAGGTT
CTAC
GAAGACCAATCCCGCCCGCCAAATGCGGAGCTTACCGCCTGCGTCATTACCCAGG
GCCAC
ATTCTTGGCCAATTGCAAGCCATCAACAAAGCCCGCCAAGAGTTTCTGCTACGAA
AGGGA
CGGGGGGTTTACTTGGACCCCCAGTCCGGCGAGGAGCTCAACCCAATCCCCCCGC
CGCCG
CAGCCCTATCAGCAGCAGCCGCGGGCCCTTGCTTCCCAGGATGGCACCCAAAAAG
AAGCT

GCAGCTGCCGCCGCCACCCACGGACGAGGAGGAATACTGGGACAGTCAGGCAGA
GGAGGT
TTTGGACGAGGAGGAGGAGGACATGATGGAAGACTGGGAGAGCCTAGACGAGGA
AGCTTC
CGAGGTCGAAGAGGTGTCAGACGAAACACCGTCACCCTCGGTCGCATTCCCCTCG
CCGGC
GCCCCAGAAATCGGCAACCGGTTCCAGCATGGCTACAACCTCCGCTCCTCAGGCG
CCGCC
GGCACTGCCCGTTCGCCGACCCAACCGTAGATGGGACACCACTGGAACCAGGGCC
GGTAA
GTCCAAGCAGCCGCCGCCGTTAGCCCAAGAGCAACAACAGCGCCAAGGCTACCG
CTCATG
GCGCGGGCACAAGAACGCCATAGTTGCTTGCTTGCAAGACTGTGGGGGCAACATC
TCCTT
CGCCCGCCGCTTTCTTCTCTACCATCACGGCGTGGCCTTCCCCCGTAACATCCTGC
ATTA
CTACCGTCATCTCTACAGCCCATACTGCACCGGCGGCAGCGGCAGCGGCAGCAAC
AGCAG
CGGCCACACAGAAGCAAAGGCGACCGGATAGCAAGACTCTGACAAAGCCCAAGA
AATCCA
CAGCGGCGGCAGCAGCAGGAGGAGGAGCGCTGCGTCTGGCGCCCAACGAACCCG
TATCGA
CCCGCGAGCTTAGAAACAGGATTTTTCCCACTCTGTATGCTATATTTCAACAGAGC
AGGG

GCCAAGAACAAGAGCTGAAAATAAAAAACAGGTCTCTGCGATCCCTCACCCGCA

GCTGCC

TGTATCACAAAAGCGAAGATCAGCTTCGGCGCACGCTGGAAGACGCGGAGGCTCT

CTTCA

GTAAATACTGCGCGCTGACTCTTAAGGACTAGTTTCGCGCCCTTTCTCAAATTTAA

GCGC

GAAAACTACGTCATCTCCAGCGGCCACACCCGGCGCCAGCACCTGTCGTCAGCGC

CATTA

TGAGCAAGGAAATTCCCACGCCCTACATGTGGAGTTACCAGCCACAAATGGGACT

TGCGG

CTGGAGCTGCCCAAGACTACTCAACCCGAATAAACTACATGAGCGCGGACCCCA

CATGA

TATCCCGGGTCAACGGAATCCGCGCCCACCGAAACCGAATTCTCTTGGAACAGGC

GGCTA

TTACCACCACACCTCGTAATAACCTTAATCCCCGTAGTTGGCCCGCTGCCCTGGTG

TACC

AGGAAAGTCCCGCTCCCACCACTGTGGTACTTCCCAGAGACGCCCAGGCCGAAGT

TCAGA

TGACTAACTCAGGGGCGCAGCTTGCGGGCGGCTTTCGTCACAGGGTGCGGTCGCC

CGGGC

AGGGTATAACTCACCTGACAATCAGAGGGCGAGGTATTCAGCTCAACGACGAGTC

GGTGA

GCTCCTCGCTTGGTCTCCGTCCGGACGGGACATTTCAGATCGGCGGCGCCGGCCG

TCCTT

CATTCACGCCTCGTCAGGCAATCCTAACTCTGCAGACCTCGTCCTCTGAGCCGCGC

TCTG

GAGGCATTGGAACTCTGCAATTTATTGAGGAGTTTGTGCCATCGGTCTACTTTAAC

CCCT

TCTCGGGACCTCCCGGCCACTATCCGGATCAATTTATTCCTAACTTTGACGCGGTA

AAGG

ACTCGGCGGACGGCTACGACTGAATGTTAAGTGGAGAGGCAGAGCAACTGCGCC

TGAAAC

ACCTGGTCCACTGTCGCCGCCACAAGTGCTTTGCCCGCGACTCCGGTGAGTTTTGC

TACT

TTGAATTGCCCGAGGATCATATCGAGGGCCCGGCGCACGGCGTCCGGCTTACCGC

CCAGG

GAGAGCTTGCCCGTAGCCTGATTCGGGAGTTTACCCAGCGCCCCCTGCTAGTTGA

GCGGG

ACAGGGGACCCTGTGTTCTCACTGTGATTTGCAACTGTCCTAACCTTGGATTACAT

CAAG

ATCTTTGTTGCCATCTCTGTGCTGAGTATAATAAATACAGAAATTAAAATATACTG

GGGC

TCCTATCGCCATCCTGTAAACGCCACCGTCTTCACCCGCCCAAGCAAACCAAGGC

GAACC

TTACCTGGTACTTTTAACATCTCTCCCTCTGTGATTTACAACAGTTTCAACCCAGA

CGGA

GTGAGTCTACGAGAGAACCTCTCCGAGCTCAGCTACTCCATCAGAAAAAACACCA

CCCTC

CTTACCTGCCGGGAACGTACGAGTGCGTCACCGGCCGCTGCACCACACCTACCGC
CTGAC

CGTAAACCAGACTTTTTCCGGACAGACCTCAATAACTCTGTTTACCAGAACAGGA
GGTGA

GCTTAGAAAACCCTTAGGGTATTAGGCCAAAGGCGCAGCTACTGTGGGGTTTATG
AACAA

TTCAAGCAACTCTACGGGCTATTCTAATTCAGGTTTCTCTAGAATCGGGGTTGGGG
TTAT

TCTCTGTCTTGTGATTCTCTTTATTCTTATACTAACGCTTCTCTGCCTAAGGCTCGC
CGC

CTGCTGTGTGCACATTTGCATTTATTGTCAGCTTTTTAAACGCTGGGGTCGCCACC
CAAG

ATGATTAGGTACATAATCCTAGGTTTACTCACCCTTGCGTCAGCCCACGGTACCAC
CCAA

AAGGTGGATTTTAAGGAGCCAGCCTGTAATGTTACATTCGCAGCTGAAGCTAATG
AGTGC

ACCACTCTTATAAAATGCACCACAGAACATGAAAAGCTGCTTATTCGCCACAAAA
ACAAA

ATTGGCAAGTATGCTGTTTATGCTATTTGGCAGCCAGGTGACACTACAGAGTATA
ATGTT

ACAGTTTTCCAGGGTAAAAGTCATAAAACTTTTATGTATACTTTTCCATTTTATGA
AATG

TGCGACATTACCATGTACATGAGCAAACAGTATAAGTTGTGGCCCCCACAAAATT
GTGTG

GAAAACACTGGCACTTTCTGCTGCACTGCTATGCTAATTACAGTGCTCGCTTTGGT

CTGT

ACCCTACTCTATATTAAATACAAAAGCAGACGCAGCTTTATTGAGGAAAAGAAAA

TGCCT

TAATTTACTAAGTTACAAAGCTAATGTCACCACTAACTGCTTTACTCGCTGCTTGC

AAAA

CAAATTCAAAAAGTTAGCATTATAATTAGAATAGGATTTAAACCCCCCGGTCATT

TCCTG

CTCAATACCATTCCCCTGAACAATTGACTCTATGTGGGATATGCTCCAGCGCTACA

ACCT

TGAAGTCAGGCTTCCTGGATGTCAGCATCTGACTTTGGCCAGCACCTGTCCCGCGG

ATTT

GTTCCAGTCCAACTACAGCGACCCACCCTAACAGAGATGACCAACACAACCAACG

CGGCC

GCCGCTACCGGACTTACATCTACCACAAATACACCCCAAGTTTCTGCCTTTGTCAA

TAAC

TGGGATAACTTGGGCATGTGGTGGTTCTCCATAGCGCTTATGTTTGTATGCCTTAT

TATT

ATGTGGCTCATCTGCTGCCTAAAGCGCAAACGCGCCCGACCACCCATCTATAGTC

CCATC

ATTGTGCTACACCCAAACAATGATGGAATCCATAGATTGGACGGACTGAAACACA

TGTTC

TTTTCTCTTACAGTATGATTAAATGAGACATGATTCCTCGAGTTTTTATATTACTGA

CCC

TTGTTGCGCTTTTTTGTGCGTGCTCCACATTGGCTGCGGTTTCTCACATCGAAGTA

GACT

GCATTCCAGCCTTCACAGTCTATTTGCTTTACGGATTTGTCACCCTCACGCTCATCT

GCA

GCCTCATCACTGTGGTCATCGCCTTTATCCAGTGCATTGACTGGGTCTGTGTGCGC

TTTG

CATATCTCAGACACCATCCCCAGTACAGGGACAGGACTATAGCTGAGCTTCTTAG

AATTC

TTTAATTATGAAATTTACTGTGACTTTTCTGCTGATTATTTGCACCCTATCTGCGTT

TTG

TTCCCCGACCTCCAAGCCTCAAAGACATATATCATGCAGATTCACTCGTATATGGA

ATAT

TCCAAGTTGCTACAATGAAAAAAGCGATCTTTCCGAAGCCTGGTTATATGCAATC

ATCTC

TGTTATGGTGTTCTGCAGTACCATCTTAGCCCTAGCTATATATCCCTACCTTGACA

TTGG

CTGGAACGCAATAGATGCCATGAACCACCCAACTTTCCCCGCGCCCGCTATGCTT

CCACT

GCAACAAGTTGTTGCCGGCGGCTTTGTCCCAGCCAATCAGCCTCGCCCACCTTCTC

CCAC

CCCCACTGAAATCAGCTACTTTAATCTAACAGGAGGAGATGACTGACACCCTAGA

TCTAG

AAATGGACGGAATTATTACAGAGCAGCGCCTGCTAGAAAGACGCAGGGCAGCGG

CCGAGC

AACAGCGCATGAATCAAGAGCTCCAAGACATGGTTAACTTGCACCAGTGCAAAA
GGGGTA
TCTTTTGTCTGGTAAAGCAGGCCAAAGTCACCTACGACAGTAATACCACCGGACA
CCGCC
TTAGCTACAAGTTGCCAACCAAGCGTCAGAAATTGGTGGTCATGGTGGGAGAAAA
GCCCA
TTACCATAACTCAGCACTCGGTAGAAACCGAAGGCTGCATTCACTCACCTTGTCA
AGGAC
CTGAGGATCTCTGCACCCTTATTAAGACCCTGTGCGGTCTCAAAGATCTTATTCCC
TTTA
ACTAATAAAAAAAAATAATAAAGCATCACTTACTTAAAATCAGTTAGCAAATTTC
TGTCC
AGTTTATTCAGCAGCACCTCCTTGCCCTCCTCCCAGCTCTGGTATTGCAGCTTCCTC
CTG
GCTGCAAACTTTCTCCACAATCTAAATGGAATGTCAGTTTCCTCCTGTTCCTGTCC
ATCC
GCACCCACTATCTTCATGTTGTTGCAGATGAAGCGCGCAAGACCGTCTGAAGATA
CCTTC
AACCCCGTGTATCCATATGACACGGAAACCGGTCCTCCAACTGTGCCTTTTCTTAC
TCCT
CCCTTTGTATCCCCCAATGGGTTTCAAGAGAGTCCCCCTGGGGTACTCTCTTTGCG
CCTA
TCCGAACCTCTAGTTACCTCCAATGGCATGCTTGCGCTCAAAATGGGCAACGGCC
TCTCT

CTGGACGAGGCCGGCAACCTTACCTCCCAAAATGTAACCACTGTGAGCCCACCTC TCAAA

AAAACCAAGTCAAACATAAACCTGGAAATATCTGCACCCCTCACAGTTACCTCAG AAGCC

CTAACTGTGGCTGCCGCCGCACCTCTAATGGTCGCGGCAACACACTCACCATGC AATCA

CAGGCCCCGCTAACCGTGCACGACTCCAAACTTAGCATTGCCACCCAAGGACCCC TCACA

GTGTCAGAAGGAAAGCTAGCCCTGCAAACATCAGGCCCCCTCACCACCACCGATA GCAGT

ACCCTTACTATCACTGCCTCACCCCCTCTAACTACTGCCACTGGTAGCTTGGGCAT TGAC

TTGAAAGAGCCCATTTATACACAAAATGGAAAACTAGGACTAAAGTACGGGGCTC CTTTG

CATGTAACAGACGACCTAAACACTTTGACCGTAGCAACTGGTCCAGGTGTGACTA TTAAT

AATACTTCCTTGCAAACTAAAGTTACTGGAGCCTTGGGTTTTGATTCACAAGGCAA TATG

CAACTTAATGTAGCAGGAGGACTAAGGATTGATTCTCAAAACAGACGCCTTATAC TTGAT

GTTAGTTATCCGTTTGATGCTCAAAACCAACTAAATCTAAGACTAGGACAGGGCC CTCTT

TTTATAAACTCAGCCCACAACTTGGATATTAACTACAACAAAGGCCTTTACTTGTT TACA

GCTTCAAACAATTCCAAAAAGCTTGAGGTTAACCTAAGCACTGCCAAGGGGTTGA TGTTT

GACGCTACAGCCATAGCCATTAATGCAGGAGATGGGCTTGAATTTGGTTCACCTA ATGCA

CCAAACACAAATCCCCTCAAAACAAAAATTGGCCATGGCCTAGAATTTGATTCAA ACAAG

GCTATGGTTCCTAAACTAGGAACTGGCCTTAGTTTTGACAGCACAGGTGCCATTAC AGTA

GGAAACAAAAATAATGATAAGCTAACTTTGTGGACCACACCAGCTCCATCTCCTA ACTGT

AGACTAAATGCAGAGAAAGATGCTAAACTCACTTTGGTCTTAACAAAATGTGGCA GTCAA

ATACTTGCTACAGTTTCAGTTTTGGCTGTTAAAGGCAGTTTGGCTCCAATATCTGG AACA

GTTCAAAGTGCTCATCTTATTATAAGATTTGACGAAAATGGAGTGCTACTAAACA ATTCC

TTCCTGGACCCAGAATATTGGAACTTTAGAAATGGAGATCTTACTGAAGGCACAG CCTAT

ACAAACGCTGTTGGATTTATGCCTAACCTATCAGCTTATCCAAAATCTCACGGTAA AACT

GCCAAAAGTAACATTGTCAGTCAAGTTTACTTAAACGGAGACAAAACTAAACCTG TAACA

CTAACCATTACACTAAACGGTACACAGGAAACAGGAGACACAACTCCAAGTGCA TACTCT

ATGTCATTTTCATGGGACTGGTCTGGCCACAACTACATTAATGAAATATTTGCCAC ATCC

TCTTACACTTTTTCATACATTGCCCAAGAATAAAGAATCGTTTGTGTTATGTTTCA ACGT

GTTTATTTTTCAATTGCAGAAAATTTCAAGTCATTTTTCATTCAGTAGTATAGCCCC ACC

ACCACATAGCTTATACAGATCACCGTACCTTAATCAAACTCACAGAACCCTAGTA TTCAA

CCTGCCACCTCCCTCCCAACACACAGAGTACACAGTCCTTTCTCCCCGGCTGGCCT TAAA

AAGCATCATATCATGGGTAACAGACATATTCTTAGGTGTTATATTCCACACGGTTT CCTG

TCGAGCCAAACGCTCATCAGTGATATTAATAAACTCCCCGGGCAGCTCACTTAAG TTCAT

GTCGCTGTCCAGCTGCTGAGCCACAGGCTGCTGTCCAACTTGCGGTTGCTTAACGG GCGG

CGAAGGAGAAGTCCACGCCTACATGGGGGTAGAGTCATAATCGTGCATCAGGAT AGGGCG

GTGGTGCTGCAGCAGCGCGCGAATAAACTGCTGCCGCCGCCGCTCCGTCCTGCAG GAATA

CAACATGGCAGTGGTCTCCTCAGCGATGATTCGCACCGCCCGCAGCATAAGGCGC CTTGT

CCTCCGGGCACAGCAGCGCACCCTGATCTCACTTAAATCAGCACAGTAACTGCAG CACAG

CACCACAATATTGTTCAAAATCCCACAGTGCAAGGCGCTGTATCCAAAGCTCATG
GCGGG

GACCACAGAACCCACGTGGCCATCATACCACAAGCGCAGGTAGATTAAGTGGCG
ACCCCT

CATAAACACGCTGGACATAAACATTACCTCTTTTGGCATGTTGTAATTCACCACCT
CCCG

GTACCATATAAACCTCTGATTAAACATGGCGCCATCCACCACCATCCTAAACCAG
CTGGC

CAAAACCTGCCCGCCGGCTATACACTGCAGGGAACCGGGACTGGAACAATGACA
GTGGAG

AGCCCAGGACTCGTAACCATGGATCATCATGCTCGTCATGATATCAATGTTGGCA
CAACA

CAGGCACACGTGCATACACTTCCTCAGGATTACAAGCTCCTCCCGCGTTAGAACC
ATATC

CCAGGGAACAACCCATTCCTGAATCAGCGTAAATCCCACACTGCAGGGAAGACCT
CGCAC

GTAACTCACGTTGTGCATTGTCAAAGTGTTACATTCGGGCAGCAGCGGATGATCC
TCCAG

TATGGTAGCGCGGGTTTCTGTCTCAAAAGGAGGTAGACGATCCCTACTGTACGGA
GTGCG

CCGAGACAACCGAGATCGTGTTGGTCGTAGTGTCATGCCAAATGGAACGCCGGAC
GTAGT

CATATTTCCTGAAGCAAAACCAGGTGCGGGCGTGACAAACAGATCTGCGTCTCCG
GTCTC

GCCGCTTAGATCGCTCTGTGTAGTAGTTGTAGTATATCCACTCTCTCAAAGCATCC

AGGC

GCCCCCTGGCTTCGGGTTCTATGTAAACTCCTTCATGCGCCGCTGCCCTGATAACA

TCCA

CCACCGCAGAATAAGCCACACCCAGCCAACCTACACATTCGTTCTGCGAGTCACA

CACGG

GAGGAGCGGGAAGAGCTGGAAGAACCATGTTTTTTTTTTATTCCAAAAGATTAT

CCAAA

ACCTCAAAATGAAGATCTATTAAGTGAACGCGCTCCCCTCCGGTGGCGTGGTCAA

ACTCT

ACAGCCAAAGAACAGATAATGGCATTTGTAAGATGTTGCACAATGGCTTCCAAAA

GGCAA

ACGGCCCTCACGTCCAAGTGGACGTAAAGGCTAAACCCTTCAGGGTGAATCTCCT

CTATA

AACATTCCAGCACCTTCAACCATGCCCAAATAATTCTCATCTCGCCACCTTCTCAA

TATA

TCTCTAAGCAAATCCCGAATATTAAGTCCGGCCATTGTAAAAATCTGCTCCAGAG

CGCCC

TCCACCTTCAGCCTCAAGCAGCGAATCATGATTGCAAAAATTCAGGTTCCTCACA

GACCT

GTATAAGATTCAAAAGCGGAACATTAACAAAAATACCGCGATCCCGTAGGTCCCT

TCGCA

GGGCCAGCTGAACATAATCGTGCAGGTCTGCACGGACCAGCGCGGCCACTTCCCC

GCCAG

GAACCTTGACAAAAGAACCCACACTGATTATGACACGCATACTCGGAGCTATGCT AACCA

GCGTAGCCCCGATGTAAGCTTTGTTGCATGGGCGGCGATATAAAATGCAAGGTGC TGCTC

AAAAAATCAGGCAAAGCCTCGCGCAAAAAAGAAAGCACATCGTAGTCATGCTCA TGCAGA

TAAAGGCAGGTAAGCTCCGGAACCACCACAGAAAAAGACACCATTTTTCTCTCAA ACATG

TCTGCGGGTTTCTGCATAAACACAAAATAAAATAACAAAAAAACATTTAAACATT AGAAG

CCTGTCTTACAACAGGAAAAACAACCCTTATAAGCATAAGACGGACTACGGCCAT GCCGG

CGTGACCGTAAAAAAACTGGTCACCGTGATTAAAAAGCACCACCGACAGCTCCTC GGTCA

TGTCCGGAGTCATAATGTAAGACTCGGTAAACACATCAGGTTGATTCATCGGTCA GTGCT

AAAAAGCGACCGAAATAGCCCGGGGGAATACATACCCGCAGGCGTAGAGACAAC ATTACA

GCCCCCATAGGAGGTATAACAAAATTAATAGGAGAGAAAAACACATAAACACCT GAAAAA

CCCTCCTGCCTAGGCAAAATAGCACCCTCCCGCTCCAGAACAACATACAGCGCTT CCACA

GCGGCAGCCATAACAGTCAGCCTTACCAGTAAAAAAGAAAACCTATTAAAAAAA CACCAC

TCGACACGGCACCAGCTCAATCAGTCACAGTGTAAAAAAGGGCCAAGTGCAGAG

CGAGTA

TATATAGGACTAAAAAATGACGTAACGGTTAAAGTCCACAAAAAACACCCAGAA

AACCGC

ACGCGAACCTACGCCCAGAAACGAAAGCCAAAAAACCCACAACTTCCTCAAATC

GTCACT

TCCGTTTTCCCACGTTACGTCACTTCCCATTTTAATTAAGAAAACTACAATTCCCA

ACAC

ATACAAGTTACTCCGCCCTAAAACCTACGTCACCCGCCCCGTTCCCACGCCCCGCG

CCAC

GTCACAAACTCCACCCCCTCATTATCATATTGGCTTCAATCCAAAATAAGGTATAT

TATT

GATGATGATTACCCT

SEQ ID NO:52 is the sequence for oligonucleotide primer 1618.95.1
5'TACCGGGGTACCCAACTCCA
SEQ ID NO:53 is the sequence for oligonucleotide primer 1618.95.6
5' GACGCGGCCTGTCCGGCC

SEQ ID NO:54 is the sequence for oligonucleotide primer 1618.97.1

5' TACTTATGACTCGTACTATTGTTATTCATCCAGGCGGTAGGAGGGCCATC

ATGAA

SEQ ID NO:55 is the sequence for oligonucleotide primer 1618.97.2
5'CCTTTATTGAAAGTGTCTCTAGTAGCTAGCGGGAGGGAGGTCC
SEQ ID NO:56 is the sequence for oligonucleotide primer 1618.95.5
5'TACTAGAGACACTTTCAATAAAGG
SEQ ID NO:57 is the sequence for oligonucleotide primer 1706.83.1
5' GTT AAC ATG GTT CTG CAG AGC
SEQ ID NO:58 is the sequence for oligonucleotide primer 1706.83.2
5' GGC TCG TCC ATG GGA TCC ACC TCA AAA GTC
SEQ ID NO:59 is the sequence for oligonucleotide primer 1706.95.1
5' GGA TCC CAT GGA CGA GCC CA
SEQ ID NO:60 is the sequence for oligonucleotide primer 1618.116.3
5' CT TAT TAC CGG GGT ACC CAA CTC CTC GAG TAT TT
SEQ ID NO:61 is the sequence for oligonucleotide primer 1619.144.1
5' TACAA GTA TAC GCC ACC ATG GCT ATG ATG GAG GTC CAG

SEQ ID NO:62 is the sequence for oligonucleotide primer 1619.144.3
5' TTGTA GTATAC TTA GCC AAC TAA AAA GGC CCC

## Claims

1. An adenoviral vector comprising a chimeric L1 late region, the chimeric L1 late region **characterized by** a heterologous coding sequence (CDS), a heterologous branch point site, a heterologous splice acceptor site, an endogenous IIIa CDS, and an endogenous polyadenylation signal, wherein the heterologous CDS, the heterologous branch point site, and the heterologous splice acceptor site are located downstream of the endogenous IIIa CDS and upstream of the endogenous polyadenylation sequence, wherein the heterologous CDS is operatively linked to said endogenous polyadenylation signal, wherein the endogenous IIIa CDS is also operatively linked to said endogenous polyadenylation signal.

2. The adenoviral vector of claim 1, wherein the chimeric L1 region comprises, in sequential order, the endogenous CDS, the heterologous branch point, the heterologous splice acceptor site, the heterologous CDS and the endogenous polyadenylation signal.

3. An adenoviral vector comprising a chimeric L1 late region, the chimeric L1 late region **characterized by** an endogenous IIIa CDS, a self-processing cleavage site, a heterologous coding sequence (CDS), and an endogenous polyadenylation signal, wherein the heterologous CDS is located downstream of the endogenous IIIa CDS and upstream of the endogenous polyadenylation signal, wherein the heterologous gene CDS and the endogenous IIIa CDS are operatively linked to said self-processing cleavage site.

4. The adenoviral vector of Claim 3, wherein the chimeric L1 late region comprises in sequential order the endogenous IIIa CDS, the self-processing cleavage site, the heterologous CDS and the endogenous polyadenylation signal.

5. The adenoviral vector of claims 3 or 4, wherein said self-processing cleavage site CDS is a 2A or 2A-like sequence.

6. The adenoviral vector of claim 3 to 5, wherein said self-processing cleavage site CDS is selected from the group consisting of SEQ ID No. 1-32.

## Patentansprüche

1. Adenoviraler Vektor, umfassend eine chimäre späte Region L1, wobei die chimäre späte Region L1 durch eine heterologe codierende Sequenz (CDS), eine heterologe Verzweigungsstelle, eine heterologe Splice-Akzeptor-Stelle, eine endogene IIIa-CDS und ein endogenes Polyadenylierungssignal **gekennzeichnet** ist, wobei die heterologe CDS, die heterologe Verzweigungsstelle und die heterologe Splice-Akzeptor-Stelle stromabwärts der endogenen IIIa-CDS und stromaufwärts der endogenen Polyadenylierungssequenz lokalisiert sind, wobei die heterologe CDS funktionell mit dem endogenen Polyadenylierungssignal verbunden ist, wobei die endogene IIIa-CDS ebenfalls funktionell mit dem endogenen Polyadenylierungssignal verbunden ist.

2. Adenoviraler Vektor gemäss Anspruch 1, wobei die chimäre L1-Region in aufeinanderfolgender Reihenfolge die endogene CDS, die heterologe Verzweigungsstelle, die heterologe Splice-Akzeptor-Stelle, die heterologe CDS und das endogene Polyadenylierungssignal umfasst.

3. Adenoviraler Vektor, umfassend eine chimäre späte Region L1, wobei die chimäre späte Region L1 durch eine endogene IIIa-CDS, eine selbstprozessierende Spaltstelle, eine heterologe codierende Sequenz (CDS) und ein endogenes Polyadenylierungssignal **gekennzeichnet** ist, wobei die heterologe CDS stromabwärts der endogenen IIIa-CDS und stromaufwärts des endogenen Polyadenylierungssignals lokalisiert ist, wobei die heterologe Gen-CDS und die endogene IIIa-CDS funktionell mit der selbstprozessierenden Spaltstelle verbunden sind.

4. Adenoviraler Vektor gemäss Anspruch 3, wobei die chimäre späte Region L1 in aufeinanderfolgender Reihenfolge die endogene IIIa-CDS, die selbstprozessierende Spaltstelle, die heterologe CDS und das endogene Polyadenylierungssignal umfasst.

5. Adenoviraler Vektor gemäss Anspruch 3 oder 4, wobei die CDS der selbstprozessierenden Spaltstelle eine 2A- oder 2A-ähnliche Sequenz ist.

6. Adenoviraler Vektor gemäss den Ansprüchen 3 bis 5, wobei die CDS der selbstprozessierenden Spaltstelle ausgewählt ist aus der Gruppe bestehend aus SEQ ID NO: 1 bis 32.

**Revendications**

1. Vecteur adénoviral comprenant une région tardive L1 chimère, la région tardive L1 chimère **caractérisée par** une séquence codante hétérologue (CDS), un site de point de ramification hétérologue, un site accepteur d'épissage hétérologue, une CDS IIIa endogène, et un signal de polyadénylation endogène, dans lequel la CDS hétérologue, le site de point de ramification hétérologue, et le site accepteur d'épissage hétérologue sont localisés en aval de la CDS IIIa endogène et en amont de la séquence de polyadénylation endogène, dans lequel la CDS hétérologue est liée de manière fonctionnelle audit signal de polyadénylation endogène, dans lequel la CDS IIIa endogène est également liée de manière fonctionnelle audit signal de polyadénylation endogène.

2. Vecteur adénoviral selon la revendication 1, dans lequel la région L1 chimère comprend, dans un ordre séquentiel, la CDS endogène, le point de ramification hétérologue, le site accepteur d'épissage hétérologue, la CDS hétérologue et le signal de polyadénylation endogène.

3. Vecteur adénoviral comprenant une région tardive L1 chimère, la région tardive L1 chimère **caractérisée par** une CDS IIIa endogène, un site d'auto-clivage, une séquence codante hétérologue (CDS), et un signal de polyadénylation endogène, dans lequel la CDS hétérologue est localisée en aval de la CDS IIIa endogène et en amont du signal de polyadénylation endogène, dans lequel la CDS de gène hétérologue et la CDS IIIa endogène sont liées de manière fonctionnelle audit site d'auto-clivage.

4. Vecteur adénoviral selon la revendication 3, dans lequel la région tardive L1 chimère comprend dans un ordre séquentiel la CDS IIIa endogène, le site d'auto-clivage, la CDS hétérologue et le signal de polyadénylation endogène.

5. Vecteur adénoviral selon les revendications 3 ou 4, dans lequel ladite CDS de site d'auto-clivage est une séquence 2A ou analogue à 2A.

6. Vecteur adénoviral selon la revendication 3 à 5, dans lequel ladite CDS de site d'auto-clivage est choisie dans le groupe constitué de SEQ ID N° 1 à 32.

# Figure 1

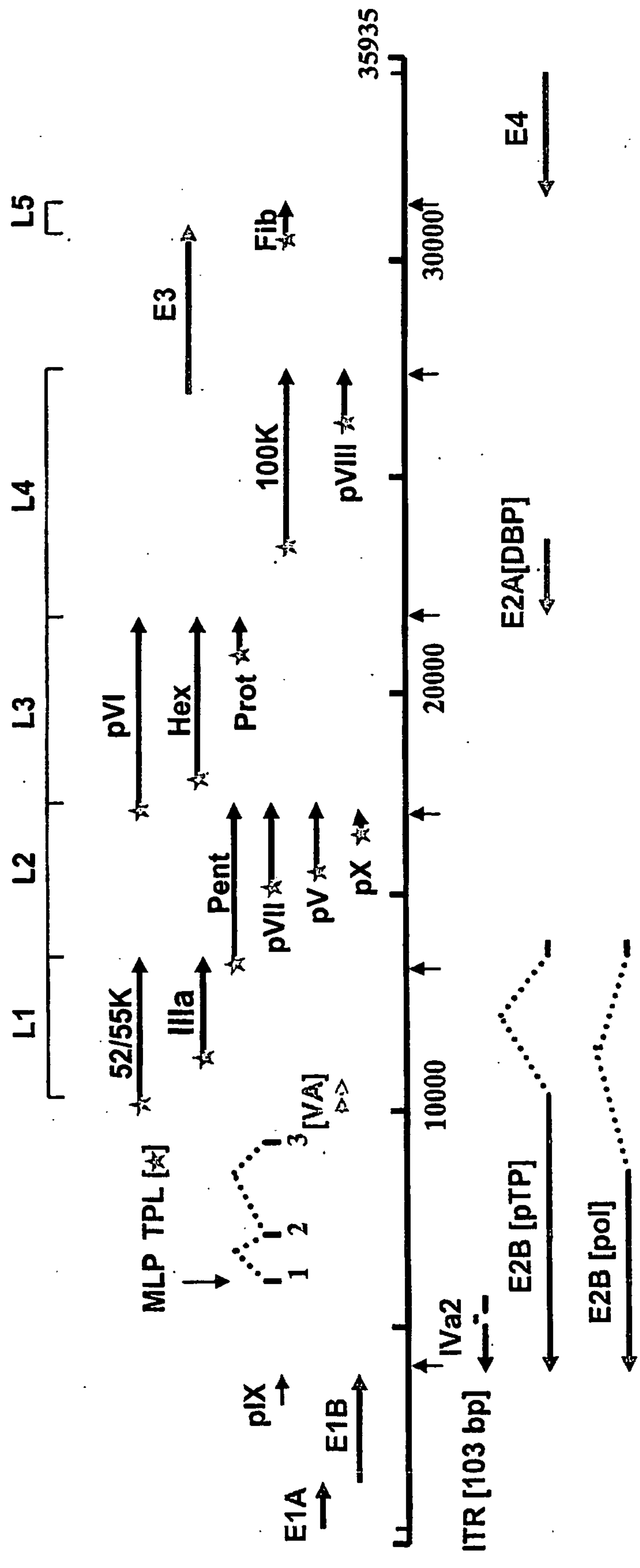


↑ = examples of insertion site for an IRES-transgene cassette

# Figure 2

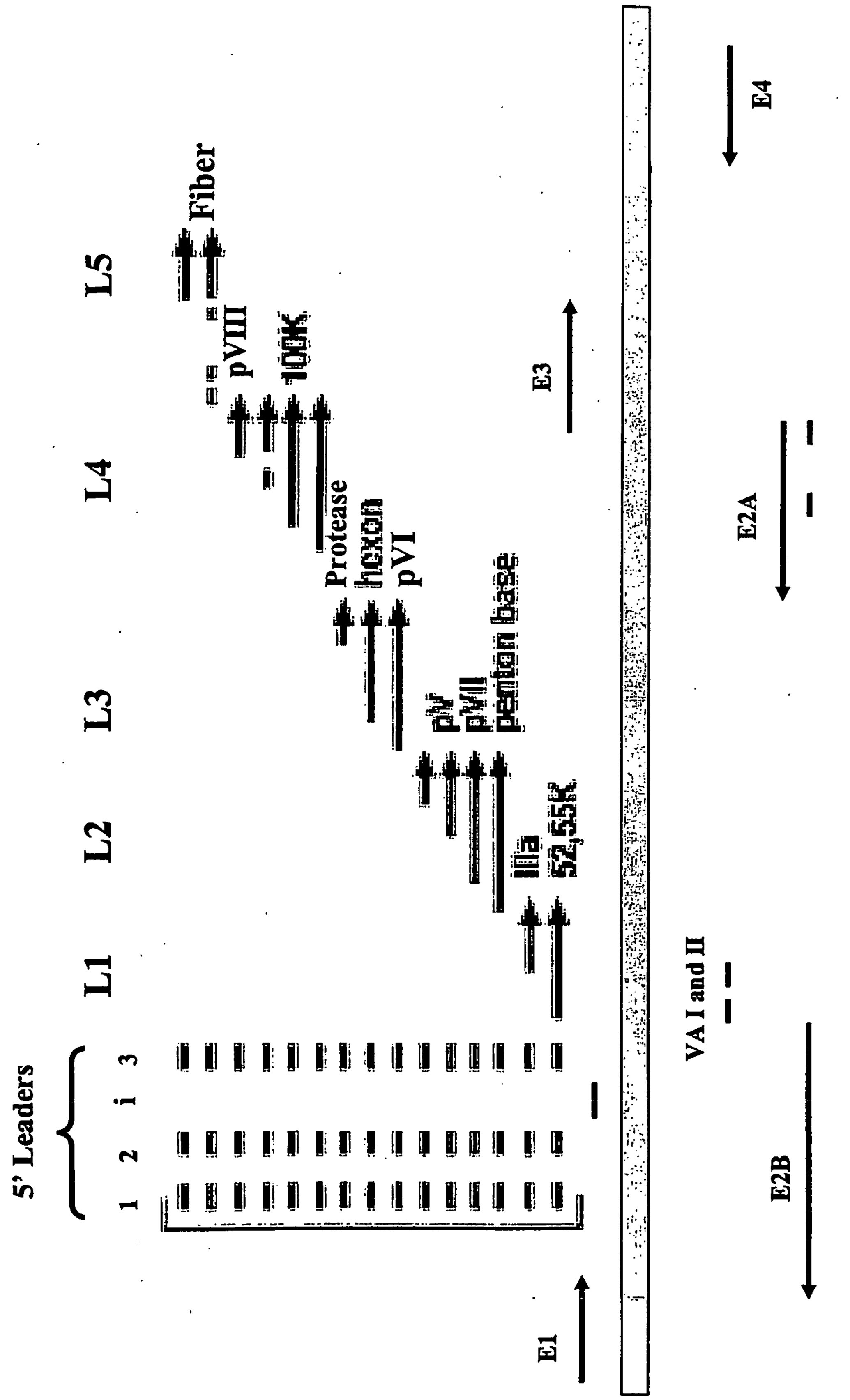
5' Leaders
1
2
i
3
L1
L2
L3
L4
L5
Fiber
pVIII
100K
Protease
hexon
pVI
pV
pVII
penton base
IIIa
52,55K
E1
E2B
VA I and II
E2A
E3
E4

# Figure 3

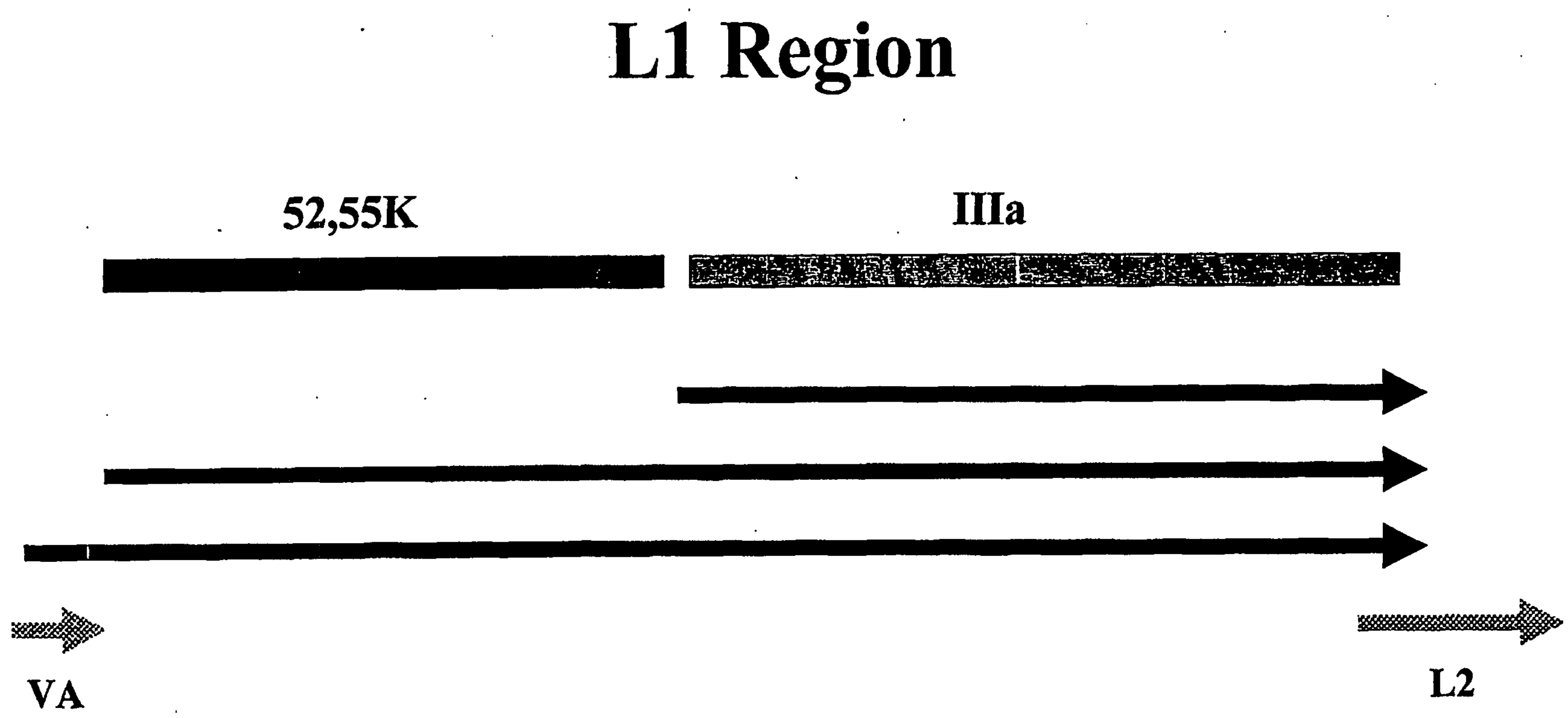

# Figure 4

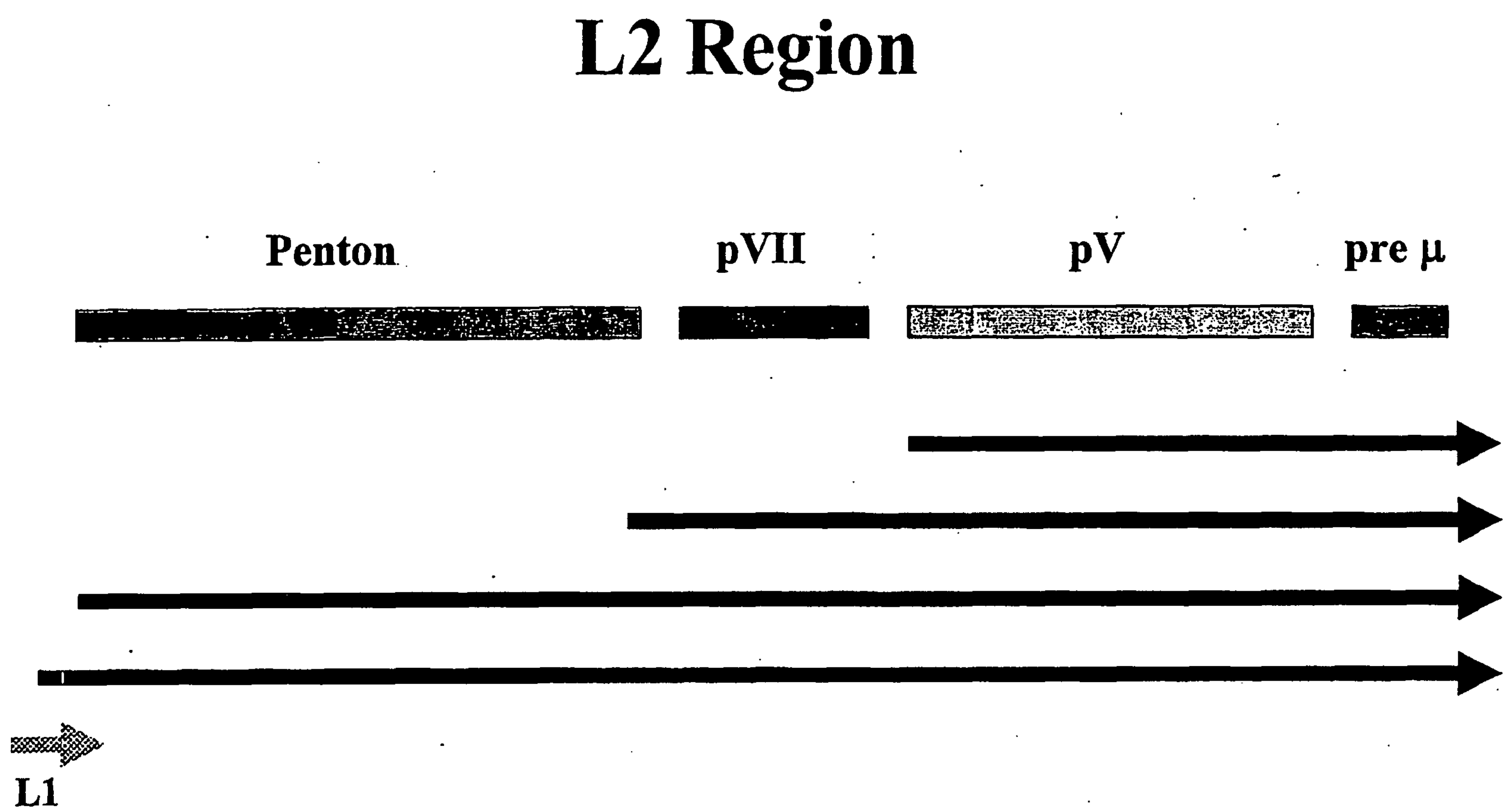

Figure 5

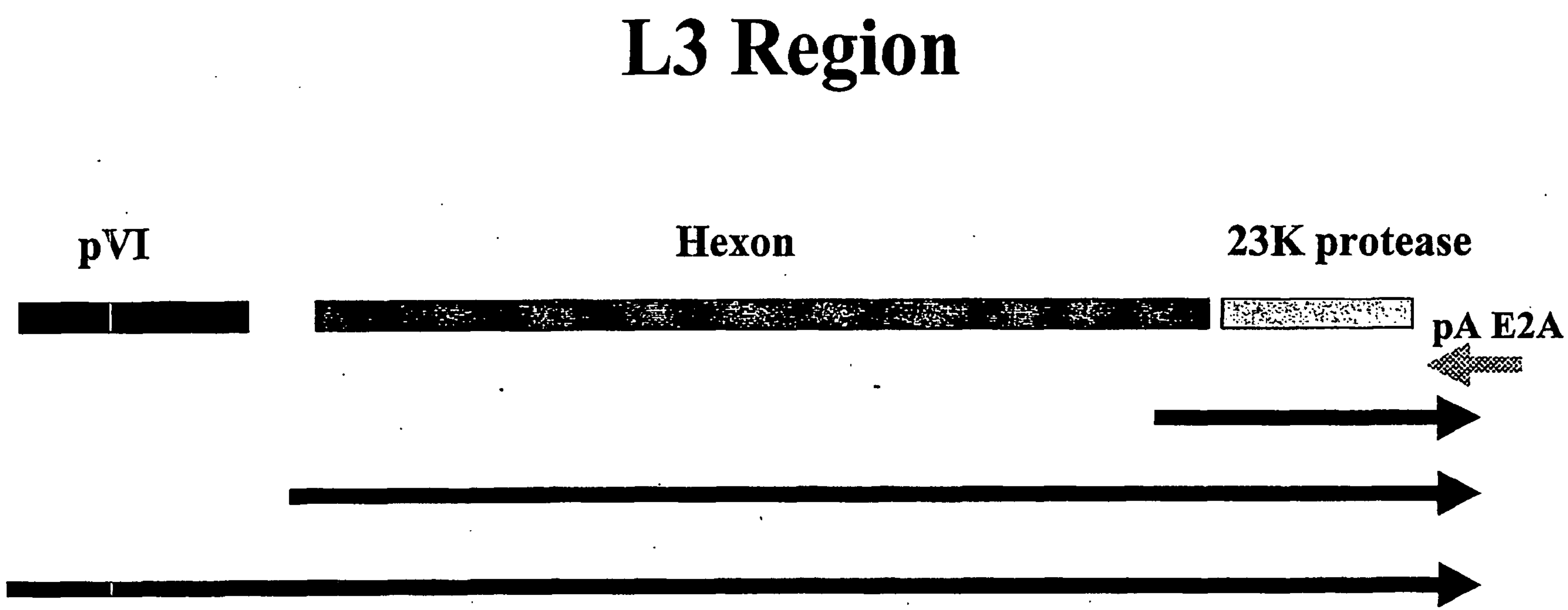

Figure 6

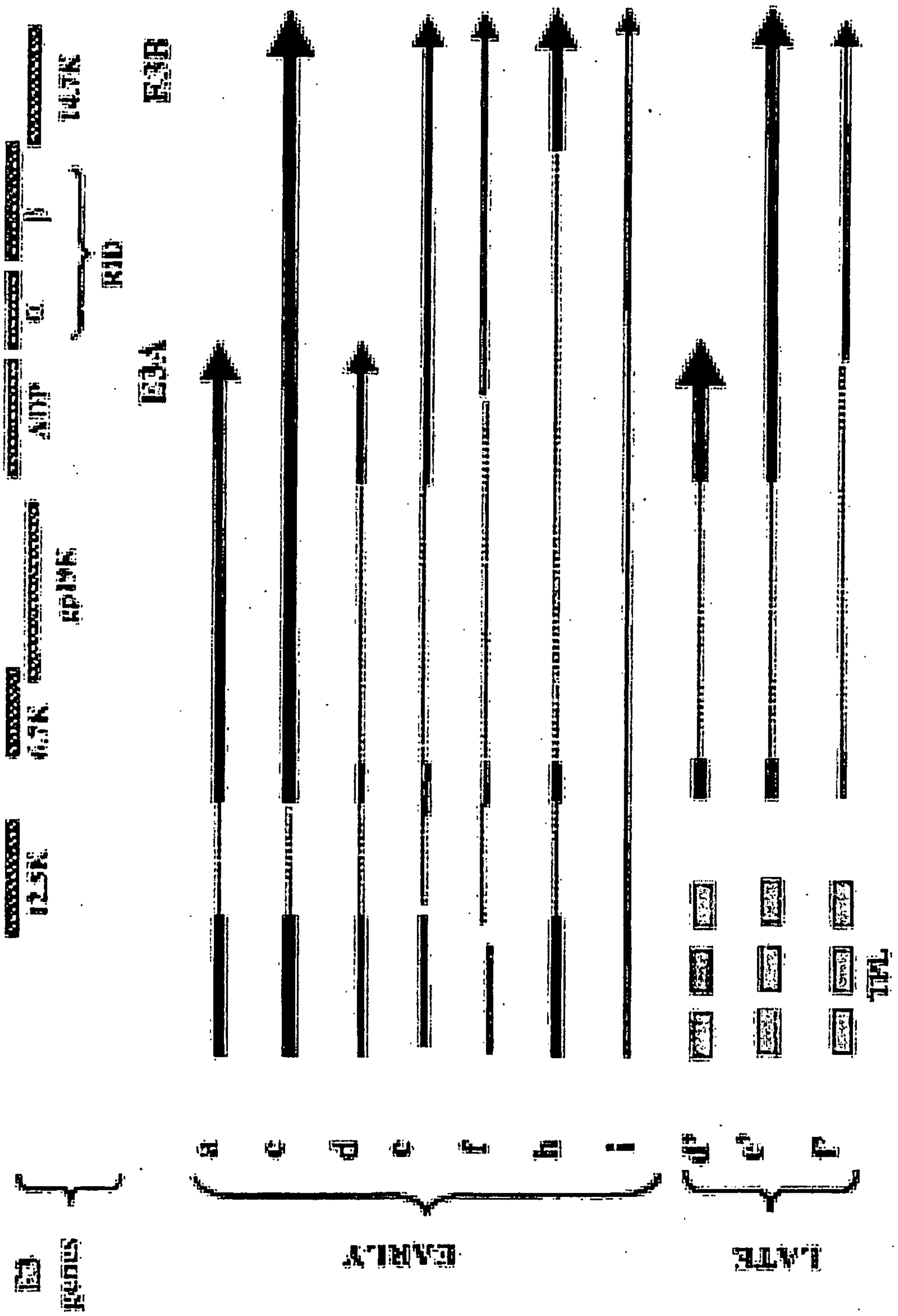

# Figure 7

## E3A Region

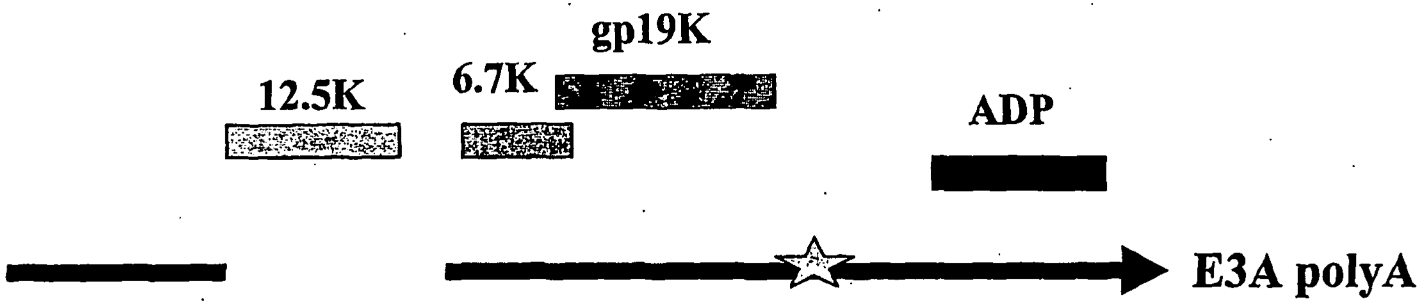


☆ Region of 192 bp in Ad5 not present in Ad2, option to delete

Figure 8

E3B Region

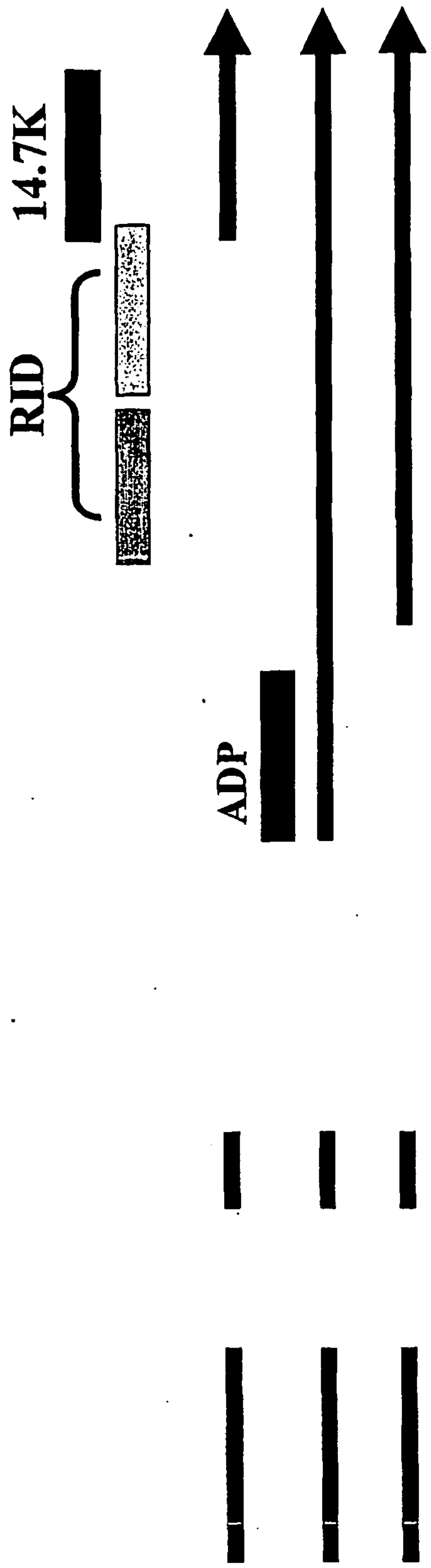

# Figure 9

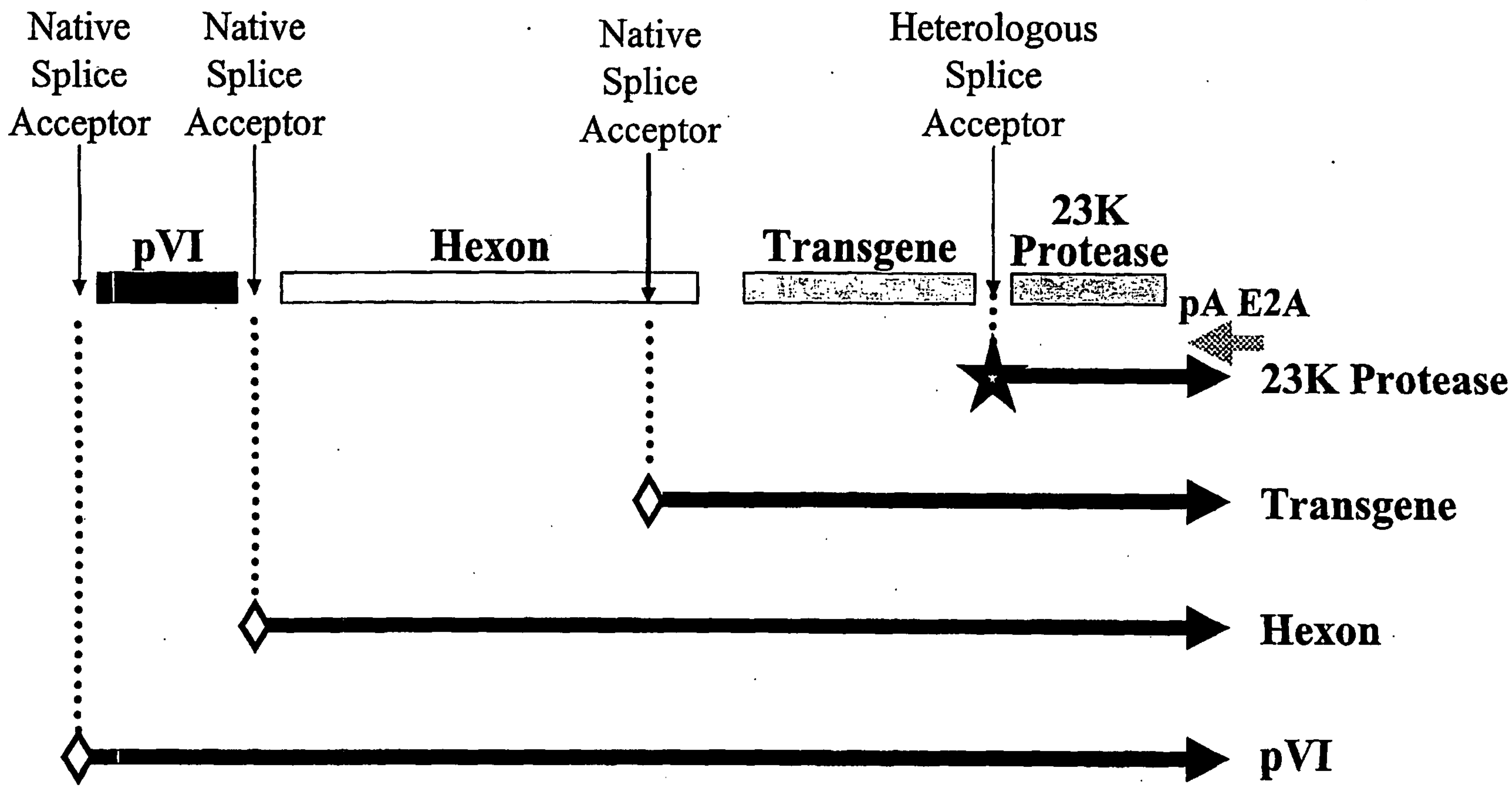


L3 Region

Figure 10

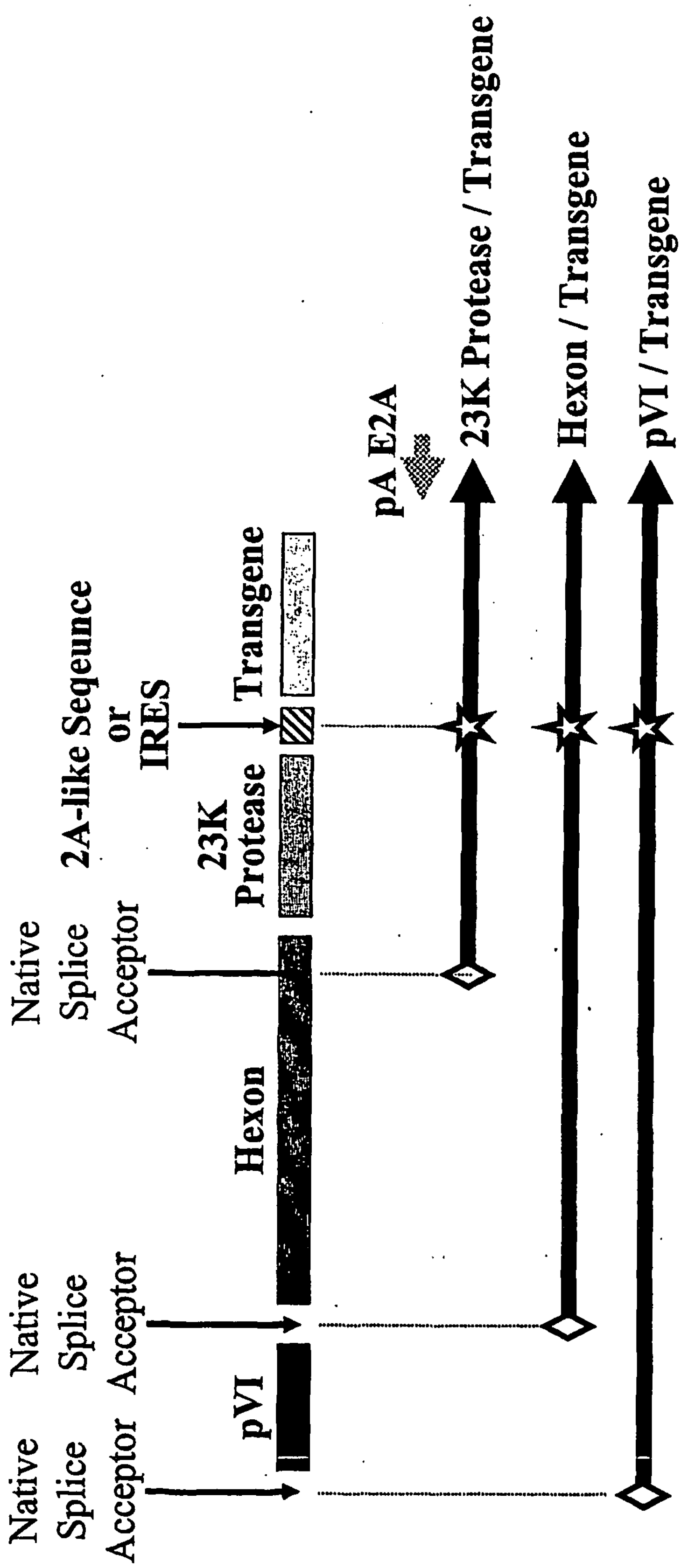


L3 Region

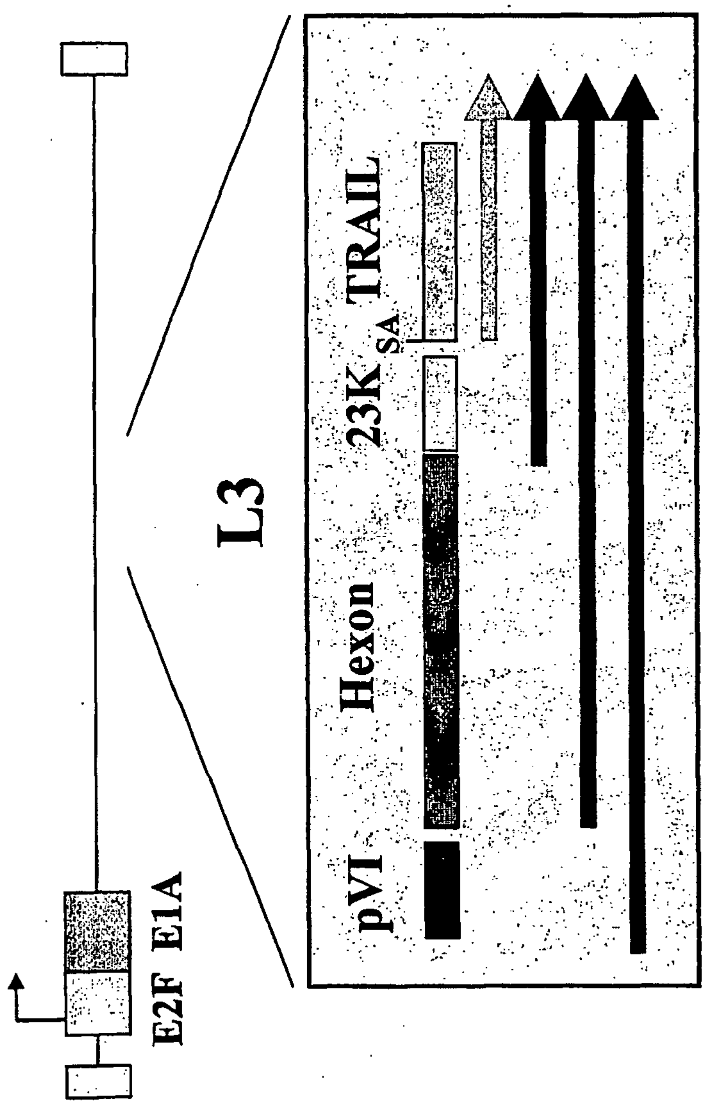


Figure 11

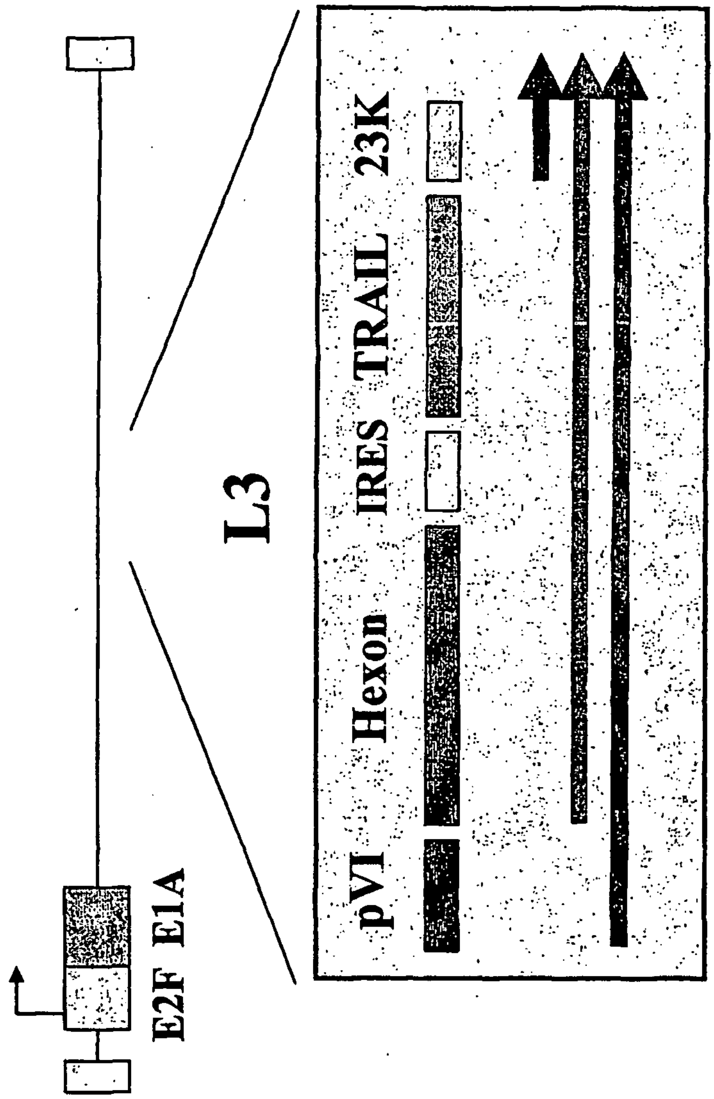


Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 58980404 P
- US 66054205 P
- WO 02067861 A
- WO 0102540 A
- WO 9839466 A
- WO 9519434 A
- WO 9701358 A
- WO 9839467 A
- WO 9839465 A
- WO 0172994 A
- WO 04009790 A
- WO 0015820 A
- WO 9814593 A
- WO 0046355 A
- WO 9839464 A
- WO 9813508 A
- WO 20004009790 A
- US 511812 P
- US 60423203 P
- US 20010053352 A
- WO 02068627 A
- WO 0155369 A
- US 6692736 B
- US 20030104625 A
- WO 9617070 A
- US 9804080 W
- US 5994128 A
- US 6033908 A
- US 0411855 W
- US 10403337 B
- US 20040002060 A
- WO 9807877 A
- WO 9939734 A
- WO 0067576 A
- WO 0192299 A
- US 5543328 A
- US 5731190 A
- US 5756086 A
- US 5770442 A
- US 5846782 A
- US 5962311 A
- US 5922315 A
- US 6057155 A
- US 6127525 A
- US 6153435 A
- US 6455314 A
- US 6555368 A
- US 6683170 A
- WO 9906576 A
- US 423203 P
- US 5846767 A, Halpin
- US 1998 A
- US SN10831304 A
- US 20030068307 A


### Non-patent literature cited in the description


- **Bett et al.** *J Virol,* 1993, vol. 67, 5911-5921
- **Fuerer et al.** *Gene Therapy,* 2004, vol. 11, 142-151
- **Sambrook et al.** Molecular Cloning: A Laboratory Manual. 1989
- Oligonucleotide Synthesis. 1984
- Animal Cell Culture. 1987
- Methods in Enzymology. Academic Press, Inc,
- Handbook of Experimental Immunology
- Gene Transfer Vectors for Mammalian Cells. 1987
- Current Protocols in Molecular Biology. 1987
- PCR: The Polymerase Chain Reaction. 1994
- Current Protocols in Immunology. 1991
- **Chao et al.** *Molecular and Cellular Biology,* 1999, vol. 19, 5588-5600
- **Jackson R J ; Howell M T ; Kaminski A.** *Trends Biochem Sci,* 1990, vol. 15 (12), 477-83
- **Jackson R J ; Kaminski, A.** *RNA,* 1995, vol. 1 (10), 985-1000
- **Jackson et al.** *Trends Biochem Sci,* 1990, vol. 15 (12), 477-483
- **Huez et al.** *Mol. Cell. Biol.,* 1998, vol. 18 (11), 6178-6190
- **Novogen, Duke et al.** *J. Virol,* 1992, vol. 66 (3), 1602-1609
- **Furler.** *Palmenberg, Ann. Rev. Microbiol.,* 1990, vol. 44, 603-623
- **Fallaux et al.** *Hum Gene Ther.,* 01 September 1998, vol. 9 (13), 1909-17
- **Graham et al.** *J Gen Virol.,* July 1977, vol. 36 (1), 59-74
- **T.T. Puck et al.** *J. Exp. Med.,* 1956, vol. 103, 273-284

- *Clontechniques,* January 2000, 10-12
- **Nevins ; Chen-Kiang.** *Adv Virus Res,* 1981, vol. 26, 1-35
- **Akusjarvi ; Stevenin.** *Curr Topics Microbiol Immunology,* 2003, vol. 272, 253-86
- **Prescott ; Falck-Pedersen.** *Mol Cell Biol,* 1994, vol. 14, 4682-4693
- **Muhlemann et al.** *J Virol,* 1995, vol. 69, 7324-7327
- **Nevins ; Wilson.** *Nature,* 12 March 1981, vol. 290 (5802), 113-8
- **Prescott ; Falck-Pederson.** *J Biol Chem,* 1992, vol. 267, 8175
- **Larsson ; Svensson ; Akusjarvi.** *J Mol Biol,* 1992, vol. 225, 287
- **Farley ; Brown ; Leppard.** *J Virol,* 2004, vol. 78, 1782
- **Rao et al.** *Proc. Natl. Acad. Sci. USA,* vol. 89, 7742-7746
- **Berkner et al.** *Nucleic Acid Research,* 1983, vol. 11, 6003-6020
- **Bridge et al.** *J. Virol.,* 1989, vol. 63, 631-638
- **McKinnon.** *Gene,* 1982, vol. 19, 33-42
- **Graham et al.** *J Gen. Virol.,* 1977, vol. 36, 59-72
- **Fallaux et al.** *Hum. Gene Ther,* 1998, vol. 9, 1909-1917
- **Wu et al.** *J Virol.,* 01 July 2003, vol. 77 (13), 7225-7235
- **Bett et al.** *Virol.,* October 1993, vol. 67 (10), 5911-21
- **Mount.** *Nucleic Acids Res,* 1982, vol. 10, 459
- **Roscigno et al.** *J Biol Chem,* 1993, vol. 268, 11222
- **Lee et al.** *Gene Therapy,* 2004, vol. 11, 94
- **Akusjarvi ; Svevenin.** *Curr Top Microbiol Immunol,* 2003, vol. 272, 253
- **Nevins ; Wilson.** *Nature,* 1981, vol. 290, 113
- **Akusjarvi ; Persson.** *J Virol,* 1981, vol. 38, 469
- **Muhlemann et al.** *J Virol,* 1995, vol. 69, 7324
- **Vandenbroucke et al.** *BMC Genomics,* 2002, vol. 3, 13
- **Hall et al.** *PNAS,* 1988, vol. 85, 704-708
- **Harris et al.** *Nucl Acid Res,* 1990, vol. 18 (10), 3015-3019
- **Liao et al.** *Virology,* 2004, vol. 323, 131
- **Zheng.** *J Biomed Sci,* 2004, vol. 11, 278
- **Parr et al.** *Nature Medicine,* 1997, vol. 3 (10), 1145-1149
- **Riccio et al.** *Nucleic Acids Res.,* 1985, vol. 13, 2759-2771
- **Cannio et al.** *Nucleic Acids Res.,* 1991, vol. 19, 2303-2308
- **Swaminathan et al.** *Curr. Topics in Micro. and Immunol.,* 1995, vol. 199, 177-194
- **Virtanen et al.** *J. Virol.,* 1984, vol. 51, 822-831
- **Weinberg et al.** *Proc. Natl. Acad. Sci.,* 1983, vol. 80, 5383-5386
- **Wold et al.** *Curr. Topics Microbiol. Immunol.,* 1995, vol. 199, 237-274
- **Tollefson et al.** *J. Virol.,* 1996, vol. 70 (4), 2296
- **Tollefson et al.** *J. Virol.,* 1992, vol. 66 (6), 3633
- **Duke et al.** *J.Virol.,* 1995, vol. 66 (3), 1602-9
- **Huez et al.** *Mol. Cell. Biol.,* 1998, vol. 18 (11), 6178-90
- **Donnelly et al.** *J Gen Virol.,* May 2001, vol. 82, 1013-25
- **de Felipe et al.** *Human Gene Therapy,* 2000, vol. 11, 1921-1931
- **Donnelly et al.** *J. Gen. Virol.,* 2001, vol. 82, 1013-1025
- **Donnelly et al.** *J Gen Virol.,* May 2001, vol. 82, 1027-41
- **Szymczak et al.** *Nature Biotechnology,* 2004, vol. 22, 589-594760
- **Ryan et al.** *Virol.,* 1989, vol. 173, 35-45
- **Halpin et al.** *Plant J,* 1999, vol. 17, 453-459
- **de Felipe et al.** *Gene Therapy,* 1999, vol. 6, 198-208
- **Ryan et al.** *EMBO J.,* 1994, vol. 13, 928-933
- **Roosien et al.** *J. Gen. Virol.,* 1990, vol. 71, 1703-1711
- **Chaplin et al.** *J. Interferon Cytokine Res.,* 1999, vol. 19, 235-241
- **Furler et al.** *Gene Ther.,* June 2001, vol. B (11), 864-73
- **Syzmczak et al.** *Nature Biotechnology,* 2004, vol. 22, 589-594760
- **Donnelly et al.** *J. Gen. Virol.,* 2001, vol. 82, 1027-1041
- **Ryan et al.** *J. Gen. Virol.,* 1991, vol. 72, 2727-2732
- **Webster et al.** *J Gen Virol,* 1989, vol. 70, 3215-3223
- **Weber.** *Curr Top Microbiol Immunol,* 1995, vol. 1991, 227-235
- **Balakirev et al.** *J of Virol,* 2002, vol. 76, 6323-6331
- **Niculescu-Duvaz et al.** *J Med Chem.,* 06 May 2004, vol. 47 (10), 2651-2658
- **Jeremias I et al.** *Eur J Immunol,* 1998, vol. 28, 143-152
- **Walczak H et al.** *Nat Med,* 1999, vol. 5, 157-163
- **Routes et al.** *J Immunol.,* 15 October 2000, vol. 165 (8), 4522-7
- **Tollefson et al.** *J Virol.,* October 2001, vol. 75 (19), 8875-87
- **Tollefson et al.** *Nature,* 16 April 1998, vol. 392 (6677), 726-30
- **Shisler et al.** *J Virol.,* November 1997, vol. 71 (11), 8299-306

- **Lichtenstein et al.** *J Virol.,* November 2002, vol. 76 (22), 11329-42
- **Tollefson et al.** *J Virol.,* 2001, vol. E3, 14.7
- **Chen et al.** *J Biol. Chem.,* 06 March 1998, vol. 273 (10), 5815-20
- **Tollefson et al.** *J Virol.,* 2001
- **Benedict et al.** *J Biol. Chem.,* 02 February 2001, vol. 276 (5), 3270-8
- **Wilson-Rawls et al.** *Virology.,* July 1993, vol. 195, 6-15
- **Ghosh-Choudhury et al.** *Gene,* 1986, vol. 50 (1-3), 161-71
- **Toietta et al.** *Mol Ther.,* February 2002, vol. 5 (2), 204-10
- **Horton et al.** *Biotechniques,* May 1990, vol. 8 (5), 528-35
- **Anton ; Graham.** *J. Virology,* 1995, vol. 69, 4600-4606